# EUROPEAN PATENT SPECIFICATION

(11) **EP 3 891 162 B1**
(45) Date of publication and mention of the grant of the patent: **06.03.2024**
(21) Application number: 19813334.0
(22) Date of filing: 05.12.2019
(51) Int. Cl.: C07H 15/234, A61K 31/7036, A61P 31/00

(54) **NOVEL DERIVATIVES OF N-3"-GUANIDINO-2,5-DIDEOXY-5-FLUORO-4,6-DISUBSTITUTED-STREPTAMINE AMINOGLYCOSIDE ANTIBIOTICS**
NEUARTIGE DERIVATE VON 4,6-DISUBSTITUIERTEN-2,5-DIDEOXY-5-FLUORO-STREPTAMIN- N-3''-GUANIDINO-AMINOGLYKOSID-ANTIBIOTIKA
NOUVEAUX DÉRIVÉS D'ANTIBIOTIQUES AMINOGLYCOSIDES À BASE DE N-3''-GUANIDINO- 2,5-DIDÉSOXY-5-FLUORO-STREPTAMINE-4,6-DISUBSTITUÉS

(30) Priority: 05.12.2018 EP 18210470
(43) Date of publication of application: 13.10.2021
(73) Proprietor: AGILeBiotics B.V., 9713 GX Groningen (NL)
(72) Inventor: BASTIAN, Andreas Alexander, 9747 AW Groningen (NL); BASTIAN, Maria, 9747 AW Groningen (NL)
(74) Representative: dompatent von Kreisler Selting Werner - Partnerschaft von Patent- und Rechtsanwälten mbB
(86) International application number: PCT/EP2019/083784
(87) International publication number: WO 2020/115190

(56) References cited:
- SHITARA T ET AL: "Synthesis of 5-deoxy-5-fluoro and 5-deoxy-5,5-difluoro derivatives of kanamycin B in its analogs. Study on structure-toxicity relationships", CARBOHYDRATE RESEARCH, PERGAMON, GB, vol. 232, no. 2, 3 August 1992 (1992-08-03), pages 273-290, XP026634507, ISSN: 0008-6215, DOI: 10.1016/0008-6215(92)80060-E [retrieved on 1992-08-03] cited in the application
- ANDRÉS G. SANTANA ET AL: "Selective modification of the 3''-amino group of kanamycin prevents significant loss of activity in resistant bacterial strains", ORGANIC & BIOMOLECULAR CHEMISTRY, vol. 14, no. 2, 1 January 2016 (2016-01-01), pages 516-525, XP055602559, ISSN: 1477-0520, DOI: 10.1039/C5OB01599E cited in the application

## Description

### FIELD OF INVENTION

The present invention relates to novel aminoglycoside compounds having antimicrobial properties and being suitable, for example, as therapeutic agents for use in the treatment of mammalian disease and in particular to novel therapeutic agents suitable for use in the treatment of microbial infection in mammals. The present invention further relates to the use of pharmaceutical compositions comprising said agents in the treatment of medical conditions in mammals, in particular in the treatment of microbial infection. The agents and pharmaceutical compositions of the invention are of particular relevance in the treatment of diseases associated with antibiotic-resistant microbes. The invention further relates to compounds for use in the treatment of diseases whose treatment is made otherwise difficult due to antibiotic-class-related bacterial resistance and provides novel therapeutic agents suitable for use in the treatment of multidrug-resistant (MDR) infections. The present invention also relates to processes for making said agents.

### BACKGROUND OF THE INVENTION

Due to antibiotic resistance constituting a serious and growing phenomenon in contemporary medicine and having emerged as one of the pre-eminent public health concerns of the 21st century (especially in the case of hospital-acquired infections), society is running out of treatment options for life-threatening bacterial infections. Existing antibiotics are losing their effectiveness against multidrug-resistance (MDR) pathogens. Moreover, while bacterial resistance is continuously increasing, the rate of new antibiotic approvals is declining. As a result of this, society is entering the "post-antibiotic era", in which hospitalized patients in critical condition cannot be effectively treated or cured. Of particularly pronounced threat to life are infections caused by the members of the so-called ESKAPE panel (*Enterococcus faecium*(+), *Staphylococcus aureus(+), Klebsiella pneunomiae*(-), *Acinetobacter baumannii(-), Pseudomonas aeruginosa*(*-*), *Enterobacter spp.*(-)) and according to data from the Centers for Disease Control and Prevention (CDC) available in 2008, these six bacteria are responsible for two thirds of all health-care-associated infections. Also, of significant threat to public health are further bacteria such as, for example, *E. coli*, in particular strains thereof which have developed resistance to known antibacterial agents.

Infection by several drug-resistant Gram-negative bacteria - such as MDR *Pseudomonas aeruginosa*, *Acinetobacter baumannii* and carbapenem-resistant *Klebsiella* species - is of great concern. The therapeutic options for these pathogens are so limited that clinicians are often forced to use older, previously discarded drugs, such as polymixins, which are associated with significant toxicity.

Aminoglycoside antibiotics, such as amikacin, are one of the last-resort antibacterials for the treatment of life threatening MDR Gram-negative infections. They are safe, induce less bacterial resistance compared to other antibiotic classes and have broad-spectrum activity against pathogens belonging to the aforementioned ESKAPE panel. However, their clinical application is under threat due to continuous emergence of bacterial resistance.

One of the most clinically relevant bacterial resistance mechanisms is based on enzymatic inactivation of the drug molecules by aminoglycoside modifying enzymes (AMEs). AMEs produced by the pathogens alter the molecular structure of aminoglycosides, reducing their binding affinity to the target protein, i.e. 30S ribosome, thus leaving them inactive against resistant pathogens. AMEs are categorized in three enzymes classes: Amino moieties in the aminoglycosides may undergo acetylation by aminoglycoside N-acetyltransferases (AACs), and hydroxyl groups in the aminoglycosides may be phosphorylated by aminoglycoside *O*-phosphotransferases (APHs) or modified by aminoglycoside *O*-nucleotidyltransferases (ANTs). In light of this, studies into the mode and site of action of AMEs have made site-specific modification of aminoglycosides a promising tool to develop antibiotics aimed at overcoming such mechanisms of bacterial resistance.

In this regard, one of the most powerful modifications of aminoglycosides has been the introduction of a (2S)-4-amino-2-hydoxybutyrate (AHB) residue into kanamycin A, which resulted in the development of the semi-synthetic antibiotic amikacin. Amikacin is currently used in the clinic against MDR bacterial infections. However, due to continuous evolution of bacterial resistance the clinical application of amikacin is under threat.

Accordingly, attempts are underway in the field to develop the next generation of semi-synthetic aminoglycoside antibiotics. However, studies often focus on a single structural modification, which results in an antibiotic molecule with limited capability to simultaneously overcome resistance caused by multiple AMEs. In addition, these semi-synthetic aminoglycosides exhibit narrow-spectrum activity since their structural modifications often increase the antibacterial activity against one bacterial class yet reduce activity against one or more other classes.

In this regard, the introduction of a guanidine group at the 3"C-position of kanamycin has been reported (Santana, A. G., Zárate, S. G., Asensio, J. L., Revuelta, J., Bastida, A. Selective modification of the 3"-amino group of kanamycin prevents significant loss of activity in resistant bacterial strains. Org. Biomol. Chem. 14, 2016, 516-525). This structural modification is, however, seen to give rise to an aminoglycoside derivative with, *inter alia,* significantly reduced potency against certain bacterial species, such as *Escherichia coli, Enterococcus faecalis*, and *Pseudomonas aeruginosa*, limiting significantly the applicability and relevance of this derivative.

Another modification, which has been reported, is the fluorination of aminoglycosides at the 5-position (also known as the 5-C position) at the 2-deoxystreptamine (2-DOS) ring. This structural modification has been taught, in certain instances, to be associated with reduced toxicity of the resultant aminoglycoside (T. Tsuchiya, T. Shitara, S. Umezawa, T. Takeuchi, M. Hamada, N. Tomono, and E. Umemura Synthesis of low-toxicity, 5-deoxy-5-fluoro and 5-deoxy-5,5-difluoro derivatives of arbekacin and its analogs, and study of structure-toxicity relationships. Carbohydr. Res. 240, 1993, 307-312; T. Shitara, Y. Kobayashi, T. Tsuchiya, and S. Umezawa. Synthesis of 5-deoxy-5-fluoro and 5-deoxy-5,5-difluoro derivatives of kanamycin B and its analogs. Study on structure-toxicity relationships, Carbohydr. Res., 232, 1992, 273-290).

Despite such studies, it remains without question a challenge to identify novel aminoglycoside compounds which are both synthetically and commercially viable, and which overcome not only bacterial resistance but also show broad, preferably improved antibacterial activity against selected bacterial strains belonging to the ESKAPE panel, in particular relative to known aminoglycosides such as, for example, amikacin and gentamicin. In this regard, the provision of compounds with improved antibacterial activity has the potential to allow effective treatment of microbial infections at lower doses which, in turn, carries the advantage of being able to treat such infections whilst simultaneously reducing the risk of drug-class-associated toxicity such as, for example, nephrotoxicity and ototoxicity. The provision of such compounds thus constitutes one object underlying the present invention.

A further object underlying the present invention is the provision of novel aminoglycoside compounds which exhibit excellent and preferably improved activity against at least one, and preferably more than one, of the following bacterial targets: *Escherichia coli (E. coli), E. coli expressing one or more of the AMEs* APH(3')IIIa, APH(3')Ia, AAC(6')Ie-APH(2")Ia, AAC(6')Ib, AAC(3)III, and AAC(3)IV (or *E. coli* expressing any one or more of the other AMEs expressed in strains against which activity data for the compounds of the invention is provided in the present application), *Enterococcus faecium*, *Enterococcus faecalis, Staphylococcus aureus, Klebsiella pneunomiae*, *Acinetobacter baumannii, Pseudomonas aeruginosa, Enterobacter spp., Morganella morgannii*, *Providencia stuartii*, and *Enterobacter cloacae* (or strains of any of these bacteria expressing any one or more of the AMEs disclosed herein), in particular strains of said bacterial targets which are multidrug-resistant (MDR) or pan-drug-resistant (PDR).

A further object underlying the present invention is the provision of novel aminoglycoside compounds which exhibit excellent and preferably improved activity against *E*. *coli*, in particular strains of *E.coli* exhibiting resistance to aminoglycoside antibiotics (e.g. Amikacin and/or Gentomycin) such as strains harboring plasmids encoding one or more AMEs such as APH(3')IIIa, APH(3')Ia, AAC(6')Ie-APH(2")Ia, AAC(6')Ib, AAC(3)III, and AAC(3)IV (or any one or more of the other AMEs expressed in strains against which activity data for the compounds of the invention is provided in the present application), and, additionally, excellent and preferably improved activity against one or more of *Enterococcus faecium, Enterococcus faecalis, Klebsiella pneumoniae, Acinetobacter baumannii, Enterobacter cloacae, Pseudomonas aeuginosa, Morganella morgannii, Providencia stuartii,* and *Staphylococcus aureus.*

The provision of compounds exhibiting excellent and preferably improved activity against any one of the targets (or any combination of two or more of the targets) recited herein and/or against which experimental data is disclosed herein constitutes an object underlying the present invention.

A further object underlying the present invention is the provision of compounds which regain activity against certain bacterial targets which is/was lost upon making structural modifications aimed at gaining activity against one or more other bacterial targets, in particular where said oter bacterial targets are resistant to one or more aminoglycoside antiobiotics. As such, an object underlying the present invention is the provision of compounds which broaden the spectrum of bacterial targets against which certain aminoglycosides exhibit activity.

### SUMMARY OF THE INVENTION

The present invention relates to novel compounds suitable for, *inter alia,* use in a medicament, in particular a medicament for use in the treatment of mammalian disease, more particular to novel therapeutic agents suitable for use in the treatment of microbial infection, in particular in mammals. Relative to amikacin-type antibiotics (4,6-disubstituted 2-deoxystreptamine (2-DOS) aminoglycosides carrying an (2*S*)-4-amino-2-hydroxybutyrate (AHB) group in the N1-position), the compounds of the present invention are seen to overcome bacterial resistance caused by AMEs and simulateously boost the antibacterial activity by up to 16-fold against bacteria of, *inter alia,* the ESKAPE panel. As is demonstrated herein, the combination of fluorination at the 5-C position (giving rise to a 5-epi-5-Fluoro group, i.e. as depicted hereinbelow in the structure of formula (I)) and the introduction of a guanidine or functionalized guanidine moiety on the NH₂ group at the 3"-C position (also referred to as the 3"-position) of the aminoglycoside scaffold results in an entirely unexpected synergistic effect leaving Amikacin-type aminoglycosides with significantly improved antibacterial activity. Moreover, the presence of substituted/functionalized guanidine moieties at the 3"-C position is entirely unknown in the art, let alone that they exhibit the excellent levels of *in vitro* and *in vivo* potency against the specific targets demonstrated herein.

Accordingly, the compounds of the present invention can be used, *inter alia,* to (1) tackle bacterial resistance, (2) boost and broaden the antibacterial activity against Gram-positive and Gram-negative bacteria, and (3) regain antibacterial activity against one or more targets lost due to making other structural modifications.

Specifically, the present invention relates to the following items:
Items:
1. A compound of formula (I) or a pharmaceutically acceptable salt thereof, 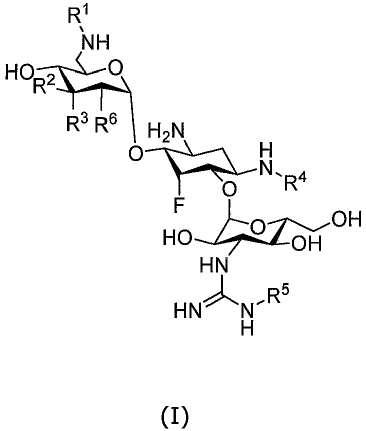 wherein
   (i) R¹ is selected from the group consisting of H, methyl, ethyl, straight chain or branched C₃₋₆alkyl, C₃₋₆cycloalkyl, 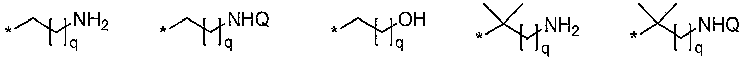 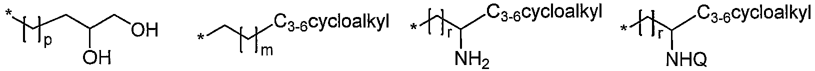 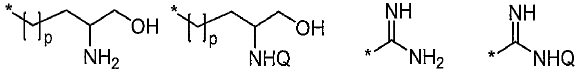 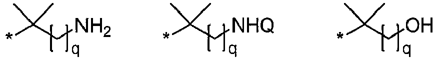 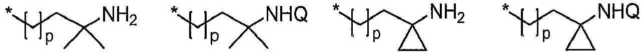 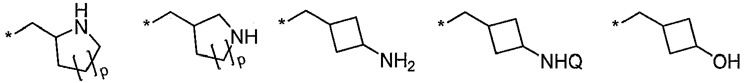 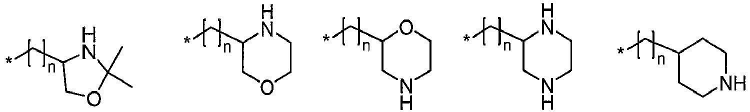 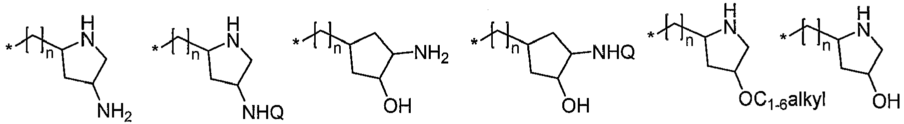 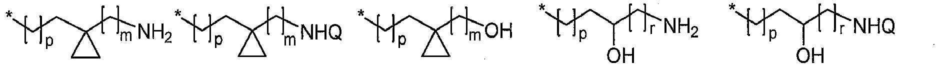 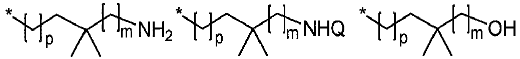 where * is the point of connection to the N atom to which R¹ is attached in formula (I);
   (ii) R² is selected from the group consisting of H, methyl, -CH₂F, -CF₃, ethyl, n-propyl, iso-propyl, cyclopropyl, halogen, hydroxyl, -OCH₃, -OEt, -OCH₂F, -OCF₃, -NH₂, -NHCH₃, -NHEt, -N(CH₃)₂, -N(Et)₂, -NHCH₂F, -NHCF₃, and -NHQ;
      wherein when R² is ethyl, n-propyl, iso-propyl, cyclopropyl, -OEt, -NHEt, or -N(Et)₂, the alkyl and cycloalkyl moieties in said R² groups may optionally be substituted with one or more substituents independently selected from the group consisting of halogen, hydroxyl, -OCH₃, -OEt, -NH₂, -NHCH₃, -NHEt, -N(CH₃)₂, -N(Et)₂, and -NHQ; with the proviso that when R² is -OEt, -NHEt, or -N(Et)₂, the carbon atom joining the Et group of said OEt, -NHEt, or -N(Et)₂ group to the O or N atom of said OEt, -NHEt, or -N(Et)₂ group may only be substituted with one or more substituents independently selected from halogen;
   (iii) R³ is selected from the group consisting of H, halogen, hydroxyl, -OCH₃, -OCH₂F, -OCF₃, -OEt, -OC₃₋₈alkyl, -OC₃₋₆cycloalkyl, -OCH₂C₃₋₆cycloalkyl, -NH₂, -NHCH₃, -NHCH₂F, -NHCF₃, -NHEt, -NHC₃₋₈alkyl, -N(CH₃)₂, -N(Et)₂, -N(C₃₋₈alkyl)₂, and -NHQ;
      wherein when R³ is -OEt, -OC₃₋₈alkyl, -OC₃₋₆cycloalkyl, -OCH₂C₃₋₆cycloalkyl, -NHEt, -NHC₃₋₈alkyl, -N(Et)₂, -N(C₃₋₈alkyl)₂, the alkyl and cycloalkyl moieties in said R³ groups may optionally be substituted with one or more substituents independently selected from the group consisting of halogen, hydroxyl, -OCH₃, -OCH₂F, -OCF₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom in each of said R³ groups which is directly bonded to the O or N atom in each of said R³ groups may only be substituted with one or more substituents independently selected from halogen; and
      wherein when R³ is -OCH₃ or -NHCH₃ it may be optionally substituted on the CH₃ moiety of said -OCH₃ or -NHCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
   (iv) R⁴ is selected from the group consisting of H, methyl, ethyl, -CH₂F, -CF₃, straight chain or branched C₃₋₆alkyl, substituted straight chain C₂₋₆alkyl, substituted branched C₃₋₆alkyl, optionally substituted C₃₋₆cycloalkyl, optionally substituted -CH₂C₃₋₆cycloalkyl, formyl, optionally substituted phenyl, optionally substituted 5- or 6-membered heteroaryl, 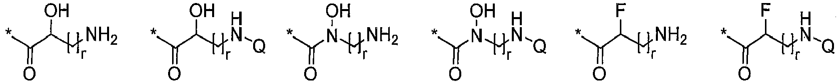 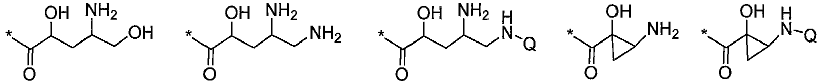 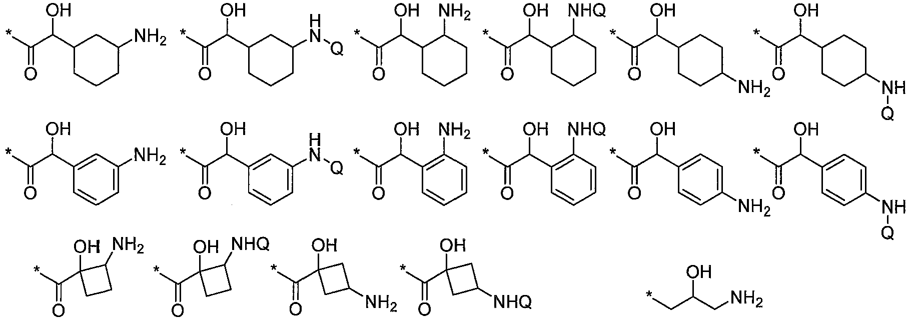 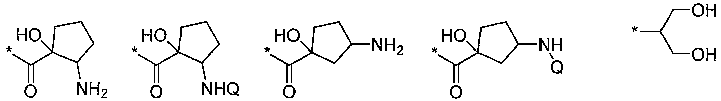 where * is the point of connection to the N atom to which R⁴ is attached in formula (I), and wherein
      when R⁴ is substituted straight chain C₂₋₆alkyl, substituted branched C₃₋₆alkyl, substituted C₃₋₆cycloalkyl, or substituted -CH₂C₃₋₆cycloalkyl, it is substituted with one or more substituents independently selected from the group consisting of halogen, hydroxyl, -OCH₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom of said R⁴ group which is directly bonded to the N atom to which R⁴ is connected in the structure of formula (I) may only be substituted with one or more substituents independently selected from halogen;
   (v) R⁵ is selected from the group consisting of H; methyl; -CH₂F; -CF₃; ethyl; straight chain or branched C₃₋₈alkyl; substituted straight chain C₂₋₈alkyl; substituted branched C₃₋₈alkyl; optionally substituted C₃₋₆cycloalkyl; optionally substituted -CH₂C₃₋₆cycloalkyl; optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; optionally substituted 6-membered non-aromatic heterocycloalkyl containing 1, 2 or 3 heteroatoms selected from O, N, and S; -C(=NH)NH₂; -C(=NR⁷)NH₂; -C(=NH)NHR⁸; -C(=NR⁷)NHR⁸; -C(=NR⁷)NR⁸R⁹; and
      -X-Z, wherein
         X is selected from the group consisting of methylene, ethylene, straight chain or branched C₃₋₈alkylene; each of which may in addition to being attached to Z be optionally substituted with one or more substituents independently selected from the group consisting of methyl, -CH₂F, -CF₃, ethyl, straight chain or branched C₃₋₆alkyl, halogen, -OH, -OCH₃, -OCH₂F, -OCF₃, -OC₂₋₆alkyl, -NH₂, -NHQ, -NHR¹⁰, -NR¹⁰R¹¹, -CO₂H, -CO₂CH₃, -CO₂C₂₋₆alkyl, -OCOCH₃, -OCOC₂₋₆alkyl, -CN, -CONHR¹², -CONR¹²R¹³, -NHCOCH₃, -NHCOC₂₋₆alkyl, -NR¹⁴COCH₃, and -NR¹⁵COC₂₋₆alkyl; with the proviso that the atom of said X group which connects it to the N atom to which R⁵ is connected in the structure of formula (I) cannot be directly connected to a further O or N atom;
            and
         Z is selected from the group consisting of optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; optionally substituted 6-membered non-aromatic heterocycloalkyl containing 1, 2 or 3 heteroatoms selected from O, N, and S; optionally substituted C₃₋₆cycloalkyl;
      wherein each of R⁷ to R¹⁵ is independently selected from the group consisting of H, methyl, ethyl, C₃₋₆alkyl, and C₃₋₆cycloalkyl;
   (vi) R⁶ is OH or NH₂;
   (vii) with the proviso for all compounds of formula (I) or pharmaceutically acceptable salts thereof that when the atom of R² which connects it to the tetrahydropyran ring to which both it and R³ are connected in the structure of formula (I) is an O or N atom, the atom of R³ which connects it to said tetrahydropyran ring cannot be an O or N atom; and the proviso that when the atom of R³ which connects it to the tetrahydropyran ring to which both it and R² are connected in the structure of formula (I) is an O or N atom, the atom of R² which connects it to said tetrahydropyran ring cannot be an O or N atom; and
   (viii) wherein for each of the groups disclosed
      m = 0, 1, 2, 3, 4, or 5;
      n = 1 or 2;
      p = 0, 1, or 2;
      q = 1, 2, 3, 4, or 5;
      r = 1, 2, 3, or 4; and
   (ix) wherein in respect of all of the above substituents and groups containing the moiety Q,
      Q is 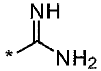
      where * is the point of connection to the N atom to which Q is attached.
2. The compound or pharmaceutically acceptable salt thereof according to item 1, wherein R² is selected from the group consisting of H, methyl, -CH₂F, -CF₃, ethyl, n-propyl, iso-propyl, cyclopropyl, halogen, hydroxyl, -OCH₃, -OEt, -OCH₂F, -OCF₃, -NH₂, -NHCH₃, -NHEt, -N(CH₃)₂, -N(Et)₂, -NHCH₂F, -NHCF₃, and -NHQ.
3. The compound or pharmaceutically acceptable salt thereof according to any of the preceding items, wherein R² is selected from the group consisting of H, methyl, ethyl, n-propyl, iso-propyl, cyclopropyl, -F, hydroxyl, -OCH₃, -OEt, -OCH₂F, and -OCF₃.
4. The compound or pharmaceutically acceptable salt thereof according to any of the preceding items, wherein R² is selected from the group consisting of H, methyl, and hydroxyl.
5. The compound or pharmaceutically acceptable salt thereof according to any of the preceding items, wherein R² is selected from the group consisting of H and methyl.
6. The compound or pharmaceutically acceptable salt thereof according to any of the preceding items, wherein R² is H.
7. The compound or pharmaceutically acceptable salt thereof according to any of items 1 to 5, wherein R² is methyl.
8. The compound or pharmaceutically acceptable salt thereof according to any of items 1 to 4, wherein R² is hydroxyl.
9. The compound or pharmaceutically acceptable salt thereof according to any of the preceding items, wherein R³ is selected from the group consisting of H, halogen, hydroxyl, -OCH₃, -OCH₂F, -OCF₃, -OEt, -OC₃₋₈alkyl,
   - OC₃₋₆cycloalkyl, and -OCH₂C₃₋₆cycloalkyl,
      wherein when R³ is -OEt, -OC₃₋₈alkyl, -OC₃₋₆cycloalkyl, or
   - OCH₂C₃₋₆cycloalkyl, the alkyl and cycloalkyl moieties in said R³ groups may be optionally substituted with one or more substituents independently selected from the group consisting of halogen, hydroxyl, -OCH₃, -OCH₂F, -OCF₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom in each of said R³ groups which is directly bonded to the O atom in each of said R³ groups may only be substituted with one or more substituents independently selected from halogen; and
      wherein when R³ is -OCH₃ it may be optionally substituted on the CH₃ moiety of said -OCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S.
10. The compound or pharmaceutically acceptable salt thereof according to any of the preceding items, wherein when R³ may be -OCH₃ substituted on the CH₃ moiety of said -OCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
   optionally substituted means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; wherein each of R¹⁶ to R³⁸ is independently selected from the group consisting of H, methyl, ethyl, C₃₋₆alkyl, and C₃₋₆cycloalkyl; with the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸.
11. The compound or pharmaceutically acceptable salt thereof according to any of the preceding items, wherein when R³ may be -NHCH₃ substituted on the CH₃ moiety of said -NHCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
   optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; wherein each of R¹⁶ to R³⁸ is independently selected from the group consisting of H, methyl, ethyl, C₃₋₆alkyl, and C₃₋₆cycloalkyl; with the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸.
12. The compound or pharmaceutically acceptable salt thereof according to any of the preceding items, wherein R³ is selected from the group consisting of H, halogen, hydroxyl, -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); and -OCH₂(optionally substituted 6-membered non-aromatic heterocycloalkyl containing 1, 2 or 3 heteroatoms selected from O, N, and S).
13. The compound or pharmaceutically acceptable salt thereof according to any of the preceding items, wherein R² is H or methyl, and R³ is selected from the group consisting of H, hydroxyl, -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); and -OCH₂(optionally substituted 6-membered non-aromatic heterocycloalkyl containing 1, 2 or 3 heteroatoms selected from O, N, and S).
14. The compound or pharmaceutically acceptable salt thereof according to any of the preceding items, wherein R³ is selected from the group consisting of H, hydroxyl, -OCH₃, -OCH₂F, -OCF₃, -OEt, -OC₃₋₈alkyl, -OC₃₋₆cycloalkyl, and -OCH₂C₃₋₆cycloalkyl.
15. The compound or pharmaceutically acceptable salt thereof according to any of the preceding items, wherein R³ is selected from the group consisting of H and hydroxyl.
16. The compound or pharmaceutically acceptable salt thereof according to any of the preceding items, wherein R³ is H.
17. The compound or pharmaceutically acceptable salt thereof according to any of items 1 to 15, wherein R³ is hydroxyl.
18. The compound or pharmaceutically acceptable salt thereof according to any of the preceding items, wherein the compound of formula (I) is selected from the compounds of formula (Ia) to (If) 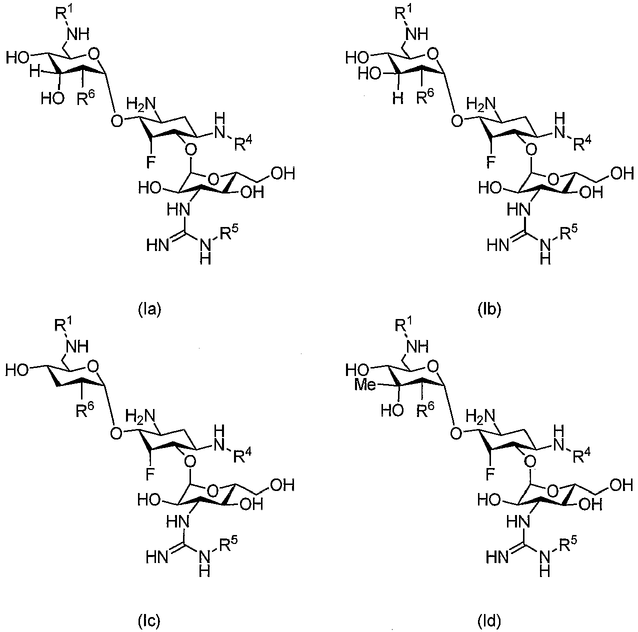 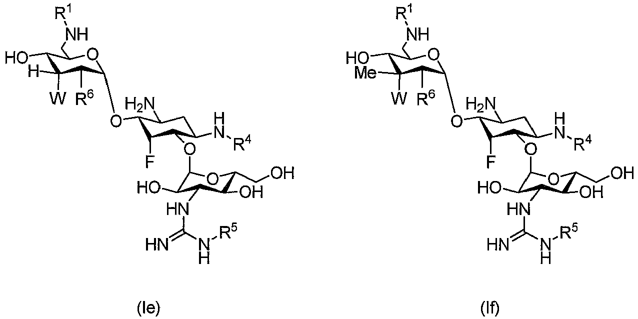 wherein independently for each of the compounds of formula (Ia) to (If) R¹, R⁴, R⁵ and R⁶ are as defined in any of the preceding items, and wherein W is independently selected for each of the compounds of formula (Ie) to (If) from the group consisting of -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); and -OCH₂(optionally substituted 6-membered non-aromatic heterocycloalkyl containing 1, 2 or 3 heteroatoms selected from O, N, and S).
19. The compound or pharmaceutically acceptable salt thereof according to any of the preceding items, wherein R⁶ is OH.
20. The compound or pharmaceutically acceptable salt thereof according to any of the preceding items, wherein R⁶ is NH₂.
21. The compound or pharmaceutically acceptable salt thereof according to any of the preceding items, wherein the compound of formula (I) is selected from the compounds of formula (Ig) to (Ip) 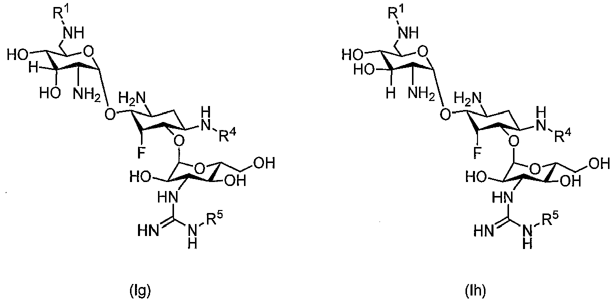 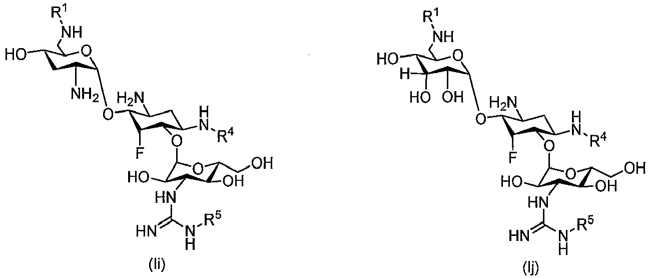 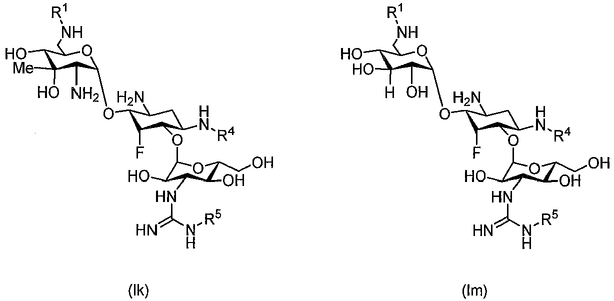 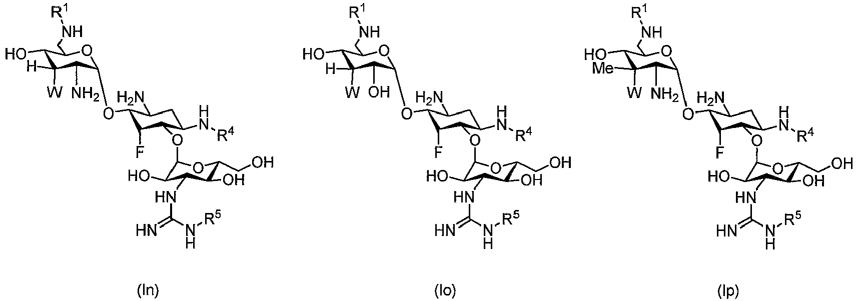 wherein independently for each of the compounds of formula (Ig) to (Ip) R¹, R⁴, and R⁵ are as defined in any of the preceding items, and wherein W is independently selected for each of the compounds of formula (In) to (Ip) from the group consisting of -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S; and -OCH₂(optionally substituted 6-membered non-aromatic heterocycloalkyl containing 1, 2 or 3 heteroatoms selected from O, N, and S).
22. The compound or pharmaceutically acceptable salt thereof according to any of items 18 to 21, wherein when W may be -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S; -OCH₂(optionally substituted 6-membered non-aromatic heterocycloalkyl containing 1, 2 or 3 heteroatoms selected from O, N, and S);
   optionally substituted means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; wherein each of R¹⁶ to R³⁸ is independently selected from the group consisting of H, methyl, ethyl, C₃₋₆alkyl, C₃₋₆cycloalkyl; with the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸.
23. The compound or pharmaceutically acceptable salt thereof according to any of the preceding items, wherein R¹ is selected from the group consisting of H, methyl, ethyl, straight chain or branched C₃₋₆alkyl, C₃₋₆cycloalkyl, 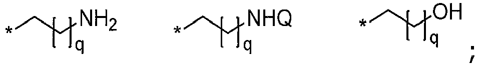
   preferably from the group consisting of H, 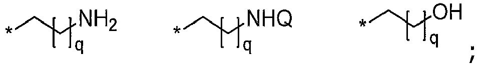
   wherein in said R¹ moieties q and Q are as defined in item 1.
24. The compound or pharmaceutically acceptable salt thereof according to any of the preceding items, wherein R¹ is selected from the group consisting of H, 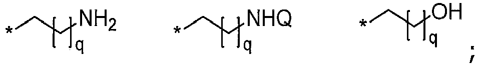 and
   wherein Q is as defined in item 1 and q is, independently for each moiety, either 1, 2, or 3 in said R¹ moieties, most preferably wherein q is 1 in said R¹ moieties.
25. The compound or pharmaceutically acceptable salt thereof according to any of the preceding items, wherein R¹ is H.
25a. The compound or pharmaceutically acceptable salt thereof according to any of items 1 to 24, wherein R¹ is *-CH₂CH₂OH (i.e. 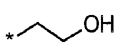 ), where * is the point of connection to the N atom to which R¹ is attached in formula (I).
26. The compound or pharmaceutically acceptable salt thereof according to any of the preceding items, wherein R⁴ is selected from the group consisting of H, 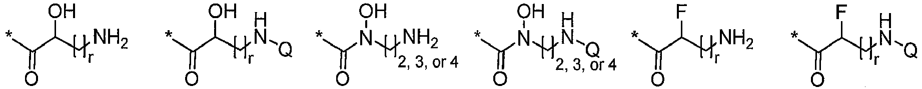 and wherein in said R⁴ moieties r and Q are as defined in item 1.
27. The compound or pharmaceutically acceptable salt thereof according to any of the preceding items, wherein R⁴ is selected from the group consisting of H, 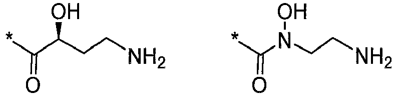 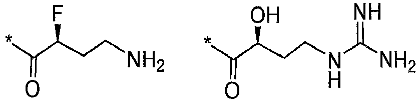
28. The compound or pharmaceutically acceptable salt thereof according to any of the preceding items, wherein R⁴ is H.
29. The compound or pharmaceutically acceptable salt thereof according to any of items 1 to 27, wherein R⁴ is 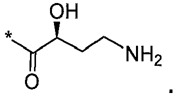
30. The compound or pharmaceutically acceptable salt thereof according to any of items 1 to 27, wherein R⁴ is 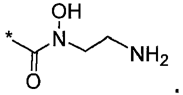
31. The compound or pharmaceutically acceptable salt thereof according to any of items 1 to 27, wherein R⁴ is 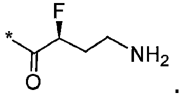
32. The compound or pharmaceutically acceptable salt thereof according to any of items 1 to 27, wherein R⁴ is 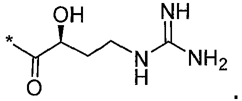
33. The compound or pharmaceutically acceptable salt thereof according to any of the preceding items, wherein when R⁵ may be optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
   said optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; are selected from the group consisting of optionally substituted azetidinyl, optionally substituted diazetidinyl, optionally substituted oxetanyl, optionally substituted thietanyl, optionally substituted pyrrolidinyl, optionally substituted tetrahydrofuranyl, optionally substituted tetrahydrothiophenyl, optionally substituted piperidinyl, optionally substituted tetrahydropyranyl, optionally substituted thianyl, optionally substituted imidazolidinyl, optionally substituted pyrazolidinyl, optionally substituted oxazolidinyl, optionally substituted isooxazolidinyl, optionally substituted thiazolidinyl, optionally substituted isothiazolidinyl, optionally substituted dioxolanyl, optionally substituted dithiolanyl, optionally substituted piperazinyl, optionally substituted morpholinyl, optionally substituted thiomorpholinyl, optionally substituted 1,3-dioxanyl, optionally substituted 1,4-dioxanyl, optionally substituted 1,3-dithianyl, and optionally substituted 1,4-dithianyl.
34. The compound or pharmaceutically acceptable salt thereof according to any of the preceding items, wherein when R⁵ may be optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
   said optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; are selected from the group consisting of 4-piperidinyl, 3-piperidinyl, 2-piperidinyl, 2- pyrrolidinyl, 3-pyrrolidinyl, 4-oxazolidinyl, 2-morpholinyl, 3-morpholinyl, 4-morpholinyl, 2-tetrahydrofuranyl, and 3-tetrahydrofuranyl.
35. The compound or pharmaceutically acceptable salt thereof according to any of the preceding items, wherein when Z may be optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
   said optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; are selected from the group consisting of optionally substituted azetidinyl, optionally substituted diazetidinyl, optionally substituted oxetanyl, optionally substituted thietanyl, optionally substituted pyrrolidinyl, optionally substituted tetrahydrofuranyl, optionally substituted tetrahydrothiophenyl, optionally substituted piperidinyl, optionally substituted tetrahydropyranyl, optionally substituted thianyl, optionally substituted imidazolidinyl, optionally substituted pyrazolidinyl, optionally substituted oxazolidinyl, optionally substituted isooxazolidinyl, optionally substituted thiazolidinyl, optionally substituted isothiazolidinyl, optionally substituted dioxolanyl, optionally substituted dithiolanyl, optionally substituted piperazinyl, optionally substituted morpholinyl, optionally substituted thiomorpholinyl, optionally substituted 1,3-dioxanyl, optionally substituted 1,4-dioxanyl, optionally substituted 1,3-dithianyl, and optionally substituted 1,4-dithianyl.
36. The compound or pharmaceutically acceptable salt thereof according to any of the preceding items, wherein when Z may be optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
   said optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; are selected from the group consisting of 4-piperidinyl, 3-piperidinyl, 2-piperidinyl, 2-pyrrolidinyl, 3-pyrrolidinyl, 4-oxazolidinyl, 2-morpholinyl, 3-morpholinyl, 4-morpholinyl, 2-tetrahydrofuranyl, and 3-tetrahydrofuranyl.
37. The compound or pharmaceutically acceptable salt thereof according to any of the preceding items, wherein R⁵ is selected from the group consisting of H, methyl, ethyl, straight chain or branched C₃₋₈alkyl, substituted straight chain C₂₋₈alkyl, substituted branched chain C₃₋₈alkyl, optionally substituted C₃₋₆cycloalkyl, 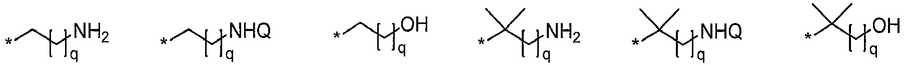 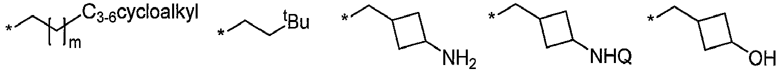 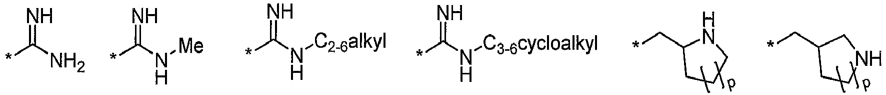 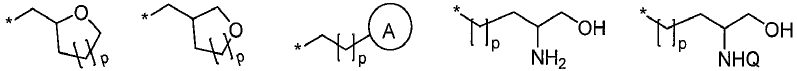 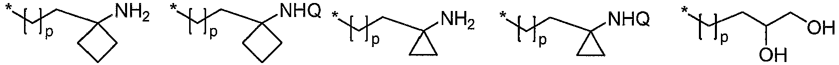 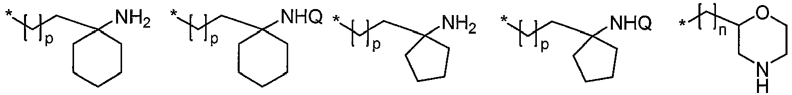 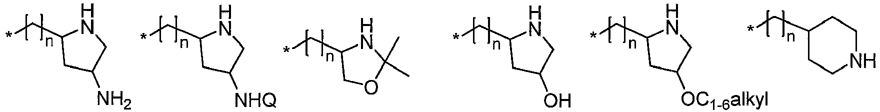 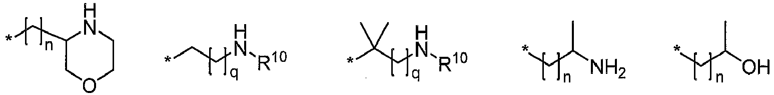 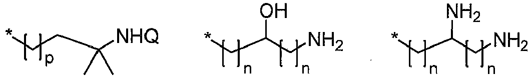
   where * is the point of connection to the N atom to which R⁵ is attached in formula (I), and
   wherein A is selected from the group consisting of optionally substituted phenyl, optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; and
   wherein m, n, q, p, and R¹⁰ in said R⁵ moieties are as defined in any of the previous items.
38. The compound or pharmaceutically acceptable salt thereof according to any of the preceding items, wherein when R⁵ may be optionally substituted straight chain C₂₋₈alkyl; optionally substituted branched C₃₋₈alkyl; optionally substituted C₃₋₆cycloalkyl; or optionally substituted -CH₂C₃₋₆cycloalkyl, optionally substituted means optionally substituted with one or more substituents independently selected from the group consisting of methyl, -CH₂F, -CF₃, ethyl, straight chain or branched C₃₋₆alkyl, halogen, -OH, -OCH₃, -OCH₂F, -OCF₃, -OC₂₋₆alkyl, -NH₂, -NHQ, -NHR¹⁰, -NR¹⁰R¹¹, -CO₂H, -CO₂CH₃, -CO₂C₂₋₆alkyl, -OCOCH₃, -OCOC₂₋₆alkyl, -CN, -CONHR⁷, -CONR¹²R¹³, -NHCOCH₃, -NHCOC₂₋₆alkyl, -NR¹⁴COCH₃, and -NR¹⁵COC₂₋₆alkyl; wherein each of R⁷ to R¹⁵ is independently selected from the group consisting of H, methyl, ethyl, C₃₋₆alkyl, and C₃₋₆cycloalkyl.
39. The compound or pharmaceutically acceptable salt thereof according to any of the preceding items, wherein when R⁵ may be optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; optionally substituted means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; with the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; wherein each of R¹⁶ to R³⁸ is independently selected from the group consisting of H, methyl, ethyl, C₃₋₆alkyl, and C₃₋₆cycloalkyl.
40. The compound or pharmaceutically acceptable salt thereof according to any of the preceding items, wherein when Z may be optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; optionally substituted means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; with the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; wherein each of R¹⁶ to R³⁸ is independently selected from the group consisting of H, methyl, ethyl, C₃₋₆alkyl, and C₃₋₆cycloalkyl.
41. The compound or pharmaceutically acceptable salt thereof according to any of the preceding items, wherein R⁵ is selected from the group consisting of H, methyl, ethyl, straight chain or branched C₃₋₆alkyl, 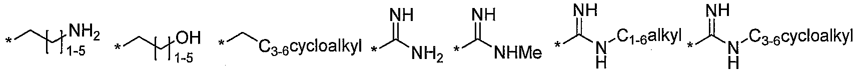 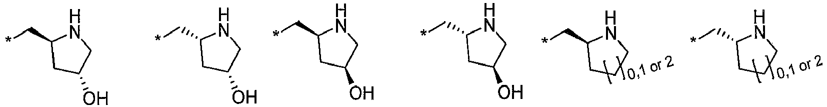 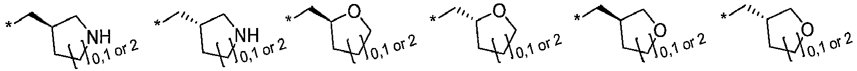 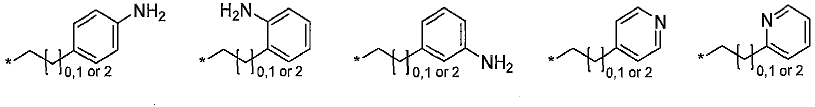 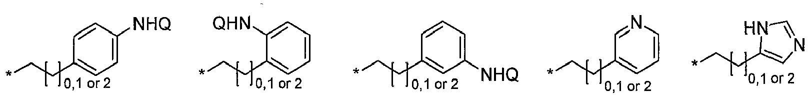 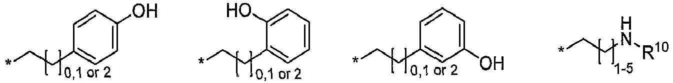 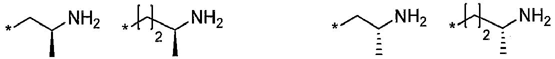 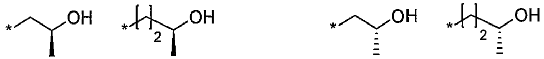 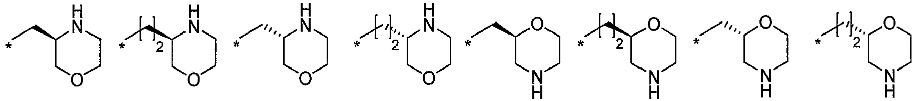 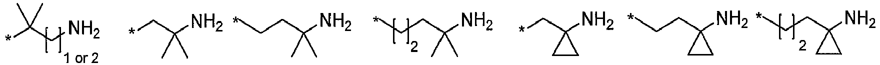 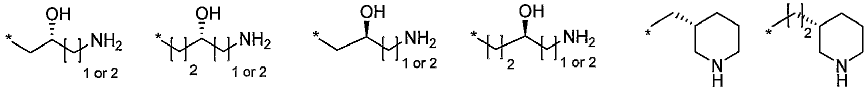 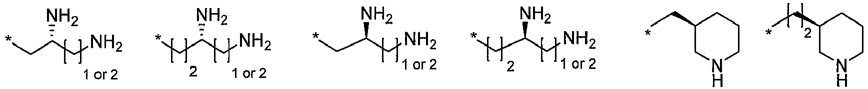 wherein Q and R¹⁰ in said R⁵ moieties are as defined in any of the previous items.
42. The compound or pharmaceutically acceptable salt thereof according to any of the preceding items, wherein R⁵ is selected from the group consisting of H, 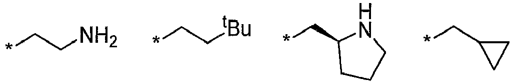 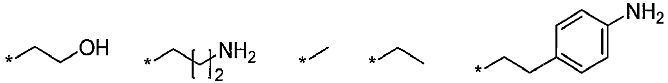 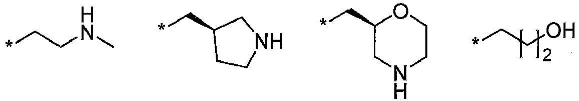 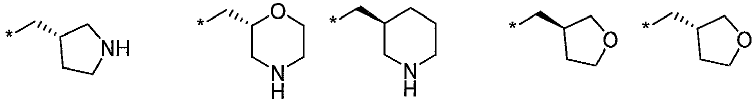 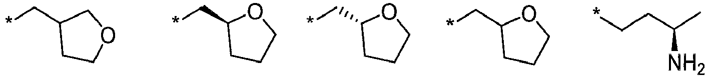 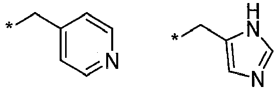
43. A composition comprising at least one compound or pharmaceutically acceptable salt thereof according to any of the preceding items.
44. A pharmaceutical composition comprising the composition, compound or pharmaceutically acceptable salt thereof according to any of the preceding items.
45. A pharmaceutical composition comprising the composition, compound or pharmaceutically acceptable salt thereof according to any of the preceding items and a pharmaceutically acceptable carrier.
46. A pharmaceutical composition comprising the composition, compound or pharmaceutically acceptable salt thereof according to any of the preceding items and a pharmaceutically acceptable carrier, diluent or excipient.
47. A composition comprising at least one compound or pharmaceutically acceptable salt thereof according to any of items 1 to 42 and at least one further antibacterial agent, wherein said at least one further antibacterial agent is different to the at least one compound or pharmaceutically acceptable salt thereof according to any of items 1 to 42.
48. The composition of item 47, wherein said at least one further antibacterial agent is a regulatory approved antibiotic, in particular a regulatory approved member of a family of antibiotics selected from the group consisting of penicillin antibiotics, cephalosporin antibiotics, carbapenem antibiotics, monobactam antibiotics, polymyxin antibiotics, rifamycin antibiotics, lipiarmycin antibiotics, quinolone antibiotics, sulfonamide antibiotics, macrolide antibiotics, lincosamide antibiotics, tetracycline antibiotics, aminoglycoside antibiotics, lipopeptide antibiotics, glycylcycline antibiotics, glycopeptide antibiotics, oxazolidinone antibiotics, and lipiarmycin.
49. The composition according to either of items 47 or 48, wherein the at least one further antibacterial agent is a beta-lactam antibiotic, in particular a beta-lactam antibiotic selected from the group consisting of penicillin antibiotics, cephalosporin antibiotics, and carbapenem antibiotics, or any combination of any thereof.
50. The composition according to any of items 47 to 49, wherein said composition further comprises at least one additional agent capable of overcoming one or more bacterial resistance mechanisms.
51. The composition according to item 50, wherein said
   (i) at least one further antibacterial agent is a beta-lactam antibiotic, in particular an existing regulatory approved beta-lactam antibiotic, more particularly an existing regulatory approved beta-lactam antibiotic selected from the group consisting of penicillin antibiotics, cephalosporin antibiotics, and carbapenem antibiotics, or any combination of any thereof; and
   (ii) said at least one additional agent capable of overcoming one or more bacterial resistance mechanisms is selected from the group consisting of clavulanic acid, sulbactam, tazobactam, avibactam, relebactam, verbobactam, and any combination of any thereof.
52. The composition according to item 51, wherein the combination of said beta-lactam antibiotic and said at least one additional agent capable of overcoming one or more bacterial resistance mechanisms is selected from the group consisting of
   (i) amoxicillin and clavulanic acid;
   (ii) Ticacillin and clavulanic acid;
   (iii) Ampicillin and Sulbactam;
   (iv) Cefoperazone and Sulbactam;
   (v) Piperacillin and Tazobactam;
   (vi) Ceftolozane and Tazobactam;
   (vii) Ceftazidime and Avibactam;
   (viii) Ceftaroline and Avibactam;
   (ix) carbapenems, in particular Impenem or Meropenem, and Relebactam; and
   (x) carbapenems, in particular Impenem or Meropenem, and Varbobactam.
52a. The composition according to any of items 43 to 52, wherein said composition further comprises at least one adjuvant capable of suppressing or preventing aminoglycoside-induced nephrotoxicity.
52b. The composition according to item 52a, wherein said at least one adjuvant capable of suppressing or preventing aminoglycoside-induced nephrotoxicity is a member of a pharmacological class selected from antibiotics, calcium channel blockers, beta blockers, cytoprotective antianginals, iNOS inhibitors, NO precursors, hormones, antiplatelets, statins, PPAR-γ agonists, TNF-α synthesis inhibitors, biguanides, antioxidants, free radical scavengers, antioxidant enzymes, superoxide dismutase mimetics, herbal extracts, and any combination of any thereof.
52c. The composition according to item 52b, wherein said at least one adjuvant capable of suppressing or preventing aminoglycoside-induced nephrotoxicity is selected from the group consisting of focfomycin, fleroxacin, nifedipine, amlodipine, carvedilol, L-NIL, L-arginine, hormones melatonin, thyroxine, trapidil, atorvastatin, rosiglitazone, pentoxifylline, metformin, probucol, aminoguanidine, L-carnitine, ebselen, N-acetylcysteine, lycopene, curcumin, thymoquinone, fish oil, vitamin E, vitamin C, sesame oil, halofuginone, resveratrol, quercetin, S-allylcysteine, diallyl sulfide, caffeic acid phenethyl ester, S-allylmercaptocysteine, superoxide dismutase, the superoxide dismutase mimetic M40403, *Rhazya stricta,* garlic, *Cassia auriculata,* soyabean, *Phylanthus amarus, Morchella esculenta,* green tea, *Nigella sativa, Ligusticum wallichi, Viscum articulatum,* gum arabic, pongamia pinnata flowers, nigella sativa oil, hemidesmus indicus, phenolic extract of soybean, green tea extract, bauhinia purpurea, sida rhomboidea, apocynin, pipercillin, and any combination of any thereof.
53. Use of a compound according to any of items 1 to 42 for preventing, inhibiting, or stopping the growth of bacteria on surfaces.
54. A compound or pharmaceutically acceptable salt thereof according to any of items 1 to 42 or a composition according to any of items 43 to 52c for use as a medicament.
55. A compound or pharmaceutically acceptable salt thereof according to any of items 1 to 42 or a composition according to any of items 43 to 52c for use in the treatment of microbial infection and/or a disorder, affliction or illness caused at least in part by microbial infection.
56. A compound, pharmaceutically acceptable salt thereof or a composition for use according to item 55, wherein said microbial infection is a bacterial infection.
57. A compound, pharmaceutically acceptable salt thereof or composition for use according to item 56, wherein said bacterial infection is caused at least in part by one or more Gram-positive bacterial species, in particular wherein said one or more Gram-positive bacterial species is selected from the group consisting of *Staphylococcus aureus, Streptococcus pneumoniae, Enterococcus faecalis, Enterococcus faecium,* and *Mycobacterium tuberculosis.*
58. A compound, pharmaceutically acceptable salt thereof or composition for use according to item 56, wherein said bacterial infection is caused at least in part by one or more Gram-negative bacterial species, in particular wherein said one or more Gram-negative bacterial species is selected from the group consisting of *Acinetobacter spp*.; *Enterobacteriaceae spp.,* in particular *Escherichia spp., Klebsiella spp.,* or *Enterobacter spp.; Mycobacterium spp.; Morganella spp.; Providencia spp.;* and *Pseudomonas spp.*; in particular from the group consisting of *Acinetobacter baumannii, Escherichia coli, Klebsiella pneumoniae, Klebsiella oxytoca, Enterobacter cloacae, Enterobacter aerogenes, Mycobacterium tuberculosis, Morganella morgannii, Providencia stuartii,* and *Pseudomonas aeruginosa.*
59. A compound, pharmaceutically acceptable salt thereof or composition for use according to item 56, wherein said bacterial infection is caused at least in part by one or more Gram-positive bacterial species and at least in part by one or more Gram-negative bacterial species, in particular wherein said one or more Gram-positive bacterial species is selected from the group consisting of *Staphylococcus aureus, Streptococcus pneumoniae, Enterococcus faecalis, Enterococcus faecium,* and *Mycobacterium tuberculosis;* and wherein said one or more Gram-negative bacterial species is selected from the group consisting of *Acinetobacter spp.*; *Enterobacteriaceae spp.*, in particular *Escherichia spp.*, *Klebsiella spp.*, or *Enterobacter spp.*; *Morganella spp.*; *Providencia spp.;* and *Pseudomonas spp.*; in particular from the group consisting of *Acinetobacter baumannii, Escherichia coli, Klebsiella pneumoniae, Klebsiella oxytoca, Enterobacter cloacae, Enterobacter aerogenes, Mycobacterium tuberculosis, Morganella morgannii, Providencia stuartii,* and *Pseudomonas aeruginosa.*
60. A compound, pharmaceutically acceptable salt thereof or composition for use according to item 56, wherein the bacterial infection is caused at least in part by a bacterial species (spp.) selected from the group consisting of *Escherichia spp., Enterococcus spp., Staphylococcus spp., Klebsiella spp., Acinetobacter spp., Pseudomonas spp., Enterobacter spp., Morganella spp., Providencia spp., Mycobacterium spp.,* and any combination of any thereof.
61. A compound, pharmaceutically acceptable salt thereof or composition for use according to item 60, wherein said *Escherichia spp.* is *Escherichia coli,* preferably wherein said *Escherichia spp.* is selected from the group consisting of Wild type *Escherichia coli* and *Escherichia coli strains* which express one or more aminoglycoside modifying enzymes.
62. A compound, pharmaceutically acceptable salt thereof or composition for use according to item 61, wherein said at least one aminoglycoside modifying enzyme is selected from the group consisting of aminoglycoside phosphotransferases, in particular APH(3')IIIa, APH(3')IIa, APH(3')Ia, APH(3")Ib, APH(3')VI, APH(6)Id, APH(3')VIa, APH(3')IIb, and APH(6)Ic; aminoglycoside acetyltransferases, in particular AAC(6')Ib, AAC(3)III, AAC(3)IV, AAC(3)Ia, AAC(3)IId, and AAC(3)Ic, more particularly AAC(3)III and AAC(3)IV; aminoglycoside nucleotidyltransferases, in particular ANT(3")Ia, ANT(2")Ia, and ANT(3"); and bifunctional aminoglycoside modifying enyzmes, in particular AAC(6')Ie-APH(2")Ia; and any combination of any thereof.
63. A compound, pharmaceutically acceptable salt thereof or composition for use according to item 60, wherein said *Enterococcus spp.* is *Enterococcus faecium or Enterococcus faecalis,* in particular *Enterococcus faecalis;* said *Staphylococcus spp.* is *Staphylococcus aureus;* said *Klebsiella spp.* is *Klebsiella pneunomiae;* said *Acinetobacter spp.* is *Acinetobacter baumannii;* said *Pseudomonas spp.* is *Pseudomonas aeruginosa;* said *Enterobacter spp.* is *Enterobacter cloacae; said Morganella spp. is Morganella morgannii; said Providencia spp. is Providencia stuartii;* and *Mycobacterium spp. is Mycobacterium tuberculosis.*
63a. A compound, pharmaceutically acceptable salt thereof or composition for use according to item 63, wherein said *Enterococcus faecium, Enterococcus faecalis, Staphylococcus aureus, Klebsiella pneunomiae, Acinetobacter baumannii, Pseudomonas aeruginosa, Enterobacter cloacae, Morganella morgannii, and*/*or Providencia stuartii* express at least one aminoglycoside modifying enzyme.
63b. A compound, pharmaceutically acceptable salt thereof or composition for use according to item 63a, wherein said at least one aminoglycoside modifying enzyme is selected from the group consisting of aminoglycoside phosphotransferases, in particular APH(3')IIIa, APH(3')IIa, APH(3')Ia, APH(3")Ib, APH(3')VI, APH(6)Id, APH(3')VIa, APH(3')IIb, and APH(6)Ic; aminoglycoside acetyltransferases, in particular AAC(6')Ib, AAC(3)III, AAC(3)IV, AAC(3)Ia, AAC(3)IId, and AAC(3)Ic, more particularly AAC(3)III and AAC(3)IV; aminoglycoside nucleotidyltransferases, in particular ANT(3")Ia, ANT(2")Ia, and ANT(3"); and bifunctional aminoglycoside modifying enyzmes, in particular AAC(6')Ie-APH(2")Ia; and any combination of any thereof.
64. A compound, pharmaceutically acceptable salt thereof or composition for use according to any of items 56 to 63b, wherein said bacterial infection is caused at least in part by a bacterial species which exhibits resistance against at least one member of a family of existing regulatory approved antibiotics, in particular by a bacterial species which exhibits resistance against at least one member of one or more families of antibiotics selected from the group consisting penicillin antibiotics, cephalosporin antibiotics, carbapenem antibiotics, monobactam antibiotics, polymyxin antibiotics, rifamycin antibiotics, lipiarmycin antibiotics, quinolone antibiotics, sulfonamide antibiotics, macrolide antibiotics, lincosamide antibiotics, tetracycline antibiotics, aminoglycoside antibiotics, lipopeptide antibiotics, glycylcycline antibiotics, glycopeptide antibiotics, oxazolidinone antibiotics, and lipiarmycin.
65. A compound, pharmaceutically acceptable salt thereof or composition for use according to any of items 56 to 64, wherein said bacterial infection is selected from one or more infections and infectious diseases from the list consisting of respiratory tract infections, complicated skin and soft tissue infections, complicated intra-abdominal infections, community acquired pneumonia, hospital-acquired pneumonia, ventilator-associated pneumonia, urinary tract infections, bacterial meningitis, infective endocarditis, sepsis, osteomyelitis, septic arthritis, septicemia, anthrax, osteomyelitis, tuberculosis, leprosy, necrotizing fasciitis, scarlet fever, rheumatic fever, postpartum fever, and streptococcal toxic shock syndrome, and additional nosocomial infections, for example infections caused by the use of intravascular catheters.

In the remainder of the application, unless specified otherwise all generic groups R¹, R², R³, R⁴, R⁵, R⁶, X, W, Y, Z, m, n, p, q, r, etc. are as defined for the moiety of formula (I) and regardless of whether these groups have been further defined and/or restricted relative to their broadest, most generic definition in the structural moiety of formula (I), the provisos stipulated for the structural moiety of formula (I) apply for all structures, formulae and embodiments herein.

Hereinafter, when reference is made to a group/substituent being "as defined for formula (I)", "as defined for the compounds of formula (I)" etc, e.g. "R¹, R⁴, R⁵, R⁶ are as defined for the compounds formula (I)", this is to be understood to mean all possible definitions of the respective groups/substituents as described herein for formula (I), including, for each individual group/substituent, the broadest definition of said group disclosed herein as well as any narrower definitions and any of the "preferred", "more preferred", "most preferred", "in particular" etc. variants of a particular group/substituent. Moreover, any specific examples or lists of groups or substituents which are stated herein to be "preferred", "more preferred" etc for the compounds of formula (I) are also to be understood to be "preferred", "more preferred" etc for any of the compounds and formulae listed herein which fall under the broadest scope of formula (I).

Hereinafter, when a formula or specific compound is depicted in such a way that the full valency of an atom, for example a heteroatom, would appear not to be satisfied, it is to be understood that the valency of said atom is satisfied through the presence of non-depicted hydrogen atoms, i.e. the depicted species is not a radical, cation, anion, carbine etc., but instead a neutral species. By way of example, the following structures/situations are presented:
Structural moieties depicted as 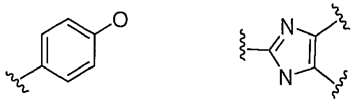
are to be understood to respectively represent

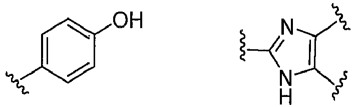

Moreover, in certain cases herein graphical depictions for a given specific structural moiety which are equally understood by the skilled person but which are different from one another are used. These are to be understood to represent the structural moiety that the skilled person would understand upon analyzing said moiety in isolation and not through comparison to other depictions of the same moiety presented elsewhere herein. For example, a methyl group may be depicted by explicitly writing the formula "CH₃" or "Me", or may be equally depicted using the standard "linear" formula which is equally well understood by the skilled person. The following two structures thus both comprise a methyl group:

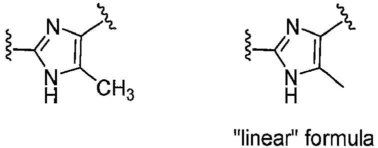

The compounds of the present invention (the compounds of formula (I)) are depicted throughout the present application in a form in which the 3 six-membered rings of the aminoglycoside core are drawn in the form of chair conformations. As such, the various substituents attached to said rings are depicted as being either equatorial or axial, this depiction showing, the respective stereochemistry (where present) of a given stereocentre. To avoid any confusion in this regard in, for instance, the case that a bond angle of an equatorial or axial substituent has not been replicated with 100% accuracy (due, for example, to constraints within the drawing package used), the following explanation of the structures set forth herein is provided:

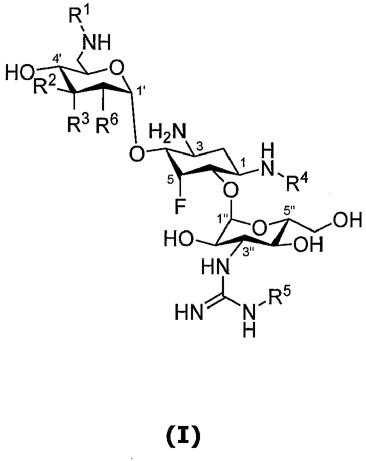

The compounds of the present invention are represented by the structure of formula (I). For clarity, above is shown the conventional numbering of the various atoms of the 3 six-membered rings of the aminoglycoside core (this numbering being adopted herein). As shown in the above structure and as depicted in all other structures herein irrespective of the accuracy of reproduction of the true bond angle, the NHR⁴ group at the 1-position is equatorial. As shown in the above structure and as depicted in all other structures herein irrespective of the accuracy of reproduction of the true bond angle, the NH₂ group at the 3-position is equatorial. As shown in the above structure and as depicted in all other structures herein irrespective of the accuracy of reproduction of the true bond angle, the O atom at the 4-position is equatorial. As shown in the above structure and as depicted in all other structures herein irrespective of the accuracy of reproduction of the true bond angle, the F atom at the 5-position is axial. As shown in the above structure and as depicted in all other structures herein irrespective of the accuracy of reproduction of the true bond angle, the O atom at the 6-position is equatorial. As shown in the above structure and as depicted in all other structures herein irrespective of the accuracy of reproduction of the true bond angle, the O atom at the 1'-position is axial. As shown in the above structure and as depicted in all other structures herein irrespective of the accuracy of reproduction of the true bond angle, the R⁶ group at the 2'-position is equatorial. As shown in the above structure and as depicted in all other structures herein irrespective of the accuracy of reproduction of the true bond angle, the R² group at the 3'-position is equatorial. As shown in the above structure and as depicted in all other structures herein irrespective of the accuracy of reproduction of the true bond angle, the R³ group at the 3'-position is axial. As shown in the above structure and as depicted in all other structures herein irrespective of the accuracy of reproduction of the true bond angle, the OH group at the 4'-position is equatorial. As shown in the above structure and as depicted in all other structures herein irrespective of the accuracy of reproduction of the true bond angle, the CH₂NHR¹ group at the 5'-position is equatorial. As shown in the above structure and as depicted in all other structures herein irrespective of the accuracy of reproduction of the true bond angle, the O atom at the 1"-position is axial. As shown in the above structure and as depicted in all other structures herein irrespective of the accuracy of reproduction of the true bond angle, the OH group at the 2"-position is equatorial. As shown in the above structure and as depicted in all other structures herein irrespective of the accuracy of reproduction of the true bond angle, the guanidyl group at the 3"-position is equatorial. As shown in the above structure and as depicted in all other structures herein irrespective of the accuracy of reproduction of the true bond angle, the OH group at the 4"-position is equatorial. As shown in the above structure and as depicted in all other structures herein irrespective of the accuracy of reproduction of the true bond angle, the CH₂OH group at the 5"-position is equatorial.

As such, the above depiction of the compounds of formula (I) is, following convention in the field of organic chemistry, to be understood to represent the following structures (two conventional, slightly differing ways of representing the same structure and stereochemistry) showing the following stereochemistry:

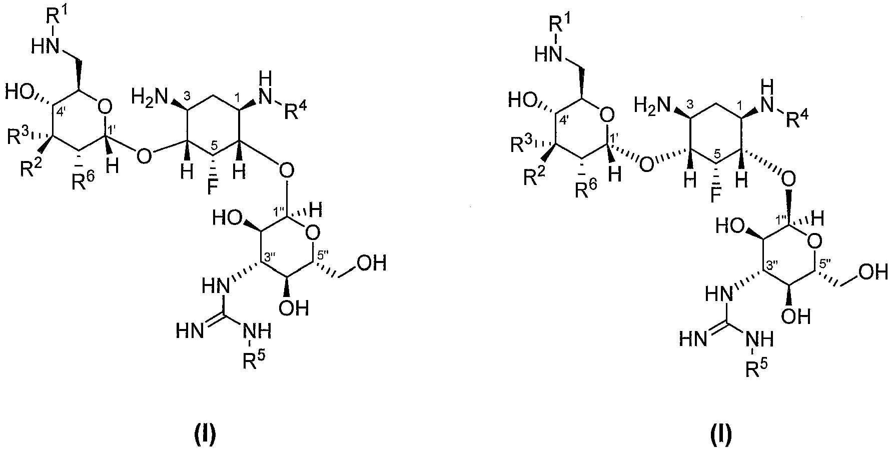

### DETAILED DESCRIPTION OF THE INVENTION

In its most general form, the present invention relates to a compound of formula (I) or a pharmaceutically acceptable salt thereof, 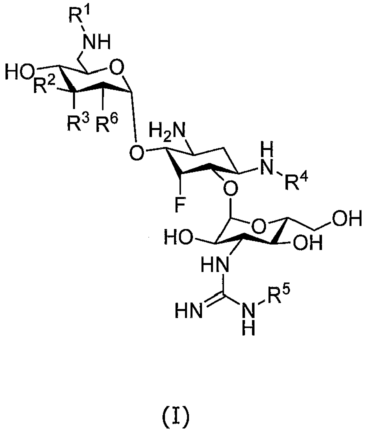 in which each of the groups R¹, R², R³, R⁴, R⁵ and R⁶ are as defined in the items herein above.

Accordingly, in the compounds of the present invention R¹ is selected from the group consisting of H, methyl, ethyl, straight chain or branched C₃₋₆alkyl, C₃₋₆cycloalkyl, 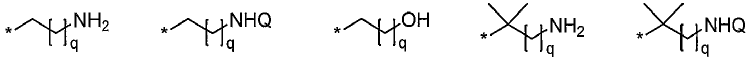 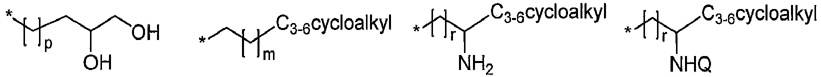 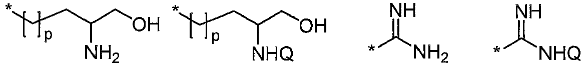 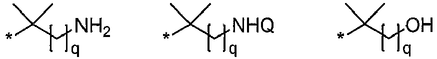 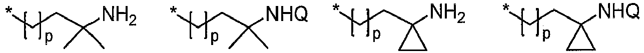 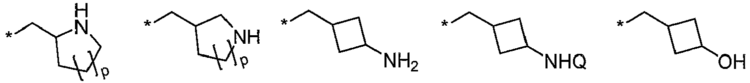 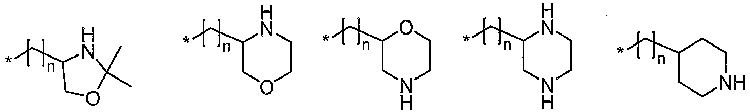 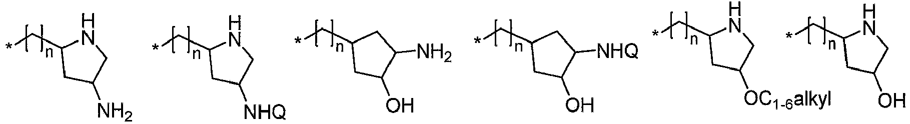 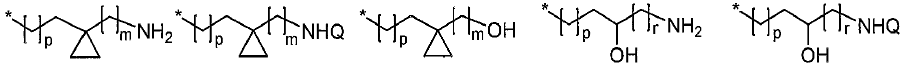 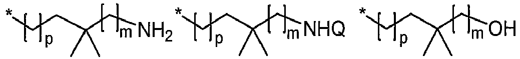
where * is the point of connection to the N atom to which R¹ is attached in formula (I) and wherein m, n, p, q, r and Q are as defined herein above for the compounds of formula (I). R¹ is preferably selected from the group consisting of H, methyl, ethyl, straight chain or branched C₃₋₆alkyl, C₃₋₆cycloalkyl, 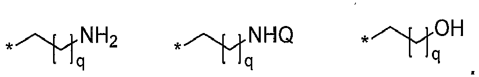
R¹ is more preferably selected from the group consisting of H, 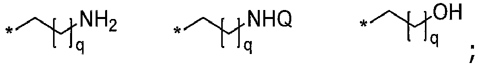
wherein, independently for each moiety, q is either 1, 2, or 3, most preferably 1, in said R¹ moieties.

In the compounds of the present invention, R¹ is most preferably H.

In the compounds of the present invention, R² is selected from the group consisting of H, methyl, -CH₂F, -CF₃, ethyl, n-propyl, iso-propyl, cyclopropyl, halogen, hydroxyl, -OCH₃, -OEt, -OCH₂F, -OCF₃, -NH₂, -NHCH₃, -NHEt, -N(CH₃)₂, -N(Et)₂, -NHCH₂F, -NHCF₃, and -NHQ;
wherein when R² is ethyl, n-propyl, iso-propyl, cyclopropyl, -OEt, -NHEt, or -N(Et)₂, the alkyl and cycloalkyl moieties in said R² groups may optionally be substituted with one or more substituents independently selected from the group consisting of halogen, hydroxyl, -OCH₃, -OEt, -NH₂, -NHCH₃, -NHEt, -N(CH₃)₂, -N(Et)₂, and -NHQ; with the proviso that when R² is -OEt, -NHEt, or -N(Et)₂, the carbon atom joining the Et group of said -OEt, -NHEt, or -N(Et)₂ group to the O or N atom of said -OEt, -NHEt, or -N(Et)₂ group may only be substituted with one or more substituents independently selected from halogen.

In said R² groups, Q is as defined herein above for the compounds of formula (I).

The wording "wherein when R² is ethyl, n-propyl, iso-propyl, cyclopropyl, -OEt, -NHEt, or -N(Et)₂, the alkyl and cycloalkyl moieties in said R² groups may optionally be substituted with..." is to be understood to mean that the ethyl group itself, the n-propyl group itself, the cyclopropyl group itself, the ethyl moiety of the -OEt group, the ethyl moiety of the -NHEt group, and the ethyl moieties of the -N(Et)₂ group may optionally be substituted with the subsequently listed substituents.

The wording "with the proviso that when R² is -OEt, -NHEt, or -N(Et)₂, the carbon atom joining the Et group of said -OEt, -NHEt, or -N(Et)₂ group to the O or N atom of said -OEt, -NHEt, or -N(Et)₂ group may only be substituted with one or more substituents independently selected from halogen" is to be understood to mean, taking R² = -OEt as an example, that where R² is -OEt, the underscored carbon atom -OCH₂CH₃ (i.e. the carbon atom joining the Et group of said -OEt, -NHEt, or -N(Et)₂ group to the O or N atom of said -OEt, -NHEt, or -N(Et)₂ group) may only be substituted with one or more substituents independently selected from halogen, whereas the non-underscored carbon atom (that of the CH₃ group of the -OEt substituent) may be substituted with any of the previously recited allowable substituents, i.e. it may optionally be substituted with one or more substituents independently selected from the group consisting of halogen, hydroxyl, -OCH₃, -OEt, -NH₂, -NHCH₃, -NHEt, -N(CH₃)₂, -N(Et)₂, and -NHQ.

In the compounds of the present invention, R² is preferably selected from the group consisting of H, methyl, -CH₂F, -CF₃, ethyl, n-propyl, iso-propyl, cyclopropyl, halogen, hydroxyl, -OCH₃, -OEt, -OCH₂F, -OCF₃, -NH₂, -NHCH₃, -NHEt, -N(CH₃)₂, -N(Et)₂, -NHCH₂F, -NHCF₃, and -NHQ. In the compounds of the present invention, R² is more preferably selected from the group consisting of H, methyl, ethyl, n-propyl, iso-propyl, cyclopropyl, -F, hydroxyl, -OCH₃, -OEt, -OCH₂F, and -OCF₃. In the compounds of the present invention, R² is still more preferably selected from the group consisting of H, methyl, and hydroxyl; and is most preferably H or methyl. R² may be hydroxyl. R² may be H. R² may be methyl.

In the compounds of the present invention, R³ is selected from the group consisting of H, halogen, hydroxyl, -OCH₃, -OCH₂F, -OCF₃, -OEt, -OC₃₋₈alkyl, -OC₃₋₆cycloalkyl, -OCH₂C₃₋₆cycloalkyl, -NH₂, -NHCH₃, -NHCH₂F, -NHCF₃, -NHEt, -NHC₃₋₈alkyl, -N(CH₃)₂, -N(Et)₂, -N(C₃₋₈alkyl)₂, and -NHQ;
wherein when R³ is -OEt, -OC₃₋₈alkyl, -OC₃₋₆cycloalkyl, -OCH₂C₃₋₆cycloalkyl, -NHEt, -NHC₃₋₈alkyl, -N(Et)₂, or -N(C₃₋₈alkyl)₂, the alkyl and cycloalkyl moieties in said R³ groups may optionally be substituted with one or more substituents independently selected from the group consisting of halogen, hydroxyl, -OCH₃, -OCH₂F, -OCF₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom directly bonded to the O or N atom in each of said R³ groups may only be substituted with one or more substituents independently selected from halogen; and
wherein when R³ is -OCH₃ or -NHCH₃ it may be optionally substituted on the CH₃ moiety of said -OCH₃ or -NHCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S.

In said R³ groups, Q is as defined herein above for the compounds of formula (I).

The wording "wherein when R³ is -OEt, -OC₃₋₈alkyl, -OC₃₋₆cycloalkyl, -OCH₂C₃₋₆cycloalkyl, -NHEt, -NHC₃₋₈alkyl, -N(Et)₂, -N(C₃₋₈alkyl)₂, the alkyl and cycloalkyl moieties in said R³ groups may optionally be substituted with ..." is to be understood to mean that only the ethyl moiety of the -OEt group, the C₃₋₈alkyl moiety of the -OC₃₋₈alkyl group, and so on may optionally be substituted with the subsequently listed substituents. In other words, the O atom of the -OEt group can, for example, not be substituted with the subsequently listed groups (such a substitution would make no technical sense as said O atom would then be bonded to 3 different groups when O has only a valency of 2), rather only the Et moeity (the alkyl moiety) of the -OEt group can be further substituted as defined.

Furthermore, the wording "with the proviso that the carbon atom in each of said R³ groups which is directly bonded to the O or N atom in each of said R³ groups may only be substituted with one or more substituents independently selected from halogen " is to be understood to mean, taking R³ = -OEt as an example, that where R³ is -OEt, the underscored carbon atom -OCH₂CH₃ (i.e. the carbon atom joining the respective Et group to the O or N atom) may only be substituted with one or more substituents independently selected from halogen, whereas the non-underscored carbon atom (that of the CH₃ group of the -OEt substituent) may be substituted with any of the previously recited allowable substituents, i.e. it may be substituted with one or more substituents independently selected from the group consisting of halogen, hydroxyl, -OCH₃, -OCH₂F, -OCF₃, -OC₂₋₄alkyl, -NH₂, -NHCH₃, -NHC₂₋₄alkyl, -N(CH₃)₂, -N(C₂₋₄alkyl)₂, and -NHQ. The reference to "the carbon atom in each of said R³ groups which is directly bonded to the O or N atom in each of said R³ groups" is to be understood to mean the carbon atom as explicitly recited in the listed R³ groups, i.e. within the R³ group itself (e.g. the underscored carbon atom -OCH₂CH₃ of the -Oet substituent) , and not the aminoglycoside ring carbon atom to which R³ is joined in the structure of formula (I) (a carbon atom not in said R³ group).

As outlined above, when R³ is -OCH₃ or -NHCH₃ in the compounds of the present invention it may be optionally substituted on the CH₃ moiety of said -OCH₃ or -NHCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; optionally substituted 6-membered non-aromatic heterocycloalkyl containing 1, 2 or 3 heteroatoms selected from O, N, and S. This is to be understood to mean that, by way of example, where R³ is -OCH₃ the CH₃ moiety of said -OCH₃ group may be substituted with any of the listed substituents such to give rise to a group where R³ is then -OCH₂-substituent. In the case that the -OCH₃ group is substituted with, for example, optionally substituted phenyl, the resultant R³ group would be -OCH₂(optionally substituted phenyl). Examples thereof would be -OCH₂Ph and -OCH₂-(4-fluorophenyl).

In the compounds of the present invention, R³ is preferably selected from the group consisting of H, halogen, hydroxyl, -OCH₃, -OCH₂F, -OCF₃, -OEt, -OC₃₋₈alkyl, -OC₃₋₆cycloalkyl, and -OCH₂C₃₋₆cycloalkyl,
wherein when R³ is -OEt, -OC₃₋₈alkyl, -OC₃₋₆cycloalkyl, or -OCH₂C₃₋₆cycloalkyl, the alkyl and cycloalkyl moieties in said R³ groups may be optionally substituted with one or more substituents independently selected from the group consisting of halogen, hydroxyl, -OCH₃, -OCH₂F, -OCF₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom in each of said R³ groups which is directly bonded to the O atom in each of said R³ moieties may only be substituted with one or more substituents independently selected from halogen; and
wherein when R³ is -OCH₃ it may be optionally substituted on the CH₃ moiety of said -OCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S. Q is as defined herein above for the compounds of formula (I).

In the compounds of the present invention, R³ is more preferably selected from the group consisting of H, halogen, hydroxyl, -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S; and -OCH₂(optionally substituted 6-membered non-aromatic heterocycloalkyl containing 1, 2 or 3 heteroatoms selected from O, N, and S).

In the compounds of the present invention, R³ is still more preferably selected from the group consisting of H, hydroxyl, -OCH₃, -OCH₂F, -OCF₃, -OEt, -OC₃₋₈alkyl, -OC₃₋₆cycloalkyl, -OCH₂C₃₋₆cycloalkyl, in particular from the group consisting of H and hydroxyl. Accordingly, in particularly preferred compounds R³ may be H. Accordingly, in further particularly preferred compounds R³ may be hydroxyl.

In a series of particularly preferred compounds of the present invention or pharmaceutically acceptable salts thereof, R² is H or methyl, and R³ is selected from the group consisting of H, hydroxyl, -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCHz(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); -OCH₂(optionally substituted 6-membered non-aromatic heterocycloalkyl containing 1, 2 or 3 heteroatoms selected from O, N, and S); and R¹, R⁴, R⁵, and R⁶ are as defined herein for the compounds of formula (I) (said definitions being either the broadest definition disclosed herein for said groups or any of the narrower definitions disclosed for said group including those stipulated as being "preferred", "more preferred", "most preferred" etc. or words to that effect).

In the compounds of the present invention, R⁴ is selected from the group consisting of H, methyl, ethyl, -CH₂F, -CF₃, straight chain or branched C₃₋₆alkyl, substituted straight chain C₂₋₆alkyl, substituted branched C₃₋₆alkyl, optionally substituted C₃₋₆cycloalkyl, optionally substituted -CH₂C₃₋₆cycloalkyl, formyl, optionally substituted phenyl, optionally substituted 5- or 6-membered heteroaryl, 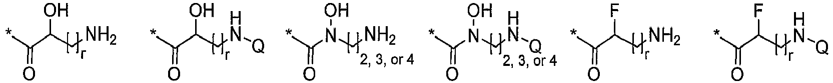 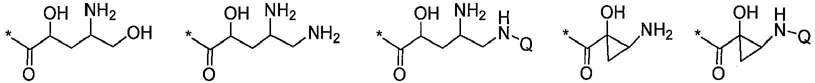 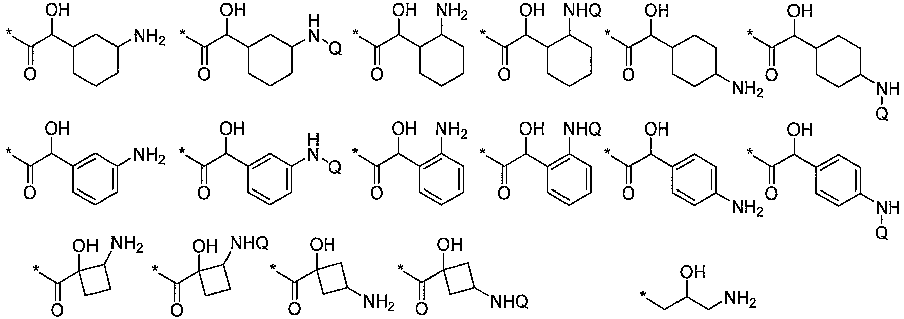 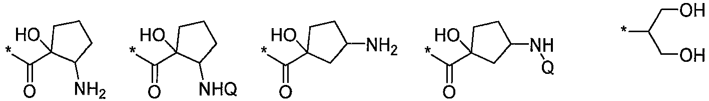 where * is the point of connection to the N atom to which R⁴ is attached in formula (I), and wherein
when R⁴ is substituted straight chain C₂₋₆alkyl, substituted branched C₃₋₆alkyl, substituted C₃₋₆cycloalkyl, or substituted -CH₂C₃₋₆cycloalkyl, it is substituted with one or more substituents independently selected from the group consisting of halogen, hydroxyl, -OCH₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom of said R⁴ group which is directly bonded to the N atom to which R⁴ is connected in the structure of formula (I) may only be substituted with one or more substituents independently selected from halogen.

In said R⁴ groups, r and Q are as defined herein above for the compounds of formula (I).

In this regard, the wording "with the proviso that the carbon atom of said R⁴ group which is directly bonded to the N atom to which R⁴ is connected in the structure of formula (I) may only be substituted with one or more substituents independently selected from halogen" is to be understood to mean, taking R⁴ = substituted ethyl as an example, that where R⁴ is substituted ethyl, the underscored carbon atom -CH₂CH₃ (i.e. the carbon atom joining the -Et group to the N atom to which R⁴ is attached in formula (I)) may only be substituted with one or more substituents independently selected from halogen, whereas the non-underscored carbon atom (that of the CH₃ group of the -Et substituent) may be substituted with any of the previously recited allowable substituents, i.e. it may be substituted with one or more substituents independently selected from the group consisting of halogen, hydroxyl, -OCH₃, -OC₂₋₄alkyl, -NH₂, -NHCH₃, -NHC₂₋₄alkyl, -N(CH₃)₂, -N(C₂₋₄alkyl)₂, and -NHQ.

In the compounds of the present invention, R⁴ is preferably selected from the group consisting of H, 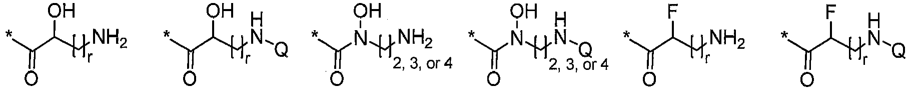 wherein in said R⁴ moieties r and Q are as defined herein above for the compounds of formula (I).

In the compounds of the present invention, R⁴ is more preferably selected from the group consisting of H,

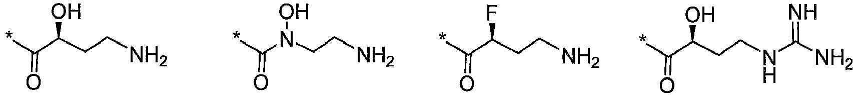

Accordingly, R⁴ may be H.

Accordingly, in particularly preferred compounds of the invention, R⁴ may be

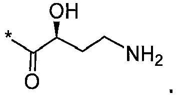

Accordingly, in further particularly preferred compounds of the invention R⁴ may be

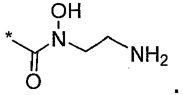

Accordingly, in further particularly preferred compounds of the invention R⁴ may be

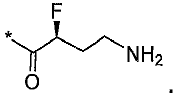

Accordingly, in further particularly preferred compounds of the invention R⁴ may be

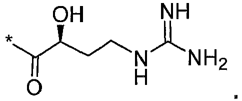

In the compounds of the present invention, R⁵ is selected from the group consisting of H; methyl; -CH₂F; -CF₃; ethyl; straight chain or branched C₃₋₈alkyl; substituted straight chain C₂₋₈alkyl; substituted branched C₃₋₈alkyl; optionally substituted C₃₋₆cycloalkyl; optionally substituted -CH₂C₃₋₆cycloalkyl; optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; optionally substituted 6-membered non-aromatic heterocycloalkyl containing 1, 2 or 3 heteroatoms selected from O, N, and S; -C(=NH)NH₂; -C(=NR⁷)NH₂; -C(=NH)NHR⁸; -C(=NR⁷)NHR⁸; -C(=NR⁷)NR⁸R⁹; and
-X-Z, wherein
   X is selected from the group consisting of methylene, ethylene, straight chain or branched C₃₋₈alkylene; each of which may in addition to being attached to Z be optionally substituted with one or more substituents independently selected from the group consisting of methyl, -CH₂F, -CF₃, ethyl, straight chain or branched C₃₋₆alkyl, halogen, -OH, -OCH₃, -OCH₂F, -OCF₃, -OC₂₋₆alkyl, -NH₂, -NHQ, -NHR¹⁰, -NR¹⁰R¹¹, -CO₂H, -CO₂CH₃, -CO₂C₂₋₆alkyl, -OCOCH₃, -OCOC₂₋₆alkyl, -CN, -CONHR¹², -CONR¹²R¹³, -NHCOCH₃, -NHCOC₂₋₆alkyl, -NR¹⁴COCH₃, and -NR¹⁵COC₂₋₆alkyl; with the proviso that the atom of said X group which connects it to the N atom to which R⁵ is connected in the structure of formula (I) cannot be directly connected to a further O or N atom; and
   Z is selected from the group consisting of optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; optionally substituted 6-membered non-aromatic heterocycloalkyl containing 1, 2 or 3 heteroatoms selected from O, N, and S; optionally substituted C₃₋₆cycloalkyl;
wherein each of R⁷ to R¹⁵ is independently selected from the group consisting of H, methyl, ethyl, C₃₋₆alkyl, and C₃₋₆cycloalkyl.

In this regard, the wording "with the proviso that the atom of said X group which connects it to the N atom to which R⁵ is connected in the structure of formula (I) cannot be directly connected a further O or N atom" is to be understood to mean, taking X = ethylene as an example (the X group may be methylene, ethylene, or straight chain or branched C₃₋₈alkylene), that where X is substituted ethylene, the underscored carbon atom -CH₂CH₂-Z of the ethylene group (i.e. the atom of said X group which connects it to the N atom in the structure of formula (I) to which R⁵ is connected) cannot be substituted with a group which would give rise to said underscored carbon atom being directly attached to an O or N atom of said substituent. In other words, the only N or O atom that the underscored carbon atom can be attached to is the N atom in the structure of formula (I) to which R⁵ is connected. This proviso does not apply to the non-underscored carbon atom of the -CH₂CH₂-Z group.

Where in the compounds of the present invention R⁵ is substituted straight chain C₂₋₈alkyl, substituted branched C₃₋₈alkyl, substituted C₃₋₆cycloalkyl, or substituted -CH₂C₃₋₆cycloalkyl, it may be substituted with any substituent. In this context, R⁵ is however preferably substituted with one or more substituents independently selected from the group consisting of methyl, -CH₂F, -CF₃, ethyl, straight chain or branched C₃₋₆alkyl, halogen, -OH, -OCH₃, -OCH₂F, -OCF₃, -OC₂₋₆alkyl, -NH₂, -NHQ, -NHR¹⁰, -NR¹⁰R¹¹, -CO₂H, -CO₂CH₃, -CO₂C₂₋₆alkyl, -OCOCH₃, -OCOC₂₋₆alkyl, -CN, -CONHR⁷, -CONR¹²R¹³, -NHCOCH₃, -NHCOC₂₋₆alkyl, -NR¹⁴COCH₃, and -NR¹⁵COC₂₋₆alkyl; wherein each of R⁷ to R¹⁵ is independently selected from the group consisting of H, methyl, ethyl, C₃₋₆alkyl, and C₃₋₆cycloalkyl. In this context, the proviso that the atom of said C₂₋₈alkyl, substituted branched C₃₋₈alkyl, substituted C₃₋₆cycloalkyl, or substituted -CH₂C₃₋₆cycloalkyl group which connects it to the N atom to which R⁵ is connected in the structure of formula (I) cannot be directly connected to a further O or N atom preferably applies.

In the compounds of the present invention, when R⁵ may be optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; said heterocycloalkyl groups may preferably be selected from the group consisting of optionally substituted azetidinyl, optionally substituted diazetidinyl, optionally substituted oxetanyl, optionally substituted thietanyl, optionally substituted pyrrolidinyl, optionally substituted tetrahydrofuranyl, optionally substituted tetrahydrothiophenyl, optionally substituted piperidinyl, optionally substituted tetrahydropyranyl, optionally substituted thianyl, optionally substituted imidazolidinyl, optionally substituted pyrazolidinyl, optionally substituted oxazolidinyl, optionally substituted isooxazolidinyl, optionally substituted thiazolidinyl, optionally substituted isothiazolidinyl, optionally substituted dioxolanyl, optionally substituted dithiolanyl, optionally substituted piperazinyl, optionally substituted morpholinyl, optionally substituted thiomorpholinyl, optionally substituted 1,3-dioxanyl, optionally substituted 1,4-dioxanyl, optionally substituted 1,3-dithianyl, and optionally substituted 1,4-dithianyl; wherein the optional substituents are as defined elsewhere herein for said heterocycloalkyl groups. In this context, said optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; may more preferably be selected from the group consisting of 4-piperidinyl, 3-piperidinyl, 2-piperidinyl, 2-pyrrolidinyl, 3-pyrrolidinyl, 4-oxazolidinyl, 2-morpholinyl, 3-morpholinyl, 4-morpholinyl, 2-tetrahydrofuranyl, and 3-tetrahydrofuranyl.

In the compounds of the present invention, where R⁵ or Z is optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; optionally substituted means optionally substituted with one or more of any substituent. In this context, optionally substituted however preferably means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; wherein each of R¹⁶ to R³⁸ is independently selected from the group consisting of H, methyl, ethyl, C₃₋₆alkyl, and C₃₋₆cycloalkyl. In this context, the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸, preferably applies. In this context, the proviso that when one of said substituents is joined directly to a N-atom in the respective heteroaryl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸, preferably applies.

In the compounds of the present invention, R⁵ is preferably selected from the group consisting of H, methyl, ethyl, straight chain or branched C₃₋₈alkyl, substituted straight chain C₂₋₈alkyl, substituted branched chain C₃₋₈alkyl; optionally substituted C₃₋₆cycloalkyl, 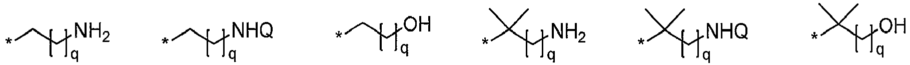 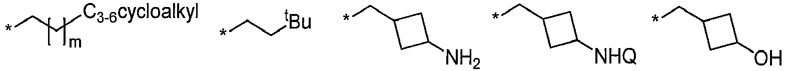 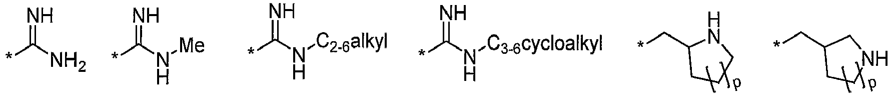 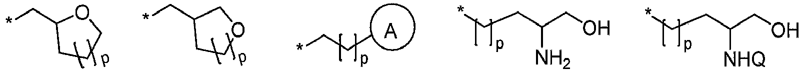 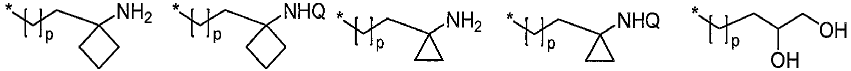 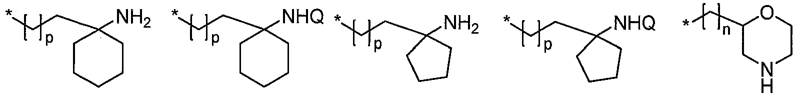 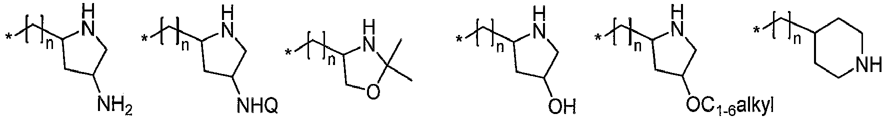 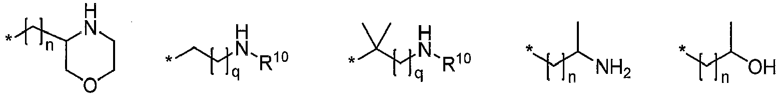 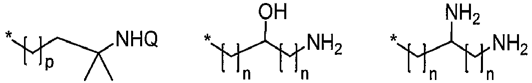
where * is the point of connection to the N atom to which R⁵ is attached in formula (I), and
wherein A is selected from the group consisting of optionally substituted phenyl, optionally substituted 5-membered heteroaryl, and optionally substituted 6-membered heteroaryl, optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; and
wherein m, n, q, p, and R¹⁰ in said R⁵ moieties are as defined herein for the compounds of formula (I) (said definitions being either the broadest definition disclosed hereinabove for said groups or any of the narrower definitions disclosed for said groups including those stipulated as being "preferred", "more preferred", "most preferred" etc. or words to that effect).

In this context, where A is optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; optionally substituted means optionally substituted with one or more of any substituent. In this context, optionally substituted however preferably means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸; wherein each of R¹⁶ to R³⁸ is independently selected from the group consisting of H, methyl, ethyl, C₃₋₆alkyl, and C₃₋₆cycloalkyl. In this context, the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸, preferably applies. In this context, the proviso that when one of said substituents is joined directly to a N-atom in the respective heteroaryl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸, preferably applies.

In the compounds of the present invention, R⁵ is more preferably selected from the group consisting of H, methyl, ethyl, straight chain or branched C₃₋₆alkyl, 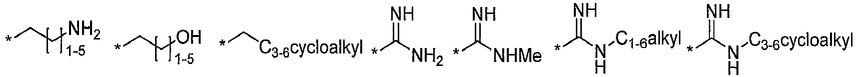 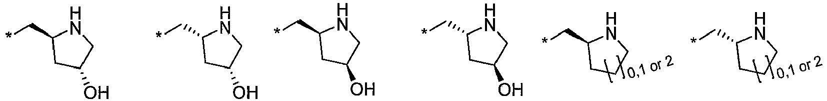 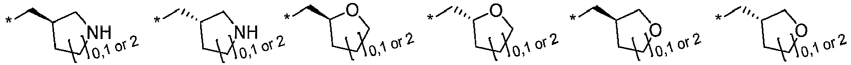 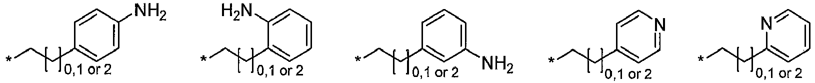 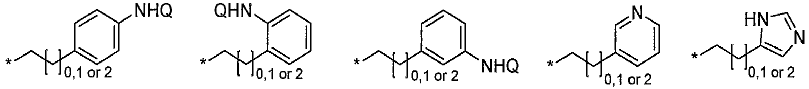 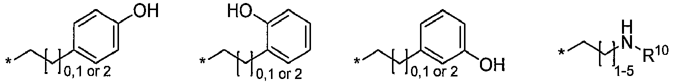 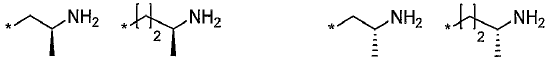 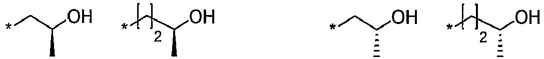 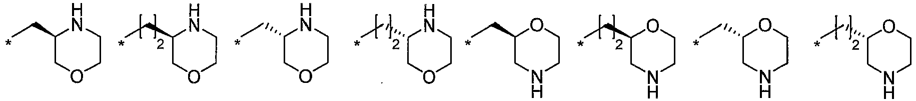 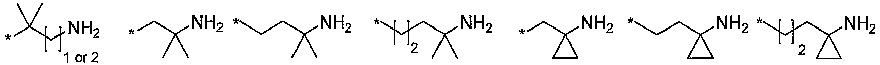 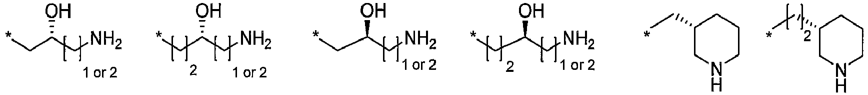 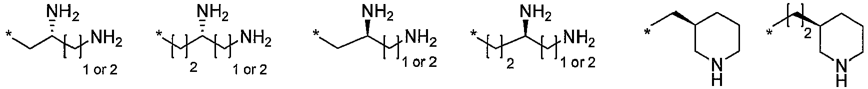 wherein Q and R¹⁰ in said R⁵ moieties are as defined herein for the compounds of formula (I) (said definitions being either the broadest definition disclosed hereinabove for said groups or any of the narrower definitions disclosed for said groups including those stipulated as being "preferred", "more preferred", "most preferred" etc. or words to that effect).

In the compounds of the present invention, R⁵ is most preferably selected from the group consisting of H,

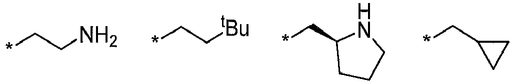

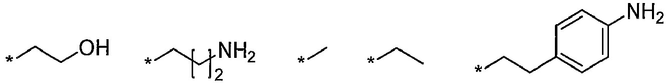

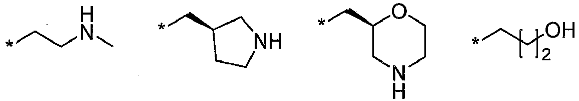

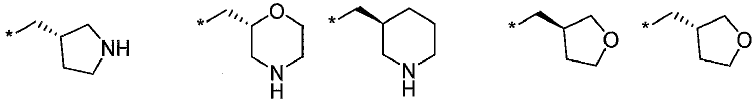

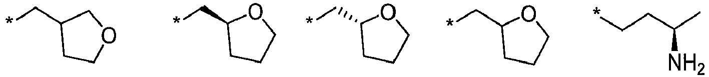

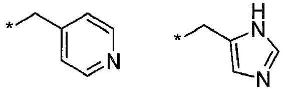

In the present invention, R⁶ is selected from the group consisting of OH and NH₂. In preferred compounds, R⁶ is OH. In other preferred compounds, R⁶ is NH₂.

The compound of formula (I) or pharmaceutically acceptable salt thereof may preferably be selected from the compounds of formula (Ia) to (If) 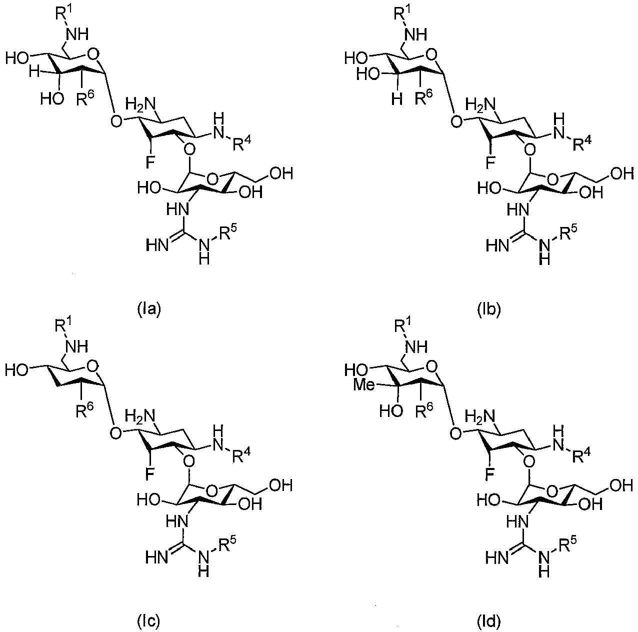 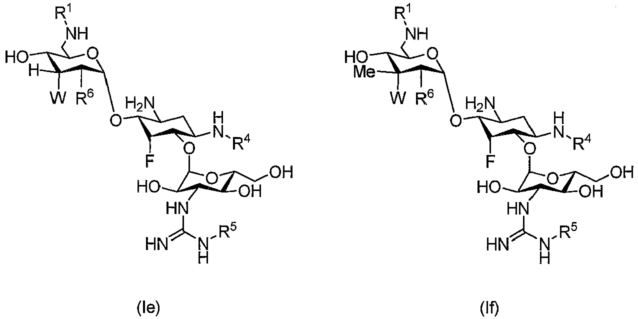 wherein independently for each of the compounds of formula (Ia) to (If) R¹, R⁴, R⁵ and R⁶ are as defined for the compounds of formula (I) (said definitions being either the broadest definition disclosed hereinabove for said groups or any of the narrower definitions disclosed for said group including those stipulated as being "preferred", "more preferred", "most preferred" etc. or words to that effect), and wherein W is independently selected for each of the compounds of formula (Ie) to (If) from the group consisting of -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); and -OCH₂(optionally substituted 6-membered non-aromatic heterocycloalkyl containing 1, 2 or 3 heteroatoms selected from O, N, and S).

Particulary preferred in this context are compounds of formula (Ia) to (If) in which R¹ is H. Further preferred compounds in this context are those in which in the compounds of formula (Ia) to (If) R⁶ is OH. Accordingly, in particulary preferred compounds of formula (Ia) to (If) R¹ is H and R⁶ is OH. Further preferred compounds in this context are those in which in the compounds of formula (Ia) to (If) R⁶ is NH₂. Accordingly, in particulary preferred compounds of formula (Ia) to (If) R¹ is H and R⁶ is NH₂.

Further preferred in this context are compounds of formula (Ia) to (If) in which R⁴ is selected from the group consisting of H, 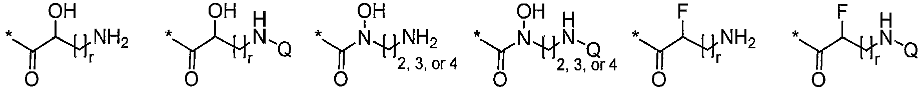 wherein in said R⁴ moieties r and Q are as defined for the compounds of formula (I). In particulary preferred compounds of formula (Ia) to (If) R⁴ is selected from the group consisting of H,

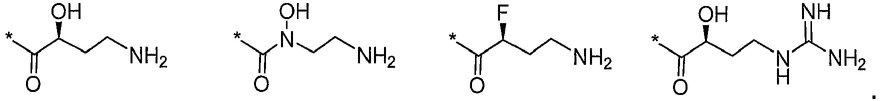

Accordingly, in particulary preferred compounds of formula (Ia) to (If) R¹ is H and R⁴ is selected from the group consisting of H, 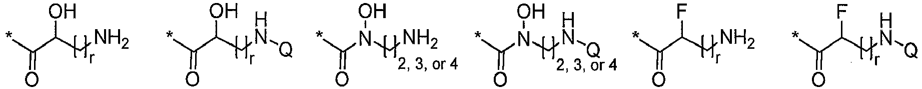 wherein in said R⁴ moieties r and Q are as defined for the compounds of formula (I), most preferably from the group consisting of H,

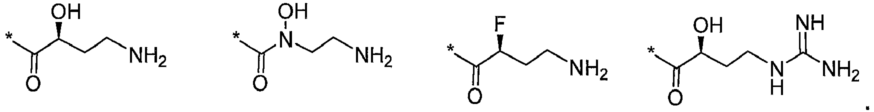

In this context, R⁶ may be OH. In this context, R⁶ may be NH₂.

Within the context of all of the foregoing relating to the compounds of formula (Ia) to (If), especially preferred are compounds of formula (Ic). Accordingly, even more especially preferred are compounds of formula (Ic) wherein R¹ is H, in particular wherein R¹ is H, R⁶ is OH and R⁴ is selected from the group consisting of H, 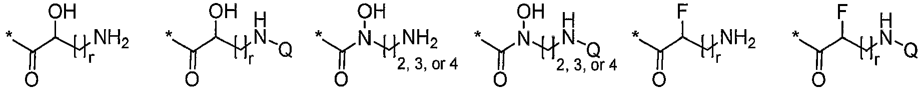 wherein in said R⁴ moieties r and Q are as defined for the compounds of formula (I), most preferably from the group consisting of H,

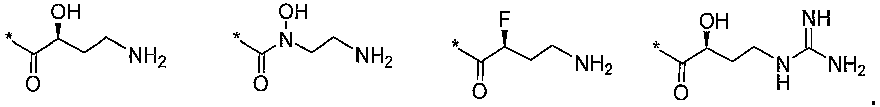

Accordingly, further even more especially preferred are compounds of formula (Ic) wherein R¹ is H, in particular wherein R¹ is H, R⁶ is NH₂ and R⁴ is selected from the group consisting of H, 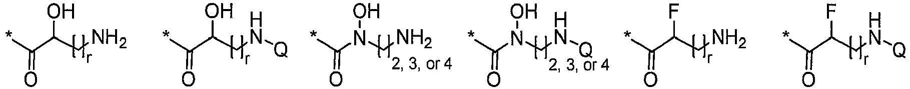 wherein in said R⁴ moieties r and Q are as defined for the compounds of formula (I), most preferably from the group consisting of H,

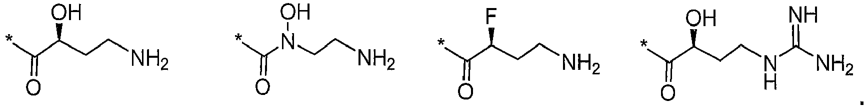

Further particulary preferred in the above context relating to compounds of formula (Ia) to (If) are compounds of formula (Ia) to (If) in which R¹ is *-CH₂CH₂OH (where * denotes the point of connection to the N atom to which R⁵ is attached in each of formula (Ia) to (If)). Further preferred compounds in this context are those in which in the compounds of formula (Ia) to (If) R⁶ is OH. Accordingly, in particulary preferred compounds of formula (Ia) to (If) R¹ is *-CH₂CH₂OH and R⁶ is OH. Further preferred compounds in this context are those in which in the compounds of formula (Ia) to (If) R⁶ is NH₂. Accordingly, in particulary preferred compounds of formula (Ia) to (If) R¹ is *-CH₂CH₂OH and R⁶ is NH₂.

Further preferred in this context are compounds of formula (Ia) to (If) in which R⁴ is selected from the group consisting of H, 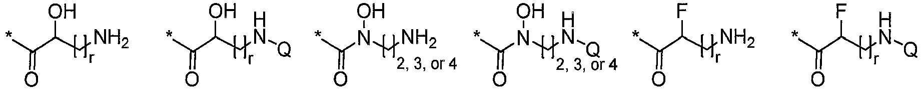 wherein in said R⁴ moieties r and Q are as defined for the compounds of formula (I). In particulary preferred compounds of formula (Ia) to (If) R⁴ is selected from the group consisting of H,

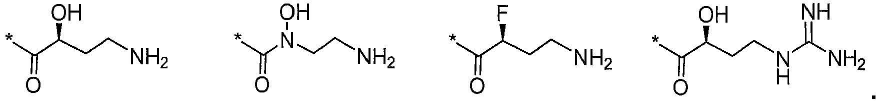

Accordingly, in particulary preferred compounds of formula (Ia) to (If) R¹ is *-CH₂CH₂OH and R⁴ is selected from the group consisting of H, 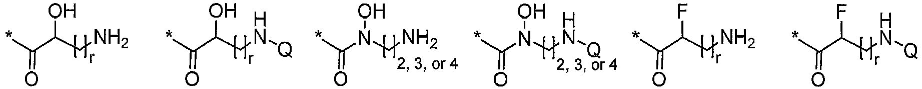 wherein in said R⁴ moieties r and Q are as defined for the compounds of formula (I), most preferably from the group consisting of H,

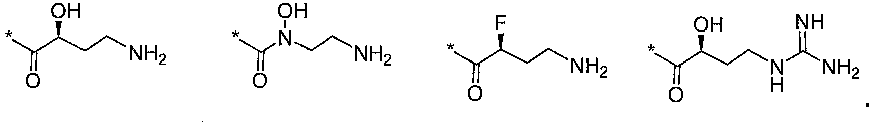

In this context, R⁶ may be OH. In this context, R⁶ may be NH₂.

Within the context of all of the foregoing relating to the compounds of formula (Ia) to (If), especially preferred are compounds of formula (Ic). Accordingly, even more especially preferred are compounds of formula (Ic) wherein R¹ is *-CH₂CH₂OH, in particular wherein R¹ is *-CH₂CH₂OH, R⁶ is OH and R⁴ is selected from the group consisting of H, 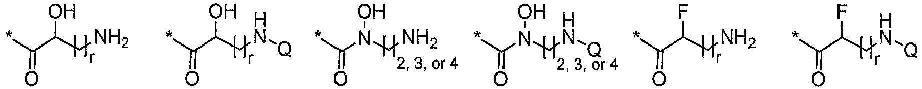 wherein in said R⁴ moieties r and Q are as defined for the compounds of formula (I), most preferably from the group consisting of H,

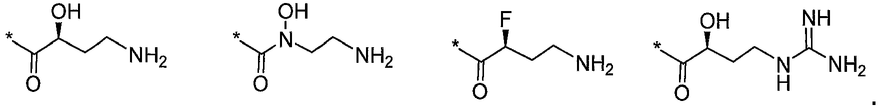

Accordingly, further even more especially preferred are compounds of formula (Ic) wherein R¹ is *-CH₂CH₂OH, in particular wherein R¹ is *-CH₂CH₂OH, R⁶ is NH₂ and R⁴ is selected from the group consisting of H, 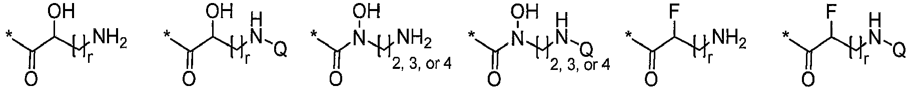 wherein in said R⁴ moieties r and Q are as defined for the compounds of formula (I), most preferably from the group consisting of H,

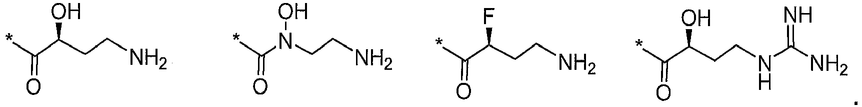

The compound of formula (I) or pharmaceutically acceptable salt thereof may be even more preferably selected from the compounds of formula (Ig) to (Ip) 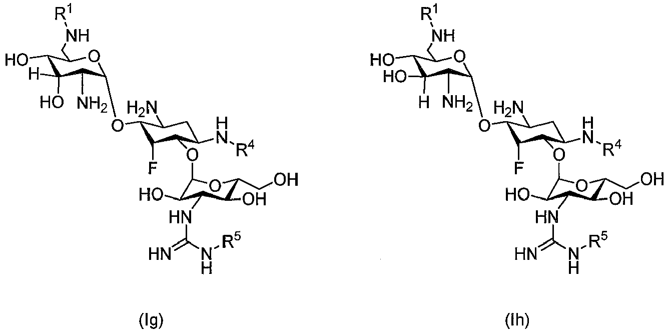 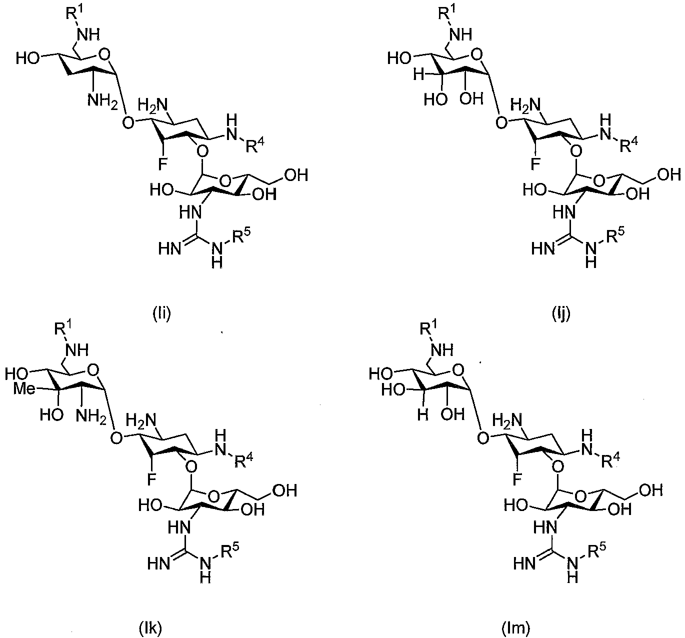 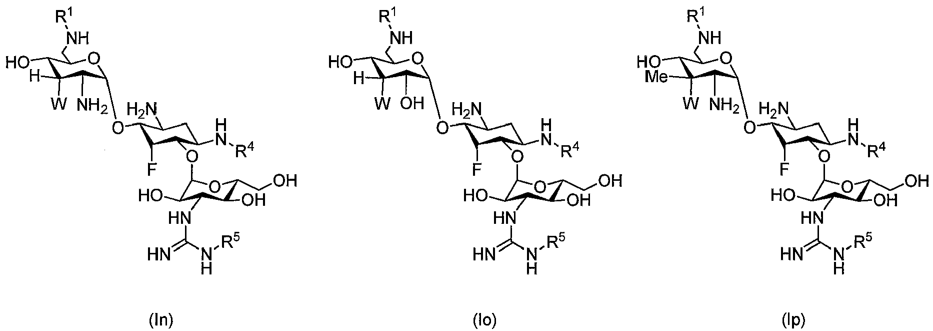 wherein independently for each of the compounds of formula (Ig) to (Ip) R¹, R⁴, and R⁵ are as defined for the compounds of formula (I) (said definitions being either the broadest definition disclosed hereinabove for said groups or any of the narrower definitions disclosed for said group including those stipulated as being "preferred", "more preferred", "most preferred" etc. or words to that effect), and wherein W is independently selected for each of the compounds of formula (In) to (Ip) from the group consisting of -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); and -OCH₂(optionally substituted 6-membered non-aromatic heterocycloalkyl containing 1, 2 or 3 heteroatoms selected from O, N, and S).

Particulary preferred in this context are compounds of formula (Ig) to (Ip) in which R¹ is H.

Further preferred in this context are compounds of formula (Ig) to (Ip) in which R⁴ is selected from the group consisting of H, 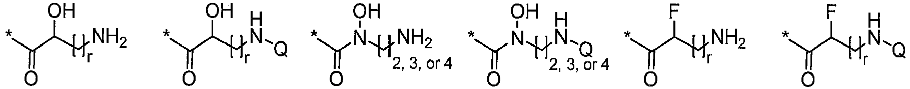 wherein in said R⁴ moieties r and Q are as defined for the compounds of formula (I). In particulary preferred compounds of formula (Ip) to (Ig) R⁴ is selected from the group consisting of H,

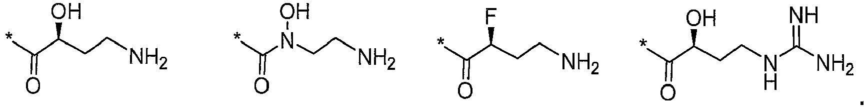

Accordingly, in particulary preferred compounds of formula (Ig) to (Ip) R¹ is H and R⁴ is selected from the group consisting of H, 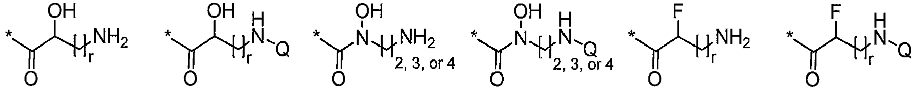 wherein in said R⁴ moieties r and Q are as defined for the compounds of formula (I), most preferably from the group consisting of H,

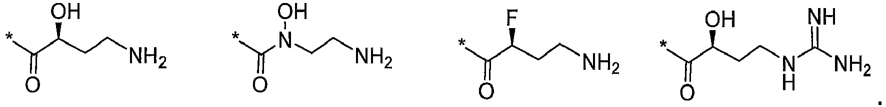

Within the context of all of the foregoing relating to the compounds of formula (Ig) to (Ip), especially preferred are compounds of formula (Ii). Accordingly, even more especially preferred are compounds of formula (Ii) wherein R¹ is H, in particular wherein R¹ is H, and R⁴ is selected from the group consisting of H, 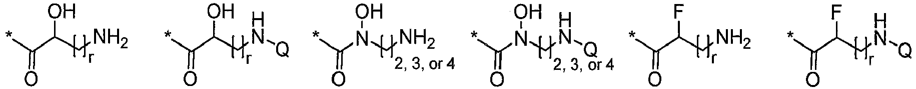 wherein in said R⁴ moieties r and Q are as defined for the compounds of formula (I), most preferably from the group consisting of H,

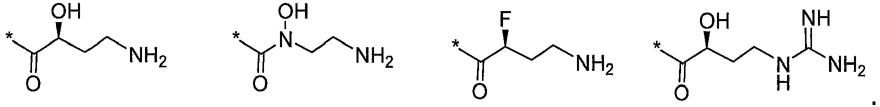

Further particulary preferred in the above context relating to compounds of formula (Ig) to (Ip) are compounds of formula (Ig) to (Ip) in which R¹ is *-CH₂CH₂OH (where * denotes the point of connection to the N atom to which R⁵ is attached in each of formula (Ig) to (Ip)).

Further preferred in this context are compounds of formula (Ig) to (Ip) in which R⁴ is selected from the group consisting of H, 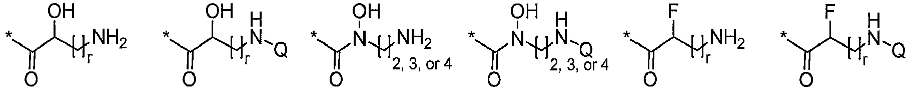 wherein in said R⁴ moieties r and Q are as defined for the compounds of formula (I). In particulary preferred compounds of formula (Ip) to (Ig) R⁴ is selected from the group consisting of H,

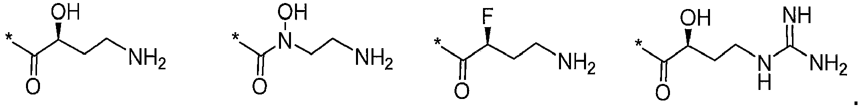

Accordingly, in particulary preferred compounds of formula (Ig) to (Ip) R¹ is *-CH₂CH₂OH and R⁴ is selected from the group consisting of H, 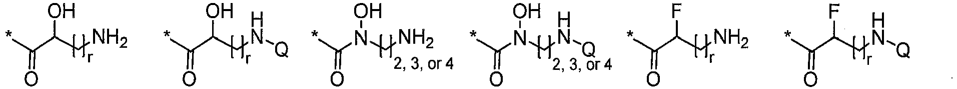 wherein in said R⁴ moieties r and Q are as defined for the compounds of formula (I), most preferably from the group consisting of H,

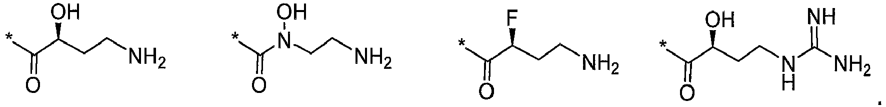

Within the context of all of the foregoing relating to the compounds of formula (Ig) to (Ip), especially preferred are compounds of formula (Ii). Accordingly, even more especially preferred are compounds of formula (Ii) wherein R¹ is *-CH₂CH₂OH, in particular wherein R¹ is *-CH₂CH₂OH, and R⁴ is selected from the group consisting of H, 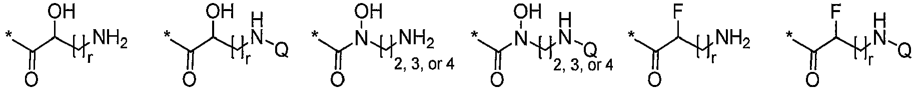 wherein in said R⁴ moieties r and Q are as defined for the compounds of formula (I), most preferably from the group consisting of H,

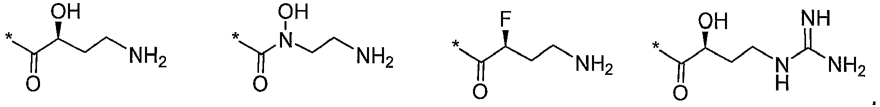

Within the context of all of the foregoing relating to compounds of formula (Ia) to (If) and (Ig) to (Ip) including the recited preferred compounds and preferred combinations of R¹, R⁴ and/or R⁶ in respect of compounds of formula (Ia) to (If) and (Ig) to (Ip), R⁵ is preferably selected from the group consisting of H, methyl, ethyl, straight chain or branched C₃₋₈alkyl, substituted straight chain C₂₋₈alkyl, substituted branched chain C₂₋₈alkyl; optionally substituted C₃₋₆cycloalkyl, 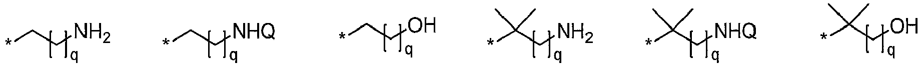 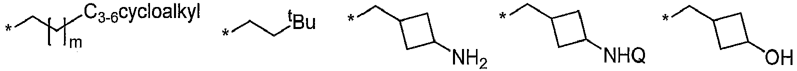 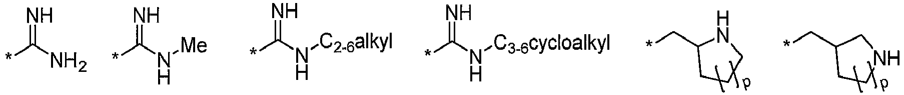 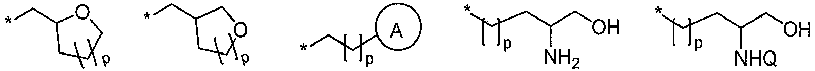 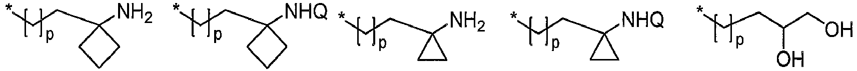 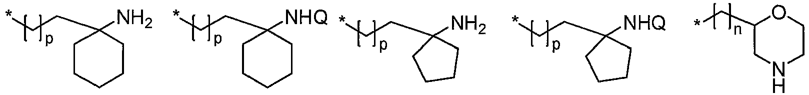 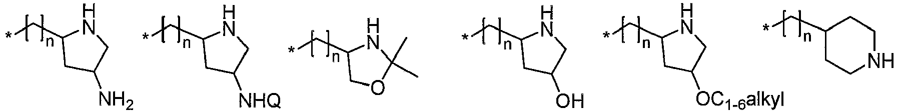 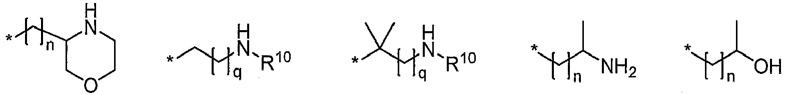 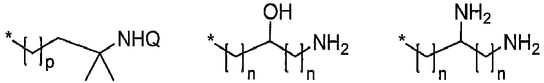
where * is the point of connection to the N atom to which R⁵ is attached in formula (I), and
wherein A is selected from the group consisting of optionally substituted phenyl, optionally substituted 5-membered heteroaryl, and optionally substituted 6-membered heteroaryl, optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; and
wherein m, n, q, p, and R¹⁰ in said R⁵ moieties are as defined herein for the compounds of formula (I) (said definitions being either the broadest definition disclosed hereinabove for said groups or any of the narrower definitions disclosed for said groups including those stipulated as being "preferred", "more preferred", "most preferred" etc. or words to that effect).

In this context, where A is optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; optionally substituted means optionally substituted with one or more of any substituent. In this context, optionally substituted however preferably means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and - C(=NR³⁷)NHR³⁸; wherein each of R¹⁶ to R³⁸ is independently selected from the group consisting of H, methyl, ethyl, C₃₋₆alkyl, and C₃₋₆cycloalkyl. In this context, the proviso that when one of said substituents is joined directly to a N-atom in the respective non-aromatic heterocycloalkyl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸, preferably applies. In this context, the proviso that when one of said substituents is joined directly to a N-atom in the respective heteroaryl moiety it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸, preferably applies.

Within the context of all of the foregoing relating to compounds of formula (Ia) to (If) and (Ig) to (Ip) including the recited preferred compounds and preferred combinations of R¹, R⁴ and/or R⁶ in respect of compounds of formula (Ia) to (If) and (Ig) to (Ip), R⁵ is more preferably selected from the group consisting of H, methyl, ethyl, straight chain or branched C₃₋₆alkyl, 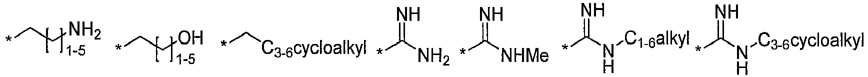 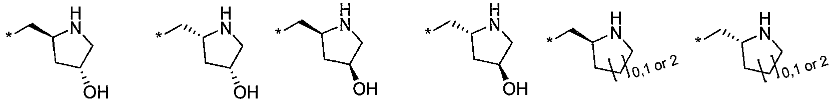 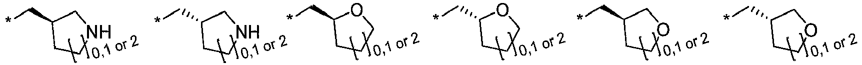 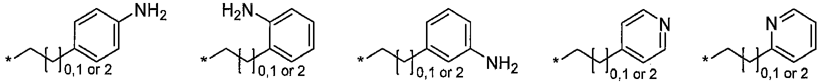 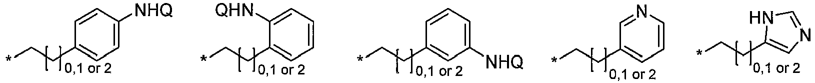 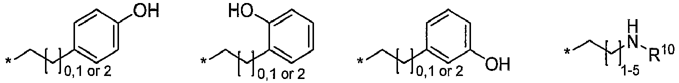 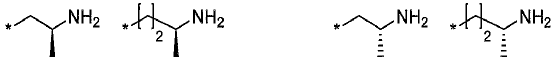 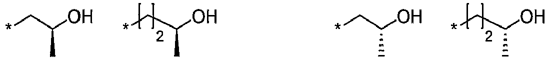 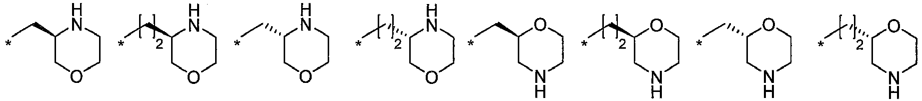 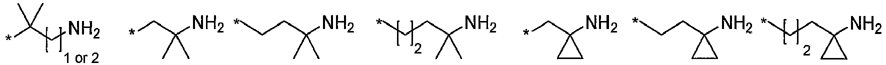 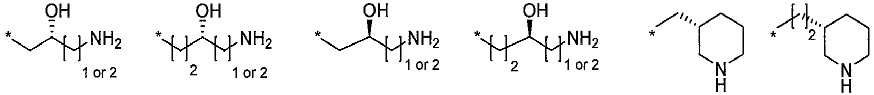 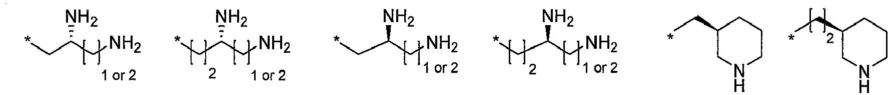 wherein Q and R¹⁰ in said R⁵ moieties are as defined herein for the compounds of formula (I) (said definitions being either the broadest definition disclosed hereinabove for said groups or any of the narrower definitions disclosed for said groups including those stipulated as being "preferred", "more preferred", "most preferred" etc. or words to that effect).

Within the context of all of the foregoing relating to compounds of formula (Ia) to (If) and (Ig) to (Ip) including the recited preferred compounds and preferred combinations of R¹, R⁴ and/or R⁶ in respect of compounds of formula (Ia) to (If) and (Ig) to (Ip), R⁵ is most preferably selected from the group consisting of H,

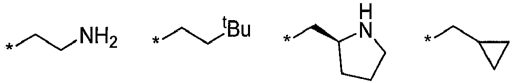

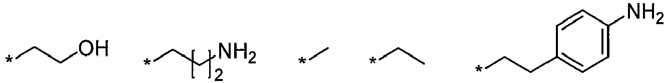

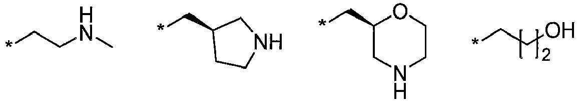

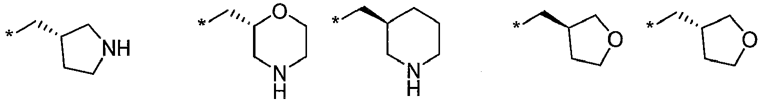

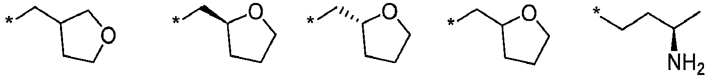

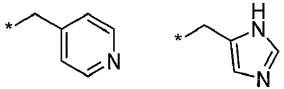

Other preferred compounds of the present invention are compounds of formula (Iq) 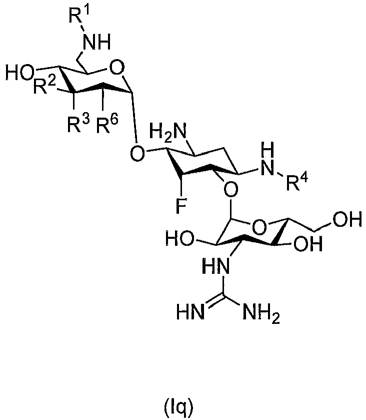 wherein R¹, R², R³, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect). Accordingly, particularly preferred compounds within this context include the compounds of formula (Iq-a) to (Iq-f) 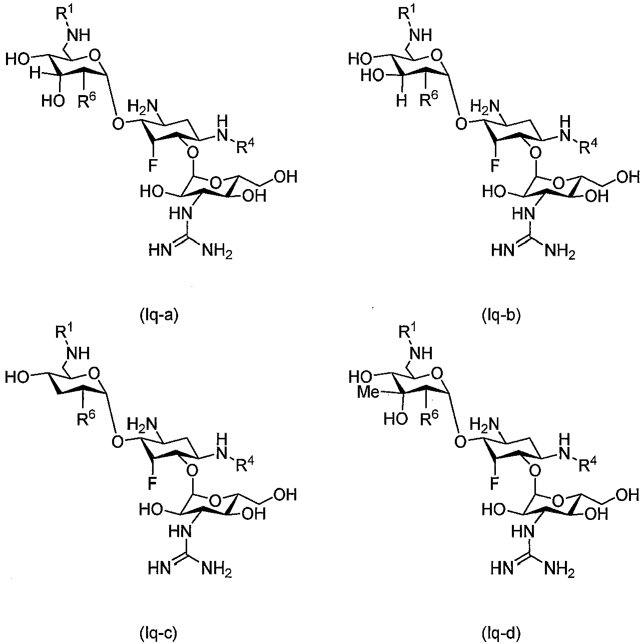 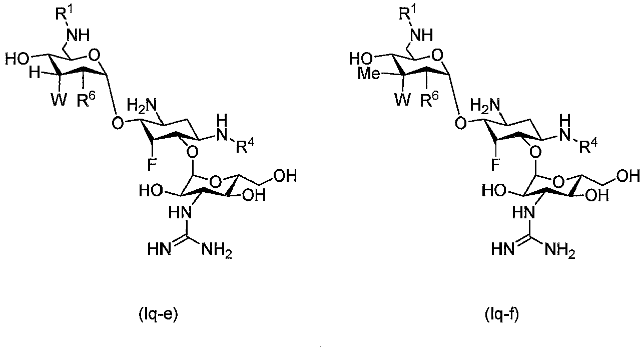 wherein R¹, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect).

Particulary preferred in this context are compounds of formula (Iq-a) to (Iq-f) in which R¹ is H. Further preferred compounds in this context are those in which in the compounds of formula (Iq-a) to (Iq-f) R⁶ is OH. Accordingly, in particulary preferred compounds of formula (Iq-a) to (Iq-f) R¹ is H and R⁶ is OH. Further preferred compounds in this context are those in which in the compounds of formula (Iq-a) to (Iq-f) R⁶ is NH₂. Accordingly, in particulary preferred compounds of formula (Iq-a) to (Iq-f) R¹ is H and R⁶ is NH₂.

Further preferred in this context are compounds of formula (Iq-a) to (Iq-f) in which R⁴ is selected from the group consisting of H, 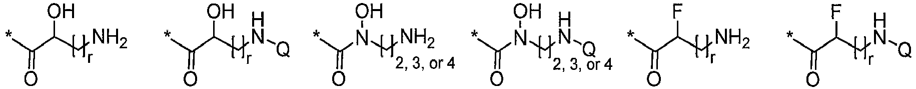 wherein in said R⁴ moieties r and Q are as defined for the compounds of formula (I). In particulary preferred compounds of formula (Iq-a) to (Iq-f) R⁴ is selected from the group consisting of H,

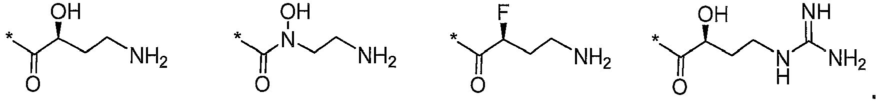

Accordingly, in particulary preferred compounds of formula (Iq-a) to (Iq-f) R¹ is H and R⁴ is selected from the group consisting of H, 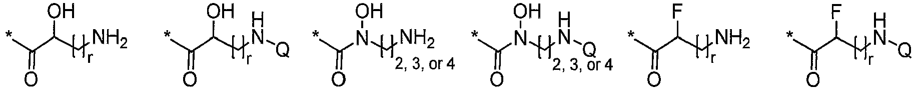 wherein in said R⁴ moieties r and Q are as defined for the compounds of formula (I), most preferably from the group consisting of H,

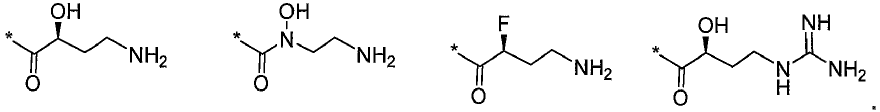

In this context, R⁶ may be OH. In this context, R⁶ may be NH₂.

Within the context of all of the foregoing relating to the compounds of formula (Iq-a) to (Iq-f), especially preferred are compounds of formula (Iq-c).

Accordingly, even more especially preferred are compounds of formula (Iq-c) wherein R¹ is H, in particular wherein R¹ is H, R⁶ is OH and R⁴ is selected from the group consisting of H, 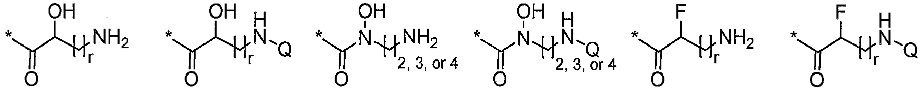 wherein in said R⁴ moieties r and Q are as defined for the compounds of formula (I), most preferably from the group consisting of H,

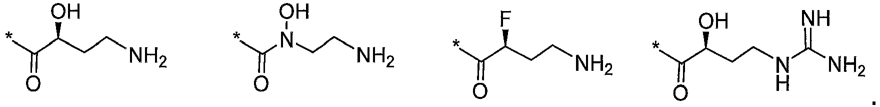

Accordingly, further even more especially preferred are compounds of formula (Iq-c) wherein R¹ is H, in particular wherein R¹ is H, R⁶ is NH₂ and R⁴ is selected from the group consisting of H, 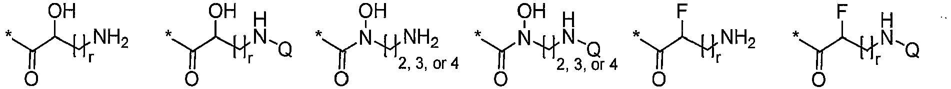 wherein in said R⁴ moieties r and Q are as defined for the compounds of formula (I), most preferably from the group consisting of H,

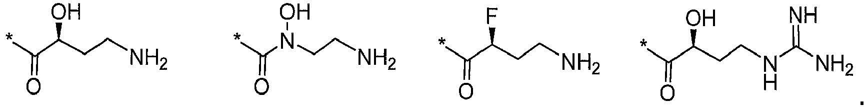

Further particulary preferred in the above context relating to compounds of formula (Iq-a) to (Iq-f) are compounds of formula (Iq-a) to (Iq-f) in which R¹ is *-CH₂CH₂OH (where * denotes the point of connection to the N atom to which R⁵ is attached in each of formula (Iq-a) to (Iq-f)). Further preferred compounds in this context are those in which in the compounds of formula (Iq-a) to (Iq-f) R⁶ is OH. Accordingly, in particulary preferred compounds of formula (Iq-a) to (Iq-f) R¹ is *-CH₂CH₂OH and R⁶ is OH. Further preferred compounds in this context are those in which in the compounds of formula (Iq-a) to (Iq-f) R⁶ is NH₂. Accordingly, in particulary preferred compounds of formula (Iq-a) to (Iq-f) R¹ is *-CH₂CH₂OH and R⁶ is NH₂.

Further preferred in this context are compounds of formula (Iq-a) to (Iq-f) in which R⁴ is selected from the group consisting of H, 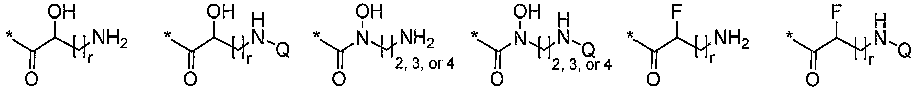 wherein in said R⁴ moieties r and Q are as defined for the compounds of formula (I). In particulary preferred compounds of formula (Iq-a) to (Iq-f) R⁴ is selected from the group consisting of H,

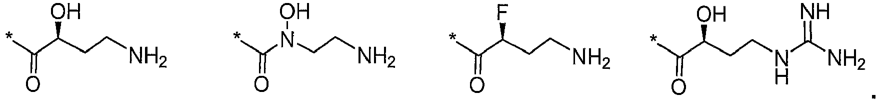

Accordingly, in particulary preferred compounds of formula (Iq-a) to (Iq-f) R¹ is *-CH₂CH₂OH and R⁴ is selected from the group consisting of H, 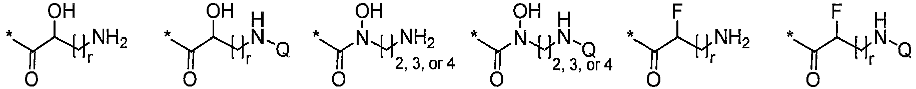 wherein in said R⁴ moieties r and Q are as defined for the compounds of formula (I), most preferably from the group consisting of H,

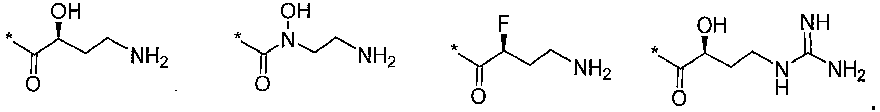

In this context, R⁶ may be OH. In this context, R⁶ may be NH₂.

Within the context of all of the foregoing relating to the compounds of formula (Iq-a) to (Iq-f), especially preferred are compounds of formula (Iq-c). Accordingly, even more especially preferred are compounds of formula (Iq-c) wherein R¹ is *-CH₂CH₂OH, in particular wherein R¹ is *-CH₂CH₂OH, R⁶ is OH and R⁴ is selected from the group consisting of H, 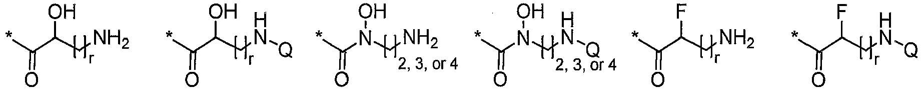 wherein in said R⁴ moieties r and Q are as defined for the compounds of formula (I), most preferably from the group consisting of H,

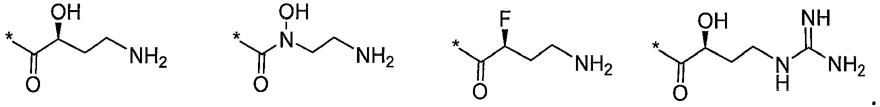

Accordingly, further even more especially preferred are compounds of formula (Iq-c) wherein R¹ is *-CH₂CH₂OH, in particular wherein R¹ is *-CH₂CH₂OH, R⁶ is NH₂ and R⁴ is selected from the group consisting of H, 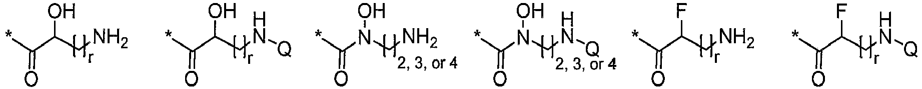 wherein in said R⁴ moieties r and Q are as defined for the compounds of formula (I), most preferably from the group consisting of H,

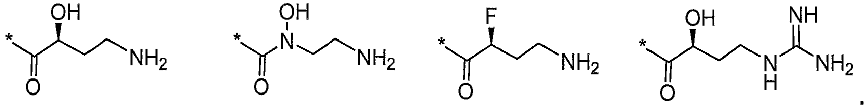

Other preferred compounds of the present invention are compounds of formula (Ir) 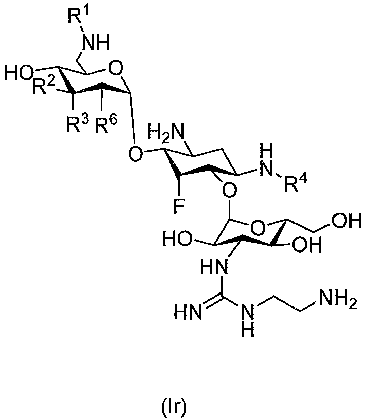 wherein R¹, R², R³, R⁴, and R⁶ are as defined herein above for compounds of formula (I) (said definitions being either the broadest definition disclosed hereinabove for said groups or any of the narrower definitions disclosed for said groups including those stipulated as being preferred", "more preferred", "most preferred" etc. or words to that effect). Accordingly, particularly preferred compounds within this context include the compounds of formula (Ir-a) to (Ir-f) 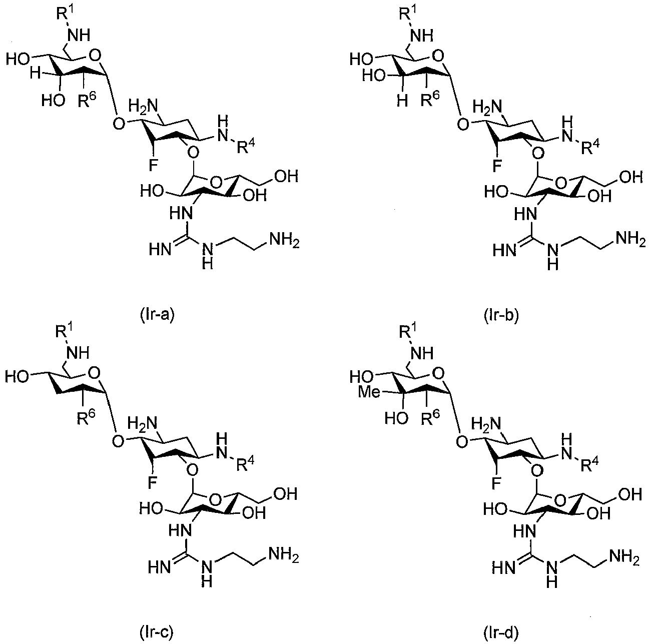 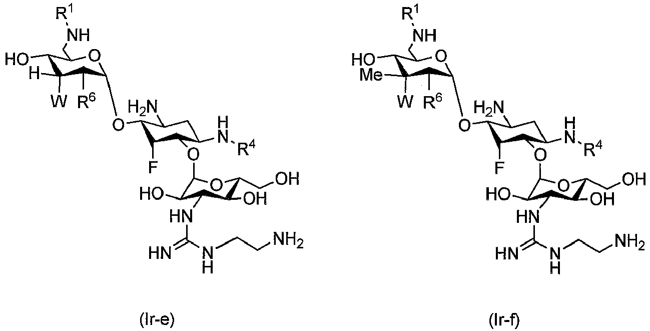 wherein R¹, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect).

Particulary preferred in this context are compounds of formula (Ir-a) to (Ir-f) in which R¹ is H. Further preferred compounds in this context are those in which in the compounds of formula (Ir-a) to (Ir-f) R⁶ is OH. Accordingly, in particulary preferred compounds of formula (Ir-a) to (Ir-f) R¹ is H and R⁶ is OH. Further preferred compounds in this context are those in which in the compounds of formula (Ir-a) to (Ir-f) R⁶ is NH₂. Accordingly, in particulary preferred compounds of formula (Ir-a) to (Ir-f) R¹ is H and R⁶ is NH₂.

Further preferred in this context are compounds of formula (Ir-a) to (Ir-f) in which R⁴ is selected from the group consisting of H, 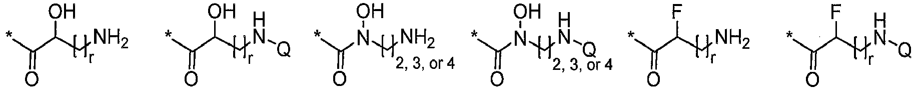 wherein in said R⁴ moieties r and Q are as defined for the compounds of formula (I). In particulary preferred compounds of formula (Ir-a) to (Ir-f) R⁴ is selected from the group consisting of H,

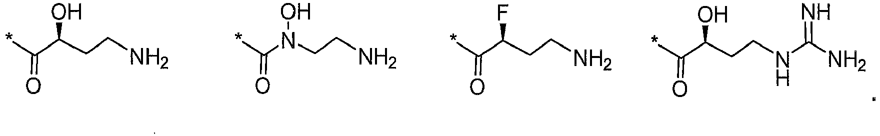

Accordingly, in particulary preferred compounds of formula (Ir-a) to (Ir-f) R¹ is H and R⁴ is selected from the group consisting of H, 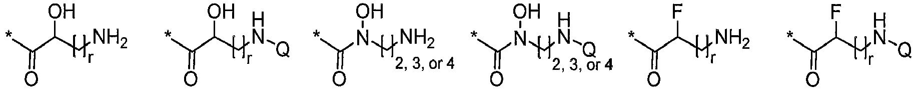 wherein in said R⁴ moieties r and Q are as defined for the compounds of formula (I), most preferably from the group consisting of H,

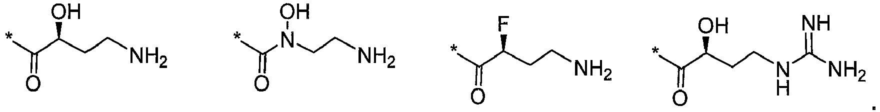

In this context, R⁶ may be OH. In this context, R⁶ may be NH₂.

Within the context of all of the foregoing relating to the compounds of formula (Ir-a) to (Ir-f), especially preferred are compounds of formula (Ir-c). Accordingly, even more especially preferred are compounds of formula (Ir-c) wherein R¹ is H, in particular wherein R¹ is H, R⁶ is OH and R⁴ is selected from the group consisting of H, 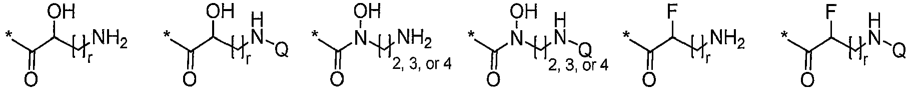 wherein in said R⁴ moieties r and Q are as defined for the compounds of formula (I), most preferably from the group consisting of H,

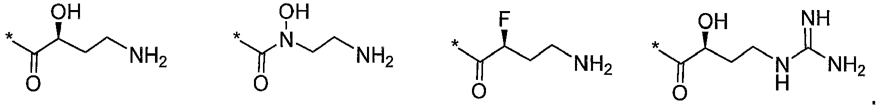

Accordingly, further even more especially preferred are compounds of formula (Ir-c) wherein R¹ is H, in particular wherein R¹ is H, R⁶ is NH₂ and R⁴ is selected from the group consisting of H, 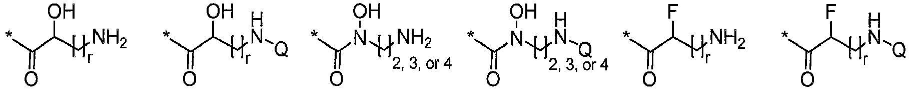 wherein in said R⁴ moieties r and Q are as defined for the compounds of formula (I), most preferably from the group consisting of H,

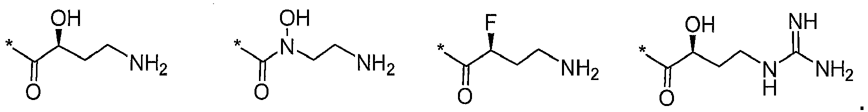

Further particulary preferred in the above context relating to compounds of formula (Ir-a) to (Ir-f) are compounds of formula (Ir-a) to (Ir-f) in which R¹ is *-CH₂CH₂OH (where * denotes the point of connection to the N atom to which R⁵ is attached in each of formula (Ir-a) to (Ir-f)). Further preferred compounds in this context are those in which in the compounds of formula (Ir-a) to (Ir-f) R⁶ is OH. Accordingly, in particulary preferred compounds of formula (Ir-a) to (Ir-f) R¹ is *-CH₂CH₂OH and R⁶ is OH. Further preferred compounds in this context are those in which in the compounds of formula (Ir-a) to (Ir-f) R⁶ is NH₂. Accordingly, in particulary preferred compounds of formula (Ir-a) to (Ir-f) R¹ is *-CH₂CH₂OH and R⁶ is NH₂.

Further preferred in this context are compounds of formula (Ir-a) to (Ir-f) in which R⁴ is selected from the group consisting of H, 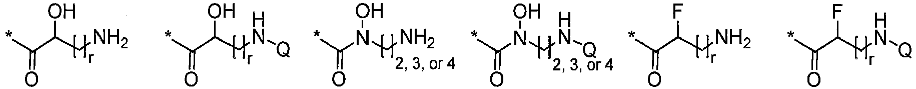 wherein in said R⁴ moieties r and Q are as defined for the compounds of formula (I). In particulary preferred compounds of formula (Ir-a) to (Ir-f) R⁴ is selected from the group consisting of H,

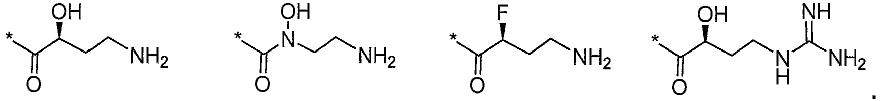

Accordingly, in particulary preferred compounds of formula (Ir-a) to (Ir-f) R¹ is H and R⁴ is selected from the group consisting of H, 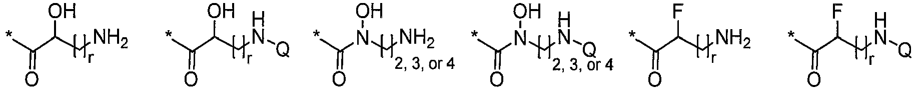 wherein in said R⁴ moieties r and Q are as defined for the compounds of formula (I), most preferably from the group consisting of H,

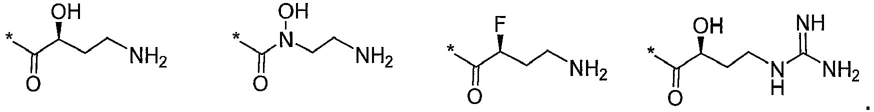

In this context, R⁶ may be OH. In this context, R⁶ may be NH₂.

Within the context of all of the foregoing relating to the compounds of formula (Ir-a) to (Ir-f), especially preferred are compounds of formula (Ir-c). Accordingly, even more especially preferred are compounds of formula (Ir-c) wherein R¹ is *-CH₂CH₂OH, in particular wherein R¹ is *-CH₂CH₂OH, R⁶ is OH and R⁴ is selected from the group consisting of H, 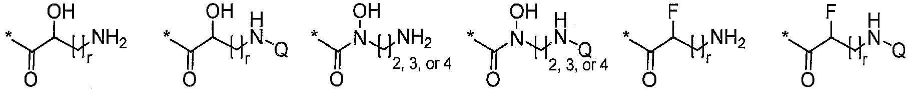 wherein in said R⁴ moieties r and Q are as defined for the compounds of formula (I), most preferably from the group consisting of H,

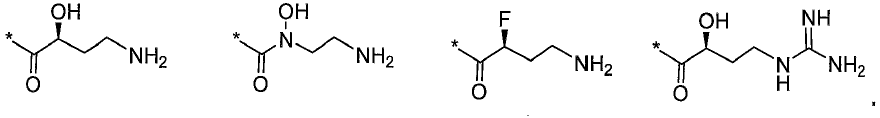

Accordingly, further even more especially preferred are compounds of formula (Ir-c) wherein R¹ is *-CH₂CH₂OH, in particular wherein R¹ is *-CH₂CH₂OH, R⁶ is NH₂ and R⁴ is selected from the group consisting of H, 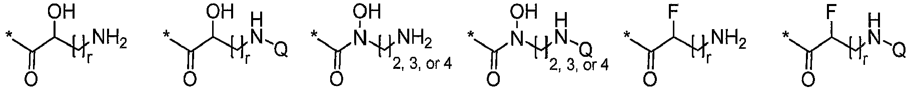 wherein in said R⁴ moieties r and Q are as defined for the compounds of formula (I), most preferably from the group consisting of H,

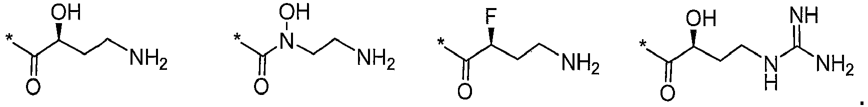

Other preferred compounds of the present invention are compounds of formula (Is) 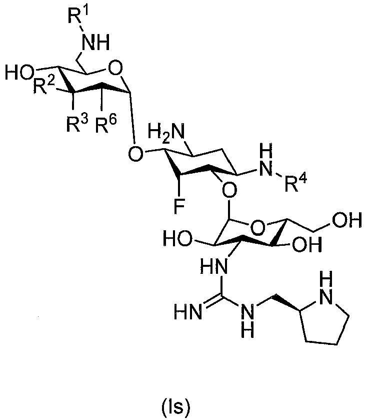 wherein R¹, R², R³, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect). Accordingly, particularly preferred compounds within this context include the compounds of formula (Is-a) to (Is-f) 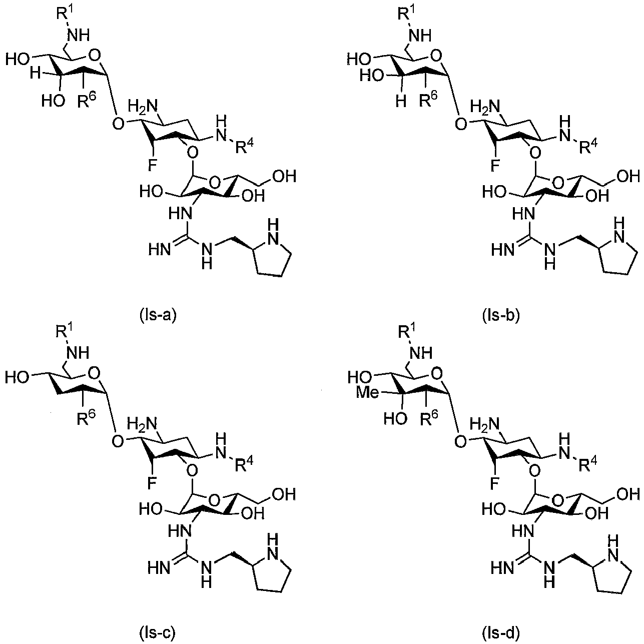 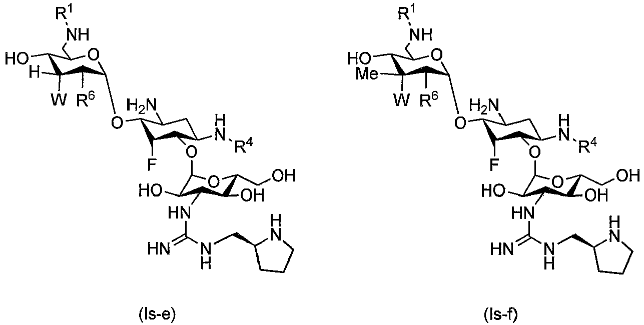 wherein R¹, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect).

Particulary preferred in this context are compounds of formula (Is-a) to (Is-f) in which R¹ is H. Further preferred compounds in this context are those in which in the compounds of formula (Is-a) to (Is-f) R⁶ is OH. Accordingly, in particulary preferred compounds of formula (Is-a) to (Is-f) R¹ is H and R⁶ is OH. Further preferred compounds in this context are those in which in the compounds of formula (Is-a) to (Is-f) R⁶ is NH₂. Accordingly, in particulary preferred compounds of formula (Is-a) to (Is-f) R¹ is H and R⁶ is NH₂.

Further preferred in this context are compounds of formula (Is-a) to (Is-f) in which R⁴ is selected from the group consisting of H, 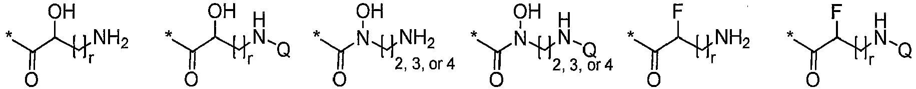 wherein in said R⁴ moieties r and Q are as defined for the compounds of formula (I). In particulary preferred compounds of formula (Is-a) to (Is-f) R⁴ is selected from the group consisting of H,

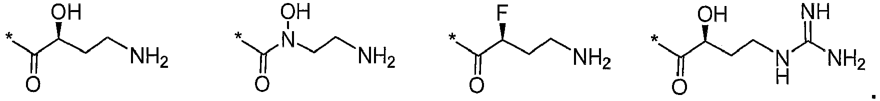

Accordingly, in particulary preferred compounds of formula (Is-a) to (Is-f) R¹ is H and R⁴ is selected from the group consisting of H, 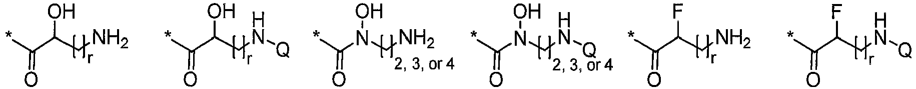 wherein in said R⁴ moieties r and Q are as defined for the compounds of formula (I), most preferably from the group consisting of H,

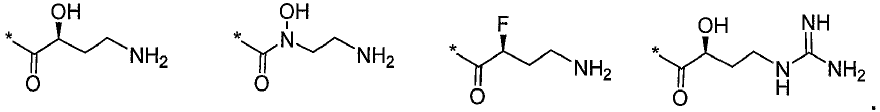

In this context, R⁶ may be OH. In this context, R⁶ may be NH₂.

Within the context of all of the foregoing relating to the compounds of formula (Is-a) to (Is-f), especially preferred are compounds of formula (Is-c).

Accordingly, even more especially preferred are compounds of formula (Is-c) wherein R¹ is H, in particular wherein R¹ is H, R⁶ is OH and R⁴ is selected from the group consisting of H, 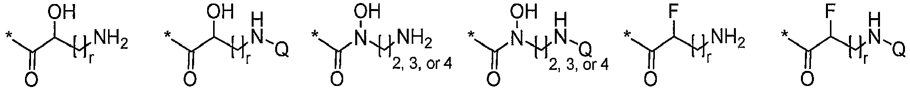 wherein in said R⁴ moieties r and Q are as defined for the compounds of formula (I), most preferably from the group consisting of H,

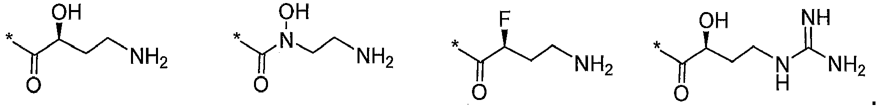

Accordingly, further even more especially preferred are compounds of formula (Is-c) wherein R¹ is H, in particular wherein R¹ is H, R⁶ is NH₂ and R⁴ is selected from the group consisting of H, 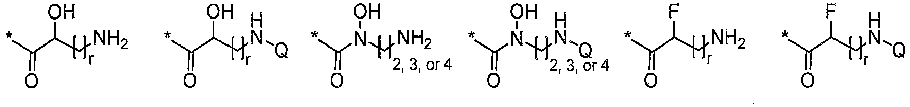 wherein in said R⁴ moieties r and Q are as defined for the compounds of formula (I), most preferably from the group consisting of H,

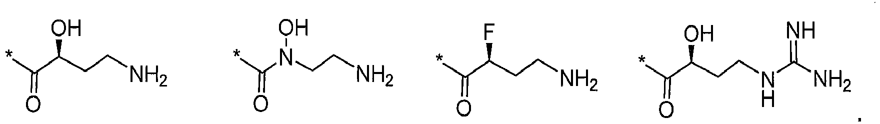

Further particulary preferred in the above context relating to compounds of formula (Is-a) to (Is-f) are compounds of formula (Is-a) to (Is-f) in which R¹ is *-CH₂CH₂OH (where * denotes the point of connection to the N atom to which R⁵ is attached in each of formula (Is-a) to (Is-f)). Further preferred compounds in this context are those in which in the compounds of formula (Is-a) to (Is-f) R⁶ is OH. Accordingly, in particulary preferred compounds of formula (Is-a) to (Is-f) R¹ is *-CH₂CH₂OH and R⁶ is OH. Further preferred compounds in this context are those in which in the compounds of formula (Is-a) to (Is-f) R⁶ is NH₂. Accordingly, in particulary preferred compounds of formula (Is-a) to (Is-f) R¹ is *-CH₂CH₂OH and R⁶ is NH₂.

Further preferred in this context are compounds of formula (Is-a) to (Is-f) in which R⁴ is selected from the group consisting of H, 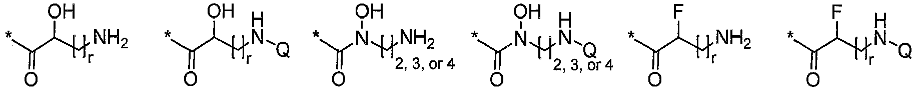 wherein in said R⁴ moieties r and Q are as defined for the compounds of formula (I). In particulary preferred compounds of formula (Is-a) to (Is-f) R⁴ is selected from the group consisting of H,

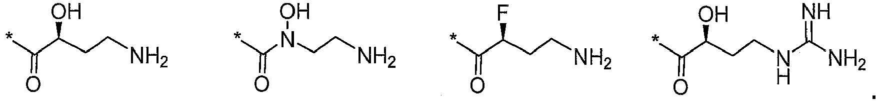

Accordingly, in particulary preferred compounds of formula (Is-a) to (Is-f) R¹ is H and R⁴ is selected from the group consisting of H, 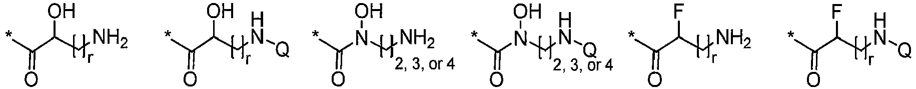 wherein in said R⁴ moieties r and Q are as defined for the compounds of formula (I), most preferably from the group consisting of H,

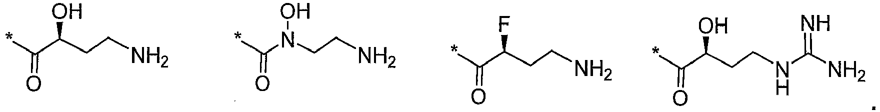

In this context, R⁶ may be OH. In this context, R⁶ may be NH₂.

Within the context of all of the foregoing relating to the compounds of formula (Is-a) to (Is-f), especially preferred are compounds of formula (Is-c). Accordingly, even more especially preferred are compounds of formula (Is-c) wherein R¹ is *-CH₂CH₂OH, in particular wherein R¹ is *-CH₂CH₂OH, R⁶ is OH and R⁴ is selected from the group consisting of H, 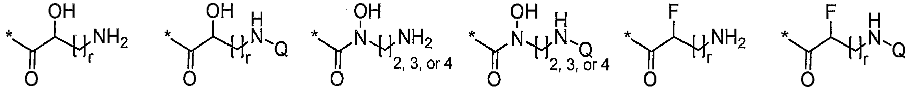 wherein in said R⁴ moieties r and Q are as defined for the compounds of formula (I), most preferably from the group consisting of H,

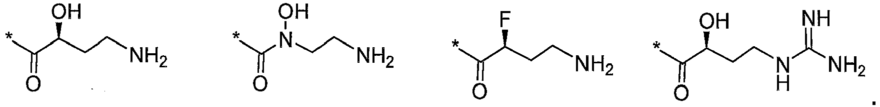

Accordingly, further even more especially preferred are compounds of formula (Is-c) wherein R¹ is *-CH₂CH₂OH, in particular wherein R¹ is *-CH₂CH₂OH, R⁶ is NH₂ and R⁴ is selected from the group consisting of H, 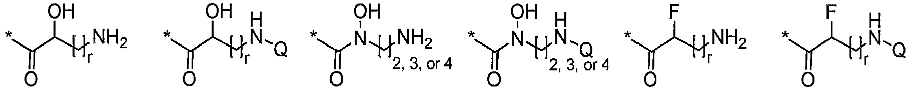 wherein in said R⁴ moieties r and Q are as defined for the compounds of formula (I), most preferably from the group consisting of H,

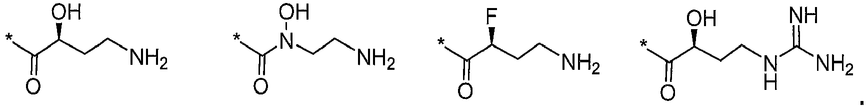

Other preferred compounds of the present invention are compounds of formula (It) 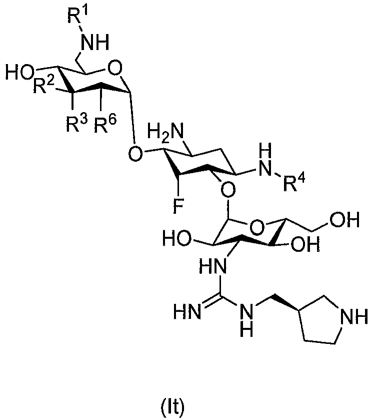 wherein R¹, R², R³, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect). Accordingly, particularly preferred compounds within this context include the compounds of formula (It-a) to (It-f) 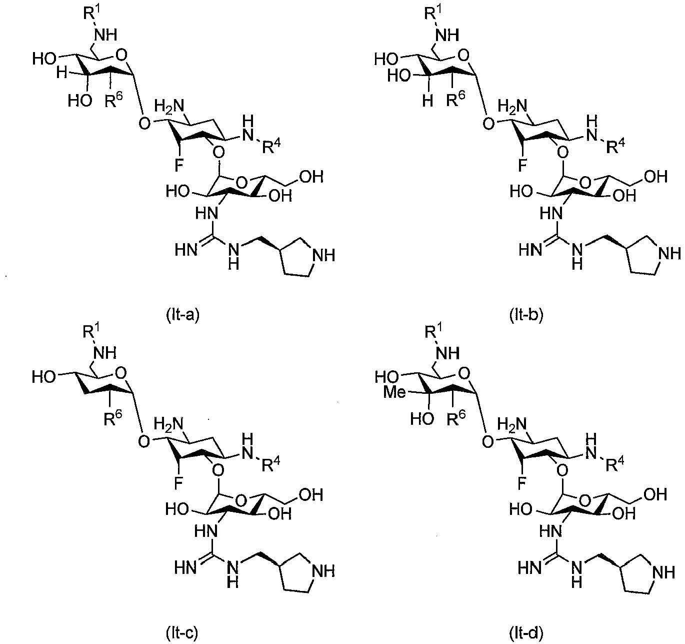 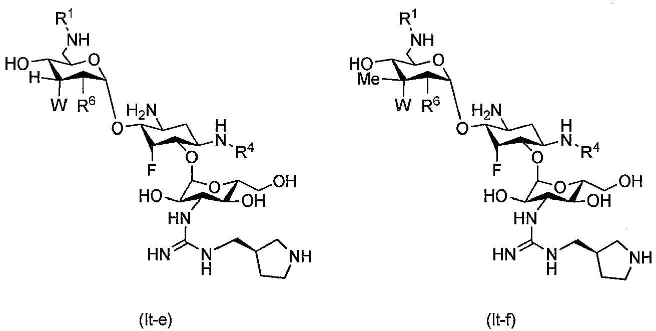 wherein R¹, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect).

Particulary preferred in this context are compounds of formula (It-a) to (It-f) in which R¹ is H. Further preferred compounds in this context are those in which in the compounds of formula (It-a) to (It-f) R⁶ is OH. Accordingly, in particulary preferred compounds of formula (It-a) to (It-f) R¹ is H and R⁶ is OH. Further preferred compounds in this context are those in which in the compounds of formula (It-a) to (It-f) R⁶ is NH₂. Accordingly, in particulary preferred compounds of formula (It-a) to (It-f) R¹ is H and R⁶ is NH₂.

Further preferred in this context are compounds of formula (It-a) to (It-f) in which R⁴ is selected from the group consisting of H, 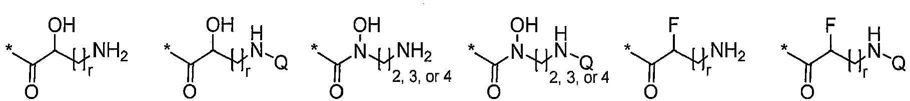 wherein in said R⁴ moieties r and Q are as defined for the compounds of formula (I). In particulary preferred compounds of formula (It-a) to (It-f) R⁴ is selected from the group consisting of H,

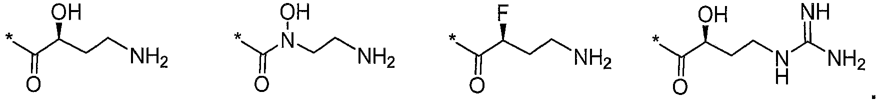

Accordingly, in particulary preferred compounds of formula (It-a) to (It-f) R¹ is H and R⁴ is selected from the group consisting of H, 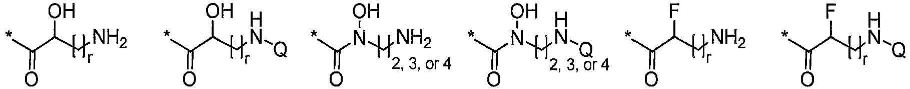 wherein in said R⁴ moieties r and Q are as defined for the compounds of formula (I), most preferably from the group consisting of H,

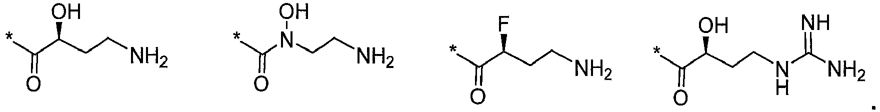

In this context, R⁶ may be OH. In this context, R⁶ may be NH₂.

Within the context of all of the foregoing relating to the compounds of formula (It-a) to (It-f), especially preferred are compounds of formula (It-c). Accordingly, even more especially preferred are compounds of formula (It-c) wherein R¹ is H, in particular wherein R¹ is H, R⁶ is OH and R⁴ is selected from the group consisting of H, 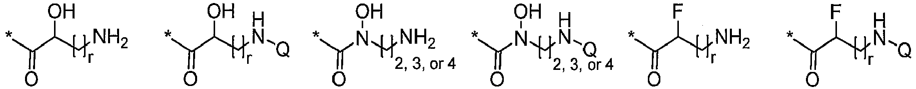 wherein in said R⁴ moieties r and Q are as defined for the compounds of formula (I), most preferably from the group consisting of H,

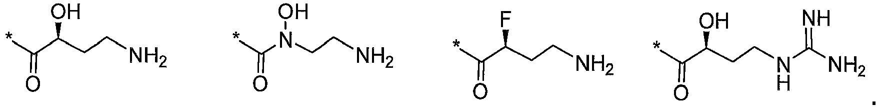

Accordingly, further even more especially preferred are compounds of formula (It-c) wherein R¹ is H, in particular wherein R¹ is H, R⁶ is NH₂ and R⁴ is selected from the group consisting of H, 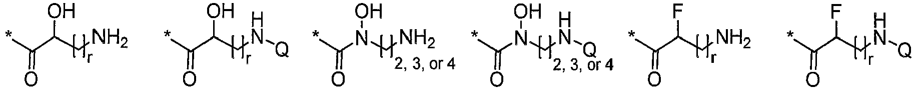 wherein in said R⁴ moieties r and Q are as defined for the compounds of formula (I), most preferably from the group consisting of H,

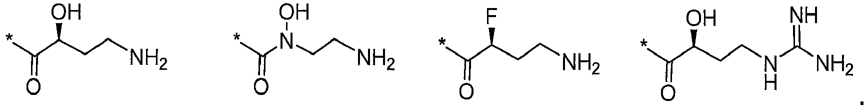

Further particulary preferred in the above context relating to compounds of formula (It-a) to (It-f) are compounds of formula (It-a) to (It-f) in which R¹ is *-CH₂CH₂OH (where * denotes the point of connection to the N atom to which R⁵ is attached in each of formula (It-a) to (It-f)). Further preferred compounds in this context are those in which in the compounds of formula (It-a) to (It-f) R⁶ is OH. Accordingly, in particulary preferred compounds of formula (It-a) to (It-f) R¹ is *-CH₂CH₂OH and R⁶ is OH. Further preferred compounds in this context are those in which in the compounds of formula (It-a) to (It-f) R⁶ is NH₂. Accordingly, in particulary preferred compounds of formula (It-a) to (It-f) R¹ is *-CH₂CH₂OH and R⁶ is NH₂.

Further preferred in this context are compounds of formula (It-a) to (It-f) in which R⁴ is selected from the group consisting of H, 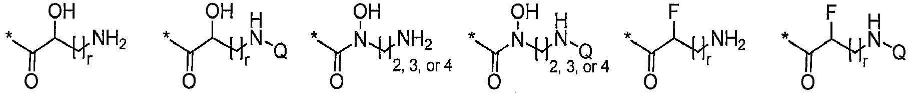 wherein in said R⁴ moieties r and Q are as defined for the compounds of formula (I). In particulary preferred compounds of formula (It-a) to (It-f) R⁴ is selected from the group consisting of H,

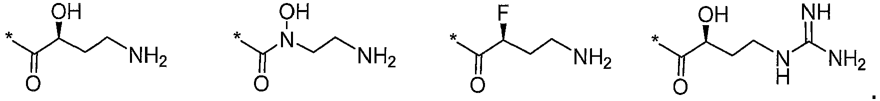

Accordingly, in particulary preferred compounds of formula (It-a) to (It-f) R¹ is *-CH₂CH₂OH and R⁴ is selected from the group consisting of H, 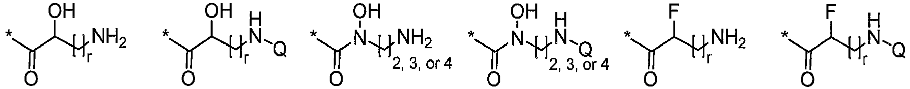 wherein in said R⁴ moieties r and Q are as defined for the compounds of formula (I), most preferably from the group consisting of H,

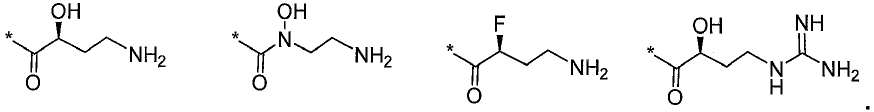

In this context, R⁶ may be OH. In this context, R⁶ may be NH₂.

Within the context of all of the foregoing relating to the compounds of formula (It-a) to (It-f), especially preferred are compounds of formula (It-c). Accordingly, even more especially preferred are compounds of formula (It-c) wherein R¹ is *-CH₂CH₂OH, in particular wherein R¹ is *-CH₂CH₂OH, R⁶ is OH and R⁴ is selected from the group consisting of H, 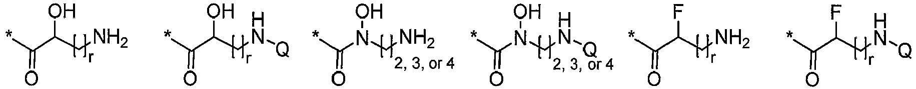 wherein in said R⁴ moieties r and Q are as defined for the compounds of formula (I), most preferably from the group consisting of H,

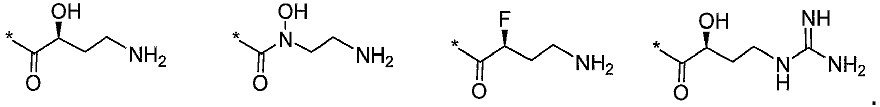

Accordingly, further even more especially preferred are compounds of formula (It-c) wherein R¹ is *-CH₂CH₂OH, in particular wherein R¹ is *-CH₂CH₂OH, R⁶ is NH₂ and R⁴ is selected from the group consisting of H, 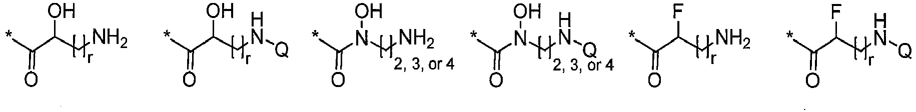 wherein in said R⁴ moieties r and Q are as defined for the compounds of formula (I), most preferably from the group consisting of H,

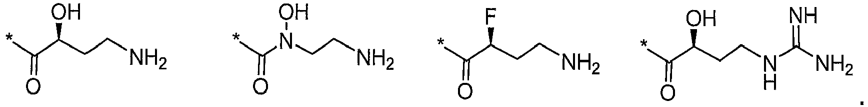

Other preferred compounds of the present invention are compounds of formula (Iu) 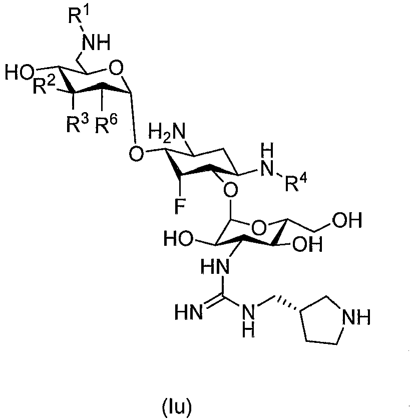 wherein R¹, R², R³, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect). Accordingly, particularly preferred compounds within this context include the compounds of formula (Iu-a) to (Iu-f) 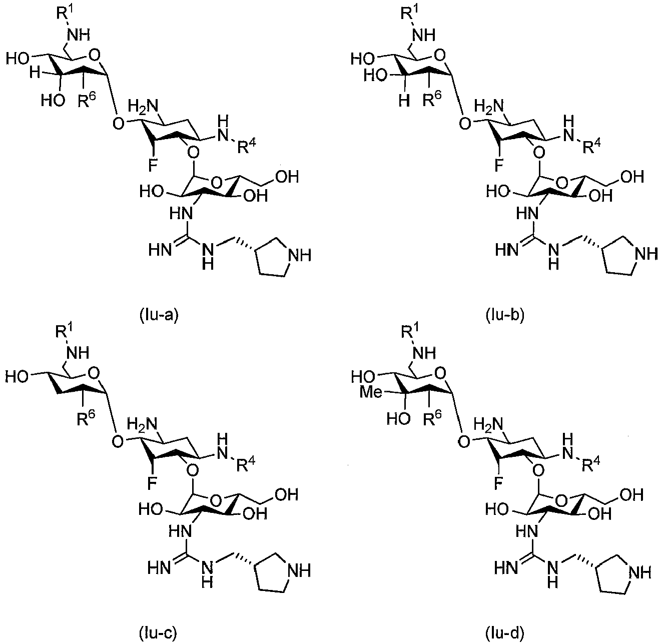 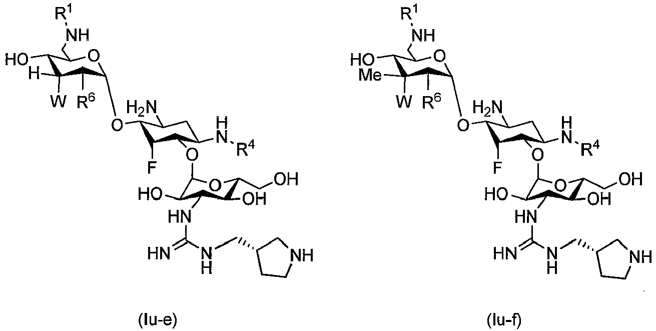 wherein R¹, R⁴, and R⁶ are as defined herein above for compounds of formula (I) (said definitions being either the broadest definition disclosed hereinabove for said groups or any of the narrower definitions disclosed for said groups including those stipulated as being "preferred", "more preferred", "most preferred" etc. or words to that effect).

Particulary preferred in this context are compounds of formula (Iu-a) to (Iu-f) in which R¹ is H. Further preferred compounds in this context are those in which in the compounds of formula (Iu-a) to (Iu-f) R⁶ is OH. Accordingly, in particulary preferred compounds of formula (Iu-a) to (Iu-f) R¹ is H and R⁶ is OH. Further preferred compounds in this context are those in which in the compounds of formula (Iu-a) to (Iu-f) R⁶ is NH₂. Accordingly, in particulary preferred compounds of formula (Iu-a) to (Iu-f) R¹ is H and R⁶ is NH₂.

Further preferred in this context are compounds of formula (Iu-a) to (Iu-f) in which R⁴ is selected from the group consisting of H, 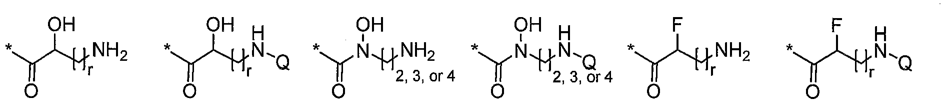 wherein in said R⁴ moieties r and Q are as defined for the compounds of formula (I). In particulary preferred compounds of formula (Iu-a) to (Iu-f) R⁴ is selected from the group consisting of H,

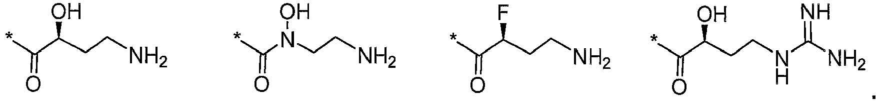

Accordingly, in particulary preferred compounds of formula (Iu-a) to (Iu-f) R¹ is H and R⁴ is selected from the group consisting of H, 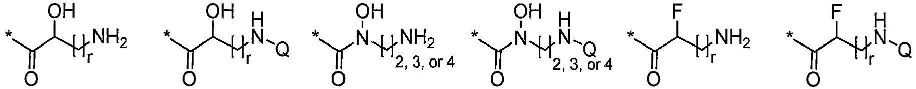 wherein in said R⁴ moieties r and Q are as defined for the compounds of formula (I), most preferably from the group consisting of H,

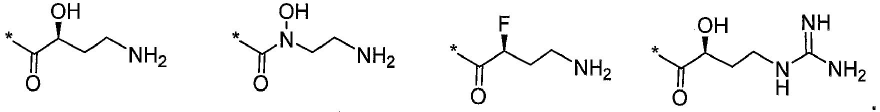

In this context, R⁶ may be OH. In this context, R⁶ may be NH₂.

Within the context of all of the foregoing relating to the compounds of formula (Iu-a) to (Iu-f), especially preferred are compounds of formula (Iu-c).

Accordingly, even more especially preferred are compounds of formula (Iu-c) wherein R¹ is H, in particular wherein R¹ is H, R⁶ is OH and R⁴ is selected from the group consisting of H, 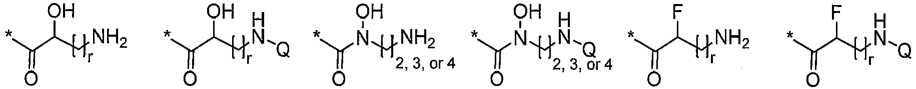 wherein in said R⁴ moieties r and Q are as defined for the compounds of formula (I), most preferably from the group consisting of H,

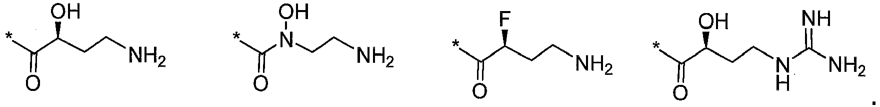

Accordingly, further even more especially preferred are compounds of formula (Iu-c) wherein R¹ is H, in particular wherein R¹ is H, R⁶ is NH₂ and R⁴ is selected from the group consisting of H, 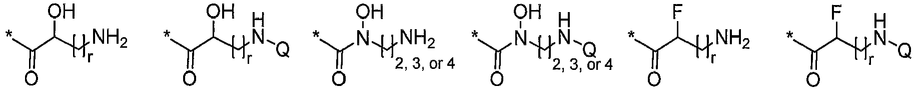 wherein in said R⁴ moieties r and Q are as defined for the compounds of formula (I), most preferably from the group consisting of H,

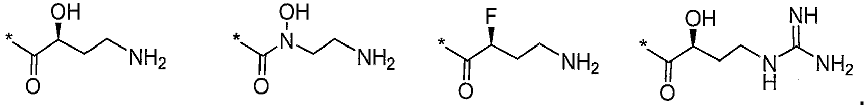

Further particulary preferred in the above context relating to compounds of formula (Iu-a) to (Iu-f) are compounds of formula (Iu-a) to (Iu-f) in which R¹ is *-CH₂CH₂OH (where * denotes the point of connection to the N atom to which R⁵ is attached in each of formula (Iu-a) to (Iu-f)). Further preferred compounds in this context are those in which in the compounds of formula (Iu-a) to (Iu-f) R⁶ is OH. Accordingly, in particulary preferred compounds of formula (Iu-a) to (Iu-f) R¹ is *-CH₂CH₂OH and R⁶ is OH. Further preferred compounds in this context are those in which in the compounds of formula (Iu-a) to (Iu-f) R⁶ is NH₂. Accordingly, in particulary preferred compounds of formula (Iu-a) to (Iu-f) R¹ is *-CH₂CH₂OH and R⁶ is NH₂.

Further preferred in this context are compounds of formula (Iu-a) to (Iu-f) in which R⁴ is selected from the group consisting of H, 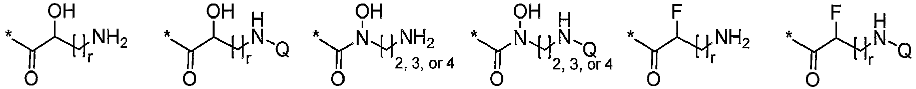 wherein in said R⁴ moieties r and Q are as defined for the compounds of formula (I). In particulary preferred compounds of formula (Iu-a) to (Iu-f) R⁴ is selected from the group consisting of H,

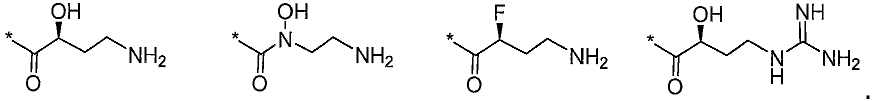

Accordingly, in particulary preferred compounds of formula (Iu-a) to (Iu-f) R¹ is *-CH₂CH₂OH and R⁴ is selected from the group consisting of H, 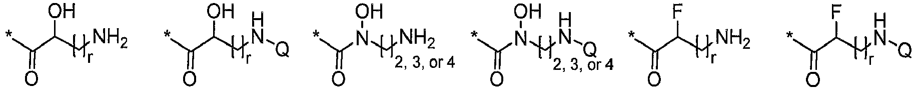 wherein in said R⁴ moieties r and Q are as defined for the compounds of formula (I), most preferably from the group consisting of H,

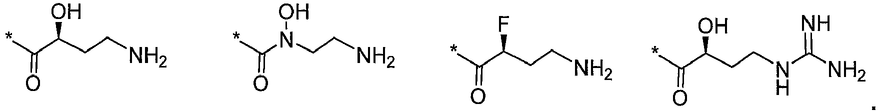

In this context, R⁶ may be OH. In this context, R⁶ may be NH₂. Within the context of all of the foregoing relating to the compounds of formula (Iu-a) to (Iu-f), especially preferred are compounds of formula (Iu-c). Accordingly, even more especially preferred are compounds of formula (Iu-c) wherein R¹ is *-CH₂CH₂OH, in particular wherein R¹ is *-CH₂CH₂OH, R⁶ is OH and R⁴ is selected from the group consisting of H, 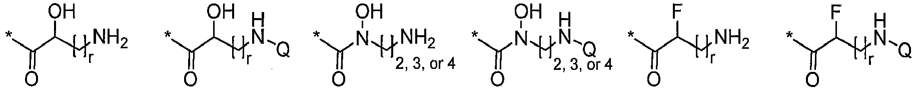 wherein in said R⁴ moieties r and Q are as defined for the compounds of formula (I), most preferably from the group consisting of H,

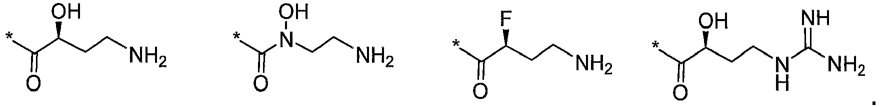

Accordingly, further even more especially preferred are compounds of formula (Iu-c) wherein R¹ is *-CH₂CH₂OH, in particular wherein R¹ is *-CH₂CH₂OH, R⁶ is NH₂ and R⁴ is selected from the group consisting of H, 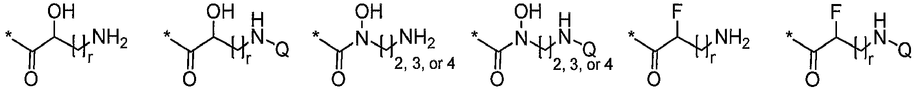 wherein in said R⁴ moieties r and Q are as defined for the compounds of formula (I), most preferably from the group consisting of H,

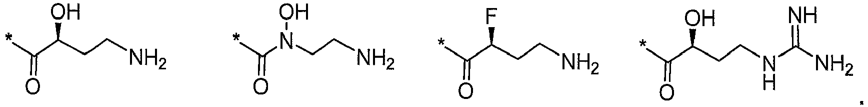

The compounds of the present invention may be selected from the group consisting of:

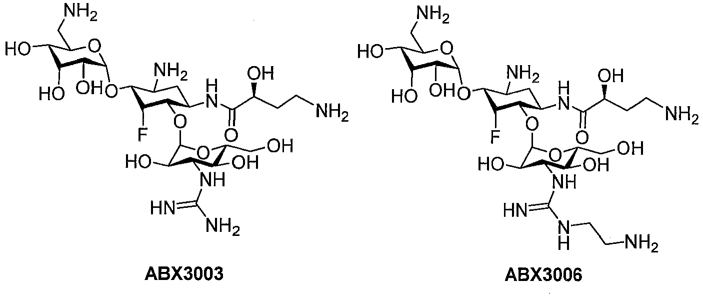

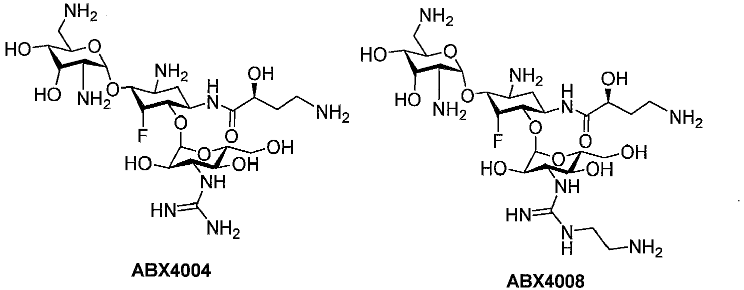

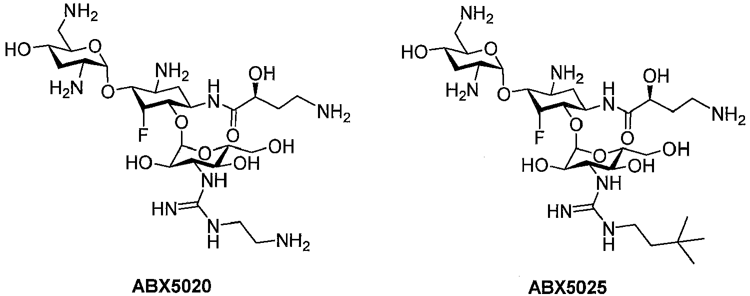

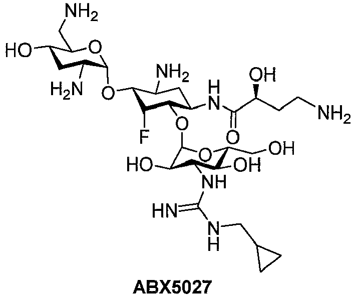

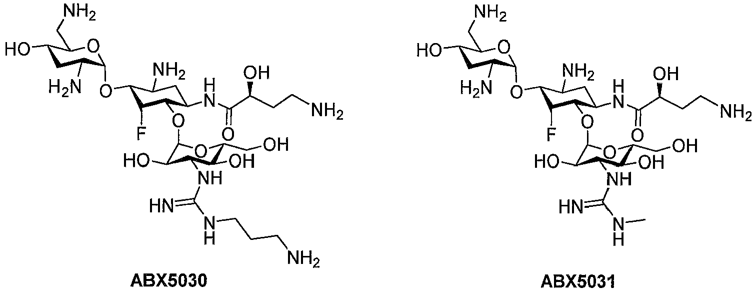

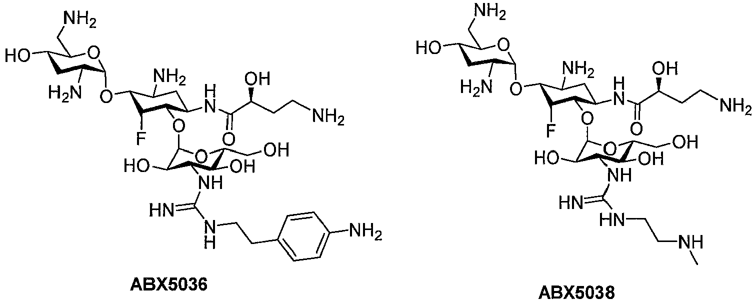

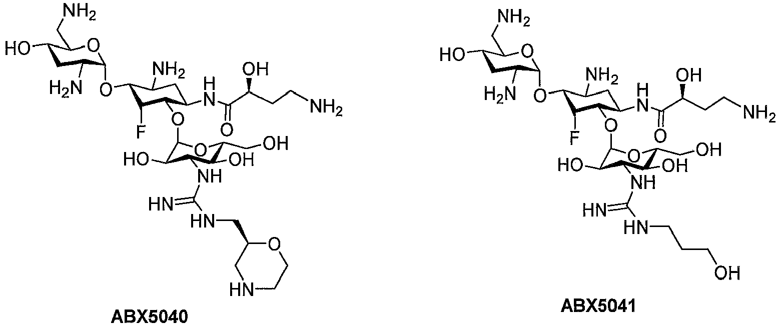

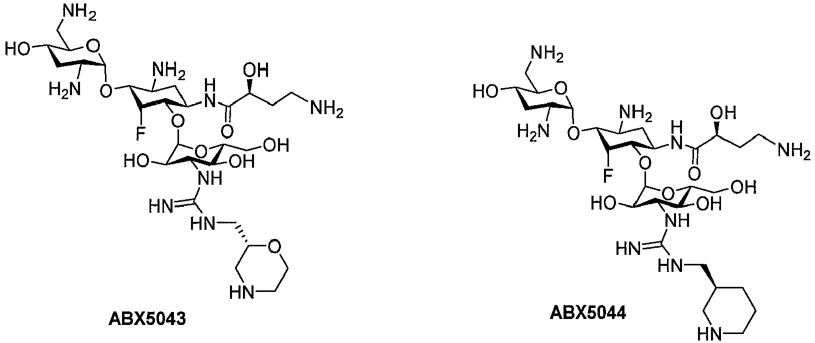

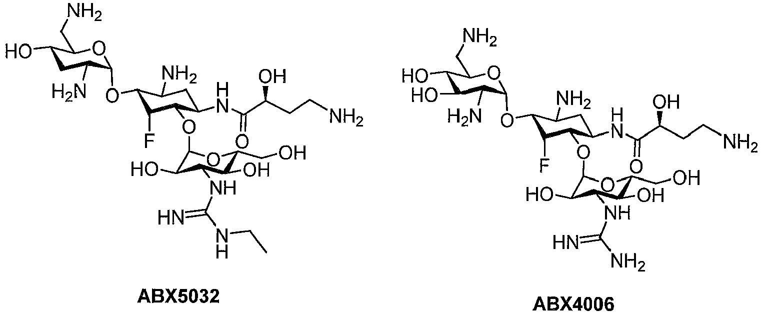

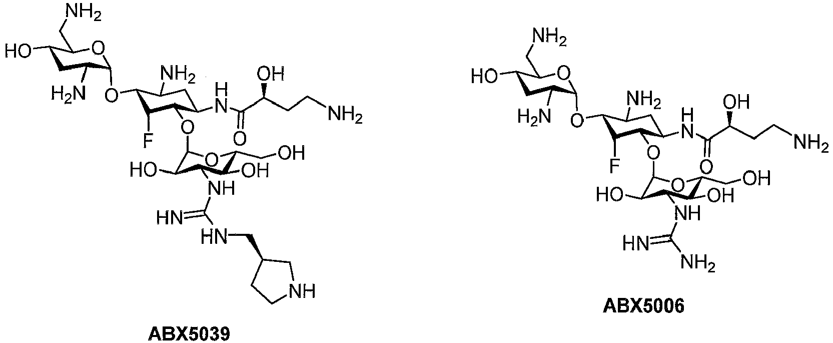

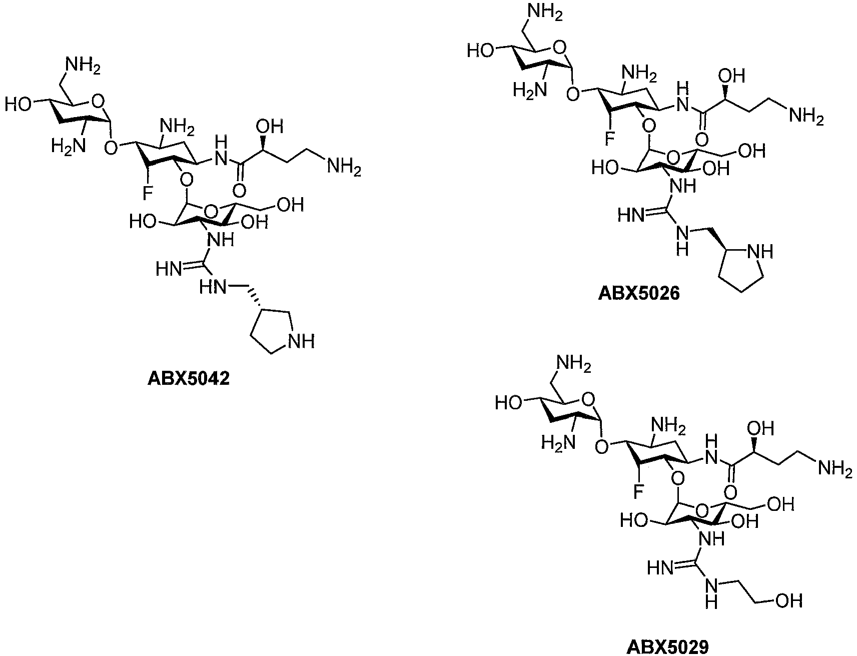

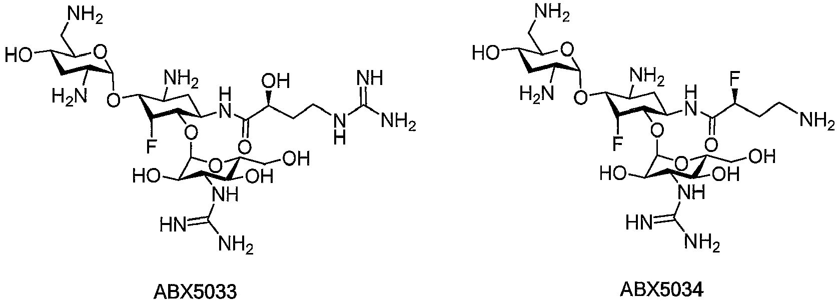

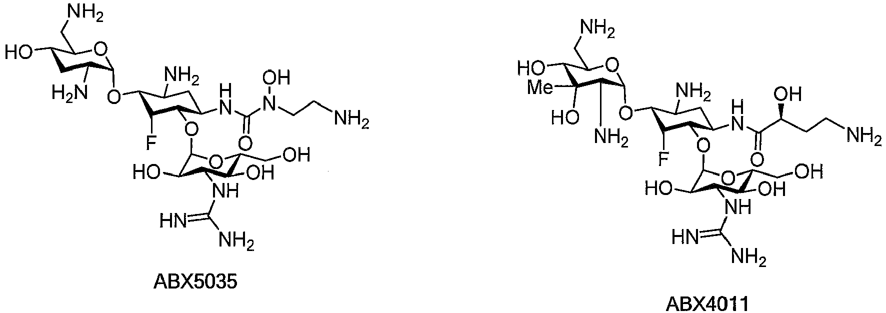

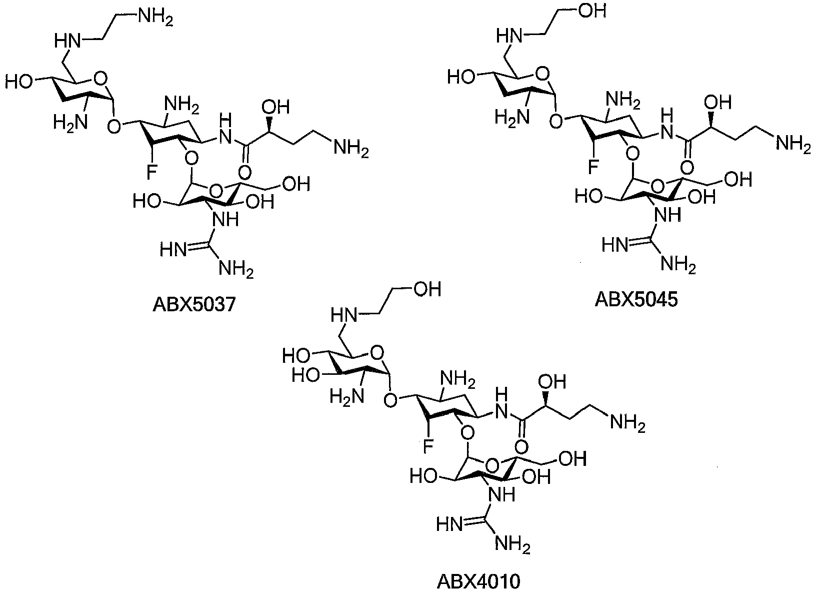

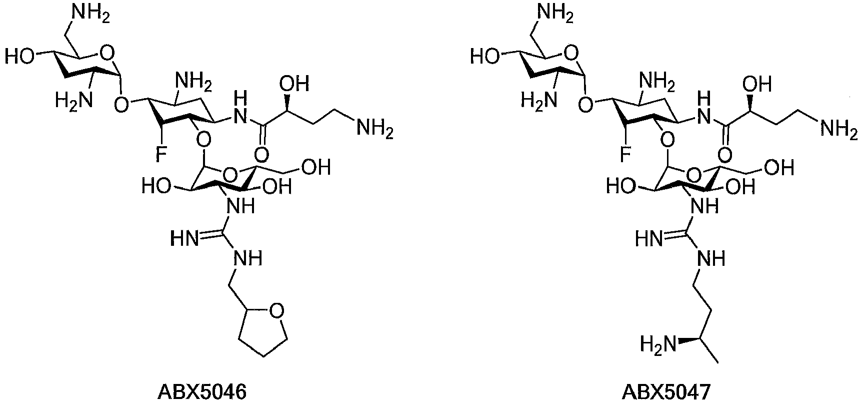

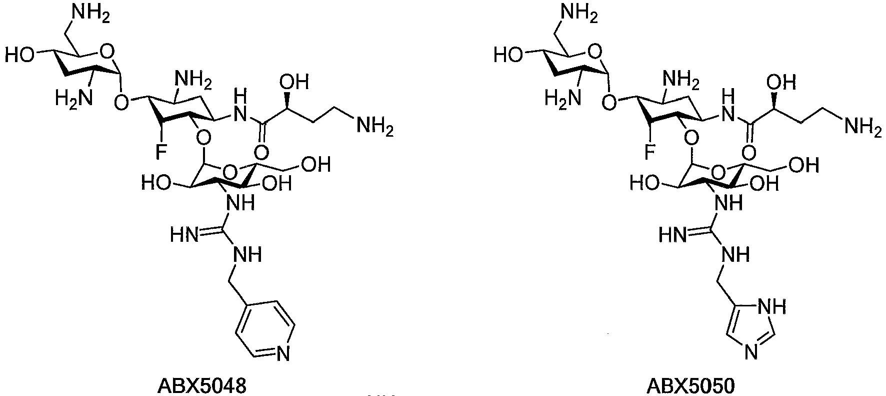

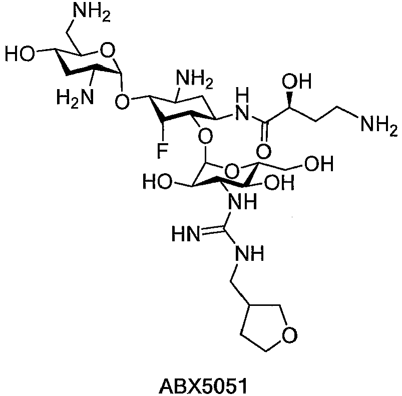

In the compounds of the present invention, where a moiety or group may be or contains (or reference is made herein to) optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; optionally substituted means, unless otherwise stipulated, optionally substituted with any substituent. Preferably, optionally substituted means optionally substituted with one or more substituents independently selected from the group consisting of methyl, -CH₂F, -CF₃, =O, =NH, =S, ethyl, straight chain or branched C₃₋₈alkyl, halogen, -OH, -OCH₃, -OCH₂F, -OCF₃, -OC₂₋₆alkyl, -NH₂, -NHQ, -NHR¹⁶, -NR¹⁷R¹⁸, -CO₂H, -CO₂CH₃, -CO₂C₂₋₆alkyl, -OCOCH₃, -OCOC₂₋₆alkyl, -CN, -CONHR¹⁹, -CONR²⁰R²¹, -NHCOCH₃, -NHCOC₂₋₆alkyl, -NR²²COCH₃, -NR²³COC₂₋₆alkyl, -C(=NH)R²⁴, -C(=NR²⁵)R²⁶, -C(=N-OH)R²⁷, -C(=N-OR²⁸)R²⁹, -C(=NH)NH₂, -C(=NR³⁰)NH₂, - C(=NH)NHR³¹, -C(=NH)NR³²R³³, -C(=NR³⁴)NR³⁵R³⁶, and -C(=NR³⁷)NHR³⁸, wherein each of R¹⁶ to R³⁸ is independently selected from the group consisting of H, methyl, ethyl, C₃₋₆alkyl, and C₃₋₆cycloalkyl; with the proviso that when one of said substituents is joined directly to a N-atom in one of the listed non-aromatic heterocycloalkyl moieties it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸. Furthermore, phenyl, 5-membered heteroaryl, and substituted 6-membered heteoraryl cannot be substituted with =O, =NH, =S, or any other substituent which requires that a double bond be formed to an atom of said phenyl, 5-membered heteroaryl, or substituted 6-membered heteoraryl ring where this would result in said ring no longer being aromatic.

The meaning of the wording "with the proviso that when one of said substituents is joined directly to a N-atom in one of the respective non-aromatic heterocycloalkyl moieties it can only be selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸" will now be explained using R³ = -OCH₂(4-piperidine) as an example. The piperidine ring is optionally substituted and, in this context, the N atom of the piperidine ring can only be substituted with a substituent selected from the group consisting of methyl, -CH₂F, -CF₃, ethyl, straight chain or branched C₃₋₈alkyl, -CO₂CH₃, -CO₂C₂₋₆alkyl, -CN, -CONHR¹⁹, -CONR²⁰R²¹, -C(=NH)R²⁴, -C(=N-OH)R²⁷, -C(=N-OR²⁸)R²⁹, -C(=NH)NH₂, -C(=NR³⁰)NH₂, -C(=NH)NHR³¹, -C(=NH)NR³²R³³, -C(=NR³⁴)NR³⁵R³⁶, and -C(=NR³⁷)NHR³⁸. As such, no heteroatom-heteroatom bonds result as a consequence of substitution on the N atom of the piperidine. In contrast, this proviso does not apply to the carbon atoms of said piperidine ring which may be substituted with any of the substituents listed.

Where chemical bonds between atoms of which at least one represents a stereocentre are depicted in the present disclosure using "straight line" bonds (as opposed to the stereochemical "wedge" (defined single stereochemistry) or "squiggly" (racemate) bonds used as standard in the art to depict single isomers or racemates respectively), this is to be understood within the context of the present application to be a direct and unambiguous disclosure of all stereoisomers falling thereunder, such a depiction also being standard in the art to represent all possible isomers and being employed in the present application merely for the purposes of imparting maximum conciseness on the disclosure.
Accordingly, 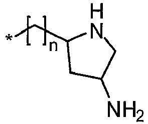 (an example of a moiety having two stereocentres from which "straight line" bonds connect the atom at the stereocentre to the adjacent atom) is to be understood to directly and unambiguously disclose each of 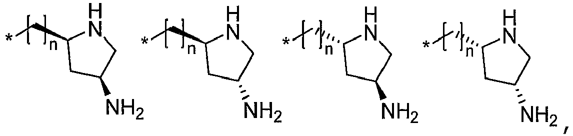 i.e.

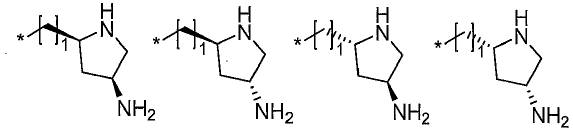

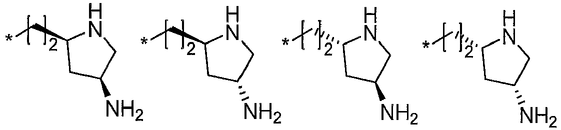

Accordingly, 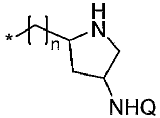 is to be understood to directly and unambiguously disclose each of

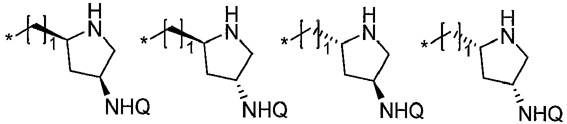

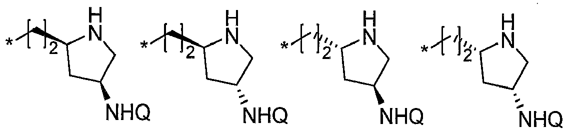

Accordingly, 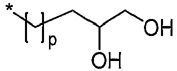 is to be understood to directly and unambiguously disclose each of

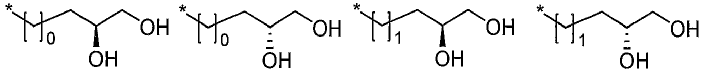

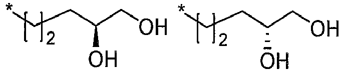

Accordingly, 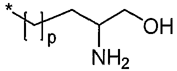 is to be understood to directly and unambiguously disclose each of

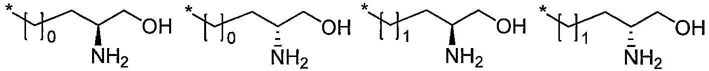

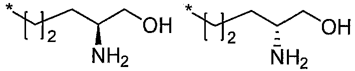

Accordingly, 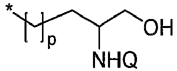 is to be understood to directly and unambiguously disclose each of

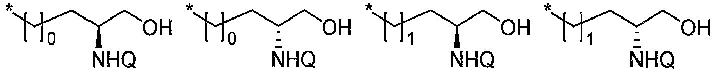

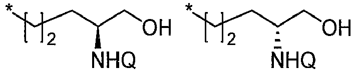

Accordingly, 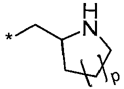 is to be understood to directly and unambiguously disclose each of

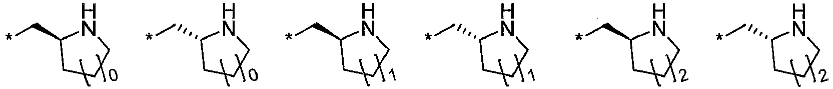

Accordingly, 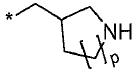 is to be understood to directly and unambiguously disclose each of

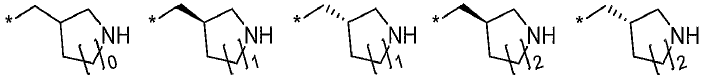

Accordingly, 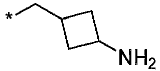 is to be understood to directly and unambiguously disclose each of

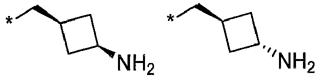

Accordingly, 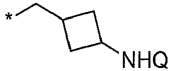 is to be understood to directly and unambiguously disclose each of

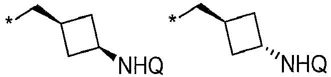

Accordingly, 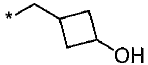 is to be understood to directly and unambiguously disclose each of

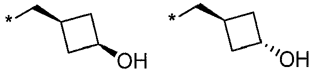

Accordingly, 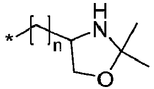 is to be understood to directly and unambiguously disclose each of

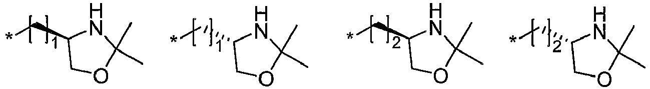

Accordingly, 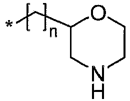 is to be understood to directly and unambiguously disclose each of

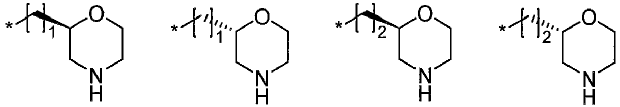

Accordingly, 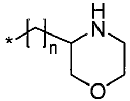 is to be understood to directly and unambiguously disclose each of

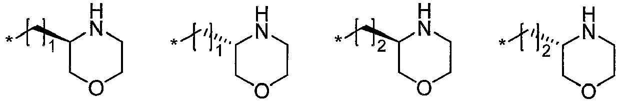

Accordingly, 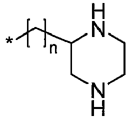 is to be understood to directly and unambiguously disclose each of

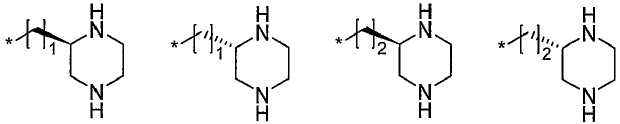

Accordingly, 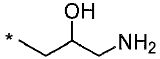 is to be understood to directly and unambiguously disclose each of

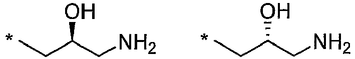

Accordingly, 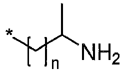 is to be understood to directly and unambiguously disclose each of

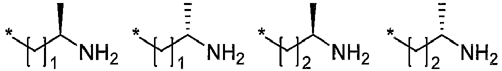

Accordingly, 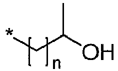 is to be understood to directly and unambiguously disclose each of

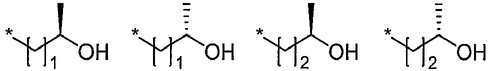

Accordingly, 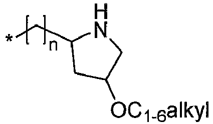 is to be understood to directly and unambiguously disclose each of

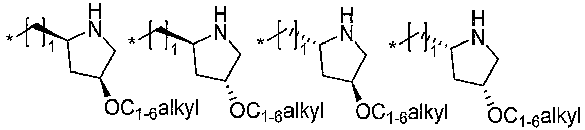

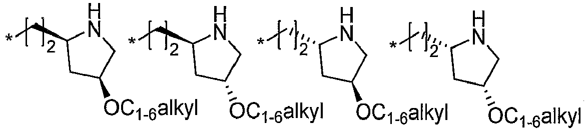

Accordingly, 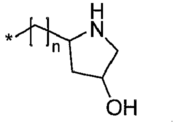 is to be understood to directly and unambiguously disclose each of

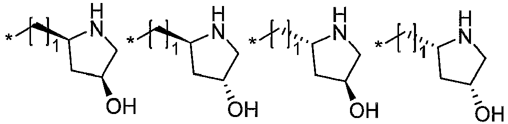

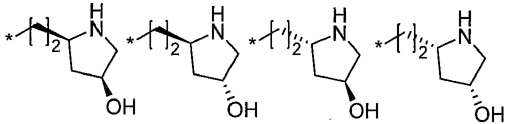

Accordingly, 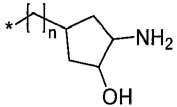 is to be understood to directly and unambiguously disclose each of

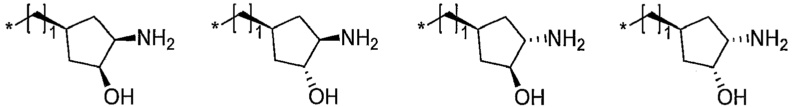

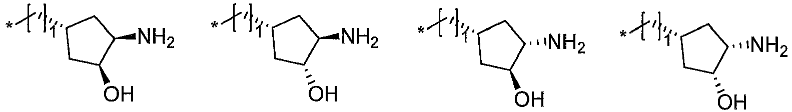

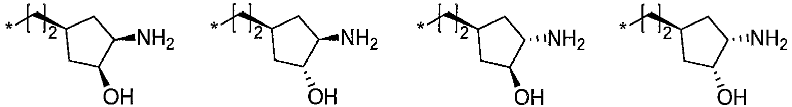

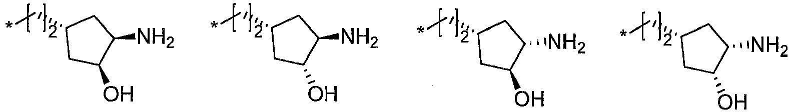

Accordingly, 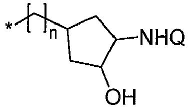 is to be understood to directly and unambiguously disclose each of

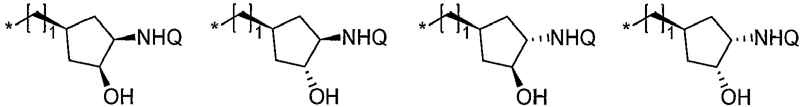

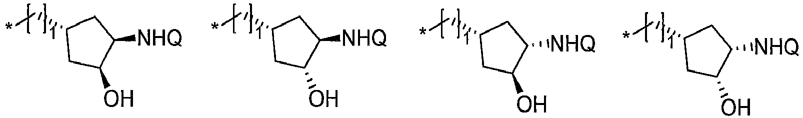

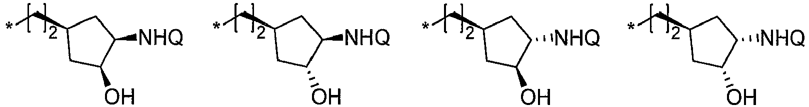

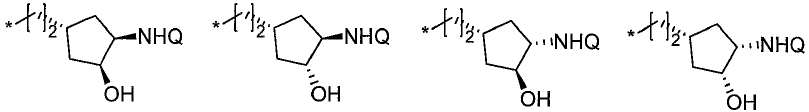

Accordingly, 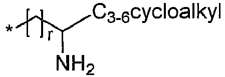 is to be understood to directly and unambiguously disclose each of

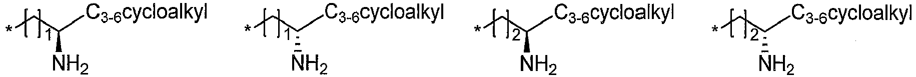

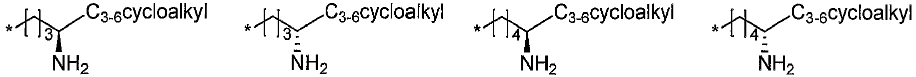

Accordingly, 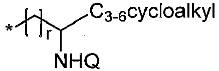 is to be understood to directly and unambiguously disclose each of

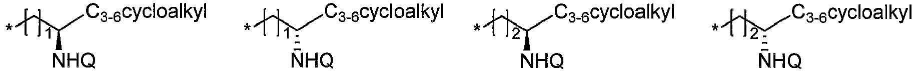

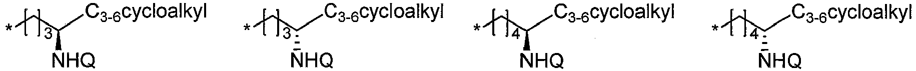

Accordingly, 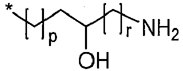 is to be understood to directly and unambiguously disclose each of

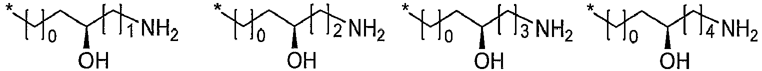

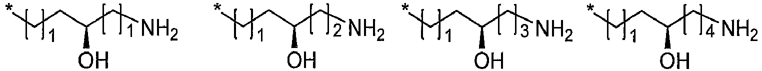

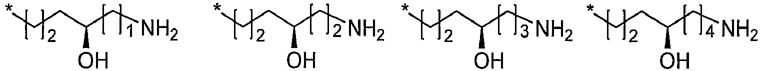

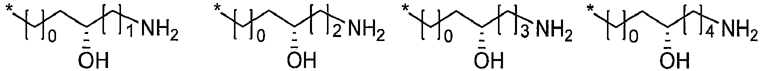

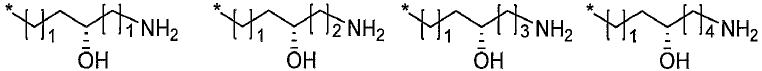

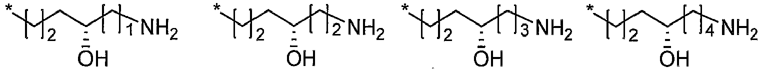

Accordingly, 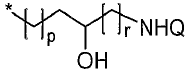 is to be understood to directly and unambiguously disclose each of

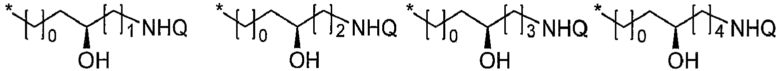

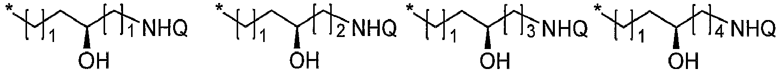

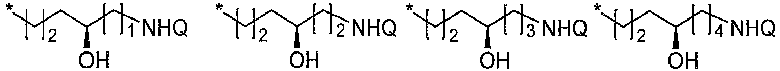

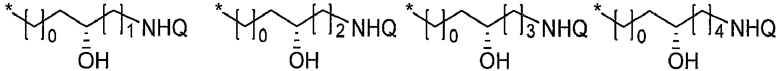

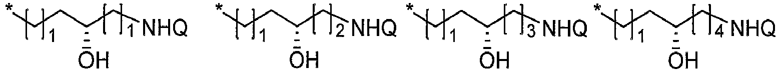

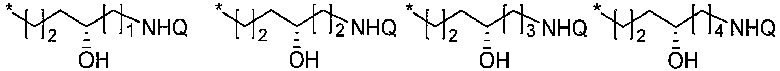

Accordingly, 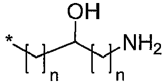 is to be understood to directly and unambiguously disclose each of

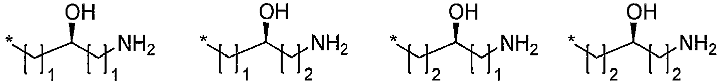

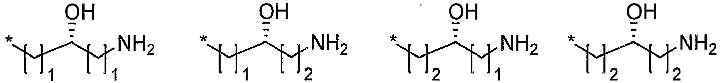

Accordingly, 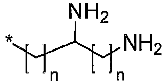 is to be understood to directly and unambiguously disclose each of

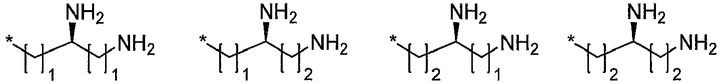

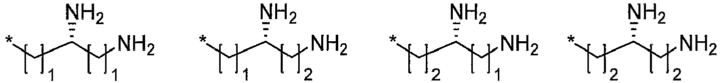

Accordingly, 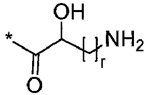 is to be understood to directly and unambiguously disclose each of

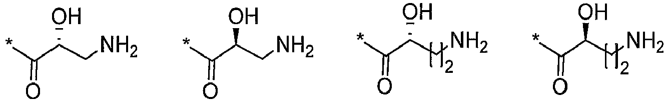

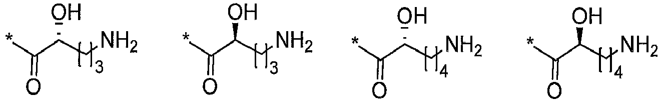

Accordingly, 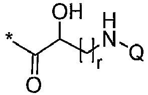 is to be understood to directly and unambiguously disclose each of

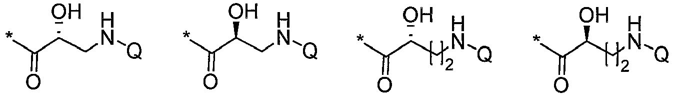

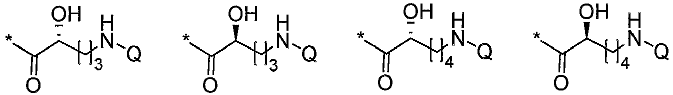

Accordingly, 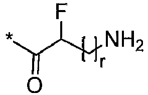 is to be understood to directly and unambiguously disclose each of

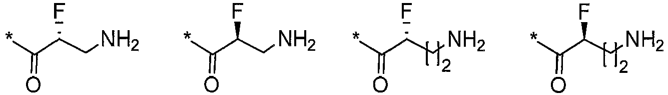

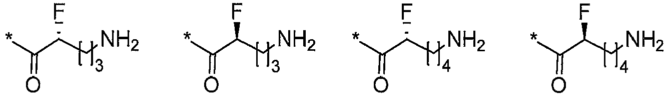

Accordingly, 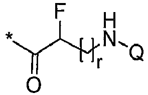 is to be understood to directly and unambiguously disclose each of

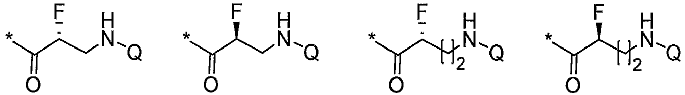

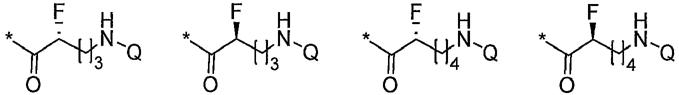

Accordingly, 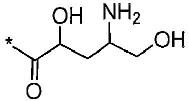 is to be understood to directly and unambiguously disclose each of

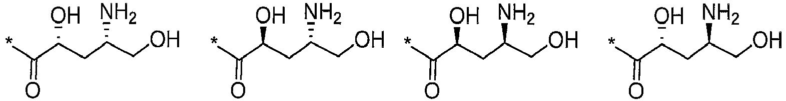

Accordingly, 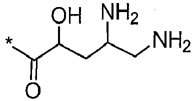 is to be understood to directly and unambiguously disclose each of

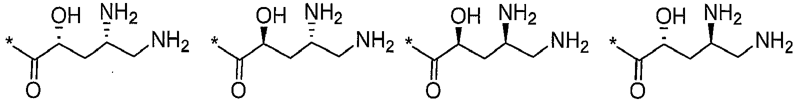

Accordingly, 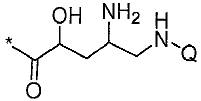 is to be understood to directly and unambiguously disclose each of

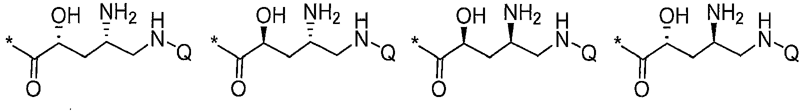

Accordingly, 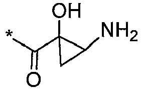 is to be understood to directly and unambiguously disclose each of

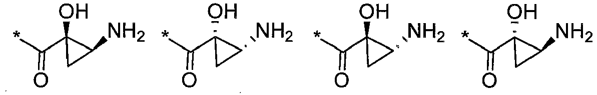

Accordingly, 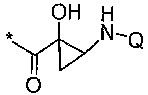 is to be understood to directly and unambiguously disclose each of

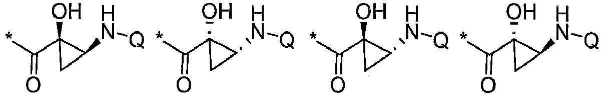

Accordingly, 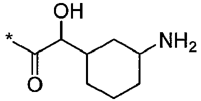 is to be understood to directly and unambiguously disclose each of

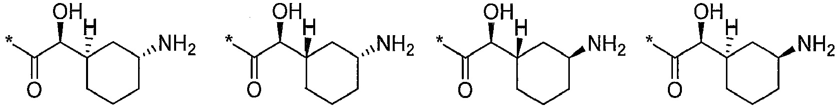

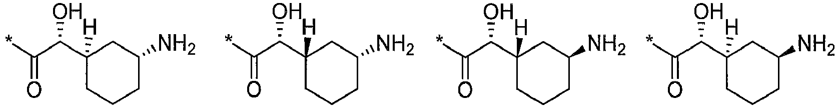

Accordingly, 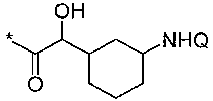 is to be understood to directly and unambiguously disclose each of

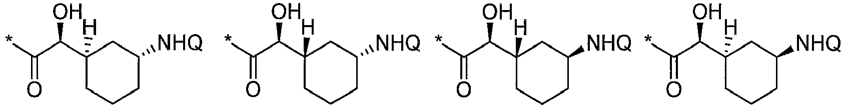

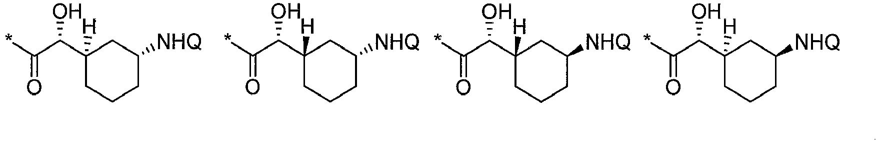

Accordingly, 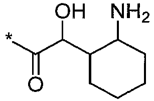 is to be understood to directly and unambiguously disclose each of

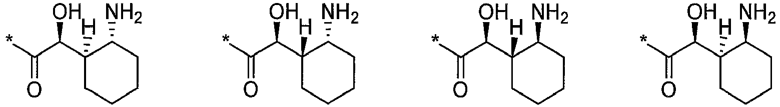

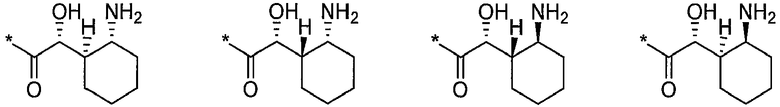

Accordingly, 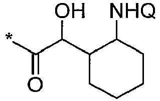 is to be understood to directly and unambiguously disclose each of

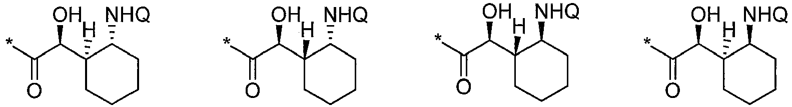

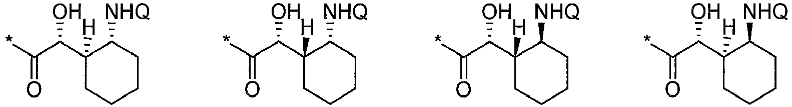

Accordingly, 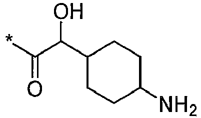 is to be understood to directly and unambiguously disclose each of

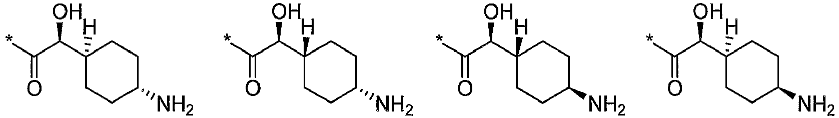

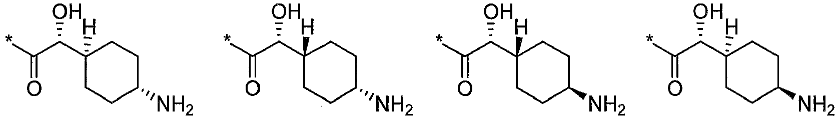

Accordingly, 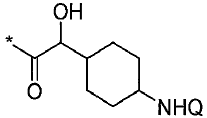 is to be understood to directly and unambiguously disclose each of

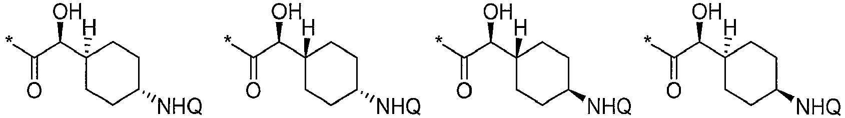

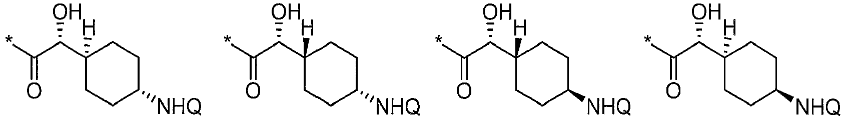

Accordingly, 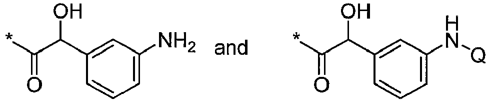 are to be understood to respectively directly and unambiguously disclose each of

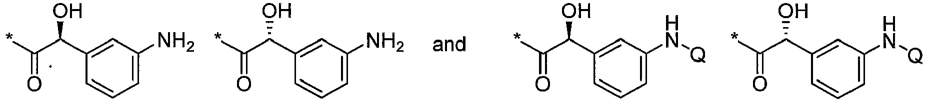

Accordingly, 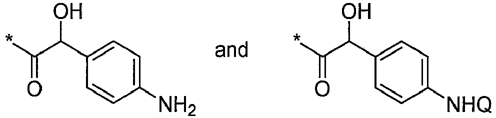 are to be understood to respectively directly and unambiguously disclose each of

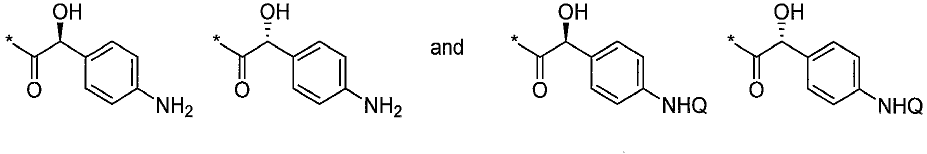

Accordingly, 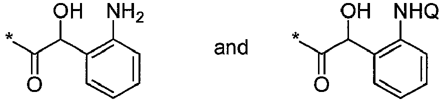 are to be understood to respectively directly and unambiguously disclose each of

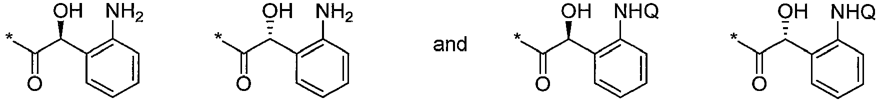

Accordingly, 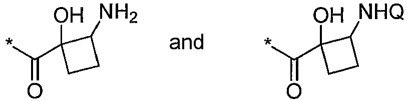 are to be understood to respectively directly and unambiguously disclose each of 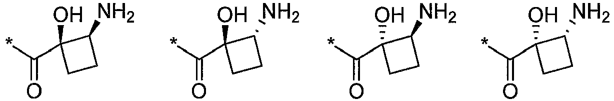 and

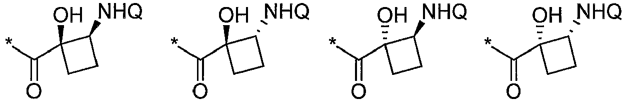

Accordingly, 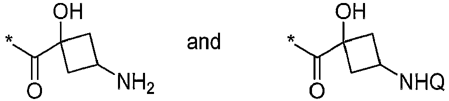 are to be understood to respectively directly and unambiguously disclose each of 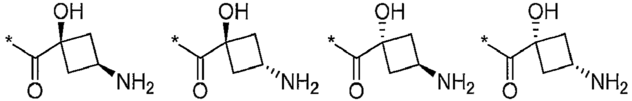 and

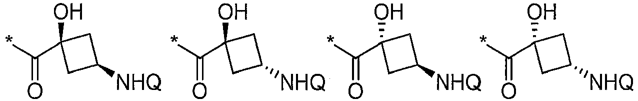

Accordingly, 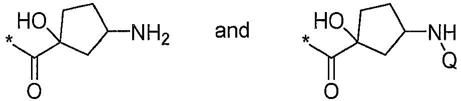 are to be understood to respectively directly and unambiguously disclose each of and

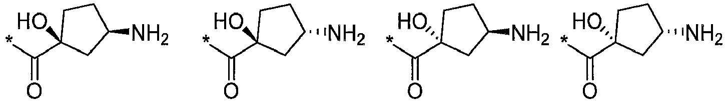

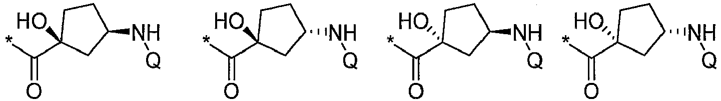

Accordingly, 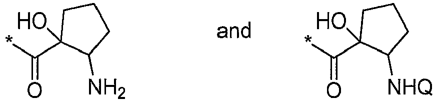 are to be understood to respectively directly and unambiguously disclose each of

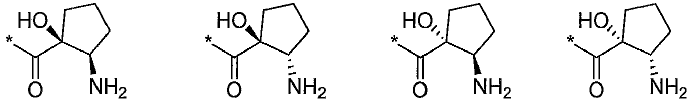

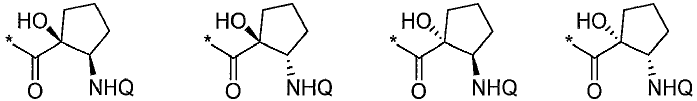

Accordingly, 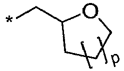 is to be understood to directly and unambiguously disclose each of

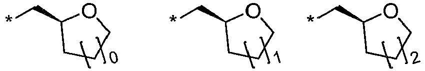

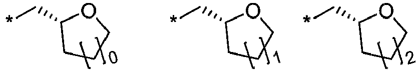

Accordingly, 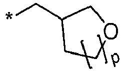 is to be understood to directly and unambiguously disclose each of

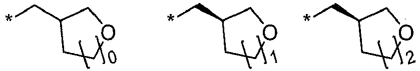

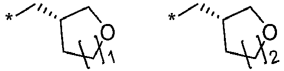

In the case that any structures are depicted in the present application in such a manner using "straight line" bonds at stereocentres but are not explicitly listed hereinabove, it is to be understood that the clear rules and teaching outlined above regarding the direct and unambiguous disclosure of each of the respective stereiosomers falling under said structures also applies to such structures.

Additionally, 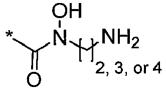 is to be understood to directly and unambiguously disclose each of

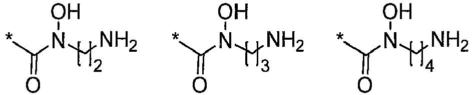

Additionally, 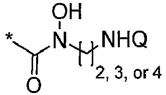 is to be understood to directly and unambiguously disclose each of

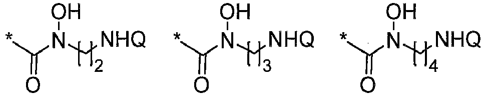

Additionally, 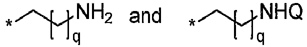 are to be understood to respectively directly and unambiguously disclose each of 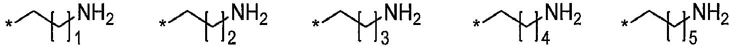 and

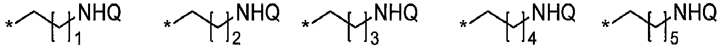

Additionally, 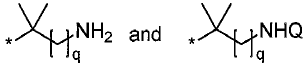 are to be understood to respectively directly and unambiguously disclose each of 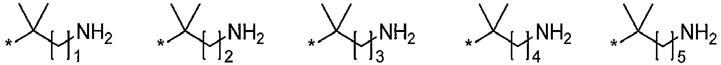 and

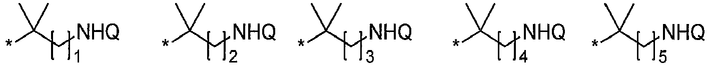

Additionally, 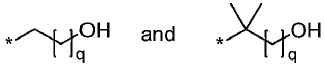 are to be understood to respectively directly and unambiguously disclose each of 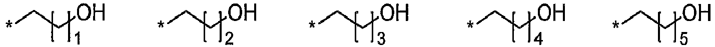 and

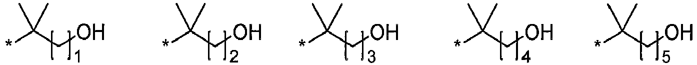

Additionally, 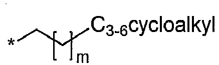 is to be understood to directly and unambiguously disclose each of

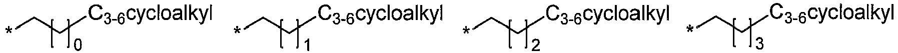

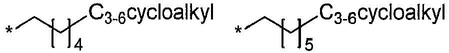

Additionally, 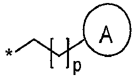 is to be understood to directly and unambiguously disclose each of

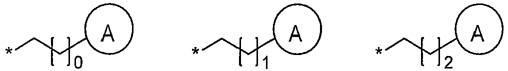

Additionally, 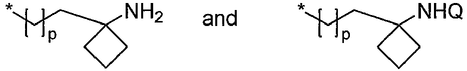 are to be understood to respectively directly and unambiguously disclose each 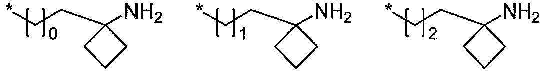 and of

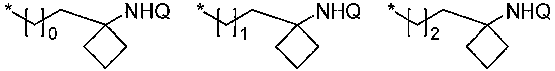

Additionally, 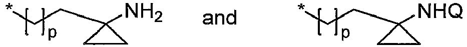 are to be understood to respectively directly and unambiguously disclose each 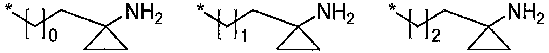 and of

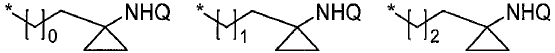

Additionally, 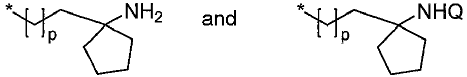 are to be understood to respectively directly and unambiguously disclose each of 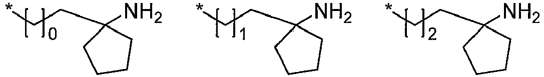 and

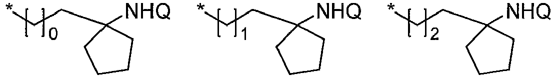

Additionally, 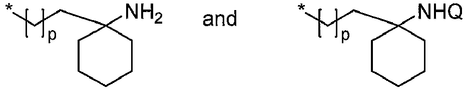 are to be understood to respectively directly and unambiguously disclose each of 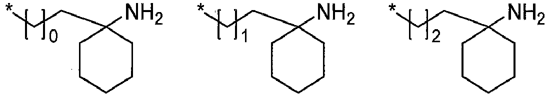 and

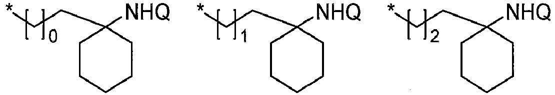

Additionally, 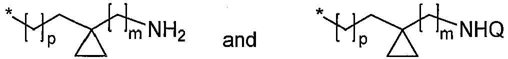 are to be understood to respectively directly and unambiguously disclose each of 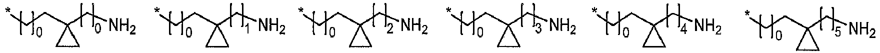 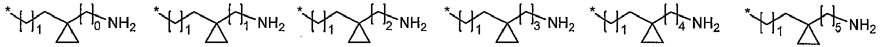 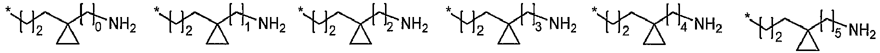 and

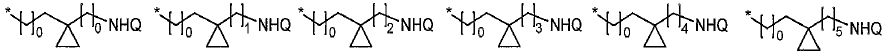

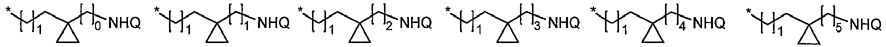

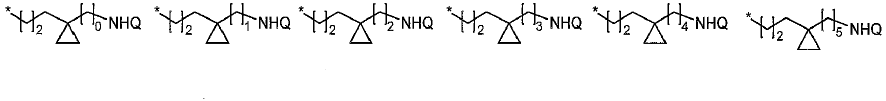

Additionally, 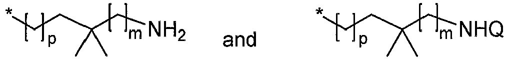 are to be understood to respectively directly and unambiguously disclose each of 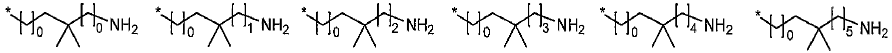 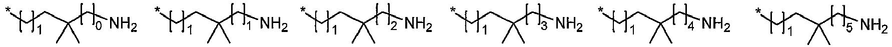 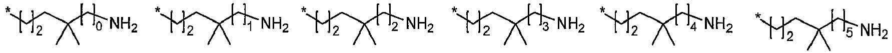 and

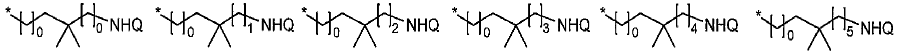

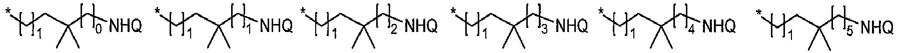

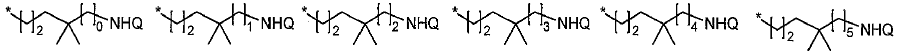

Additionally, 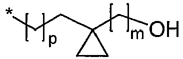 is to be understood to respectively directly and unambiguously disclose each of

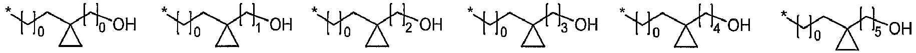

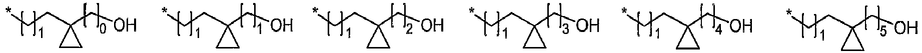

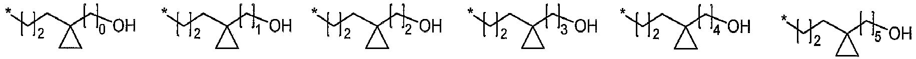

Additionally, 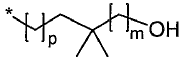 is to be understood to respectively directly and unambiguously disclose each of

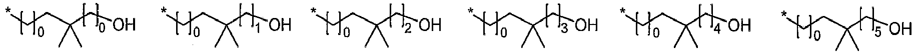

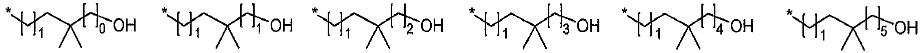

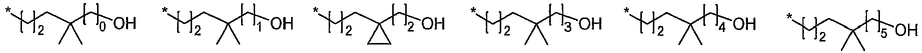

Additionally, 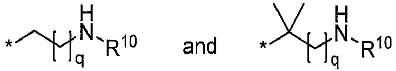 are to be understood to respectively directly and unambiguously disclose each of 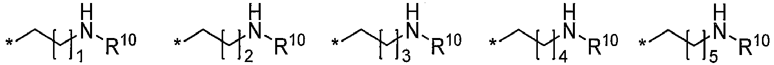 and

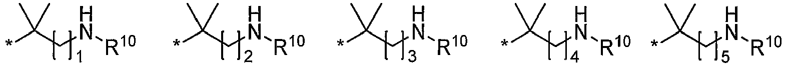

Additionally, 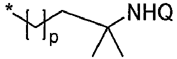 is to be understood to respectively directly and unambiguously disclose each of

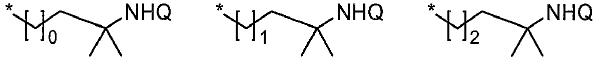

Additionally, 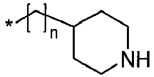 is to be understood to respectively directly and unambiguously disclose each of

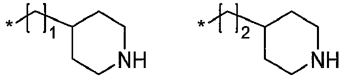

As used throughout herein, unless stated otherwise, "alkyl" means hydrocarbon chains which may be linear (straight chain) or branched. As used herein, C₂ₜₒ₄alkyl means a list of groups comprising or consisting of ethyl, n-propyl, isopropyl, n-, iso-, sec- and tert-butyl. As used herein, C₂ₜₒ₆alkyl means a list of groups comprising or consisting of ethyl, n-propyl, isopropyl, n-butyl, iso-butyl, sec-butyl, tert-butyl, n-pentyl, 1,1-dimethylpropyl, 1-methylbutyl, 1-ethylpropyl, 2,2-dimethylpropyl, 3-methylbutyl, 1,2-dimethylpropyl, 2-methylbutyl, n-hexyl, 2-methylpentyl, 3-methylpentyl, 2,2-dimethylbutyl, and 2,3-dimethylbutyl. As used herein, C₁ₜₒ₆alkyl means a list of groups comprising or consisting of methyl and each of those recited above in the definition of C₂ₜₒ₆alkyl. As used herein, straight chain C₂ₜₒ₆alkyl means a list of groups consisting of ethyl, n-propyl, n-butyl, n-pentyl, and n-hexyl. As used herein, straight chain C₃ₜₒ₆alkyl means a list of groups consisting of n-propyl, n-butyl, n-pentyl, and n-hexyl. As used herein, straight chain C₃ₜₒ₈alkyl means a list of groups consisting of n-propyl, n-butyl, n-pentyl, n-hexyl, n-heptyl, and n-octyl. As used herein, straight chain C₂ₜₒ₆alkyl means a list of groups consisting of ethyl, n-propyl, n-butyl, n-pentyl, n-hexyl, n-heptyl, and n-octyl. As used herein, straight chain C₃ₜₒ₆alkylene means a list of groups consisting of n-propylene, n-butylene, n-pentylene, n-hexylene, n-heptylene, and n-octylene.

As used herein, branched C₃ₜₒ₆alkyl means a list of groups comprising or consisting of isopropyl, iso-butyl, sec-butyl, tert-butyl, 1,1-dimethylpropyl, 1-methylbutyl, 1-ethylpropyl, 2,2-dimethylpropyl, 3-methylbutyl, 1,2-dimethylpropyl, 2-methylbutyl, 2-methylpentyl, 3-methylpentyl, 2,2-dimethylbutyl, and 2,3-dimethylbutyl.

As used herein, branched C₃ₜₒ₈alkyl means a list of groups comprising or consisting of isopropyl, iso-butyl, sec-butyl, tert-butyl, 1,1-dimethylpropyl, 1-methylbutyl, 1-ethylpropyl, 2,2-dimethylpropyl, 3-methylbutyl, 1,2-dimethylpropyl, 2-methylbutyl, 2-methylpentyl, 3-methylpentyl, 2,2-dimethylbutyl, 2,3-dimethylbutyl, 2-methylhexyl, 3-methylhexyl, 2,2-dimethylpentyl, 2,3-dimethylpentyl, 2,4-dimethylpentyl, 3,3-dimethylpentyl, 3-ethylpentyl, 2,2,3-trimethylbutyl, 2-methylheptyl, 3-methylheptyl , 4-methylheptyl, 3-ethylhexyl, 2,2-dimethylhexyl, 2,3-dimethylhexyl, 2,4-dimethylhexyl, 2,5-dimethylhexyl, 3,3-dimethylhexyl, 3,4-dimethylhexyl, 3-ethyl-2-methylpentyl, 3-ethyl-3-methylpentyl, 2,2,3-trimethylpentyl, 2,2,4-trimethylpentyl, 2,3,3-trimethylpentyl, 2,3,4-trimethylpentyl, and 2,2,3,3-tetramethylbutyl.

As used herein, C₃ₜₒ₈alkyl means a list of groups comprising or consisting of both those listed above in the definition of straight chain C₃ₜₒ₆alkyl and those listed above in the definition of branched C₃ₜₒ₈alkyl.

As used herein, branched C₃ₜₒ₈alkylene means a list of groups comprising or consisting of isopropylene, iso-butylene, sec-butylene, tert-butylene, 1,1-dimethylpropylene, 1-methylbutylene, 1-ethylpropylene, 2,2-dimethylpropylene, 3-methylbutylene, 1,2-dimethylpropylene, 2-methylbutylene, 2-methylpentylene, 3-methylpentylene, 2,2-dimethylbutylene, 2,3-dimethylbutylene, 2-methylhexylene, 3-methylhexylene, 2,2-dimethylpentylene, 2,3-dimethylpentylene, 2,4-dimethylpentylene, 3,3-dimethylpentylene, 3-ethylpentylene, 2,2,3-trimethylbutylene, 2-methylheptylene, 3-methylheptylene, 4-methylheptylene, 3-ethylhexylene, 2,2-dimethylhexylene, 2,3-dimethylhexylene, 2,4-dimethylhexylene, 2,5-dimethylhexylene, 3,3-dimethylhexylene, 3,4-dimethylhexylene, 3-ethyl-2-methylpentylene, 3-ethyl-3-methylpentylene, 2,2,3-trimethylpentylene, 2,2,4-trimethylpentylene, 2,3,3-trimethylpentylene, 2,3,4-trimethylpentylene, and 2,2,3,3-tetramethylbutylene.

As used throughout herein, unless stated otherwise, "cycloalkyl" means rings of which the atoms forming the ring itself are exclusively carbon atoms. As used herein, C₃₋₆cycloalkyl means a list of groups consisting of cyclopropyl, cyclobutyl, cyclopentyl, and cyclohexyl.

The term "halogen" as used throughout herein means, unless otherwise stated, F, Cl, Br or I. As used herein, the term "halogen" preferably means F, Cl or Br, most preferably F.

The term "heteroaryl" or "heteroaromatic" as used throughout herein refers to an aryl ring system having one to four heteroatoms (e.g., O, S, N, or combinations thereof) as ring atoms in a heteroaromatic ring system, wherein the remainder of the atoms forming the ring system are carbon atoms. The heteroaryl ring or group may consist of a single ring or a fused ring system. A typical fused heteroaryl ring system is a 9- or 10-membered ring system containing one to four heteroatoms selected from oxygen, sulphur and nitrogen. A typical single heteroaryl ring in this sense is a 5- to 6-membered ring containing one to four, preferably one to three heteroatoms selected from oxygen, sulfur and nitrogen. Examples of 5-membered heteroaryl include pyrrolyl, furanyl, thienyl, imidazolyl, pyrazolyl, oxazolyl, isoxazolyl, thiazolyl, isothiazolyl, triazolyl, furazanyl, oxadiazolyl, thiadiazolyl, dithiazolyl, and tetrazolyl. Examples of 6-membered heteroaryl include pyridyl, pyrazinyl, pyrimidyl, pyridazinyl, triazinyl, and tetrazinyl. As used throughout herein, 5-membered heteroaryl may include pyrrolyl (e.g. 1-, 2-,3-, 4-, or 5-pyrrolyl), furanyl (e.g. 2-, 3-, 4-, or 5- furanyl), thienyl (e.g. 2-, 3-, 4-, or 5-thienyl), imidazolyl (e.g. 1-, 2-, 4-, or 5- imidazolyl), pyrazolyl (e.g. 1-, 3-, 4-, or 5-pyrazolyl), oxazolyl (e.g. 2-, 4-, or 5-oxazolyl), isoxazolyl (e.g. 3-, 4-, or 5-isoxazolyl), thiazolyl (e.g. 2-, 4-, or 5-thiazolyl), isothiazolyl (e.g. 3-, 4-, or 5-isothiazolyl), triazolyl (e.g. 4-, or 5-(1*H*-1,2,3-triazol)-yl; 4-, or 5-(2H-1,2,3-triazol)-yl; 3-, or 5-(1*H*-1,2,4-triazol)-yl; 3-, or 5-(4*H*-1,2,4-triazol)-yl), furazanyl (e.g. 3-, or 4-furazanyl), oxadiazolyl (e.g. 3-, or 4-(1,2,5-oxadiazol)-yl; 3-, or 5-(1,2,4-oxadiazol)-yl; 4-, or 5-(1,2,3-oxadiazol)-yl; 2-, or 5-(1,3,4-oxadiazol)-yl), thiadiazolyl (e.g. 3-, or 4-(1,2,5-thiadiazol)-yl; 3-, or 5-(1,2,4-thiadiazol)-yl; 4-, or 5-(1,2,3-thiadiazol)-yl; 2-, or 5-(1,3,4-thiadiazol)-yl), and tetrazolyl (e.g. 1-, or 5-(1*H*-tetrazol)-yl; 1-, or 4-(2H-tetrazol)-yl. As used throughout herein 6-membered heteroaryl may include pyridyl (e.g. 2-, 3-, 4-, 5- or 6-pyridyl), pyrazinyl (e.g. 2-, 3-, 5- or 6-pyrazinyl), pyrimidyl (e.g. 2-, 4-, 5- or 6-pyrimidyl), pyridazinyl (e.g. 3-, 4-, 5- or 6-pyridazinyl), triazinyl (e.g. 4-, or 5-, or 6-(1,2,3-triazin)-yl; 3-, or 5-, or 6-(1,2,4-triazin)-yl; 2-, or 4-, or 6-(1,3,5-triazin)-yl).

As used throughout herein, a 4, 5 or 6-membered non-aromatic heterocycloalkyl group or moiety or a non-aromatic heterocyclic ring may be understood to refer to a 4, 5 or 6-membered cycloaliphatic ring in which 1, 2 or 3 of the ring carbon atoms have independently been replaced with O, N or 5 atoms. The resultant ring is non-aromatic. For example, the replacement of a carbon atom in the cycloaliphatic compound cyclohexane with a nitrogen atom in order to generate the non-aromatic heterocyclic compound piperidine or the replacement of a carbon atom in the cycloaliphatic group, cyclopentyl with a nitrogen atom in order to generate the 5-membered non-aromatic heterocycloalkyl group (containing 1 heteroatom), pyrrolidyl.

As used throughout herein, optionally substituted 4-membered non-aromatic heterocycloalkyl groups may include optionally substituted azetidinyl (for example, optionally substituted 1-azetidinyl, 2-azetidinyl, 3-azetidinyl, or 4-azetidinyl), optionally substituted 1,3-diazetidyl (for example, optionally substituted 1- diazetidyl, 2- diazetidyl, 3-diazetidyl, or 4-diazetidyl), optionally substituted oxetanyl (for example, optionally substituted 2-oxetanyl, 3-oxetanyl, or 4-oxetanyl), and optionally substituted thietanyl (for example, optionally substituted 2- thietanyl, 3-thietanyl, or 4-thietanyl).

As used throughout herein, optionally substituted 5-membered non-aromatic heterocycloalkyl groups may include optionally substituted pyrrolidinyl (for example, optionally substituted 1-pyrrolidinyl, 2-pyrrolidinyl, 3-pyrrolidinyl, 4-pyrrolidinyl, or 5-pyrrolidinyl), optionally substituted pyrazolidinyl (for example, optionally substituted 1-pyrazolidinyl, 2- pyrazolidinyl, 3-pyrazolidinyl, 4-pyrazolidinyl, or 5-pyrazolidinyl), optionally substituted imidazolidinyl (for example, optionally substituted 1-imidazolidinyl, 2-imidazolidinyl, 3-imidazolidinyl, 4-imidazolidinyl, or 5-imidazolidinyl), optionally substituted tetrahydrofuranyl (for example, optionally substituted 2-tetrahydrofuranyl, 3-tetrahydrofuranyl, 4-tetrahydrofuranyl, or 5-tetrahydrofuranyl), optionally substituted 1,3-dioxolanyl (for example, optionally substituted 1,3-dioxolan-2-yl, 1,3-dioxolan-4-yl, or 1,3-dioxolan-5-yl), optionally substituted tetrahydrothiophenyl (for example, optionally substituted 2-tetrahydrothiophenyl, 3-tetrahydrothiophenyl, 4-tetrahydrothiophenyl, or 5-tetrahydrothiophenyl), optionally substituted oxazolidinyl (for example, optionally substituted 2-oxazolidinyl, 4-oxazolidinyl, or 5-oxazolidinyl), optionally substituted isoxazolidinyl (for example, optionally substituted 3-isoxazolidinyl, 4-isoxazolidinyl, or 5-isoxazolidinyl), optionally substituted 1,2-oxathiolanyl (for example, optionally substituted 1,2-oxathiolan-3-yl , 1,2-oxathiolan-4-yl, or 1,2-oxathiolan-5-yl), optionally substituted 1,3-oxathiolanyl (for example, optionally substituted 1,3-oxathiolan-2-yl, 1,3-oxathiolan-4-yl, or 1,3-oxathiolan-5-yl), optionally substituted thiazolidinyl (for example, optionally substituted 2-thiazolidinyl, 3-thiazolidinyl, 4-thiazolidinyl, or 5-thiazolidinyl), and optionally substituted isothiazolidinyl (for example, optionally substituted 2-isothiazolidinyl, 3-isothiazolidinyl, 4-isothiazolidinyl, or 5-isothiazolidinyl), optionally substituted 1,3-dithiolanyl (for example, optionally substituted 3-dithiolanyl, 4-dithiolanyl, or 5-dithiolanyl).

As used throughout herein, optionally substituted 6-membered non-aromatic heterocycloalkyl groups may include optionally substituted piperidinyl (for example, optionally substituted 1-piperidinyl, 2-piperidinyl, 3-piperidinyl, 4-piperidinyl, 5-piperidinyl, or 6-piperidinyl), optionally substituted tetrahydropyranyl (for example, optionally substituted 2-tetrahydropyranyl, 3-tetrahydropyranyl, 4-tetrahydropyranyl, 5-tetrahydropyranyl, or 6-tetrahydropyranyl), optionally substituted thianyl (for example, optionally substituted 2-thianyl, 3-thianyl, 4-thianyl, 5-thianyl, or 6-thianyl), optionally substituted piperazinyl (for example, optionally substituted 1-piperazinyl, 2-piperazinyl, 3-piperazinyl, 4-piperazinyl, 5-piperazinyl, or 6-piperazinyl), optionally substituted morpholinyl (for example, optionally substituted 2-morpholinyl, 3-morpholinyl, 4-morpholinyl, 5-morpholinyl, or 6-morpholinyl), optionally substituted thiomorpholinyl (for example, optionally substituted 2-thiomorpholinyl, 3-thiomorpholinyl, 4-thiomorpholinyl, 5-thiomorpholinyl, or 6-thiomorpholinyl), optionally substituted 1,3-dioxanyl (for example, optionally substituted 1,3-dioxan-2-yl, 1,3-dioxan-4-yl, 1,3-dioxan-5-yl, or 1,3-dioxan-6-yl), optionally substituted 1,4-dioxanyl (for example, optionally substituted 1,4-dioxan-2-yl, or 1,2-dioxan-3-yl), optionally substituted 1,3-dithianyl (for example, optionally substituted 1,3-dithian-2-yl, 1,3-dithian-4-yl, 1,3-dithian-5-yl, or 1,3-dithian-6-yl), optionally substituted 1,4-dithianyl (for example, optionally substituted 1,4-dithian-2-yl, or 1,2-dithian-3-yl).

As used throughout herein, "aryl" preferably means an unsaturated aromatic carbocyclic group of from 6 to 14 carbon atoms having a single ring (e.g. phenyl) or multiple condensed rings (e.g. naphthyl or anthryl). Particular aryls include phenyl, biphenyl, naphthyl and the like.

Examples of bicyclic fused heteroaryl ring systems include indolyl (e.g. 1-, 2-, 3-, 4-, 5-, 6-, or 7-indolyl), benzofuranyl (e.g. 2-, 3-, 4-, 5-, 6-, or 7-benzofuranyl), indazolyl (e.g. 1-, 3-, 4-, 5-, 6-, or 7-indazolyl), oxindolyl (e.g. 1-, 3-, 4-, 5-, 6-, or 7-oxindolyl), benzimidazolyl (e.g. 1-, 2-, 4-, 5-, 6-, or 7-benzimidazolyl), benzothiophenyl (e.g. 2-, 3-, 4-, 5-, 6-, or 7-benzothiophenyl), benzoxazolyl (e.g. 2-, 4-, 5-, 6-, or 7-benzoxazolyl), benzo[d]thiazolyl (e.g. 2-, 4-, 5-, 6-, or 7-benzo[d]thiazolyl), quinolinyl (e.g. 2-, 3-, 4-, 5-, 6-, 7-, or 8-quinolinyl), isoquinolinyl (e.g. 1-, 3-, 4-, 5-, 6-, 7-, or 8-isoquinolinyl), coumarinyl (e.g. 3-, 4-, 5-, 6-, 7-, or 8- coumarinyl), purinyl (e.g. 2-, 6-, 8-, or 9- purinyl), 1,2-diazanaphthyl (e.g. 3-, 4-, 5-, 6-, 7-, 8-(1,2-diazanaphth)-yl), 1,3-diazanaphthyl (e.g. 2-, 4-, 5-, 6-, 7-, 8-(1,3-diazanaphth)-yl), 1,4-diazanaphthyl (e.g. 2-, 3-, 5-, 6-, 7-, 8-(1,4-diazanaphth)-yl), 1,5-diazanaphthyl (e.g. 2-, 3-, 4-, 6-, 7-, 8-(1,5-diazanaphth)-yl), 1,6-diazanaphthyl (e.g. 2-, 3-, 4-, 5-, 7-, 8-(1,6-diazanaphth)-yl), 1,7-diazanaphthyl (e.g. 2-, 3-, 4-, 5-, 6-, 8-(1,7-diazanaphth)-yl), 1,8-diazanaphthyl (e.g. 2-, 3-, 4-, 5-, 6-, 7-(1,8-diazanaphth)-yl), 2,3-diazanaphthyl (e.g. 1-, 4-, 5-, 6-, 7-, 8-(2,3-diazanaphth)-yl), 2,6-diazanaphthyl (e.g. 1-, 3-, 4-, 5-, 7-, 8-(2,6-diazanaphth)-yl), and 2,7-diazanaphthyl (e.g. 1-, 3-, 4-, 5-, 6-, 8-(2,7-diazanaphth)-yl).

Where reference is made herein to groups or moieties which may be "substituted or unsubstituted", "optionally substituted", "substituted with any substituent" or "substituted", when substituted, they may be, unless otherwise stipulated, substituted with one or more of any substituent. Where reference is made herein to groups or moieties which may be "substituted or unsubstituted", "optionally substituted", "substituted with any substituent" or "substituted", when substituted, it is preferred that the introduction of said substituent does not directly give rise to a carbon atom bearing more than one O, N, or 5 atom. Likewise preferred is that the introduction of said substituent does not directly give rise to a heteroatom-heteroatom bond (e.g. a N-N, N-O, S-N, O-S, O-O, or S-S bond).

Examples of such substituents are those found in the exemplary compounds and embodiments disclosed herein, as well as, for example, halo (e.g., chloro, iodo, bromo, or fluoro); C_{1- or 2-8} alkyl; C_{1- or 2-8} alkyl substituted with one or more substituents independently selected from OH, NH₂, NHCH₃, N(CH₃)₂, NH(C₂₋₆alkyl), N(C₂₋₆alkyl)₂, SH, SCH₃, SC₂₋₆alkyl, CN, CONH₂, CO₂H, NO₂, SO₂H, SO₂CH₃, and SO₂Aryl; C₂₋₈ alkenyl; C₂₋₈ alkynyl; hydroxyl; C_{1-or 2-8} alkoxyl; -NH₂; -NHCH₃; -NHC₂₋₆ alkyl; -N(CH₃)₂; -N(C₂₋₆ alkyl)₂; amino (primary, secondary, or tertiary); -NO₂; -SH; -SCH₃; -SC₂-₆ alkyl; -C=NH; -C=NCH₃; -C=NC₂₋₆ alkyl; -CN; -CONH₂; -CONHCH₃; -CONHC₂₋₆ alkyl; -CON(CH₃)₂; -CON(C₂₋₆alkyl)₂; phosphonato; -P(O)(OH)₂; -P(O)(OH)(OCH₃); -P(O)(OCH₃)₂; -P(O)(OH)(OC₂₋₆ alkyl); -P(O)(OC₂₋₆ alkyl)₂; -OP(O)(OH)₂; -OP(O)(OH)(OCH₃); -OP(O)(OCH₃)₂; -OP(O)(OH)(OC₂₋₆ alkyl); -OP(O)(OC₂₋₆ alkyl)₂; phosphine; -P(CH₃)₂; -P(C₂₋₆ alkyl)₂; -P(C₃₋₆cycloalkyl)₂; -P(Aryl)₂; -P(Phenyl)₂; -P(Heteroaryl)₂; carboxyl; -CO₂H; carbamoyl; -OCONH₂; -OCONHCH₃; -OCON(CH₃)C₂₋₆ alkyl; -OCON(CH₃)C₃₋₆cycloalkyl; -OCON(CH₃)Aryl; -OCON(CH₃)Phenyl; -OCON(CH₃)Heteroaryl; -OCON(CH₃)₂; -OCONHC₂₋₆alkyl; -OCON(C₂₋₆alkyl)C₃₋₆cycloalkyl; -OCON(C₂₋₆alkyl)Aryl; -OCON(C₂₋₆alkyl)Phenyl; -OCON(C₂₋₆alkyl)Heteroaryl; -OCON(C₂₋₆ alkyl)₂; -OCONHC₃₋₆cycloalkyl; -OCON(C₃₋₆cycloalkyl)Aryl; -OCON(C₃₋₆cycloalkyl)Phenyl; -OCON(C₃₋₆ cycloalkyl)Heteroaryl; -OCON(C₃₋₆ cycloalkyl)₂; -OCONHAryl; -OCON(Aryl)Phenyl; -OCON(Aryl)Heteroaryl; -OCON(Aryl)₂; -OCONHPhenyl; -OCON(Phenyl)Heteroaryl; -OCON(Phenyl)₂; -OCONHHeteroaryl; -OCON(Heteroaryl)₂; -OCON(CH₃)₂ ; -OCON(C₂₋₆ alkyl)₂; -OCONCH₃(C₂₋₆alkyl); -NHCOOCH₃; -NHCOOC₂₋₆ alkyl; -NHCOOC₃₋₆cycloalkyl; -NHCOOAryl; -NHCOOPhenyl; -NHCOO(Heteroaryl); -N(CH₃)COOCH₃; -N(CH₃)COOC₂₋₆alkyl; -N(CH₃)COOC₃₋₆cycloalkyl; -N(CH₃)COOAryl; -N(CH₃)COOPhenyl; -N(CH₃)COO(Heteroaryl); -N(C₂₋₆alkyl)COOCH₃; -N(C₂₋₆alkyl)COOC₂₋₆alkyl; -N(C₂₋₆alkyl)COOC₃₋₆cycloalkyl; -N(C₂₋₆alkyl)COOAryl; -N(C₂₋₆alkyl)COOPhenyl; -N(C₂₋₆alkyl)COO(Heteroaryl); -N(COOC₃₋₆cycloalkyl)COOCH₃; -N(COOC₃₋₆cycloalkyl)COOC₂₋₆alkyl; -N(COOC₃₋₆cycloalkyl)COOC₃₋₆cycloalkyl; -N(COOC₃₋₆cycloalkyl)COOAryl; -N(COOC₃₋₆cycloalkyl)COOPhenyl; -N(COOC₃₋₆cycloalkyl)COO(Heteroaryl); -N(Aryl)COOCH₃; -N(Aryl)COOC₂₋₆ alkyl; -N(Aryl)COOC₃₋₆cycloalkyl; -N(Aryl)COOAryl; -N(Aryl)COOPhenyl; -N(Aryl)COO(Heteroaryl); -N(Phenyl)COOCH₃; -N(Phenyl)COOC₂₋₆alkyl; -N(Phenyl)COOC₃₋₆cycloalkyl; -N(Phenyl)COOAryl; -N(Phenyl)COOPhenyl; -N(Phenyl)COO(Heteroaryl); -N(Heteroaryl)COOCH₃; -N(Heteroaryl)COOC₂₋₆alkyl; -N(Heteroaryl)COOC₃₋₆cycloalkyl; -N(Heteroaryl)COOAryl; -N(Heteroaryl)COOPhenyl; -N(Heteroaryl)COO(Heteroaryl); -NCOOCH₃; -N(CH₃)COOC₂₋₆alkyl; -N(C₂₋₆ alkyl)COOCH₃; -N(C₂₋₆ alkyl)COOC₂₋₆alkyl; carbamate; acetal; urea; -NHCONH₂; -NHCONH(CH₃); NHCON(CH₃)₂; -NHCONH(C₂₋₆ alkyl); -NHCON(C₂₋₆alkyl)₂; -NHCONHC₃₋₆cycloalkyl; -NHCON(C₃₋₆cycloalkyl)₂; -NHCONHAryl; -NHCON(Aryl)₂; -NHCONHPhenyl; -NHCON(Phenyl)₂; -NHCONHHeteroaryl; -NHCON(Heteroaryl)₂;-NHCON(CH₃)(C₂₋₆ alkyl); -NHCON(CH₃)(C₃₋₆ cycloalkyl); -NHCON(CH₃)(Aryl); -NHCON(CH₃)(Phenyl); -NHCON(CH₃)(Heteroaryl); -NHCON(C₂₋₆alkyl)(C₃₋₆cycloalkyl); -NHCON(C₂₋₆alkyl)(Aryl); -NHCON(C₂₋₆ alkyl)(Phenyl); -NHCON(C₂₋₆alkyl)(Heteroaryl); -NHCON(C₃₋₆ cycloalkyl)(Aryl); -NHCON(C₃₋₆cycloalkyl)(Phenyl); -NHCON(C₃₋₆cycloalkyl)(Heteroaryl); -NHCON(Aryl)(Phenyl); -NHCON(Aryl)(Heteroaryl); -NHCON(Phenyl)(Heteroaryl); -N(CH₃)CONH₂; -N(CH₃)CONH(CH₃); N(CH₃)CON(CH₃)₂; -N(CH₃)CONH(C₂₋₆alkyl); -N(CH₃)CON(C₂₋₆alkyl)₂; -N(CH₃)CONHC₃₋₆cycloalkyl; -N(CH₃)CON(C₃₋₆cycloalkyl)₂; -N(CH₃)CONHAryl; -N(CH₃)CON(Aryl)₂; -N(CH₃)CONHPhenyl; -N(CH₃)CON(Phenyl)₂; -N(CH₃)CONHHeteroaryl; -N(CH₃)CON(Heteroaryl)₂; -N(CH₃)CON(CH₃)(C₂₋₆alkyl); -N(CH₃)CON(CH₃)(C₃₋₆cycloalkyl); -N(CH₃)CON(CH₃)(Aryl); -N(CH₃)CON(CH₃)(Phenyl); -N(CH₃)CON(CH₃)(Heteroaryl); -N(CH₃)CON(C₂₋₆alkyl)(C₃₋₆cycloalkyl); -N(CH₃)CON(C₂₋₆alkyl)(Aryl); -N(CH₃)CON(C₂₋₆alkyl)(Phenyl); -N(CH₃)CON(C₂₋₆alkyl)(Heteroaryl); -N(CH₃)CON(C₃₋₆cycloalkyl)(Aryl); -N(CH₃)CON(C₃₋₆cycloalkyl)(Phenyl); -N(CH₃)CON(C₃₋₆cycloalkyl)(Heteroaryl); -N(CH₃)CON(Aryl)(Phenyl); -N(CH₃)CON(Aryl)(Heteroaryl); -N(CH₃)CON(Phenyl)(Heteroaryl); -N(C₂₋₆alkyl)CONH₂; -N(C₂₋₆alkyl)CONH(CH₃); -N(C₂₋₆alkyl)CON(CH₃)₂; -N(C₂₋₆alkyl)CONH(C₂₋₆alkyl); -N(C₂₋₆alkyl)CON(C₂₋₆alkyl)₂; -N(C₂₋₆alkyl)CONHC₃₋₆cycloalkyl; -N(C₂₋₆ alkyl)CON(C₃₋₆ cycloalkyl)₂; -N(C₂₋₆ alkyl)CONHAryl; -N(C₂₋₆alkyl)CON(Aryl)₂; -N(C₂₋₆alkyl)CONHPhenyl; -N(C₂₋₆alkyl)CON(Phenyl)₂; -N(C₂₋₆alkyl)CONHHeteroaryl; -N(C₂₋₆alkyl)CON(Heteroaryl)₂; -N(C₂₋₆alkyl)CON(CH₃)(C₂₋₆alkyl); -N(C₂₋₆alkyl)CON(CH₃)(C₃₋₆cycloalkyl); -N(C₂₋₆alkyl)CON(CH₃)(Aryl); -N(C₂₋₆ alkyl)CON(CH₃)(Phenyl); -N(C₂₋₆alkyl)CON(CH₃)(Heteroaryl); -N(C₂₋₆alkyl)CON(C₂₋₆alkyl)(C₃₋₆ cycloalkyl); -N(C₂₋₆ alkyl)CON(C₂₋₆ alkyl)(Aryl); -N(C₂₋₆ alkyl)CON(C₂₋₆ alkyl)(Phenyl); -N(C₂₋₆alkyl)CON(C₂₋₆alkyl)(Heteroaryl); -N(C₂₋₆alkyl)CON(C₃₋₆cycloalkyl)(Aryl); -N(C₂₋₆alkyl)CON(C₃₋₆cycloalkyl)(Phenyl); -N(C₂₋₆alkyl)CON(C₃₋₆cycloalkyl)(Heteroaryl); -N(C₂₋₆alkyl)CON(Aryl)(Phenyl); -N(C₂₋₆alkyl)CON(Aryl)(Heteroaryl); -N(C₂₋₆alkyl)CON(Phenyl)(Heteroaryl); -N(C₃₋₆cycloalkyl)CONH₂; -N(C₃₋₆ cycloalkyl)CONH(CH₃); N(C₃₋₆ cycloalkyl)CON(CH₃)₂; -N(C₃₋₆ cycloalkyl)CONH(C₂₋₆ alkyl); -N(C₃₋₆ cycloalkyl)CON(C₂₋₆ alkyl)₂; -N(C₃₋₆cycloalkyl)CONHC₃₋₆ cycloalkyl; -N(C₃₋₆ cycloalkyl)CON(C₃₋₆ cycloalkyl)₂; -N(C₃₋₆cycloalkyl)CONHAryl; -N(C₃₋₆cycloalkyl)CON(Aryl)₂; -N(C₃₋₆cycloalkyl)CONHPhenyl; -N(C₃₋₆cycloalkyl)CON(Phenyl)₂; -N(C₃₋₆cycloalkyl)CONHHeteroaryl; -N(C₃₋₆cycloalkyl)CON(Heteroaryl)₂; -N(C₃₋₆cycloalkyl)CON(CH₃)(C₂₋₆ alkyl); -N(C₃₋₆ cycloalkyl)CON(CH₃)(C₃₋₆ cycloalkyl); -N(C₃₋₆ cycloalkyl)CON(CH₃)(Aryl); -N(C₃₋₆ cycloalkyl)CON(CH₃)(Phenyl); -N(C₃₋₆cycloalkyl)CON(CH₃)(Heteroaryl); -N(C₃₋₆cycloalkyl)CON(C₂₋₆alkyl)(C₃₋₆cycloalkyl); -N(C₃₋₆ cycloalkyl)CON(C₂₋₆ alkyl)(Aryl); -N(C₃₋₆ cycloalkyl)CON(C₂₋₆alkyl)(Phenyl); -N(C₃₋₆cycloalkyl)CON(C₂₋₆alkyl)(Heteroaryl); -N(C₃₋₆cycloalkyl)CON(C₃₋₆cycloalkyl)(Aryl); -N(C₃₋₆cycloalkyl)CON(C₃₋₆cycloalkyl)(Phenyl); -N(C₃₋₆ cycloalkyl)CON(C₃₋₆ cycloalkyl)(Heteroaryl); -N(C₃₋₆cycloalkyl)CON(Aryl)(Phenyl); -N(C₃₋₆cycloalkyl)CON(Aryl)(Heteroaryl); -N(C₃₋₆cycloalkyl)CON(Phenyl)(Heteroaryl); -N(Aryl)CONH₂; -N(Aryl)CONH(CH₃); -N(Aryl)CON(CH₃)₂; -N(Aryl)CONH(C₂₋₆alkyl); -N(Aryl)CON(C₂₋₆alkyl)₂; -N(Aryl)CONHC₃₋₆cycloalkyl;-N(Aryl)CON(C₃₋₆cycloalkyl)₂; -N(Aryl)CONHAryl; -N(Aryl)CON(Aryl)₂; -N(Aryl)CONHPhenyl; -N(Aryl)CON(Phenyl)₂; -N(Aryl)CONHHeteroaryl; -N(Aryl)CON(Heteroaryl)₂; -N(Aryl)CON(CH₃)(C₂₋₆alkyl); -N(Aryl)CON(CH₃)(C₃₋₆cycloalkyl); -N(Aryl)CON(CH₃)(Aryl); -N(Aryl)CON(CH₃)(Phenyl); -N(Aryl)CON(CH₃)(Heteroaryl); -N(Aryl)CON(C₂₋₆alkyl)(C₃₋₆cycloalkyl); -N(Aryl)CON(C₂₋₆alkyl)(Aryl); -N(Aryl)CON(C₂₋₆ alkyl)(Phenyl); -N(Aryl)CON(C₂₋₆alkyl)(Heteroaryl); -N(Aryl)CON(C₃₋₆cycloalkyl)(Aryl); -N(Aryl)CON(C₃₋₆cycloalkyl)(Phenyl); -N(Aryl)CON(C₃₋₆cycloalkyl)(Heteroaryl); -N(Aryl)CON(Aryl)(Phenyl); -N(Aryl)CON(Aryl)(Heteroaryl); -N(Aryl)CON(Phenyl)(Heteroaryl); -N(Phenyl)CONH₂; -N(Phenyl)CONH(CH₃); -N(Phenyl)CON(CH₃)₂; -N(Phenyl)CONH(C₂₋₆alkyl); -N(Phenyl)CON(C₂₋₆alkyl)₂; -N(Phenyl)CONHC₃₋₆cycloalkyl; -N(Phenyl)CON(C₃₋₆cycloalkyl)₂; -N(Phenyl)CONHAryl; -N(Phenyl)CON(Aryl)₂; -N(Phenyl)CONHPhenyl; -N(Phenyl)CON(Phenyl)₂; -N(Phenyl)CONHHeteroaryl; -N(Phenyl)CON(Heteroaryl)₂; -N(Phenyl)CON(CH₃)(C₂₋₆alkyl); -N(Phenyl)CON(CH₃)(C₃₋₆cycloalkyl); -N(Phenyl)CON(CH₃)(Aryl); -N(Phenyl)CON(CH₃)(Phenyl); -N(Phenyl)CON(CH₃)(Heteroaryl); -N(Phenyl)CON(C₂₋₆alkyl)(C₃₋₆cycloalkyl); -N(Phenyl)CON(C₂₋₆alkyl)(Aryl); -N(Phenyl)CON(C₂₋₆alkyl)(Phenyl); -N(Phenyl)CON(C₂₋₆alkyl)(Heteroaryl); -N(Phenyl)CON(C₃₋₆cycloalkyl)(Aryl); -N(Phenyl)CON(C₃₋₆cycloalkyl)(Phenyl); -N(Phenyl)CON(C₃₋₆cycloalkyl)(Heteroaryl);-N(Phenyl)CON(Aryl)(Phenyl); -N(Phenyl)CON(Aryl)(Heteroaryl); -N(Phenyl)CON (Phenyl)(Heteroaryl); -N(Heteroaryl)CONH₂; -N(Heteroaryl)CONH(CH₃); -N(Heteroaryl)CON(CH₃)₂; -N(Heteroaryl)CONH(C₂₋₆alkyl); -N(Heteroaryl)CON(C₂₋₆alkyl)₂; -N(Heteroaryl)CONHC₃₋₆cycloalkyl; -N(Heteroaryl)CON(C₃₋₆cycloalkyl)₂; -N(Heteroaryl)CONHAryl; -N(Heteroaryl)CON(Aryl)₂; -N(Heteroaryl)CONHPhenyl; -N(Heteroaryl)CON(Phenyl)₂; -N(Heteroaryl)CONHHeteroaryl; -N(Heteroaryl)CON(Heteroaryl)₂; -N(Heteroaryl)CON(CH₃)(C₂₋₆alkyl); -N(Heteroaryl)CON(CH₃)(C₃₋₆cycloalkyl); -N(Heteroaryl)CON(CH₃)(Aryl); -N(Heteroaryl)CON(CH₃)(Phenyl); -N(Heteroaryl)CON(CH₃)(Heteroaryl); -N(Heteroaryl)CON(C₂₋₆alkyl)(C₃₋₆cycloalkyl); -N(Heteroaryl)CON(C₂₋₆alkyl)(Aryl); -N(Heteroaryl)CON(C₂₋₆alkyl)(Phenyl); -N(Heteroaryl)CON(C₂₋₆alkyl)(Heteroaryl); -N(Heteroaryl)CON(C₃₋₆cycloalkyl)(Aryl); -N(Heteroaryl)CON(C₃₋₆cycloalkyl)(Phenyl); -N(Heteroaryl)CON(C₃₋₆cycloalkyl)(Heteroaryl); -N(Heteroaryl)CON(Aryl)(Phenyl); -N(Heteroaryl)CON(Aryl)(Heteroaryl); -N(Heteroaryl)CON(Phenyl)(Heteroaryl); -NHCONH(CH₃); -NHCON(CH₃)₂; -NHCONH(C₂₋₆alkyl); -NHCON(C₂₋₆alkyl)₂; -NHCON(CH₃)(C₂₋₆ alkyl); -N(C₂₋₆alkyl)CONH₂; -N(C₂₋₆alkyl)CONH(CH₃); -N(C₂₋₆alkyl)CON(CH₃)₂; -N(C₂₋₆alkyl)CONH(C₂₋₆alkyl); -N(C₂₋₆alkyl)CON(C₂₋₆alkyl)₂; -N(C₂₋₆alkyl)CON(CH₃)(C₂₋₆alkyl); thiocarbonyl; -C(S)CH₃; -C(S)C₂₋₆ alkyl; -C(S)C₃₋₆cycloalkyl; -C(S)Aryl; -C(S)Phenyl; -C(S)Heteroaryl; sulfonyl; -SO₂CH₃; -SO₂C₂₋₆alkyl; -SO₂C₃₋₆cycloalkyl; -SO₂Aryl; -SO₂Ph; -SO₂Heteroaryl; sulfinyl; -SOCH₃; -SOC₂₋₆alkyl; -SOC₃₋₆cycloalkyl; -SOAryl; -SOPh; -SOHeteroaryl; sulfate; -OSO₂CH₃; -OSO₂C₂₋₆alkyl; -OSO₂C₃₋₆cycloalkyl; -OSO₂Aryl; -OSO₂Phenyl; -OSO₂Heteroaryl; sulfonamide; -SO₂NH₂; -SO₂NHCH₃; -SO₂NHC₂₋₆alkyl; SO₂NHC₃₋₆cycloalkyl; -SO₂NHAryl; -SO₂NHPh; -SO₂NHHeteroaryl; -SO₂N(CH₃)₂; -SO₂N(C₂₋₆alkyl)₂; -SO₂N(C₃₋₆cycloalkyl)₂; SO₂N(Aryl)₂; -SO₂N(Ph)₂; -SO₂N(Heteroaryl)₂; -SO₂N(CH₃)(C₂₋₆alkyl ); -SO₂N(CH₃)(C₃₋₆cycloalkyl); -SO₂N(CH₃)(Aryl); -SO₂N(CH₃)(Phenyl); -SO₂N(CH₃)( Heteroaryl); -SO₂N(C₂₋₆alkyl)(C₃₋₆cycloalkyl); -SO₂N(C₂₋₆alkyl)(Aryl); -SO₂N(C₂₋₆ alkyl)(Phenyl); -SO₂N(C₂₋₆alkyl)(Heteroaryl); -SO₂N(C₃₋₆cycloalkyl)(Aryl); -SO₂N(C₃₋₅cycloalkyl)(Phenyl); -SO₂N(C₃₋₆cycloalkyl)(Heteroaryl); -SO₂N(Aryl)(Phenyl); -SO₂N(Aryl)(Heteroaryl); -SO₂N (Phenyl)(heteroaryl); -NHSO₂CH₃; -NHSO₂C₂₋₆alkyl; -NHSO₂C₃₋₆cycloalkyl; -NHSO₂Aryl; -NHSO₂Phenyl; -NHSO₂Heteroaryl; -N(CH₃)SO₂CH₃; -N(CH₃)SO₂C₂₋₆alkyl; -N(CH₃)SO₂C₃₋₆cycloalkyl; -N(CH₃)SO₂Aryl; -N(CH₃)SO₂Phenyl; -N(CH₃)SO₂Heteroaryl; -N(C₂₋₆alkyl)SO₂CH₃; -N(C₂₋₆alkyl)SO₂C₂₋₆alkyl; -N(C₂₋₆alkyl)SO₂C₃₋₆cycloalkyl -N(C₂₋₆alkyl)SO₂Aryl; -N(C₂₋₆alkyl)SO₂Phenyl; -N(C₂₋₆alkyl)SO₂Heteroaryl; -N(C₃₋₆cycloalkyl)SO₂CH₃; -N(C₃₋₆cycloalkyl)SO₂C₂₋₆alkyl; -N(C₃₋₆cycloalkyl)SO₂C₃₋₆cycloalkyl; -N(C₃₋₆cycloalkyl)SO₂Aryl; -N(C₃₋₆cycloalkyl)SO₂Phenyl; -N(C₃₋₆cycloalkyl)SO₂Heteroaryl; -N(Aryl)SO₂CH₃; -N(Aryl)SO₂C₂₋₆alkyl; -N(Aryl)SO₂C₃₋₆cycloalkyl; -N(Aryl)SO₂Aryl; -N(Aryl)SO₂Phenyl; -N(Aryl)SO₂Heteroaryl; -N(Phenyl)SO₂CH₃; -N(Phenyl)SO₂C₂₋₆alkyl; -N(Phenyl)SO₂C₃₋₆cycloalkyl; -N(Phenyl)SO₂Aryl; -N(Phenyl)SO₂Phenyl; -N(Phenyl)SO₂Heteroaryl; -N(Heteroaryl)SO₂CH₃; -N(Heteroaryl)SO₂C₂₋₆alkyl; -N(Heteroaryl)SO₂C₃₋₆cycloalkyl; -N(Heteroaryl)SO₂Aryl; -N(Heteroaryl)SO₂Phenyl; -N(Heteroaryl)SO₂Heteroaryl; oxime; =NOH; =NOCH₃; =NOC₂₋₆alkyl; =NOC₃₋₆cycloalkyl; =NOAryl; =NOPhenyl; =NOHeteroaryl; -CH=NOH; -CH=NOCH₃; -CH=NOC₂₋₆alkyl; -CH=NOC₃₋₆cycloalkyl; -CH=NOAryl; -CH=NOPhenyl; -CH=NOHeteroaryl; -C(CH₃)=NOH; -C(CH₃)=NOCH₃; -C(CH₃)=NOC₂₋₆alkyl; -C(CH₃)=NOC₃₋₆cycloalkyl; -C(CH₃)=NOAryl; -C(CH₃)=NOPhenyl; -C(CH₃)=NOHeteroaryl; -C(C₂₋₆alkyl)=NOH; -C(C₂₋₆alkyl)=NOCH₃; -C(C₂₋₆alkyl)=NOC₂₋₆alkyl; -C(C₂₋₆alkyl)=NOC₃₋₆cycloalkyl; -C(C₂₋₆alkyl)=NOAryl; -C(C₂₋₆alkyl)=NOPhenyl; -C(C₂₋₆alkyl)=NOHeteroaryl; -C(C₃₋₆cycloalkyl)=NOH; -C(C₃₋₆cycloalkyl)=NOCH₃; -C(C₃₋₆cycloalkyl)=NOC₂₋₆alkyl; -C(C₃₋₆cycloalkyl)=NOC₃₋₆cycloalkyl; -C(C₃₋₆cycloalkyl)=NOAryl; -C(C₃₋₆cycloalkyl)=NOPhenyl; -C(C₃₋₆cycloalkyl)=NOHeteroaryl; -C(Aryl)=NOH; -C(Aryl)=NOCH₃; -C(Aryl)=NOC₂₋₆alkyl; -C(Aryl)=NOC₃₋₆cycloalkyl; -C(Aryl)=NOAryl; -C(Aryl)=NOPhenyl; -C(Aryl)=NOHeteroaryl; -C(Phenyl)=NOH; -C(Phenyl)=NOCH₃; -C(Phenyl)=NOC₂₋₆alkyl; -C(Phenyl)=NOC₃₋₆cycloalkyl; -C(Phenyl)=NOAryl; -C(Phenyl)=NOPhenyl; -C(Phenyl)=NOHeteroaryl; -C(Heteroaryl)=NOH; -C(Heteroaryl)=NOCH₃; -C(Heteroaryl)=NOC₂₋₆alkyl; -C(Heteroaryl)=NOC₃₋₆cycloalkyl; -C(Heteroaryl)=NOAryl; -C(Heteroaryl)=NOPhenyl; -C(Heteroaryl)=NOHeteroaryl; -ON=CH(CH₃); -ON=CH(C₂₋₆alkyl); -ON=CH(C₃₋₆cycloalkyl); -ON=CH(Aryl); -ON=CH(Phenyl); -ON=CH(heteroaryl); -ON=C(CH₃)₂; -ON=C(CH₃)(C₂₋₆alkyl); -ON=C(CH₃)(C₃₋₆cycloalkyl); -ON=C(CH₃)(Aryl); -ON=C(CH₃)(Phenyl); -ON=C(CH₃)(heteroaryl); -ON=C(C₂₋₆alkyl)₂; -ON=C(C₂₋₆alkyl) (C₃₋₆cycloalkyl); -ON=C(C₂₋₆alkyl)(Aryl); -ON=C(C₂₋₆alkyl)(Phenyl); -ON=C(C₂₋₆alkyl)(Heteroaryl); -ON=C(C₃₋₆cycloalkyl)₂; -ON=C(C₃₋₆cycloalkyl)(Aryl); -ON=C(C₃₋₆cycloalkyl)(Phenyl); -ON=C(C₃₋₆cycloalkyl)(Heteroaryl);-ON=C(Aryl)₂; -ON=C(Aryl)(Phenyl); -ON=C(Aryl)(Heteroaryl);-ON=C(Phenyl)₂; -ON=C(Phenyl)(Heteroaryl); -ON=C(Heteroaryl)₂; imine; =NH; =NCH₃; =NC₂₋₆alkyl; =NC₃₋₆cycloalkyl; =NAryl; =NPhenyl; =NHeteroaryl; -CH=NH; -CH=NCH₃; -CH=NC₂₋₆alkyl; -CH=NC₃₋₆cycloalkyl; -CH=NAryl; -CH=NPhenyl; -CH=NHeteroaryl; -C(CH₃)=NH; -C(CH₃)=NCH₃; -C(CH₃)=NC₂₋₆alkyl; -C(CH₃)=NC₃₋₆cycloalkyl; -C(CH₃)=NAryl; -C(CH₃)=NPhenyl; -C(CH₃)=NHeteroaryl; -C(C₂₋₆alkyl)=NH; -C(C₂₋₆alkyl)=NCH₃; -C(C₂₋₆alkyl)=NC₂₋₆alkyl; -C(C₂₋₆alkyl)=NC₃₋₆cycloalkyl; -C(C₂₋₆alkyl)=NAryl; -C(C₂₋₆alkyl)=NPhenyl; -C(C₂₋₆alkyl)=NHeteroaryl; -C(C₃₋₆cycloalkyl) = NH; -C(C₃₋₆cycloalkyl)=NCH₃; -C(C₃₋₆cycloalkyl)=NC₂₋₆alkyl; -C(C₃₋₆cycloalkyl)=NC₃₋₆cycloalkyl; -C(C₃₋₆cycloalkyl)=NAryl; -C(C₃₋₆cycloalkyl)=NPhenyl; -C(C₃₋₆cycloalkyl)=NHeteroaryl; -C(Aryl)=NH; -C(Aryl)=NCH₃; -C(Aryl)=NC₂₋₆alkyl; -C(Aryl)=NC₃₋₆cycloalkyl; -C(Aryl)=NAryl; -C(Aryl)=NPhenyl; -C(Aryl)=NHeteroaryl; -C(Phenyl)=NH; -C(Phenyl)=NCH₃; -C(Phenyl)=NC₂₋₆alkyl; -C(Phenyl)=NC₃₋₆cycloalkyl; -C(Phenyl)=NAryl; -C(Phenyl)=NPhenyl; -C(Phenyl)=NHeteroaryl; -C(Heteroaryl)=NH; -C(Heteroaryl)=NCH₃; -C(Heteroaryl)=NC₂₋₆alkyl; -C(Heteroaryl)=NC₃. ₆cycloalkyl; -C(Heteroaryl)=NAryl; -C(Heteroaryl)=NPhenyl; -C(Heteroaryl)=NHeteroaryl; -N=CH(CH₃); -N=CH(C₂₋₆alkyl); -N=CH(C₃₋₆cycloalkyl); -N=CH(Aryl); -N=CH(Phenyl); -N=CH(heteroaryl); -N=C(CH₃)₂; -N=C(CH₃)(C₂₋₆alkyl); -N=C(CH₃)(C₃₋₆cycloalkyl); -N=C(CH₃)(Aryl); -N=C(CH₃)(Phenyl); -N=C(CH₃)(heteroaryl); -N=C(C₂₋₆alkyl)₂; -N=C(C₂₋₆alkyl) (C₃₋₆cycloalkyl); -N=C(C₂₋₆alkyl)(Aryl); -N=C(C₂₋₆alkyl)(Phenyl); -N=C(C₂₋₆alkyl)(Heteroaryl); -N=C(C₃₋₆cycloalkyl)₂; -N=C(C₃₋₆cycloalkyl)(Aryl); -N=C(C₃₋₆cycloalkyl)(Phenyl); -N=C(C₃₋₆cycloalkyl)(Heteroaryl);-N=C(Aryl)₂; -N=C(Aryl)(Phenyl); -N=C(Aryl)(Heteroaryl);-N=C(Phenyl)₂; -N=C(Phenyl)(Heteroaryl); -N=C(Heteroaryl)₂; ketone; acetyl; -C(O)CH₃; -C(O)C₂₋₆alkyl; -C(O)C₃₋₆cycloalkyl; -C(O)Aryl; -C(O)Phenyl; -C(O)Heteroaryl; -CH₂C(O)CH₃; -CH₂C(O)C₂₋₆alkyl; -CH₂C(O)C₃₋₆cycloalkyl; -CH₂C(O)Aryl; -CH₂C(O)Phenyl; -CH₂C(O)Heteroaryl; -C₂₋₆alkylC(O)C₂₋₆alkyl; -C₂₋₆alkylC(O)C₃₋₆cycloalkyl; - C₂₋₆alkyl C(O)Aryl; -C₂₋₆alkylC(O)Phenyl; -C₂₋₆alkylC(O)Heteroaryl; -C(O)CH₂CH₃; -C(O)CH₂C₂₋₆alkyl; -C(O)CH₂C₃₋ₑcycloalkyl; -C(O)CH₂Aryl; -C(O)CH₂Phenyl; -C(O)CH₂Heteroaryl; -C(O)C₂₋₆alkylCH₃; -C(O)C₂₋₆alkylC₂₋₆alkyl; -C(O)C₂₋₆alkylC₃₋₆cycloalkyl; -C(O)C₂₋₆alkylAryl; -C(O)C₂₋₆alkylPhenyl; -C(O)C₂₋₆alkylHeteroaryl; aldehyde; -CHO; -CH₂CHO; -C₂₋₆alkylCHO; ester; -CO₂CH₃; -CO₂C₂₋₆alkyl; -CO₂C₃₋₆cycloalkyl; -CO₂Aryl; -CO₂Phenyl; -CO₂Heteroaryl; reverse ester; acetoxy; -OCOCH₃; -OCOC₂₋₆alkyl; -OCOC₃₋₆cycloalkyl; -OCOAryl; -OCOPhenyl; -OCOHeteroaryl;oxygen (=O); hydrazinyl; -NHNH₂;-N(CH₃)NH₂; -N(C₂₋₆alkyl)NH₂; -N(C₃₋₆cycloalkyl)NH₂; -N(Aryl)NH₂; -N(Phenyl)NH₂; -N(Heteroaryl)NH₂; -N(COCH₃)NH₂; -N(COC₂₋₆alkyl)NH₂; -N(COC₃₋₆cycloalkyl)NH₂; -N(COAryl)NH₂; -N(COPhenyl)NH₂; -N(COHeteroaryl)NH₂; -NHNH(CH₃); -NHNH(C₂₋₆alkyl); -NHNH(C₃₋₆cycloalkyl); -NHNH(Aryl); -NHNH(Phenyl); -NHNH(Heteroaryl); -NHNH(COCH₃); -NHNH(COC₂₋₆alkyl); -NHNH(COC₃₋₆cycloalkyl); -NHNH(COAryl); -NHNH(COPhenyl); -NHNH(COHeteroaryl);-N(CH₃)NH(CH₃); -N(CH₃)NH(C₂₋₆alkyl); -N(CH₃)NH(C₃₋₆cycloalkyl); -N(CH₃)NH(Aryl); -N(CH₃)NH(Phenyl); -N(CH₃)NH(Heteroaryl); -N(CH₃)NH(COCH₃); -N(CH₃)NH(COC₂₋₆alkyl); -N(CH₃)NH(COC₃₋₆cycloalkyl); -N(CH₃)NH(COAryl); -N(CH₃)NH(COPhenyl); -N(CH₃)NH(COHeteroaryl);-N(C₂₋₆alkyl)NH(CH₃); -N(C₂₋₆alkyl)NH(C₂₋₆alkyl); -N(C₂₋₆alkyl)NH(C₃₋₆cycloalkyl); -N(C₂₋₆alkyl)NH(Aryl); -N(C₂₋₆alkyl)NH(Phenyl); -N(C₂₋₆alkyl)NH(Heteroaryl); -N(C₂₋₆alkyl)NH(COCH₃); -N(C₂₋₆alkyl)NH(COC₂₋₆alkyl); -N(C₂₋₆alkyl)NH(COC₃₋₆cycloalkyl); -N(C₂₋₆alkyl)NH(COAryl); -N(C₂₋₆alkyl)NH(COPhenyl); -N(C₂₋₆alkyl)NH(COHeteroaryl);-N(C₃₋₆cycloalkyl)NH(CH₃); -N(C₃₋₆cycloalkyl)NH(C₂₋₆alkyl); -N(C₃₋₆cycloalkyl)NH(C₃₋₆cycloalkyl); -N(C₃₋₆cycloalkyl)NH(Aryl); -N(C₃₋₆cycloalkyl)NH(Phenyl); -N(C₃₋₆cycloalkyl)NH (Heteroaryl); -N(C₃₋₆cycloalkyl)NH(COCH₃); -N(C₃₋₆cycloalkyl)NH(COC₂₋₆alkyl); -N(C₃₋₆cycloalkyl)NH(COC₃₋₆cycloalkyl); -N(C₃₋₆cycloalkyl)NH(COAryl); -N(C₃₋₆cycloalkyl)NH(COPhenyl); -N(C₃₋₆cycloalkyl)NH(COHeteroaryl); -N(Aryl)NH(CH₃); -N(Aryl)NH(C₂₋₆alkyl); -N(Aryl)NH(C₃₋₆cycloalkyl); -N(Aryl)NH(Aryl); -N(Aryl)NH(Phenyl); -N(Aryl)NH(Heteroaryl); -N(Aryl)NH(COCH₃); -N(Aryl)NH(COC₂₋₆alkyl); -N(Aryl)NH(COC₃₋₆cycloalkyl); -N(Aryl)NH(COAryl); -N(Aryl)NH(COPhenyl); -N(Aryl)NH(COHeteroaryl);-N(Phenyl)NH(CH₃); -N(Phenyl)NH(C₂₋₆alkyl); -N(Phenyl)NH(C₃₋₆cycloalkyl); -N(Phenyl)NH(Aryl); -N(Phenyl)NH(Phenyl); -N(Phenyl)NH(Heteroaryl); -N(Phenyl)NH(COCH₃); -N(Phenyl)NH(COC₂₋₆alkyl); -N(Phenyl)NH(COC₃₋₆cycloalkyl); -N(Phenyl)NH(COAryl); -N(Phenyl)NH(COPhenyl); -N(Phenyl)NH(COHeteroaryl); -N(Heteroaryl)NH(CH₃); -N(Heteroaryl)NH(C₂₋₆alkyl); -N(Heteroaryl)NH(C₃₋₆cycloalkyl); -N(Heteroaryl)NH(Aryl); -N(Heteroaryl)NH(Phenyl); -N(Heteroaryl)NH(Heteroaryl); -N(Heteroaryl)NH(COCH₃); -N(Heteroaryl)NH(COC₂₋₆alkyl); -N(Heteroaryl)NH(COC₃₋₆cycloalkyl); -N(Heteroaryl)NH(COAryl); -N(Heteroaryl)NH(COPhenyl); -N(Heteroaryl)NH(COHeteroaryl);-N(COCH₃)NH(CH₃); -N(COCH₃)NH(C₂₋₆alkyl); -N(COCH₃)NH(C₃₋₆cycloalkyl); -N(COCH₃)NH(Aryl); -N(COCH₃)NH(Phenyl); -N(COCH₃)NH(Heteroaryl); -N(COCH₃)NH(COCH₃); -N(COCH₃)NH(COC₂₋₆alkyl); -N(COCH₃)NH(COC₃₋₆cycloalkyl); -N(COCH₃)NH(COAryl); -N(COCH₃)NH(COPhenyl); -N(COCH₃)NH(COHeteroaryl); -N(COC₂₋₆alkyl)NH(CH₃); -N(COC₂₋₆alkyl)NH(C₂₋₆alkyl); -N(COC₂₋₆alkyl)NH(C₃₋₆cycloalkyl); -N(COC₂₋₆alkyl)NH(Aryl); -N(COC₂₋₆alkyl)NH(Phenyl); -N(COC₂₋₆alkyl)NH(Heteroaryl); -N(COC₂₋₆alkyl)NH(COCH₃); -N(COC₂₋₆alkyl)NH(COC₂₋₆alkyl); -N(COC₂₋₆alkyl)NH(COC₃₋₆cycloalkyl); -N(COC₂₋₆alkyl)NH(COAryl); -N(COC₂₋₆alkyl)NH(COPhenyl); -N(COC₂₋₆alkyl)NH(COHeteroaryl);-N(COC₃₋₆cycloalkyl)NH(CH₃); -N(COC₃₋₆cycloalkyl)NH(C₂₋₆alkyl); -N(COC₃₋₆cycloalkyl)NH(C₃₋₆cycloalkyl); -N(COC₃₋₆cycloalkyl)NH(Aryl); -N(COC₃₋₆cycloalkyl)NH(Phenyl); -N(COC₃₋₆cycloalkyl)NH(Heteroaryl); -N(COC₃₋₆cycloalkyl)NH(COCH₃); -N(COC₃₋₆cycloalkyl)NH(COC₂₋₆alkyl); -N(COC₃₋₆cycloalkyl)NH(COC₃₋₆cycloalkyl); -N(COC₃₋₆cycloalkyl)NH(COAryl); -N(COC₃₋₆cycloalkyl)NH(COPhenyl); -N(COC₃₋₆cycloalkyl)NH(COHeteroaryl); -N(COAryl)NH(CH₃); -N(COAryl)NH(C₂₋₆alkyl); -N(COAryl)NH(C₃₋₆cycloalkyl); -N(COAryl)NH(Aryl); -N(COAryl)NH(Phenyl); -N(COAryl)NH(Heteroaryl); -N(COAryl)NH(COCH₃); -N(COAryl)NH(COC₂₋₆alkyl); -N(COAryl)NH(COC₃₋₆cycloalkyl); -N(COAryl)NH(COAryl); -N(COAryl)NH(COPhenyl); -N(COAryl)NH(COHeteroaryl);-N(COPhenyl)NH(CH₃); -N(COPhenyl)NH(C₂₋₆alkyl); -N(COPhenyl)NH(C₃₋₆cycloalkyl); -N(COPhenyl)NH(Aryl); -N(COPhenyl)NH(Phenyl); -N(COPhenyl)NH(Heteroaryl); -N(COPhenyl)NH(COCH₃); -N(COPhenyl)NH(COC₂₋₆alkyl); -N(COPhenyl)NH(COC₃₋₆cycloalkyl); -N(COPhenyl)NH(COAryl); -N(COPhenyl)NH(COPhenyl); -N(COPhenyl)NH(COHeteroaryl); -N(COHeteroaryl)NH(CH₃); -N(COHeteroaryl)NH(C₂₋₆alkyl); -N(COHeteroaryl)NH(C₃₋₆cycloalkyl); -N(COHeteroaryl)NH(Aryl); -N(COHeteroaryl)NH(Phenyl); -N(COHeteroaryl)NH(Heteroaryl); -N(COHeteroaryl)NH(COCH₃); -N(COHeteroaryl)NH(COC₂₋₆alkyl); -N(COHeteroaryl)NH(COC₃₋₆cycloalkyl); -N(COHeteroaryl)NH(COAryl); -N(COHeteroaryl)NH(COPhenyl); -N(COHeteroaryl)NH(COHeteroaryl); -NHN(CH₃)₂; -NHN(CH₃)(C₂₋₆alkyl); -NHN(CH₃)(C₃₋₆cycloalkyl); -NHN(CH₃)(Aryl); -NHN(CH₃)(Phenyl); -NHN(CH₃)(Heteroaryl); -NHN(CH₃)(COCH₃); -NHN(CH₃)(COC₂₋₆alkyl); -NHN(CH₃)(COC₃₋₆cycloalkyl); -NHN(CH₃)(COAryl); -NHN(CH₃)(COPhenyl); -NHN(CH₃)(COHeteroaryl);-NHN(C₂₋₆alkyl)₂; -NHN(C₂₋₆alkyl)(C₃₋₆cycloalkyl); -NHN(C₂₋₆alkyl)(Aryl); -NHN(C₂₋₆alkyl)(Phenyl); -NHN(C₂₋₆alkyl)(Heteroaryl); -NHN(C₂₋₆alkyl)(COCH₃); -NHN(C₂₋₆alkyl)(COC₂₋₆alkyl); -NHN(C₂₋₆alkyl)(COC₃₋₆cycloalkyl); -NHN(C₂₋₆alkyl)(COAryl); -NHN(C₂₋₆alkyl)(COPhenyl); -NHN(C₂₋₆alkyl)(COHeteroaryl);-NHN(C₃₋₆cycloalkyl)₂; -NHN(C₃₋₆cycloalkyl)(Aryl); -NHN(C₃₋₆cycloalkyl)(Phenyl); -NHN(C₃₋₆cycloalkyl)(Heteroaryl); -NHN(C₃₋₆cycloalkyl)(COCH₃); -NHN(C₃₋₆cycloalkyl)(COC₂₋₆alkyl); -NHN(C₃₋₆cycloalkyl)(COC₃₋₆cycloalkyl); -NHN(C₃₋₆cycloalkyl)(COAryl); -NHN(C₃₋₆cycloalkyl)(COPhenyl); -NHN(C₃₋₆cycloalkyl)(COHeteroaryl);-NHN(Aryl)₂; -NHN(Aryl)(Phenyl); -NHN(Aryl)(Heteroaryl); -NHN(Aryl)(COCH₃); -NHN(Aryl)(COC₂₋₆alkyl); -NHN(Aryl)(COC₃₋₆cycloalkyl); -NHN(Aryl)(COAryl); -NHN(Aryl)(COPhenyl); -NHN(Aryl)(COHeteroaryl); -NHN(Phenyl)₂; -NHN(Phenyl)(Heteroaryl); -NHN(Phenyl)(COCH₃); -NHN(Phenyl)(COC₂₋₆alkyl); -NHN(Phenyl)(COC₃₋₆cycloalkyl); -NHN(Phenyl)(COAryl); -NHN(Phenyl)(COPhenyl); -NHN(Phenyl)(COHeteroaryl);-NHN(Heteroaryl)₂; -NHN(Heteroaryl)(COCH₃); -NHN(Heteroaryl)(COC₂₋₆alkyl); -NHN(Heteroaryl)(COC₃₋₆cycloalkyl); -NHN(Heteroaryl)(COAryl); -NHN(Heteroaryl)(COPhenyl); -NHN(Heteroaryl)(COHeteroaryl); -NHN(COCH₃)₂; -NHN(COCH₃)(COC₂₋₆alkyl); -NHN(COCH₃)(COC₃₋₆cycloalkyl); -NHN(COCH₃)(COAryl); -NHN(COCH₃)(COPhenyl); -NHN(COCH₃)(COHeteroaryl);-NHN(COC₂₋₆alkyl)₂; -NHN(COC₂₋₆alkyl)(COC₃₋₆cycloalkyl); -NHN(COC₂₋₆alkyl)(COAryl); -NHN(COC₂₋₆alkyl)(COPhenyl); -NHN(COC₂₋₆alkyl)(COHeteroaryl); -NHN(COC₃₋₆cycloalkyl)₂; -NHN(COC₃₋₆cycloalkyl)(COAryl); -NHN(COC₃₋₆cycloalkyl)(COPhenyl); -NHN(COC₃₋₆cycloalkyl)(COHeteroaryl); -NHN(COAryl)₂; -NHN(COAryl)(COPhenyl); -NHN(COAryl)(COHeteroaryl); -NHN(COPhenyl)₂; -NHN(COPhenyl)(COHeteroaryl);-NHN(COHeteroaryl)₂; -N(CH₃)N(CH₃)₂; -N(CH₃)N(CH₃)(C₂₋₆alkyl); -N(CH₃)N(CH₃)(C₃₋₆cycloalkyl); -N(CH₃)N(CH₃)(Aryl); -N(CH₃)N(CH₃)(Phenyl); -N(CH₃)N(CH₃)(Heteroaryl); -N(CH₃)N(CH₃)(COCH₃); -N(CH₃)N(CH₃)(COC₂₋₆alkyl); -N(CH₃)N(CH₃)(COC₃₋₆cycloalkyl); -N(CH₃)N(CH₃)(COAryl); -N(CH₃)N(CH₃)(COPhenyl); -N(CH₃)N(CH₃)(COHeteroaryl);-N(CH₃)N(C₂₋₆alkyl)₂; -N(CH₃)N(C₂₋₆alkyl)(C₃₋₆cycloalkyl); -N(CH₃)N(C₂₋₆alkyl)(Aryl); -N(CH₃)N(C₂₋₆alkyl)(Phenyl); -N(CH₃)N(C₂₋₆alkyl)(Heteroaryl); -N(CH₃)N(C₂₋₆alkyl)(COCH₃); -N(CH₃)N(C₂₋₆alkyl)(COC₂₋₆alkyl); -N(CH₃)N(C₂₋₆alkyl)(COC₃₋₆cycloalkyl); -N(CH₃)N(C₂₋₆alkyl)(COAryl); -N(CH₃)N(C₂₋₆alkyl)(COPhenyl); -N(CH₃)N(C₂₋₆alkyl)(COHeteroaryl);-N(CH₃)N(C₃₋₆cycloalkyl)₂; -N(CH₃)N(C₃₋₆cycloalkyl)(Aryl); -N(CH₃)N(C₃₋₆cycloalkyl)(Phenyl); -N(CH₃)N(C₃₋₆cycloalkyl)(Heteroaryl); -N(CH₃)N(C₃₋₆cycloalkyl)(COCH₃); -N(CH₃)N(C₃₋₆cycloalkyl)(COC₂₋₆alkyl); -N(CH₃)N(C₃₋₆cycloalkyl)(COC₃₋₆cycloalkyl); -N(CH₃)N(C₃₋₆cycloalkyl)(COAryl); -N(CH₃)N(C₃₋₆cycloalkyl)(COPhenyl); -N(CH₃)N(C₃₋₆cycloalkyl)(COHeteroaryl); -N(CH₃)N(Aryl)₂; -N(CH₃)N(Aryl)(Phenyl); -N(CH₃)N(Aryl)(Heteroaryl); -N(CH₃)N(Aryl)(COCH₃); -N(CH₃)N(Aryl)(COC₂₋₆alkyl); -N(CH₃)N(Aryl)(COC₃₋₆cycloalkyl); -N(CH₃)N(Aryl)(COAryl); -N(CH₃)N(Aryl)(COPhenyl); -N(CH₃)N(Aryl)(COHeteroaryl); -N(CH₃)N(Phenyl)₂; -N(CH₃)N(Phenyl)(Heteroaryl); -N(CH₃)N(Phenyl)(COCH₃); -N(CH₃)N(Phenyl)(COC₂₋₆alkyl); -N(CH₃)N(Phenyl)(COC₃₋₆cycloalkyl); -N(CH₃)N(Phenyl)(COAryl); -N(CH₃)N(Phenyl)(COPhenyl); -N(CH₃)N(Phenyl)(COHeteroaryl); -N(CH₃)N(Heteroaryl)₂; -N(CH₃)N(Heteroaryl)(COCH₃); -N(CH₃)N(Heteroaryl)(COC₂₋₆alkyl); -N(CH₃)N(Heteroaryl)(COC₃₋₆cycloalkyl); -N(CH₃)N(Heteroaryl)(COAryl); -N(CH₃)N(Heteroaryl)(COPhenyl); -N(CH₃)N(Heteroaryl)(COHeteroaryl); -N(CH₃)N(COCH₃)₂; -N(CH₃)N(COCH₃)(COC₂₋₆alkyl); -N(CH₃)N(COCH₃)(COC₃₋₆cycloalkyl); -N(CH₃)N(COCH₃)(COAryl); -N(CH₃)N(COCH₃)(COPhenyl); -N(CH₃)N(COCH₃)(COHeteroaryl); -N(CH₃)N(COC₂₋₆alkyl)₂; -N(CH₃)N(COC₂₋₆alkyl)(COC₃₋₆cycloalkyl); -N(CH₃)N(COC₂₋₆alkyl)(COAryl); -N(CH₃)N(COC₂₋₆alkyl)(COPhenyl); -N(CH₃)N(COC₂₋₆alkyl)(COHeteroaryl);-N(CH₃)N(COC₃₋₆cycloalkyl)₂; -N(CH₃)N(COC₃₋₆cycloalkyl)(COAryl); -N(CH₃)N(COC₃₋₆cycloalkyl)(COPhenyl); -N(CH₃)N(COC₃₋₆cycloalkyl)(COHeteroaryl); -N(CH₃)N(COAryl)₂; -N(CH₃)N(COAryl)(COPhenyl); -N(CH₃)N(COAryl)(COHeteroaryl); -N(CH₃)N(COPhenyl)₂; -N(CH₃)N(COPhenyl)(COHeteroaryl); -N(CH₃)N(COHeteroaryl)₂; -N(Phenyl)N(CH₃)₂; -N(Phenyl)N(CH₃)(C₂₋₆alkyl); -N(Phenyl)N(CH₃)(C₃₋₆cycloalkyl); -N(Phenyl)N(CH₃)(Aryl); -N(Phenyl)N(CH₃)(Phenyl); -N(Phenyl)N(CH₃)(Heteroaryl); -N(Phenyl)N(CH₃)(COCH₃); -N(Phenyl)N(CH₃)(COC₂₋₆alkyl); -N(Phenyl)N(CH₃)(COC₃₋₆cycloalkyl); -N(Phenyl)N(CH₃)(COAryl); -N(Phenyl)N(CH₃)(COPhenyl); -N(Phenyl)N(CH₃)(COHeteroaryl); -N(Phenyl)N(C₂₋₆alkyl)₂; -N(Phenyl)N(C₂₋₆alkyl)(C₃₋₆cycloalkyl); -N(Phenyl)N(C₂₋₆alkyl)(Aryl); -N(Phenyl)N(C₂₋₆alkyl)(Phenyl); -N(Phenyl)N(C₂₋₆alkyl)(Heteroaryl); -N(Phenyl)N(C₂₋₆alkyl)(COCH₃); -N(Phenyl)N(C₂₋₆alkyl)(COC₂₋₆alkyl); -N(Phenyl)N(C₂₋₆alkyl)(COC₃₋₆cycloalkyl); -N(Phenyl)N(C₂₋₆alkyl)(COAryl); -N(Phenyl)N(C₂₋₆alkyl)(COPhenyl); -N(Phenyl)N(C₂₋₆alkyl)(COHeteroaryl); -N(Phenyl)N(C₃₋₆cycloalkyl)₂; -N(Phenyl)N(C₃₋₆cycloalkyl)(Aryl); -N(Phenyl)N(C₃₋₆cycloalkyl)(Phenyl); -N(Phenyl)N(C₃₋₆cycloalkyl)(Heteroaryl); -N(Phenyl)N(C₃₋₆cycloalkyl)(COCH₃); -N(Phenyl)N(C₃₋₆cycloalkyl)(COC₂₋₆alkyl); -N(Phenyl)N(C₃₋₆cycloalkyl)(COC₃₋₆cycloalkyl); -N(Phenyl)N(C₃₋₆cycloalkyl)(COAryl); -N(Phenyl)N(C₃₋₆cycloalkyl)(COPhenyl); -N(Phenyl)N(C₃₋₆cycloalkyl)(COHeteroaryl);-N(Phenyl)N(Aryl)₂; -N(Phenyl)N(Aryl)(Phenyl); -N(Phenyl)N(Aryl)(Heteroaryl); -N(Phenyl)N(Aryl)(COCH₃); -N(Phenyl)N(Aryl)(COC₂₋₆alkyl); -N(Phenyl)N(Aryl)(COC₃₋₆cycloalkyl); -N(Phenyl)N(Aryl)(COAryl); -N(Phenyl)N(Aryl)(COPhenyl); -N(Phenyl)N(Aryl)(COHeteroaryl); -N(Phenyl)N(Phenyl)₂; -N(Phenyl)N(Phenyl)(Heteroaryl); -N(Phenyl)N(Phenyl)(COCH₃); -N(Phenyl)N(Phenyl)(COC₂₋₆alkyl); -N(Phenyl)N(Phenyl)(COC₃₋₆cycloalkyl); -N(Phenyl)N(Phenyl)(COAryl); -N(Phenyl)N(Phenyl)(COPhenyl); -N(Phenyl)N(Phenyl)(COHeteroaryl); -N(Phenyl)N(Heteroaryl)₂; -N(Phenyl)N(Heteroaryl)(COCH₃); -N(Phenyl)N(Heteroaryl)(COC₂₋₆alkyl); -N(Phenyl)N(Heteroaryl)(COC₃₋₆cycloalkyl); -N(Phenyl)N(Heteroaryl)(COAryl); -N(Phenyl)N(Heteroaryl)(COPhenyl); -N(Phenyl)N(Heteroaryl)(COHeteroaryl); -N(Phenyl)N(COCH₃)₂; -N(Phenyl)N(COCH₃)(COC₂₋₆alkyl); -N(Phenyl)N(COCH₃)(COC₃₋₆cycloalkyl); -N(Phenyl)N(COCH₃)(COAryl); -N(Phenyl)N(COCH₃)(COPhenyl); -N(Phenyl)N(COCH₃)(COHeteroaryl); -N(Phenyl)N(COC₂₋₆alkyl)₂; -N(Phenyl)N(COC₂₋₆alkyl)(COC₃₋₆cycloalkyl); -N(Phenyl)N(COC₂₋₆alkyl)(COAryl); -N(Phenyl)N(COC₂₋₆alkyl)(COPhenyl); -N(Phenyl)N(COC₂₋₆alkyl)(COHeteroaryl); -N(Phenyl)N(COC₃₋₆cycloalkyl)₂; -N(Phenyl)N(COC₃₋₆cycloalkyl)(COAryl); -N(Phenyl)N(COC₃₋₆cycloalkyl)(COPhenyl); -N(Phenyl)N(COC₃₋₆cycloalkyl)(COHeteroaryl); -N(Phenyl)N(COAryl)₂; -N(Phenyl)N(COAryl)(COPhenyl); -N(Phenyl)N(COAryl)(COHeteroaryl); -N(Phenyl)N(COPhenyl)₂; -N(Phenyl)N(COPhenyl)(COHeteroaryl); -N(Phenyl)N(COHeteroaryl)₂; hydrazonyl; =NNH₂; =NNH(CH₃); =NNH(C₂₋₆alkyl); =NNH(C₃₋₆cycloalkyl); =NNH(Aryl); =NNH(Phenyl); =NNH(Heteroaryl); =NNH(COCH₃); =NNH(COC₂₋₆alkyl); =NNH(COC₃₋₆cycloalkyl); =NNH(COAryl); =NNH(COPhenyl); =NNH(COHeteroaryl);=NN(CH₃)₂; =NN(CH₃)(C₂₋₆alkyl); =NN(CH₃)(C₃₋₆cycloalkyl); =NN(CH₃)(Aryl); =NN(CH₃)(Phenyl); =NN(CH₃)(Heteroaryl); =NN(CH₃)(COCH₃); =NN(CH₃)(COC₂₋₆alkyl); =NN(CH₃)(COC₃₋₆cycloalkyl); =NN(CH₃)(COAryl); =NN(CH₃)(COPhenyl); =NN(CH₃)(COHeteroaryl);=NN(C₂₋₆alkyl)₂; =NN(C₂-₆alkyl)(C₃₋₆cycloalkyl); =NN(C₂₋₆alkyl)(Aryl); =NN(C₂₋₆alkyl)(Phenyl); =NN(C₂₋₆alkyl)(Heteroaryl); =NN(C₂₋₆alkyl)(COCH₃); =NN(C₂₋₆alkyl)(COC₂₋₆alkyl); =NN(C₂₋₆alkyl)(COC₃₋₆cycloalkyl); =NN(C₂₋₆alkyl)(COAryl); =NN(C₂₋₆alkyl)(COPhenyl); =NN(C₂₋₆alkyl)(COHeteroaryl); =NN(C₃₋₆cycloalkyl)₂; =NN(C₃₋₆cycloalkyl)(Aryl); =NN(C₃₋₆cycloalkyl)(Phenyl); =NN(C₃₋₆cycloalkyl)(Heteroaryl); =NN(C₃₋₆cycloalkyl)(COCH₃); =NN(C₃₋₆cycloalkyl)(COC₂₋₆alkyl); =NN(C₃₋₆cycloalkyl)(COC₃₋₆cycloalkyl); =NN(C₃₋₆cycloalkyl)(COAryl); =NN(C₃₋₆cycloalkyl)(COPhenyl); =NN(C₃₋₆cycloalkyl)(COHeteroaryl); =NN(Aryl)₂; =NN(Aryl)(Phenyl); =NN(Aryl)(Heteroaryl); =NN(Aryl)(COCH₃); =NN(Aryl)(COC₂₋₆alkyl); =NN(Aryl)(COC₃₋₆cycloalkyl); =NN(Aryl)(COAryl); =NN(Aryl)(COPhenyl); =NN(Aryl)(COHeteroaryl);=NN(Phenyl)₂; =NN(Phenyl)(Heteroaryl); =NN(Phenyl)(COCH₃); =NN(Phenyl)(COC₂₋₆alkyl); =NN(Phenyl)(COC₃₋₆cycloalkyl); =NN(Phenyl)(COAryl); =NN(Phenyl)(COPhenyl); =NN(Phenyl)(COHeteroaryl);=NN(Heteroaryl)₂; =NN(Heteroaryl)(COCH₃); =NN(Heteroaryl)(COC₂₋₆alkyl); =NN(Heteroaryl)(COC₃₋₆cycloalkyl); =NN(Heteroaryl)(COAryl); =NN(Heteroaryl)(COPhenyl); =NN(Heteroaryl)(COHeteroaryl);=NN(COCH₃)₂; =NN(COCH₃)(COC₂₋₆alkyl); =NN(COCH₃)(COC₃₋₆cycloalkyl); =NN(COCH₃)(COAryl); =NN(COCH₃)(COPhenyl); =NN(COCH₃)(COHeteroaryl); =NN(COC₂₋₆alkyl)₂; =NN(COC₂₋₆alkyl)(COC₃₋₆cycloalkyl); =NN(COC₂₋₆alkyl)(COAryl); =NN(COC₂₋₆alkyl)(COPhenyl); =NN(COC₂₋₆alkyl)(COHeteroaryl);=NN(COC₃₋₆cycloalkyl)₂; =NN(COC₃₋₆cycloalkyl)(COAryl); =NN(COC₃₋₆cycloalkyl)(COPhenyl); =NN(COC₃₋₆cycloalkyl)(COHeteroaryl); =NN(COAryl)₂; =NN(COAryl)(COPhenyl); =NN(COAryl)(COHeteroaryl); =NN(COPhenyl)₂;=NN(COPhenyl)(COHeteroaryl); =NN(COHeteroaryl)₂; haloalkyl (e.g., trifluoromethyl, difluoromethyl, fluoromethyl); substituted aminoacyl and aminoalkyl; carbocyclic C₃₋₈cycloalkyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic (e.g., pyrrolidinyl, piperidinyl, piperazinyl, morpholinyl, furanyl, or thiazinyl); carbocyclic or heterocyclic, monocyclic or fused or non-fused polycyclic aryl (e.g., phenyl, naphthyl, pyrrolyl, indolyl, furanyl, thienyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothienyl, or benzofuranyl); -O-aryl; aryl; aryl-C_{1- or 2-6}alkyl; alkoxy; -OCH₃; -OC₂₋₆alkyl; -OC₃₋₆cycloalkyl; -OPhenyl; -OPhenyl-C_{1- or 2-6}alkyl; -OHeteroaryl; -CO₂CH₃;- CONH₂; -OCH₂CONH₂; -NH₂; -N(C₁₋₄alkyl)₂; amido;-NHC(O)CH₃; -NHC(O)C_{2- 6}alkyl; -NHC(O)C_{3- 6}cycloalkyl; -NHC(O)Aryl; -NHC(O)Phenyl; -NHC(O)Heteroaryl;-N(CH₃)C(O)CH₃; -N(CH₃)C(O)C_{2- 6}alkyl; -N(CH₃)C(O)C_{3- 6}cycloalkyl; -N(CH₃)C(O)Aryl; -N(CH₃)C(O)Phenyl; -N(CH₃)C(O)Heteroaryl; -N(C_{2- 6}alkyl)C(O)CH₃; -N(C_{2- 6}alkyl)C(O)C_{2- 6}alkyl; -N(C_{2- 6}alkyl)C(O)C_{3- 6}cycloalkyl; -N(C_{2- 6}alkyl)C(O)Aryl; -N(C₂₋₆alkyl)C(O)Phenyl; -N(C_{2- 6}alkyl)C(O)Heteroaryl;-N(C_{3- 6}cycloalkyl)C(O)CH₃; -N(C_{3- 6}cycloalkyl)C(O)C_{2- 6}alkyl; -N(C_{3- 6}cycloalkyl)C(O)C_{3- 6}cycloalkyl; -N(C₃₋₆cycloalkyl)C(O)Aryl; -N(C_{3- 6}cycloalkyl)C(O)Phenyl; -N(C₃₋₆cycloalkyl)C(O)Heteroaryl;-N(Aryl)C(O)CH₃; -N(Aryl)C(O)C_{2- 6}alkyl; -N(Aryl)C(O)C_{3- 6}cycloalkyl; -N(Aryl)C(O)Aryl; -N(Aryl)C(O)Phenyl; -N(Aryl)C(O)Heteroaryl;-N(Phenyl)C(O)CH₃; -N(Phenyl)C(O)C_{2- 6}alkyl; -N(Phenyl)C(O)C_{3- 6}cycloalkyl; -N(Phenyl)C(O)Aryl; -N(Phenyl)C(O)Phenyl; -N(Phenyl)C(O)Heteroaryl;-N(Heteroaryl)C(O)CH₃; -N(Heteroaryl)C(O)C₂₋₆alkyl; -N(Heteroaryl)C(O)C_{3- 6}cycloalkyl; -N(Heteroaryl)C(O)Aryl; -N(Heteroaryl)C(O)Phenyl; -N(Heteroaryl)C(O)Heteroaryl; guanidyl; amidinyl; -SO₂NH₂; -OCHF₂; -CF₃; -OCF₃; and such moieties may also be optionally substituted by a fused-ring structure or bridge, for example -OCH₂O- or -O- C_{1- or 2-6}alkylene-O-. These substituents may optionally be further substituted with a substituent selected from such groups.

Representative compounds of the invention which may be mentioned are those provided in the Examples as the free base or a pharmaceutically acceptable salt thereof. The molecular weight of the compounds of the invention (in free base form) is preferably less than 1000 g/mol, more preferably less than 900 g/mol, most preferably less than 800 g/mol.

When the compound of the invention and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (both cupric and cuprous), ferric, ferrous, lithium, magnesium, potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include arginine, betaine, caffeine, choline, N',N'- dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Where the compounds of the invention are intended for pharmaceutical use they are preferably provided in substantially pure form, for example at least 60% pure, more suitably at least 75% pure, especially at least 98% pure (% are on a weight for weight basis).

The invention also provides a compound of the invention or pharmaceutically acceptable salt thereof for use as a pharmaceutical or mediciament. The invention also provides a pharmaceutical composition comprising at least one compound of the invention or a pharmaceutically acceptable salt thereof and a pharmaceutically acceptable carrier. The invention further provides a pharmaceutical composition comprising at least one compound of the invention or a pharmaceutically acceptable salt thereof and a pharmaceutically acceptable carrier, diluent or excipient. Preferably these compositions are comprised of a pharmaceutically acceptable carrier or pharmaceutically acceptable carrier, diluent or excipient and a non-toxic therapeutically effective amount of said at least one compound of the invention, or a pharmaceutically acceptable salt thereof.

The compounds of the invention may be used in combination with further antibacterial agents. Accordingly, the present invention further provides a composition comprising at least one compound of the invention or pharmaceutically acceptable salt thereof and at least one further antibacterial agent, wherein said at least one further antibacterial agent is different to the compound of the invention or pharmaceutically acceptable salt thereof. Preferably, said at least one further antibacterial agent is a regulatory approved antibiotic, in particular a regulatory approved member of a family of antibiotics selected from the group consisting of penicillin antibiotics, cephalosporin antibiotics, carbapenem antibiotics, monobactam antibiotics, polymyxin antibiotics, rifamycin antibiotics, lipiarmycin antibiotics, quinolone antibiotics, sulfonamide antibiotics, macrolide antibiotics, lincosamide antibiotics, tetracycline antibiotics, aminoglycoside antibiotics, lipopeptide antibiotics, glycylcycline antibiotics, glycopeptide antibiotics, oxazolidinone antibiotics, and lipiarmycin. Where the invention relates to a composition comprising at least one compound of the invention or pharmaceutically acceptable salt thereof and at least one further antibacterial agent, the at least one further antibacterial agent may be a beta-lactam antibiotic, in particular a beta-lactam antibiotic selected from the group consisting of penicillin antibiotics, cephalosporin antibiotics, and carbapenem antibiotics, or any combination of any thereof. In the context of the foregoing, the compositions may further comprise at least one additional agent capable of overcoming one or more bacterial resistance mechanisms. Examples of such additional agents include clavulanic acid, sulbactam, tazobactam, avibactam, relebactam, verbobactam, and any combination of any thereof.

Accordingly, such compositions may preferably comprise (i) at least one further antibacterial agent which is a beta-lactam antibiotic, in particular an existing regulatory approved beta-lactam antibiotic, more particularly an existing regulatory approved beta-lactam antibiotic selected from the group consisting of penicillin antibiotics, cephalosporin antibiotics, and carbapenem antibiotics, or any combination of any thereof; and (ii) at least one additional agent capable of overcoming one or more bacterial resistance mechanisms, said additional agent preferably being selected from the group consisting of clavulanic acid, sulbactam, tazobactam, avibactam, relebactam, verbobactam, and any combination of any thereof. Specific combinations of a (i) beta-lactam antibiotic with said (ii) at least one additional agent capable of overcoming one or more bacterial resistance mechanisms which are of particular interest include
(i) amoxicillin and clavulanic acid;
(ii) Ticacillin and clavulanic acid;
(iii) Ampicillin and Sulbactam;
(iv) Cefoperazone and Sulbactam;
(v) Piperacillin and Tazobactam;
(vi) Ceftolozane and Tazobactam;
(vii) Ceftazidime and Avibactam;
(viii) Ceftaroline and Avibactam;
(ix) carbapenems, in particular Impenem or Meropenem, and Relebactam; and
(x) carbapenems, in particular Impenem or Meropenem, and Varbobactam.

Accordingly, the present invention provides a composition comprising at least one compound of the invention or pharmaceutically acceptable salt thereof and any one of combinations (i) to (x). These compositions may further comprise a pharmaceutically acceptable carrier, diluent and/or excipient.

The pharmaceutical compositions disclosed herein may optionally comprise further therapeutic ingredients or adjuvants. The compositions include compositions suitable for oral, rectal, topical, pulmonary and parenteral (including subcutaneous, intramuscular, and intravenous) administration, although the most suitable route in any given case will depend on the particular host, and the nature and severity of the conditions for which the active ingredient is being administered. The pharmaceutical compositions may be conveniently presented in unit dosage form and prepared by any of the methods well known in the art of pharmacy.

In practice, the compounds of the invention, or pharmaceutically acceptable salts thereof, can be combined as the active ingredient in intimate admixture with a pharmaceutical carrier according to conventional pharmaceutical compounding techniques. The carrier may take a wide variety of forms depending on the form of preparation desired for administration, e.g. oral, parenteral (including intravenous, intramuscular, subcutaneous), topical, and pulmonary.

Thus, the pharmaceutical compositions can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound of the invention, or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation. The compounds of the invention, or pharmaceutically acceptable salts thereof, can also be included in pharmaceutical compositions in combination with one or more other therapeutically active compounds. The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques. Oral administration of the compounds of the invention may find particular use where no bioavailabilty of said compounds is necessary, for example in the treatment of infections of the intestinal tract such as infections of the colon. Alternatively, the compounds of the invention may be combined with drug delivery technologies known in the art to render aminoglycosides bioavailable.

A tablet containing the composition of this invention may be prepared by compression or molding, optionally with one or more accessory ingredients (excipients) or adjuvants. Compressed tablets may be prepared by compressing, in a suitable machine, the active ingredient in a **free-flowing** form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1mg to about 2g of the active ingredient, typically 2 mg, 5 mg, 10 mg, 25 mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof. Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, using a compound of the invention, or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds. Rectal administration of the compounds of the invention may find particular use where no bioavailabilty of said compounds is necessary, for example in the treatment of infections of the intestinal tract such as infections of the colon.

Pharmaceutical compositions of this invention can be in a form suitable for pulmonary administration, for example via inhalation using any technique known in the art for delivery of a drug into the lungs including, for example, administration via a dry-powder inhaler or a nebulizer. Suitable carriers and formulations for pharmaceutical compositions to be administered in such a fashion are known in the art and may find application with the compounds of the present invention.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of the invention, or pharmaceutically acceptable salts thereof, may also be prepared in powder or liquid concentrate form. Generally, dosage levels on the order of 0.01mg/kg to about 150mg/kg of body weight per day are useful in the treatment of the above-indicated conditions, or alternatively about 0.5mg to about 7g per patient per day. For example, microbial infection may be effectively treated by the administration of from about 0.01 to 50mg of the compound per kilogram of body weight per day, or alternatively about 0.5mg to about 3.5g per patient per day. It is understood, however, that the specific dose level for any particular patient will depend upon a variety of factors including the age, body weight, general health, sex, diet, time of administration, route of administration, rate of excretion, drug combination and the severity of the particular disease undergoing therapy. For example, it may be expected that a higher dose is required when administering the compounds of the invention via inhalation as opposed to, for example, parenterally, the dose when administering the compounds by inhalation (for example using a nebulizer) potentially being from 0.01mg/kg to about 500 mg/kg of body weight per day in the treatment of microbial infection. Actual methods of preparing compositions and dosage forms such as those referred to above are known, or will be apparent, to those skilled in this art; for example, see Remington: The Science and Practice of Pharmacy, 21st Edition (University of the Sciences in Philadelphia, 2005).

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration at the effective date of this application as being acceptable for use in humans or domestic animals.

The present invention further provides a compound of the invention or a pharmaceutically acceptable salt thereof, for use in or as a medicament or pharmaceutical.

In respect of the compounds disclosed herein, uses of the compounds disclosed herein, compositions comprising the compounds disclosed herein, and uses of the compositions comprising the compounds disclosed herein, the terms "compound(s) of the invention" and "compound(s) of formula (I)" are to be understood to be interchangeable. Accordingly, when reference is made, for example, to a use of "compound(s) of formula (I)" or a composition comprising "compound(s) of formula (I)", this is to be understood to mean the compounds of formula (I) in their broadest definition set forth herein and all further compounds which fall under this broadest definition and which, for example, further limit one or more structural features of the broadest definition of the "compound(s) of formula (I)" (for example, the compounds of formula (I) as defined in a more narrow manner in the dependent claims or compounds of formula (Ia) to (If), (Ig) to (Ip) etc. and their preferred forms).

The references to methods of treatment in the subsequent paragraphs of this description are to be interpreted as references to the compounds, pharmaceutical compositions of the present invention for use in a method for treatment of the human (or animal) body by therapy (or for diagnosis).

Where reference is made herein to a compound of the invention or a pharmaceutically acceptable salt thereof for use in or as a medicament or pharmaceutical or a method of treatment comprising the administration of a compound of the invention or a pharmaceutically acceptable salt thereof, it is to be understood that all compositions disclosed herein which comprise a compound of the invention or a pharmaceutically acceptable salt thereof may also be employed for use in said treatment or administered in said method of treatment.

Accordingly, the present invention also provides any of the compositions disclosed herein comprising at least one compound of the invention or a pharmaceutically acceptable salt thereof, for use in or as a medicament or pharmaceutical.

The compounds of the invention or pharmaceutically acceptable salts thereof or compositions comprising at least one compound of the invention or pharmaceutically acceptable salt thereof may be for use in the treatment of microbial infection and/or a disorder, affliction or illness caused at least in part by microbial infection. Where reference is made herein to the treatment of microbial infection, this is to be understood to also cover disorders, afflictions or illnesses caused at least in part by microbial infection.

As used herein, the term microbial infection preferably means bacterial infection. Where reference is made herein to the treatment of bacterial infection, this is to be understood to also cover disorders, afflictions or illnesses caused at least in part by bacterial infection.

As used herein, the term "treatment" includes both therapeutic and prophylactic treatment. The compounds of the invention may exhibit advantageous properties compared to known compounds or combination therapies for the treatment of microbial infection. The compounds of the invention, or pharmaceutically acceptable salts thereof, may be administered alone or in combination with one or more other therapeutically active compounds (including those combinations comprising at least one further antibacterial agent disclosed herein above). The other therapeutically active compounds may be for the treatment of the same disease or condition as the compounds of the invention or a different disease or condition, for example in their use in immunocompromised patients. The therapeutically active compounds may be administered simultaneously, sequentially or separately. The compounds of the invention may be administered with other active compounds for the treatment of microbial infection, for example together with penicillins, cephalosporins, polymyxins, rifamycins, quinolones, sulfonamides, macrolide antibiotics, lincosamides, tetracyclines, aminoglycosides, cyclic lipopeptides (such as daptomycin), glycylcyclines, oxazolidinones (such as linezolid). Inhibitors of bacterial efflux pumps, such as the AcrAB-TolC pump or the CmeABC efflux pump, may also be administered simultaneously (co-administered), sequentially or separately with the compounds or compositions of the invention. Any composition disclosed herein above may thus further comprise at least one inhibitor of bacterial efflux pumps, such as ihibitors of the AcrAB-TolC pump, inhibitors of the MexAB-, MexCD-, MexEF-, and MexXY pump, inhibitors of AcrD pump, or ihibitors of the CmeABC efflux pump. Examples of such inhibitors are described in X.-Z. Li, P. Plésiat, H. Nikaido, Clin. Microbiol. Rev. 2015, 28(2), 337-418, and J. Sun, Z. Deng, A. Yan, Biochem. Biophys. Res. Commun. 2014, 453, 254-267.

Co-administration within the meaning of the present invention includes administration of a formulation which includes both the compound of the invention, or a pharmaceutically acceptable salt thereof, and the other agent(s), or the simultaneous or separate administration of different formulations of each agent. Where the pharmacological profiles of the compound of the invention, or a pharmaceutically acceptable salt thereof, and the other agent(s) allow it, co-administration of the two agents may be preferred. The invention also provides the use of a compound of the invention, or a pharmaceutically acceptable salt thereof, and another agent in the manufacture of a medicament for the treatment of microbial infection. The invention also provides a pharmaceutical composition comprising at least one compound of the invention, or a pharmaceutically acceptable salt thereof, and another anti-microbial agent, i.e. other active compounds for the treatment of microbial infection, (such as those listed elsewhere herein) and a pharmaceutically acceptable carrier.

Certain aminoglycoside antibacterial agents such as, for example, Gentamicin are known to be associated with the induction of nephrotoxicity in subjects which are dosed with said agent. Gentamicin, for instance, is associated with an induction of, *inter alia,* tubular necrosis, epithelial oedema of proximal tubules, cellular desquamation, tubular fibrosis, glomerular congestion, perivascular edema and inflammation, which, in turn, may lead to renal dysfunction. Co-administering aminoglycoside antibacterial agents with adjuvants capable of suppressing or preventing the undesired nephrotoxic side-effects can greatly increase the usefulness of said agents, thus enabling their deployment without the observation of these side-effects and/or potentially allowing them to be dosed at higher levels than would otherwise be possible were such an adjuvant not to be co-administered. Examples of pharmacologicial classes of such adjuvants include other antibiotics (such as fosfomycin and /or fleroxacin), calcium channel blockers (such as nifedipine and/or amlodipine), beta blockers (such as carvedilol), cytoprotective antianginals (such as trimetazine), iNOS inhibitors (such as L-NIL), nitrogen oxide (NO) precursors (such as L-arginine), hormones (such as melatonin and/or thyroxine), antiplatelets (such as trapidil), statins (such as atorvastatin), PPAR-γ agonists (such as rosiglitazone), TNF-a synthesis inhibitors (such as pentoxifylline), biguanides (such as metformin), antioxidants (such as probucol, aminoguanidine, L-carnitine, ebselen, N-acetylcysteine, lycopene, curcumin, thymoquinone, fish oil, vitamin E, vitamin C, sesame oil, halofuginone, resveratrol, and quercetin or any combination of any thereof), free radical scavengers (such as S-allylcysteine, diallyl sulfide, caffeic acid phenethyl ester, S-allylmercaptocysteine, or any combination of any thereof), antioxidant enzymes (such as superoxide dismutase), superoxide dismutase mimetics (such as superoxide dismutase mimetic M40403), and herbal extracts (such as *Rhazya stricta,* garlic, *Cassia auriculata,* soyabean, *Phylanthus amarus, Morchella esculenta,* green tea, *Nigella sativa, Ligusticum wallichi, Viscum articulatum,* or any combination of any thereof). Further examples of such adjuvants include gum arabic, pongamia pinnata flowers, nigella sativa oil, hemidesmus indicus, PESB (phenolic extract of soybean), green tea extract, bauhinia purpurea, sida rhomboidea, apocynin, and pipercillin, as well the agents and drugs taught to reduce the neophrotoxicity of Gentamicin in Ali, B. H. et al. Basic and Clinical Pharmacology & Toxicology, 109, 225-232 and Balakumar P. et al. Pharmacological Research, 62 (2010), 179-186. The present invention thus further relates to a composition comprising at least one compound of the invention or pharmaceutically acceptable salt thereof and at least one adjuvant capable of suppressing or preventing aminoglycoside-induced nephrotoxicity, in particular wherein said at least one adjuvant is selected from the pharmacologicial classes of such adjuvants and/or the specific adjuvants listed hereinabove, or any combination of any thereof. Said composition may be any of the other compositions of the present invention disclosed herein which further comprises at least one adjuvant capable of suppressing or preventing aminoglycoside-induced nephrotoxicity, in particular wherein said at least one adjuvant is selected from the pharmacologicial classes of such adjuvants and/or the specific adjuvants listed hereinabove, or any combination of any thereof. Further, the compounds of the invention or pharmaceutically acceptable salts thereof or any of the compositions comprising at least one compound of the invention or pharmaceutically acceptable salt thereof disclosed herein may thus, in any of the uses or methods of treatment disclosed herein, be co-administered together with one or more members of any one or more of the above-listed pharmacological classes of adjuvants.

The compounds of the invention or pharmaceutically acceptable salts thereof or any of the compositions comprising at least one compound of the invention or pharmaceutically acceptable salt thereof disclosed herein may be for use in the treatment of any microbial or bacterial infection. Such microbial or bacterial infections may be caused or at least caused in part by Gram-positive bacteria, Gram-negative bacteria or a combination of both. Such microbial or bacterial infections may be caused by either Gram-positive bacteria or Gram-negative bacteria.

Gram-positive bacteria which cause at least in part the infections which may be treated by the compounds of the invention, pharmaceutically acceptable salts thereof or compositions comprising at least one compound of formula (I) or pharmaceutically acceptable salt thereof may be any Gram-positive bacteria. These Gram-positive bacteria may in particular be selected from the group consisting of *Staphylococcus aureus, Streptococcus pneumoniae, Enterococcus faecalis, Enterococcus faecium,* and *Mycobacterium tuberculosis.* Gram-negative bacteria which cause at least in part the infections which may be treated by the compounds of the invention, pharmaceutically acceptable salts thereof or compositions comprising at least one compound of the invention or pharmaceutically acceptable salt thereof may be any Gram-negative bacteria. These Gram-negative bacteria may in particular be selected from the group consisting of *Acinetobacter spp.; Enterobacteriaceae spp., in particular Escherichia spp., Klebsiella spp, or Enterobacter spp.; Morganella spp.; Providencia spp.;* and *Pseudomonas spp., in particular from the group consisting of Acinetobacter baumannii* (*A. baumannii*)*, Escherichia coli* (*E. coli*)*, Klebsiella pneumoniae (K. pneumoniae), Klebsiella oxytoca (K. oxytoca), Enterobacter cloacae (E. cloacae), Enterobacter aerogenes (E. aerogenes), Morganella morgannii (M. morgannii), Providencia stuartii (P. stuartii),* and *Pseudomonas aeruginosa (P. aeruginosa).* The *E. coli* may be selected from Wild Type *E. coli* and *E. coli* strains which express one or more aminoglycoside modifying enzymes (AMEs). Furthermore in this context, each of said *Acinetobacter spp.; Enterobacteriaceae spp., in particular Escherichia spp., Klebsiella spp, or Enterobacter spp.; Morganella spp.; Providencia spp.; Pseudomonas spp.; Acinetobacter baumannii* (*A. baumannii*)*; Escherichia coli* (*E. coli*)*; Klebsiella pneumoniae (K. pneumoniae); Klebsiella oxytoca (K. oxytoca); Enterobacter cloacae (E. cloacae); Enterobacter aerogenes (E. aerogenes); Morganella morgannii (M. morgannii); Providencia stuartii (P. stuartii);* or *Pseudomonas aeruginosa (P. aeruginosa)* may be either non-resistant strains or strains which express one or more aminoglycoside modifying enzymes (AMEs), in particular one or more AMEs belonging to any of the families of AMEs disclosed elsewhere herein or any of the specific individual AMEs disclosed elsewhere herein.

Aminoglycoside modifying enzymes are resistance-causing enzymes which may be expressed in a given bacterial species and which are capable of modifying the structure of an aminoglycoside molecule, thus potentially "deactivating" its antibacterial capabilities by reducing their affinity to the target protein, i.e. 30S ribosomal subunit. In particular, in the present invention aminoglycoside modifying enzymes present in a given bacterial species may be selected from aminoglycoside acetyltransferases (AACs), aminoglycoside phosphotransferases (APHs), and aminoglycoside nucloetidyltransferases (ANTs), bifunctional aminoglycoside modifying enyzmes, and any combination of any thereof. In this context, AACs may include AAC(3)-I (for example, AAC(3)Ia, AAC(3)Ic), AAC(3)-II (for example, AAC(3)IId), AAC(3)-III, AAC(3)-IV, AAC(3)-VI, AAC(6')-I, AAC(6')-II, AAC(6')Ie-APH(2")Ia, and AAC(2'-I). APHs may include APH(2")-I, APH(3')-I (for example, APH(3')Ia), APH(3')-II (for example, APH(3')IIa, APH(3')IIb), APH(3')-III (for example APH(3')IIIa), APH(3')-IV, APH(3')-V, APH(3')-VI (for example, APH(3')VIa), APH(3')-VII, APH(3")-I (for example, APH(3")Ib), and APH(6)-I (for example, APH(6)Ic, APH(6)Id). ANTs may include ANT(2")-I (for example, ANT(2")Ia), ANT(3"), ANT(3")-I (for example, ANT(3")Ia), ANT(4')-I, ANT(4')-II, and ANT(6)-I. Preferably, the AMEs present in bacterial species against which the compounds of the invention exhibit activity may be selected from APH(3')IIIa, APH(3')Ia, APH(3')IIb, APH(3')VIa, APH(3")Ib, APH(6)Ic, APH(6)Id, AAC(6')Ib, AAC(3)III, AAC(3)IV, AAC(3)Ia, AAC(3)Ic, AAC(3)IId, ANT(2")Ia), ANT(3"), ANT(3")Ia and AAC(6')Ie-APH(2")Ia, most preferably from APH(3')IIIa, APH(3')Ia, AAC(3)III, AAC(3)IV and AAC(6')Ie-APH(2")Ia.

Accordingly, the present invention further provides compounds of the invention, pharmaceutically acceptable salts thereof or compositions comprising at least one compound of the invention or pharmaceutically acceptable salt thereof for use in the treatment of microbial infection caused at least in part by bacteria which express one or more AMEs, in particular one or more AMEs selected from the group consisting of AAC(3)-I, AAC(3)-II, AAC(3)-III, AAC(3)-IV, AAC(3)-VI, AAC(6')-I, AAC(6')-II, AAC(6')-APH(2"), AAC(2'-I), APH(2")-I, APH(3')-I, APH(3')-II, APH(3')-III, APH(3')-IV, APH(3')-V, APH(3')-VI, APH(3')-VII, APH(3")-I, APH(6)-I, ANT(2")-I, ANT(3")-I, ANT(4')-I, ANT(4')-II, ANT(6)-I, more particularly from the group consisting of APH(3')IIIa, APH(3')Ia, AAC(3)III, AAC(3)IV and AAC(6')Ie-APH(2")Ia. Said AMEs expressed in the said bacteria responsible at least in part for the microbial infection which is treated by the aforementioned compounds, pharmaceutically acceptable salts or compositions may also be selected from the group consisting of APH(3')IIIa, APH(3')Ia, APH(3')IIa, APH(3')IIb, APH(3')VIa, APH(3")Ib, APH(6)Ic, APH(6)Id, AAC(6')Ib, AAC(3)III, AAC(3)IV, AAC(3)Ia, AAC(3)Ic, AAC(3)IId, ANT(2")Ia), ANT(3"), ANT(3")Ia, AAC(6')Ie-APH(2")Ia, or any combination of any thereof. The bacterial species which expresses such AMEs may be any bacterial species specified herein, in particular *Escherichia coli.*

The compounds of the invention, pharmaceutically acceptable salts thereof or compositions comprising a compound of the invention or pharmaceutically acceptable salt thereof are also effective for use in the treatment of microbial infection caused at least in part by bacteria which exhibit other resistance mechanisms such as, for example, other efflux and/or target modifying enzymes. Examples of such target modifying enzymes inlcude 16S RNA methylases (e.g. armA, rmtA, rmtB, etc.).

The compounds of formula (I), pharmaceutically acceptable salts thereof or compositions comprising at least one compound of formula (I) or pharmaceutically acceptable salt thereof may further be for use used in the treatment of a bacterial infection caused at least in part by a bacterial species (spp.) selected from the group consisting of *Escherichia spp., Enterococcus spp., Staphylococcus spp., Klebsiella spp., Acinetobacter spp., Pseudomonas spp., Enterobacter spp., Mycobacterium spp., Morganella spp., Providencia spp.,* and any combination of any thereof. *Escherichia spp.* may be *Escherichia coli* (*E. coli*)*,* in particular Wild Type *E. coli* or *E. coli* strains which express one or more aminoglycoside modifying enzymes (AMEs) as defined hereinabove. *Enterococcus spp.* may be *Enterococcus faecium* or *Enterococcus faecalis,* preferably *Enterococcus faecalis. Staphylococcus spp.* may be *Staphylococcus aureus, Klebsiella spp.* may be *Klebsiella pneunomiae. Acinetobacter spp.* may be *Acinetobacter baumannii. Pseudomonas spp.* may be *Pseudomonas aeruginosa. Enterobacter spp.* may be *Enterobacter cloacae. Morganella spp.* may be *Morganella* morganni. *Providencia spp.* may be *Providencia stuartii. Strains of each of said species and bacteria may be strains* which are non-resistant or strains which express one or more aminoglycoside modifying enzymes (AMEs) as defined eslsewhere herein.

The compounds of the invention, pharmaceutically acceptable salts thereof or compositions comprising at least one compound of the invention or pharmaceutically acceptable salt thereof may further be for use in the treatment of a bacterial infection wherein said bacterial infection is caused at least in part by a bacterial species which exhibits resistance against at least one member of a family of existing regulatory approved antibiotics. "Existing regulatory approved antibiotics" in this context and as used elsewhere herein (also referred to herein as "regulatory approved antibiotics") should be understood to mean existing antibiotics which have at the filing date, or where priority is claimed the priority date, of the present application been approved by at least one national, regional or international regulatory body responsible for permitting medicines or at least antibiotics for use in the respective national, regional or international territory. Said regulatory approved antibiotics against which the bacterial species exhibits resistance may be selected from the group consisting penicillin antibiotics, cephalosporin antibiotics, carbapenem antibiotics, monobactam antibiotics polymyxin antibiotics, rifamycin antibiotics, lipiarmycin antibiotics, quinolone antibiotics, sulfonamide antibiotics, macrolide antibiotics, lincosamide antibiotics, tetracycline antibiotics, aminoglycoside antibiotics, lipopeptide antibiotics, glycylcycline antibiotics, glycopeptide antibiotics, oxazolidinone antibiotics, and lipiarmycin.

Examples of exisiting regulatory approved penicillin antibiotics include Penicillin G, Penicillin K, Penicillin N, Penicillin O, Penicillin V, Methicillin, Nafcillin, Oxacillin, Cloxacillin, Dicloxacillin, Flucloxacillin, Ampicillin, Amoxicillin, Pivampicillin, Hetacillin, Bacampicillin, Metampicillin, Talampicillin, Epicillin, Carbenicillin, Ticarcillin, Temocillin, Mezlocillin, Piperacillin, Clavulanic acid, Sulbactam, Tazobactam.

Examples of existing regulatory approved cephalosporin antibiotics include Cefazolin, Cefalexin, Cefadroxil, Cefapirin, Cefazedone, Cefazaflur, Cefradine, Cefroxadine, Ceftezole, Cefaloglycin, Cefacetrile, Cefalonium, Cefaloridine, Cefalotin, Cefatrizine, Cefaclor Cefotetan, Cefoxitin, Cefprozil, Cefuroxime, Cefuroxime axetil, Cefamandole, Cefminox, Cefonicid, Ceforanide, Cefotiam, Cefbuperazone, Cefuzonam, Cefmetazole, Loracarbef, Cefixime, Ceftriaxone, Ceftazidime, Cefoperazone, Cefdinir, Cefcapene, Cefdaloxime, Ceftizoxime, Cefmenoxime, Cefotaxime, Cefpiramide, Cefpodoxime, Ceftibuten, Cefditoren, Cefetamet, Cefodizime, Cefpimizole, Cefsulodin, Cefteram, Ceftiolene, Flomoxef, Latamoxef, Cefepime, Cefozopran, Cefpirome, Cefquinome, Ceftaroline fosamil, Ceftolozane, Ceftobiprole, Ceftiofur, Cefquinome, and Cefovecin.

Examples of existing regulatory approved carbapenem antibiotics include imipenem, doripenem, meropenem, ertapenem, faropenem, and diapenem.

Examples of existing regulatory approved monobactam antibiotics include aztreonam.

Examples of existing regulatory approved polymyxin antibiotics include polymyxin Band polymxin E (colistin).

Examples of existing regulatory approved rifamycin antibiotics include rifamycin B, rifamycin SV, rifampicin (or rifampin), rifabutin, and rifamixin.

Examples of existing regulatory approved lipiarmycin antibiotics include lipiarmycin A and lipiarmycin B.

Examples of existing regulatory approved quinolone antibiotics include ciprofloxacin, fleroxacin, lomefloxacin, nadifloxacin, norfloxacin, ofloxacin, pefloxacin, rufloxacin, balofloxacin, grepafloxacin, levofloxacin, pazufloxacin, sparfloxacin, temafloxacin, clinafloxacin, gatifloxacin, moxifloxacin, sitafloxacin, prulifloxacin, besifloxacin, delafloxacin, and ozenoxacin.

Examples of existing regulatory approved sulfonamide antibiotics include mafenide, sulfacetamide, sulfadiazine, sulfadoxine, sulfamethizole, sulfamethoxazole, sulphanilamide, sulfasalazine, and sulfisoxazole.

Examples of existing regulatory approved macrolide antibiotics include azithromycin, boromycin, clarithromycin, dirithromycin, erythromycin, flurithromycin, josamycin, midecamycin, miocamycin, oleandomycin, rokitamycin, roxithromycin, spiramycin, troleandomycin, and tylosin.

Examples of existing regulatory approved lincosamide antibiotics include lincomycin, clindamycin, and pirlimycin.

Examples of existing regulatory approved tetracycline antibiotics include doxycycline, chlortetracycline, clomocycline, demeclocycline, lymecycline, meclocycline, metacycline, minocycline, omadacycline, oxytetracycline, penimepicycline, rolitetracycline, sarecycline, tetracycline, and eravacycline.

Examples of existing regulatory approved aminoglycoside antibiotics include gentamicin, tobramycin, amikacin, plazomicin, streptomycin, neomycin, and paromomycin, apramycin, arbekacin, and dibekacin.

Examples of existing regulatory approved lipopeptide antibiotics include daptomycin.

Examples of existing regulatory approved glycylcycline antibiotics include tigecycline.

Examples of existing regulatory approved glycopeptide antibiotics include vancomycin.

Examples of existing regulatory approved oxazolidinone antibiotics include linezolid and tedizolid.

The compounds of formula (I), pharmaceutically acceptable salts thereof or any of the compositions disclosed herein comprising at least one compound of formula (I), a pharmaceutically acceptable salt thereof may be for use to treat any bacterial infection. Specific examples of such bacterial infections include one or more infections and infectious diseases selected from the list consisting of respiratory tract infections, complicated skin and soft tissue infections, complicated intra-abdominal infections, community acquired pneumonia, hospital-acquired pneumonia, ventilator-associated pneumonia, urinary tract infections, bacterial meningitis, infective endocarditis, sepsis, osteomyelitis, septic arthritis, septicemia, anthrax, osteomyelitis, tuberculosis, leprosy, necrotizing fasciitis, scarlet fever, rheumatic fever, postpartum fever, and streptococcal toxic shock syndrome, and additional nosocomial infections, for example infections caused by the use of intravascular catheters.

The present invention also provides a method for the treatment of a disease or condition in which microbes play a role, said method comprising a step of administering to a subject in need thereof a therapeutically effective amount of a compound of formula (I), a pharmaceutically acceptable salt thereof or a composition comprising at least one compound of formula (I) or a pharmaceutically acceptable salt thereof as defined herein, said subject being a human or animal, in particular a mammal, more particularly a human.

Accordingly, where reference is made herein to a compound of formula (I), a pharmaceutically acceptable salt thereof or a composition comprising at least one compound of formula (I) or a pharmaceutically acceptable salt thereof, for use as a medicament or for use in the treatment of a particular type of infection, disorder, affliction, or illness, this may also cover a method of treatment of said infection, disorder, affliction, or illness comprising a step of administering to a subject in need thereof a therapeutically effective amount of a compound of formula (I), a pharmaceutically acceptable salt thereof or a composition comprising at least one compound of formula (I) or a pharmaceutically acceptable salt thereof, said subject being a human or animal, in particular a mammal, more particularly a human.

Accordingly, the present invention also provides a method for the treatment of microbial infection, said method comprising a step of administering to a subject in need thereof a therapeutically effective amount of a compound of formula (I) or a pharmaceutically acceptable salt thereof, said subject being a human or animal, in particular a mammal, more particularly a human.

The compounds of the invention may show activity against, and thus be used in the treatment of microbial infections caused by gram-positive bacteria and/or gram-negative bacteria. The compounds of the invention, pharmaceutically acceptable salts thereof or compositions comprising compounds of the invention or pharmaceutically acceptable salts thereof may be for use used in the treatment of bacterial infections caused by or at least in part by one or more members of the families *Escherichia spp., Enterococcus spp., Staphylococcus spp., Klebsiella spp., Acinetobacter spp., Pseudomonas spp., and Enterobacter spp., Mycobacterium spp., Morganella spp., Providencia spp.,* and any combination of any thereof, in particular by one or more bacteria selected from *Escherichia coli* (*E. coli*) Wild Type, *E*. *coli* APH(3')IIIa, *E. coli* APH(3')Ia, *E.coli* AAC(6')-APH(2"), *E*. *coli* AAC(6')Ib, *E.coli* AAC(3)III, *E*. *coli* AAC(3)IV, *E*. *coli* APH(3')IIb, *E*. *coli* APH(3')VIa, *E*. *coli* APH(3")Ib, *E*. *coli* APH(6)Ic, *E*. *coli* APH(6)Id, *E*. *coli* AAC(3)Ia, *E*. *coli* AAC(3)Ic, *E*. *coli* AAC(3)IId, *E. coli* ANT(2")Ia), *E. coli* ANT(3"), *E*. *coli* ANT(3")Ia, *E*. *coli* AAC(6')Ie-APH(2")Ia, *Enterococcus faecalis, Enterococcus faecium, Staphylococcus aureus, Klebsiella pneumoniae, Acinetobacter baumanii, Pseudomonas aeruginosa, Enterobacter cloacae, Morganella morgannii, Providencia stuartii, any of said bacteria expressing any of the AMEs disclosed elsewhere herein,* and any combination of any thereof. By way of example, said bacterial infections may be caused by or at least in part by one or more of *Acinetobacter baumanii, Escherichia coli, Morganella morgannii,* and *Providencia stuartii,* wherein each of said bacteria expresses one or more AMEs independently selected from AAC(3)Ia, ANT(3")Ia, APH(3")Ib, APH(3')VI, APH(6)Id, ANT(2")Ia, APH(3')-VIa, APH(3')-Ia, AAC(3)-IId, ANT(3"), APH(3')-IIa, APH(3')-IIb, APH(6)-Ic, and AAC(3)-Ic. Examples thereof include *Acinetobacter baumanii* expressing one or more AMEs selected from AAC(3)Ia, ANT(3")Ia, APH(3")Ib, APH(3')VI, and APH(6)Id (for example, *Acinetobacter baumanii expressing* AAC(3)Ia, ANT(3")Ia, APH(3")Ib, APH(3')VI, and APH(6)Id; or *Acinetobacter baumanii* expressing ANT(2")Ia, APH(3")-Ib, APH(3')-VIa, and APH(6)-Id). Further examples thereof include *Escherichia coli* expressing one or more AMEs selected from ANT(3")-Ia, and APH(3')-Ia (for example, *Escherichia coli* expressing APH(3')-Ia; or *Escherichia coli* expressing ANT(3")-Ia and APH(3')-Ia). Further examples thereof include *Morganella morganni* expressing one or more AMEs selected from AAC(3)-IId, ANT(3"), APH(3")-Ib, APH(3')-Ia, and APH(6)-Id (for example, *Morganella morgannii* expressing AAC(3)-IId, ANT(3"), APH(3")-Ib, APH(3')-Ia, and APH(6)-Id). Still further examples thereof include *Providencia stuartii* expressing one or more AMEs selected from AAC(3)-Ic, ANT(3")Ia, ANT(3"), APH(3')-IIa, APH(3')-IIb, APH(3')-VI, and APH(6)-Ic (for example, *Providencia stuartii* expressing AAC(3)-Ic, ANT(3")Ia, ANT(3"), APH(3')-IIa, APH(3')-IIb, APH(3')-VI, and APH(6)-Ic).

The compounds of the present invention and their pharmaceutically acceptable salts exhibit particularly high potency and cross-panel activity against microbial infections caused at least in part by bacteria from the ESKAPE panel as well as carbapenem-resistant *Enterobacteriaceae* (CRE) infections. As such, the compounds of the present invention or their pharmaceutically acceptable salts may be for use in the treatment of microbial infections caused at least in part by bacteria from the ESKAPE panel and/or carbapenem-resistant *Enterobacteriaceae* (CRE) infections. Accordingly, the present invention also relates to a method of treatment of a subject suffering from a microbial infection caused at least in part by bacteria from the ESKAPE panel and/or carbapenem-resistant *Enterobacteriaceae* (CRE) infections comprising administering a therapeutically effective amount of a compound of the invention or a pharmaceutically acceptable salt thereof to said subject. In particular, the compounds of the present invention or their pharmaceutically acceptable salts may be used in the treatment of microbial infections caused at least in part by *Enterobacter cloacae.* When used in treating carbapenem-resistant *Enterobacteriaceae* (CRE) infections, the compounds of the present invention or their pharmaceutically acceptable salts may be used to treat carbapenem-resistant *Enterobacteriaceae* (CRE) infections caused at least in part by at least one of *Escherichia coli, Klebsiella pneumoniae,* and *Enterobacter cloacae.*

The compounds of the invention or their pharmaceutically acceptable salts also exhibit excellent cross-panel activity against clinical isolates of selected multidrug-resistant (MDR) bacteria of the ESKAPE panel and amplified activity against *Enterococcus faecalis.* The compounds of the invention or their pharmaceutically acceptable salts find particular use in the treatment of microbial infections caused at least in part by *Klebsiella pneumonia, Acinetobacter baumannii, Pseudomonas aeruginosa,* or any combination of any thereof, including MDR microbial infections caused at least in part by *Klebsiella pneumoniae, Acinetobacter baumannii, Pseudomonas aeruginosa,* or any combination of any thereof. Examples thereof include infections caused at least in part by *Klebsiella pneumoniae* strains expressing carbapenemases such as OXA-type carbapenemases (e.g. OXA-48, etc.), NDM-type carbapenemases, VIM-type carbapenemases, and/or *Klebsiella pneumoniae carbapenemases* (*KPCs*)*,* and/or extended-spectrum beta-lactamases (ESBLs). More than one resistance-causing enzyme may be expressed by a single *Klebsiella pneumoniae* strain. Further examples thereof include infections caused at least in part by *Klebsiella pneumoniae* strains, which exhibit Colistin resistance. Said *Klebsiella pneumoniae* strains may additionally be resistant to one or more of, *inter alia,* Amikacin, Gentamicin, Tobramycin (also known as Tobramicin), Cetazidime and Meropenem. Further examples thereof include infections caused at least in part by *Acinetobacter baumannii* strains which are resistant to carbapenems (so-called carbapenem-resistant *Acinetobacter baumannii* - CRAB) and MDR *Acinetobacter baumannii* strains. Said *Acinetobacter baumannii* strains may additionally be resistant to one or more of, *inter alia,* Amikacin, Gentamicin, Tobramycin, Cetazidime and Meropenem. Further examples thereof include infections caused at least in part by *Pseudomonas aeruginosa* strains expressing carbapenemases such as OXA-type carbapenemases (e.g. OXA-2), VIM-type carbapenemases, and MDR *Pseudomonas aeruginosa* strains. Said *Pseudomonas aeruginosa* may additionally be resistant to one or more of, *inter alia,* Amikacin, Gentamicin, Tobramycin, Cetazidime and Meropenem. Further examples thereof include infections caused at least in part by Methicillin-resistant *Staphylococcus aureus* (MRSA) strains.

The compounds of the invention or their pharmaceutically acceptable salts find particular use in the treatment of microbial infections caused at least in part by *Escherichia. coli (E. coli)* strains, which are multidrug resistant, in particular against aminoglycosides (e.g. Amikacin, Gentamicin, and/or Tobramicin), or pandrug resistant.

The compounds of the invention or their pharmaceutically acceptable salts find particular use in the treatment of microbial infections caused at least in part by *Enterococcus. faecalis* strains, which are multidrug-resistant, in particular against aminoglycosides (e.g. Amikacin, Gentamicin, and/or Tobramicin), or pandrug resistant.

The compounds of the invention or pharmaceutically acceptable salts thereof or any of the compositions disclosed herein comprising at least one compound of the invention or pharmaceutically acceptable salt thereof find particular use in the treatment of microbial infections caused at least in part by *Vancomycin-resistant Enterococcus faecium (VRE).*

The compounds of the invention or pharmaceutically acceptable salts thereof or any of the compositions disclosed herein comprising at least one compound of the invention or pharmaceutically acceptable salt thereof find particular use in the treatment of microbial infections caused at least in part by *Morganella morgannii* strains, in particular strains which are multidrug-resistant (in particular against aminoglycosides (e.g. Amikacin, Gentamicin, and/or Tobramicin), or pandrug resistant).

The compounds of the invention or pharmaceutically acceptable salts thereof or any of the compositions disclosed herein comprising at least one compound of the invention or pharmaceutically acceptable salt thereof find particular use in the treatment of microbial infections caused at least in part by *Providencia stuartii* strains, in particular strains which are multidrug-resistant (in particular against aminoglycosides (e.g. Amikacin, Gentamicin, and/or Tobramicin), or pandrug resistant).

The compounds of the invention or their pharmaceutically acceptable salts find particular use in the treatment of microbial infections caused at least in part by *Klebsiella pneumoniae* strains, which are multidrug-resistant, in particular against aminoglycosides (e.g. Amikacin, Gentamicin, and/or Tobramycin), or pandrug resistant.

The compounds of the invention or their pharmaceutically acceptable salts find particular use in the treatment of microbial infections caused at least in part by *Acinetobacter baumannii* strains, which are multidrug-resistant, in particular against aminoglycosides (e.g. Amikacin, Gentamicin, and/or Tobramycin), or pandrug resistant.

The compounds of the invention or their pharmaceutically acceptable salts find particular use in the treatment of microbial infections caused at least in part by *Enterobacter. cloacae* strains, which are multidrug-resistant, in particular against aminoglycoside (e.g. Amikacin, Gentamicin, and/or Tobramycin), or pandrug resistant.

The compounds of the invention or their pharmaceutically acceptable salts find particular use in the treatment of microbial infections caused at least in part by *Pseudomonas aeruginosa* strains, which are multidrug-resistant, in particular against aminoglycosides (e.g. Amikacin, Gentamicin, and/or Tobramycin), or pandrug resistant.

The compounds of the invention or their pharmaceutically acceptable salts find particular use in the treatment of microbial infections caused at least in part by *Staphylococcus aureus* strains, which are multidrug-resistant, in particular against aminoglycosides (e.g. Amikacin, Gentamicin, and/or Tobramycin), or pandrug resistant.

In one embodiment the present invention provides a compound of formula (I), or a pharmaceutically acceptable salt thereof, for use in treating a medical condition, i.e. for use as a medicament or pharmaceutical agent, in particular for treating microbial infection and/or infectious diseases. As such, the present invention provides a compound of formula (I), or a pharmaceutically acceptable salt thereof, for use in the treatment of microbial infection and/or a disorder, affliction or illness caused at least in part by microbial infection. The present invention thus further provides a method of treating a subject suffering microbial infection and/or a disorder, affliction or illness caused at least in part by microbial infection, said method comprising administration of a therapeutically effective amount of a compound of formula (I) or a pharmaceutically acceptable salt thereof to said subject. The subject which is thus treated is a human or animal, in particular a mammal, more particularly a human. The microbial infection may be and is preferably a bacterial infection. This bacterial infection may be caused in least in part by one or more Gram-positive species and/or one or more Gram-negative species. Such Gram-positive species may be one or more species selected from the list consisting of *Staphylococcus aureus, Streptococcus pneumoniae, Enterococcus faecalis, Enterococcus faecium, Mycobacterium tuberculosis.* Such Gram-negative species may be one or more species selected from the list consisting of *Klebsiella spp., Escherichia spp., Acinetobacter spp., Morganella spp., Providencia spp., Enterobacter spp..* As such, said bacterial infection may be caused at least in part by a bacterial species (spp.) selected from the group consisting of *Escherichia spp., Enterococcus spp., Staphylococcus spp., Klebsiella spp., Acinetobacter spp., Pseudomonas spp., Enterobacter spp., Mycobacterium spp., Morganella spp., Providencia spp.,* or combinations thereof. In this context, the *Escherichia spp.* may be *Escherichia coli,* preferably selected from the group consisting of Wild type *Escherichia coli* and *Escherichia coli* strains which express one or more aminoglycoside modifying enzymes and said aminoglycoside modifying enzymes may be selected from the group consisting of aminoglycoside phosphotransferases, in particular APH(3')IIIa, and APH(3')Ia, aminoglycoside acetyltransferases, in particular AAC(3)III, and AAC(3)IV, and bifunctional aminoglycoside modifying enyzmes, in particular AAC(6')Ie-APH(2")Ia, or combinations thereof. Said aminoglycoside modifying enzymes may also be selected from the group consisting of APH(3')IIIa, APH(3')Ia, AAC(6')-APH(2"), AAC(6')Ib, AAC(3)III, AAC(3)IV, APH(3')IIa, APH(3')IIb, APH(3')VIa, APH(3")Ib, APH(6)Ic, APH(6)Id, AAC(3)Ia, AAC(3)Ic, AAC(3)IId, ANT(2")Ia), ANT(3"), ANT(3")Ia, AAC(6')Ie-APH(2")Ia, and any combination of any thereof. In this context, said *Enterococcus spp.* may be *Enterococcus faecium* or *Enterococcus faecalis,* preferably *Enterococcus faecalis,* said *Staphylococcus spp.* may be *Staphylococcus aureus,* said *Klebsiella spp.* may be *Klebsiella pneunomiae,* said *Acinetobacter spp.* may be *Acinetobacter baumannii,* said *Pseudomonas spp.* may be *Pseudomonas aeruginosa,* said *Morganella spp.* may be *Morganella morgannii,* said *Providencia spp.* may be *Providencia stuartii,* and said *Enterobacter spp.* may be *Enterobacter cloacae.* Any of these bacteria may be non-resistant strains or strains expressing one or more of the above-listed AMEs. In this context, said bacterial infection may be caused at least in part by a bacterial species which exhibits resistance against at least one member of a family of existing regulatory approved antibiotics, in particular by a bacterial species which exhibits resistance against at least one member of one or more families of antibiotics selected from the group consisting penicillin antibiotics, cephalosporin antibiotics, carbapenem antibiotics, monobactam antibiotics, polymyxin antibiotics, rifamycin antibiotics, lipiarmycin antibiotics, quinolone antibiotics, sulfonamide antibiotics, macrolide antibiotics, lincosamide antibiotics, tetracycline antibiotics, aminoglycoside antibiotics, lipopeptide antibiotics, glycylcycline antibiotics, glycopetide antibiotics, oxazolidinone antibiotics, and lipiarmycin. In this context, said bacterial infection may one or more infections and infectious diseases selected from the list consisting of respiratory tract infections, complicated skin and soft tissue infections, complicated intra-abdominal infections, community acquired pneumonia, hospital-acquired pneumonia, ventilator-associated pneumonia, urinary tract infections, bacterial meningitis, infective endocarditis, sepsis, osteomyelitis, septic arthritis, septicemia, anthrax, osteomyelitis, tuberculosis, leprosy, necrotizing fasciitis, scarlet fever, rheumatic fever, postpartum fever, and streptococcal toxic shock syndrome, and additional nosocomial infections, for example infections caused by the use of intravascular catheters.

A common disadvantage associated with known aminoglycoside antibacterial agents such as, for example, Gentamicin is that they are associated with a low volume of distribution upon *in vivo* dosing. This low level of tissue distribution limits the applicability of conventional aminoglycoside antibacterial agents such as Gentamicin in treating microbial infections whose treatment requires a high degree of tissue penetration (such as, for example, pneumonia, cystic fibrosis, skin infection, and soft tissue infection). Accordingly, aminoglycosides are generally not employed as a first-choice antimicrobial agent in treating such infections. A further object underlying the present invention is thus the provision of aminoglycoside antimicrobial agents with improved tissue distribution (measured by, for example, the pharmacokinetic parameter volume of distribution (V_{d})) relative to conventional aminoglycoside antimicrobial agents such as, for example, Gentamicin. In this regard, the compounds of the invention are seen to exhibit a significantly increased volume of distribution over conventional aminoglycoside antimicrobial agents such as Gentamicin, said increase typically being in the range of 2- to 5-fold. Accordingly, the compounds of the present invention, pharmaceutically acceptable salts thereof or any composition described herein comprising at least one compound of the invention or pharmaceutically acceptable salt thereof may be used in the treatment of a bacterial infection and/or a disorder, affliction or illness caused at least in part by bacterial infection, wherein said treatment requires a high degree of tissue penetration, in particular in the treatment of pneumonia, cystic fibrosis, skin infection, or soft tissue infection, especially in the treatment of pneumonia.

A further object underlying the invention is the provision of aminoglycoside antibacterial agents against which at least one bacterial family or strain exhibits a reduced propensity to develop resistance when compared to other known aminoglycoside antibacterial agents such as, for example, amikacin, gentamicin, or any of the other aminoglycoside antibacterial agents taught herein. A wide variety of bacterial strains are seen to exhibit such a reduced proposentity to develop resistance against the compounds of the invention relative to known aminoglycosides such as, for example, amikacin or gentamicin. Such effects have been seen in a number of *in vitro* serial passage resistance studies performed over 30 days in, *inter alia,* strains of *A. baumannii, K. pneumoniae* (including Gentamicin-resistant clinical isolates), and *E.coli.*

The compounds of the present invention or pharmaceutically acceptable salts thereof may be for use in the treatment of bacterial infection and/or a disorder, affliction or illness caused at least in part by bacterial infection, in particular wherein said bacterial infection is caused by any one of (or any combination of any of) the bacterial strains disclosed herein or against which data is provided in the experimental section herein.

A further aspect of the invention relates to methods for preventing, inhibiting, or stopping the growth of bacteria on surfaces involving the step of applying at least one compound of the invention to said surface. The compounds of the invention may be applied directly or in an alternative form, such as in solution, to said surface. The compounds of the invention may thus be formulated as an antiseptic, disinfectant or antimicrobial agent and used, for example, to prevent, inhibit, or stop the growth of bacteria on surfaces.

Such surfaces may constitute hard surfaces such as, for example, floors, worktops, bathroom surfaces, kitchen surfaces, stone surfaces, wooden surfaces, concrete surfaces, ceramic surfaces, plastic surfaces, glass surfaces, crockery, cutlery, pots, pans, devices such as household devices or medical devices including contact lenses, or soft surfaces, such as, for example, skin, hair, clothing, contact lenses and the like. The compounds of the invention may thus be formulated and used as antiseptics, disinfectants, etc. and/or used as components, additives or preservatives for medical/surgical devices, disinfectants, soaps, shampoos, hand washes, denitrifiers, household cleaning formulations, detergents for laundry and dishes, in wash and treatment solutions for topical use, instruments and devices including contact lenses, and in other disinfecting and antibacterial applications.

The present invention thus further relates to the compounds of the invention for use in preventing, inhibiting, or stopping the growth of bacteria on surfaces, said surfaces including both hard surfaces such as floors, worktops, bathroom surfaces, kitchen surfaces, stone surfaces, wooden surfaces, concrete surfaces, ceramic surfaces, plastic surfaces, glass surfaces, crockery, cutlery, pots, pans, devices such as household devices or medical devices including contact lenses, and soft surfaces such as skin, hair, clothing, contact lenses and the like.

As employed herein, the term "comprising" is to be understood to also cover the alternative in which the product/method/use in respect of which the term "comprising" is used may also "consist exclusively of" the subsequently-described elements.

As employed herein, the term "comprising" is to be understood to also cover the alternative in which the product/method/use in respect of which the term "comprising" is used may also "consist essentially of" the subsequently-described elements.

Unless otherwise indicated, each chemical or composition referred to herein should be interpreted as being a commercial grade material which may contain the isomers, by-products, derivatives, and other such materials which are normally understood to be present in the commercial grade. However, the amount of each chemical component is presented exclusive of any solvent or diluent oil, which may be customarily present in the commercial material, unless otherwise indicated.

Unless stated otherwise, all synthetic processes and parameter measurements are to be understood to have been conducted at room/ambient temperature, i.e. at 21±1 °C.

Unless otherwise indicated, the term "percent" or "%" used in respect of the quantity of a material refers to a percentage by weight (i.e. %(w/w)).

Where employed in the present application, the wording "or any combination of any thereof" is to be understood to mean that all possible combinations of the members of the list preceding said wording are directly and unambiguously disclosed in said list. This formulation represents a mere means of formulating the corresponding text (description or claim) in as concise a manner as possible as required under patent law whilst explicitly making clear that said wording is to be read and understood to directly and unambiguously disclose each and every one of the possible combinations falling arising therefrom. By way of example, a list reading "selected from A, B, C, and any combination of any thereof" is to be understood to be a single list which directly and unambiguously discloses the following alternatives: A, B, C, A+B, A+C, B+C, A+B+C.

Since all numbers, values and/or expressions specifying quantities of materials, ingredients, reaction conditions, molecular weights, number of carbon atoms, and the like, used herein and in the claims appended hereto, are subject to the various uncertainties of measurement encountered in obtaining such values, unless otherwise indicated, all are to be understood as modified in all instances by the term "about." As used herein, the term "about" used in conjunction with a numerical value should be understood to mean within the degree of error of an instrument that commonly would be used by one of ordinary skill in the art to measure the value in the context of the present disclosure and, more particularly, within a range of the stated value where no discernible function or property would be affected such to differ from that same function or property exhibited precisely at the stated value. The term "about" used in conjunction with a numerical value may mean that value ±20%. The term "about" used in conjunction with a numerical value may mean that value ±10%. The term "about" used in conjunction with a numerical value may mean that value ±5%. The term "about" used in conjunction with a numerical value may mean that value ±1%. The term "about" used in conjunction with a numerical value may mean that value ±0.5%.

In the present invention, certain R groups may be substituted or unsubstituted phenyl or 5- or 6-membered heteroaryl (as defined in the claims and/or description). The groups are therefore aromatic. "Aromatic" in the context of the present invention means that, at ambient (room) temperature (21±1 °C) and atmospheric pressure (101.325 kPa), the corresponding ring exists in the aromatic form (in the case of tautomers, in the aromatic tautomeric form) preferably to the degree of at least 10, 20, 30, 40, 50, 60, 70, 80, or 90%. Preferably, "aromatic" means that the corresponding ring exists at room temperature and atmospheric pressure in the aromatic form to the degree of at least 95%, more preferably at least 99%, still more preferably at least 99.5% or at least 99.9%.

During the synthesis of the compounds of formula (I), labile functional groups in the intermediate compounds, e.g. hydroxyl, carboxy and amino groups, may be protected. The protecting groups may be removed at any stage in the synthesis of the compounds of formula (I) or may be present on the final compound of formula (I). A comprehensive discussion of the ways in which various labile functional groups may be protected and methods for cleaving the resulting derivatives is given in, for example, Protective groups in Organic Synthesis, T.W. Greene and P.G.M Wuts, (2006) John Wiley & Sons, Inc., New York, 4th edition.

The preferred groups for variables recited herein in relation to the compounds of formula (I) also apply to the intermediate compounds.

While the preferred groups for each variable have generally been listed above separately for each respective variable, preferred compounds of this invention include those in which several or each variable in formula (I) is selected from the preferred groups for each variable. Therefore, this invention is intended to include all combinations of groups listed as being preferred.

The invention will now be described by reference to the following examples which are for illustrative purposes and are not to be construed as a limitation of the scope of the present invention.

The compounds of the present invention can be synthesised using the synthetic procedures and approaches outlined herein. The synthetic procedures and approaches outlined in, for example, the Examples section of the present application exemplify the syntheses of exemplary compounds of the present invention and these approaches and protocols may be used to access other compounds of the invention. That synthetic routes may have to be modified or the order of synthetic steps changed in order to most easily allow for the introduction of the necessary functionality to allow the specific molecules of the invention to be furnished is clear to the skilled organic chemist. The necessary amendments to the synthetic routes explicitly set forth herein and, for example, reagents etc. required to furnish specific groups and thus compounds of the invention belong to the common knowledge of the person skilled in the art.

By way of example, the compounds of the invention may be accessed from a compound of the following starting formula:

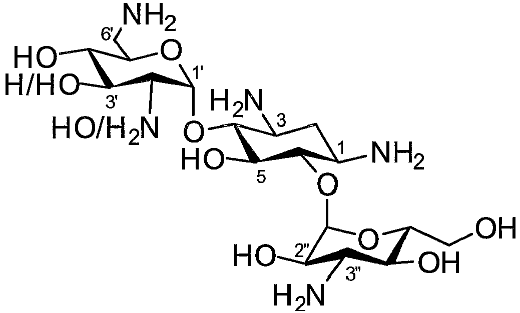

Starting from a compound of the above formula (for example, Tobramycin, Kanamycin A or Kanamycin B), one means of accessing the compounds of the invention may involve the following steps:
1) using protecting-group strategies based on known chemistry (for example, those taught in Protective Groups in Organic Synthesis, T.W. Greene and P.G.M Wuts, (2006) John Wiley & Sons, Inc., New York, 4th edition) and exploiting the known reactivity of the various heteroatoms in the above compound to furnish a compound in which the only unprotected NH₂ group is that at the 1-position. Examples of such protecting group strategies may be the protection of, where present, the 3-, 2'- and 6'-NH₂ groups with tert-Butyloxycarbonyl (Boc) protecting groups and the protection of the 3"-NH₂ group with an orthogonal protecting group such as a trifluoromethyl acetate group;
2) introducing, using known chemistry, the R⁴ group onto the unprotected NH₂ group at the 1-position in either its final form as defined for the compounds of formula (I) or in a suitably protected form known to the skilled person such to enable the remainder of the synthetic process required to furnish the compounds of the invention;
3) using known protecting-group chemistry to produce a compound in which the only unprotected OH group is the OH group at the 5-position. By way of example, this could involve the protection of all other hydroxyl groups in the molecule as their respective acetate esters (using, for example, acetic anhydride and a suitable base);
4) substituting the OH at the 5-position with an F atom using known chemistry for such fluorination reactions, in particular using a fluorination agent such as X-TalFluor-E (also known as (diethylamino)difluorosulfonium tetrafluoroborate);
5) selectively removing the N-protecting group of the NH₂ group at the 3"-position such that the only free NH₂ group is that at said position, the OH protecting groups optionally being simultaneously removed under the same reaction conditions. Where the NH₂ group at the 3"-position is protected with a trifluoromethylacetate and the OH groups are protected as esters such as their respective acetates, this could be achieved using, for example, nucleophilic basic conditions such as methoxide (e.g. sodium methoxide) in a suitable solvent (e.g. methanol);
6) reacting the free NH₂ group at the 3"-position to introduce the desired free (R⁵=H) or substituted/derivatized (R⁵≠H; including R⁵ residues carrying protected amino and/or hydroxyl functionality) guanidine group at said 3"-position using known chemistry for the conversion of NH₂ groups to the corresponding free or substituted/derivatized guanidine. Suitable chemistry for the establishment of the desired guanidine group may involve the reaction of said free 3"-NH₂ group with the appropriate N,N'-(bis-protected)-1-guanylpyrazole, e.g. a N,N'-Bis-boc-1-guanylpyrazole;
7) removal of the remaining protecting groups using known chemistry (for example, that taught in Protective Groups in Organic Synthesis, T.W. Greene and P.G.M Wuts, (2006) John Wiley & Sons, Inc., New York, 4th edition) such to furnish the compound of the invention or a salt thereof.

Where additional functionality or structural moieties are to be introduced into the compounds of the invention (e.g. where R¹ is other than H, where R³ is an OH or ether moiety, etc.) the additional synthetic steps required to introduce such structural moieties and thus ultimately realise the final compounds of the invention can again be based on chemistry well known to the skilled person. Examples of such chemistry are set forth in the Synthesis section herein. These additional steps can, for example, be incorporated into the basic process (steps 1) to 7)) outlined above, the order of the steps in the resultant process being selected dependent on the exact chemistry at hand and belonging to synthetic route planning routine to the skilled person. For example, introduction of an OH group or ether as R³ can be achieved by first oxidising the 3'-OH group to the corresponding carbonyl group and subsequently reacting said carbonyl group with an appropriate nucleophile (e.g. hydride where R²=H; an appropriate organometallic compound such as a Grignard reagent where R²= alkyl, etc.). The resultant R³ OH group can then be further elaborated to produce, *inter alia,* 3'-ether compounds using known chemistry. The above-described oxidation of the 3'-OH group to the corresponding carbonyl can generally be realised directly following step 2) above as said OH group can generally be selectively oxidised without protection of the remaining OH groups in the molecule (suitable oxidation conditions for this transformation can be found, for example, in WO 2013/195149 A1). It may be preferred to carry out the subsequent reaction of this carbonyl group with e.g. hydride (carbonyl reduction) subsequent to step 3 and prior to the deprotection of the resultant protected OH groups or with e.g. a nucleophilic organometallic reagent prior to step 3) as free OH groups may, in some cases (e.g. depending on the specific reagents used and their respect reactivities), interfere with the desired carbonyl reduction/organometallic addition (such synthetic planning belongs to the everyday routine of the skilled organic chemist). However, generally speaking, either reduction of said carbonyl group with sodium borohydride or the addition of Grignard reagents to said carbonyl group can be successfully performed without the need to protect the hydroxyl groups present.

Similarly routine synthetic planning can be used to introduce R¹ groups (where R¹ is other than H) into the compounds of the invention. This may require, for example, the use of orthogonal N-protecting groups such that, for example, following step 1) above, the 6'-NH₂ group is protected with a different (and orthogonal) protecting group to the NH₂ groups at the 2'-position, 3-position and 3"-position (for example, using a carboxybenzyl (Cbz) group to protect the 6'-NH₂ group, Boc groups to protect the 2'-NH₂ and 3-NH₂ groups and a trifluoroacetate group to protect the 3"-NH₂ group). Such protecting group orthogonality allows the flexibility to deprotect and further develop the structure at these centres at different stages of the synthetic route. The introduction of the various R¹ groups at the 6'-NH₂ group can be achieved using chemistry well known in the art.

As alluded to above, the generic synthetic process set forth in steps 1) to 7) above represents only one possible route to the compounds of the invention. As such, alternative synthetic routes including those in which the above-outlined process has been amended in one or more steps may also provide the compounds of the invention. For example, where the introduction of the R⁴ group onto the NH₂ group at the 1-position involves an amide-forming reaction (acylation of the NH₂ group at the 1-position), methods are known in the art which should potentially enable this step to be achieved without the NH₂ group at the 3"-C position requiring to be protected (i.e. it can be achieved in the presence of other nucleophilic amino groups). For example, the use of 1) PyBOP (benzotriazol-1-yl-oxytripyrrolidinophosphonium hexafluorophosphate), or 2) a combination of HOMB (N-Hydroxy-5-norbornene-2,3-dicarboxylic acid imide) and EDC (1-Ethyl-3-(3-dimethylaminopropyl)carbodiimide), as an amide coupling agent together with an appropriate acid coupling partner may allow selective amide formation at the 1-C position over the 3"-C position (such chemistry and appropriate reaction conditions can be found, for example, in US2013/0217642 A1). This may have the advantage of reducing the overall number of steps required in the synthesis. Other alternatives include the introduction of the guanidine functionality at an earlier stage in the overall synthesis, e.g. prior to introduction of the fluoro-substituent at the 5-C position. This is well-tolerated and can be carried through the remainder of the synthesis. An example of a synthetic approach to compounds of the present invention incorporating each of these two modifications is shown in the reaction scheme below.

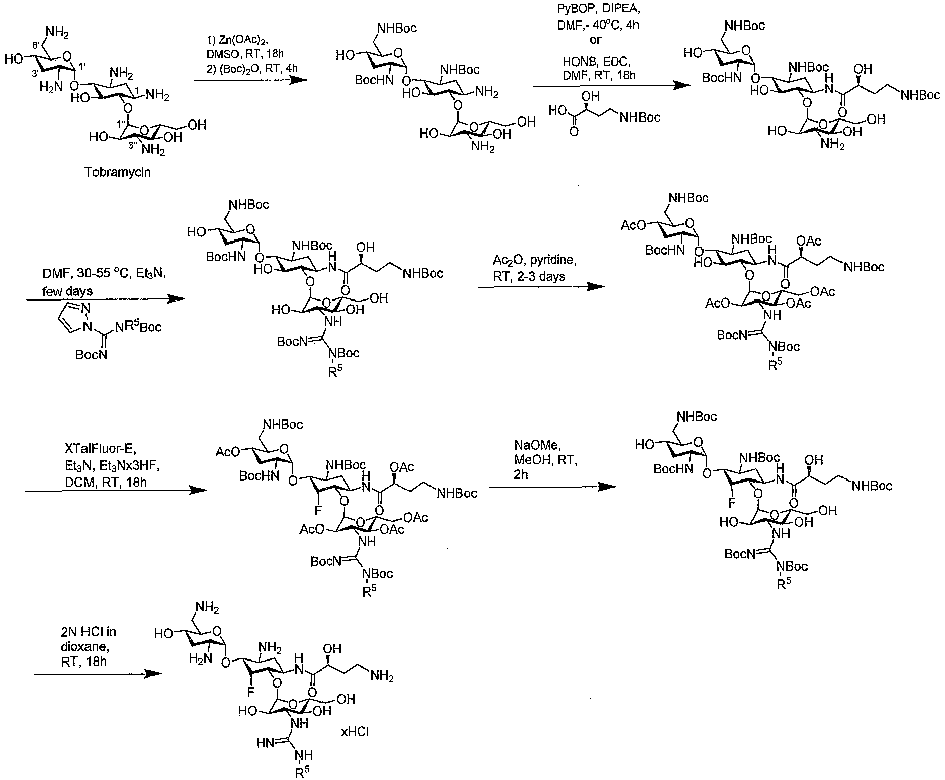

### EXAMPLES

Unless stated otherwise, all synthetic processes and parameter measurements are to be understood to have been conducted at room/ambient temperature, i.e. at 21±1 °C.

Unless otherwise indicated, each chemical or composition referred to herein should be interpreted as being a commercial grade material which may contain the isomers, by-products, derivatives, and other such materials which are normally understood to be present in the commercial grade.

All measurements conducted using instruments listed or mentioned herein were, unless otherwise specified, to be understood to have been performed following standard procedures for conducting such methods such as those well known in the art or as set forth by the manufacturer of said instruments.

Where commercial product names or trademarks are used to describe products mentioned herein, the compositions, properties, configuration etc. of said products are to be understood to be those of the corresponding products available under said names or trademarks on the filing date (priority date where applicable) of the present application.

Unless stated otherwise, all optical density values reported herein were measured at 600 nm using UV/VIS spectroscopy.

### Abbreviations:

- **CLSI**: Clinical Laboratory Standards Institute
- **MIC**: Minimal Inhibitory Concentration
- **MH**: Mueller - Hinton
- **MH2**: Mueller - Hinton 2
- **LB Broth**: Luria Bertani Broth
- **CFU**: Colony Forming Units
- **LOD**: Limit of detection
- **OD**: Optical density
- **rH**: Relative humidity
- **ATCC**: American Type Culture Collection
- **PBS**: Phosphate-buffered saline
- **DMSO**: Dimethyl Sulfoxide
- **DMF**: Dimethylformamide
- **MeOH**: Methanol
- **EtOH**: Ethanol
- **EtOAc**: Ethyl acetate
- **DCM**: Dichloromethane
- **THF**: Tetrahydrofuran
- **LC/MS**: Liquid chromatography-mass spectrometry
- **NMR**: Nuclear magnetic resonance
- **ATP**: Attached proton test
- **HSQC**: Heteronuclear single quantum coherence
- **COSY**: Correlation spectroscopy
- **MS**: Molecular sieve
- **KanaA**: Kanamycin A
- **KanaB**: Kanamycin B
- **AmikaB**: Amikacin B
- **AmikaA**: Amikacin A
- **Tobra**: Tobramycin
- **AHB**: (2S)-(-)-4-Amino-2-hydroxybutyric acid
- **AFB**: (2S)-(-)-4-Amino-2-fluorobutyric acid
- **AHCA**: (2-aminoethyl)(hydroxy)carbamic acid
- **BND**: N-Benzyloxycarbonyloxy-5-norbornene-2,3-dicarboximide
- **TFA**: Trifluoroacetic acid
- **Et₃N**: Triethylamine
- **NaOMe**: Sodium methoxide
- **DIPEA**: N,N-Diisopropylethylamine
- **LDA**: Lithium diisopropylamide
- **Me**: Methyl
- **Et**: Ethyl
- **BQ**: 1,4-Benzoquinone
- **NaBH₄**: Sodium borohydride
- **HATU**: 1-[Bis(dimethylamino)methylene]-1*H*-1,2,3-triazolo[4,5-*b*]pyridinium 3-oxid hexafluorophosphate
- **XtalFluor-E**: (Diethylamino)difluorosulfonium tetrafluoroborate
- **HCl**: Hydrochloric acid
- **Gua**: Guanidine
- **Su**: Succinimide
- **Boc**: tert-Butyloxycarbonyl
- **Cbz**: Carboxybenzyl
- **TBDMS**: tert-Butyldimethylsilyl
- **Ac**: Acetate
- **Tf**: Triflate
- ***tert***: Tertiary
- ***sec***: Secondary
- **t_{R}**: Retention time
- **R_{f}**: Retention factor
- **ACN**: Acetonitrile
- **TSB**: Tryptic soy broth

### A. Materials and Methods

### 1. Organic synthesis

**Methods.** ¹H-, ¹³C-, COSY-, HSQC- spectra were recorded on a Varian Unity Inova spectrometer (500 MHz and 125.7 MHz, respectively) and a Varian AMX400 spectrometer (400, 100.59 MHz, respectively) using CDCl₃, DMSO-*d₆*, MeOD-*d₄* or D₂O as solvent. ¹H-, and ¹⁹F spectra were recorded on a Varian VNMRS NMR 300 MHz spectrometer with a 7.05 Tesla magnet from Oxford Instruments equipped with an indirect detection probe or on a Mercury 300 spectrometer with a 7.05 Tesla magnet from Oxford Instruments equipped with and 4 nuclei auto switchable probe (300 MHz and 282 MHz, respectively) using CDCl₃, DMSO-*d₆*, MeOD-*d₄* or D₂O as solvent. Chemical shift values are reported in ppm with the solvent resonance as the internal standard (CDCl₃: δ7.26 for ¹H, δ77.2 for ¹³C; DMSO-*d₆*: δ2.50 for ¹H, δ39.5 for ¹³C; MeOD-*d₄*: δ3.31 for ¹H, δ49.2 for ¹³C; D₂O: δ4.80 for ¹H). Data are reported as follows: chemical shifts (δ), multiplicity (s = singlet, d = doublet, t = triplet, q =quartet, br = broad, m = multiplet), coupling constants J (Hz), and integration. Liquid chromatography-mass spectrometry (LCMS): System 1: The high-performance liquid chromatography-mass spectrometry (HPLC-MS) spectra were recorded on an Agilent 1200 series HPLC system equipped with a Degasser (G1379B), Binary Pump (G1312B), multi column thermostat (G7116A), autosampler (G1329B), autosampler thermostat (G1330B), UV detector (G1315C), ELSD detector (G7102A) and Agilent 6130 mass detector (G6130B). System 2: The high-performance liquid chromatography-mass spectrometry (HPLC-MS) spectra were recorded on an Agilent 1260 infinity series HPLC system equipped with a degasser (G4225A), binary pump (G1312B), multi column thermostat (G7116A), autosampler (G1329B), autosampler thermostat (G1330B), UV detector (G4212B), ELSD detector (G4260B) and Agilent 6120 mass detector (G6120B). LCMS Method A: Waters Atlantis T3 column (C18 reverse phase silica; 4.6 x 100 mm, 3 µm) with Krudcatcher; 0.05% TFA (aq)/ACN: 80/20 (2.0 min) --> (8.0 min) --> 10/90 (2.0 min) (this description of the LCMS method should be understood to mean that for the first 2 minutes the solvent ratio is steady at 80/20 0.05% TFA (aq)/ACN, followed by an 8 minute period during which the solvent ratio changes slowly until it reaches the final ratio of 10/90 0.05% TFA (aq)/ACN, followed by a final 2 minute period during which the solvent ratio is steady at 10/90 0.05% TFA (aq)/ACN); Flow: 1.0 ml/min; ELSD evaporator temp: 80 °C; ELSD nebulizer temp: 80 °C; Column temperature 22 °C. LCMS Method B: Waters Atlantis T3 column (C18 reverse phase silica; 4.6 x 100 mm, 3 µm) with Krudcatcher; 0.05% TFA (aq)/ACN: 100/20 (2.0 min) --> (3 min) --> 10/90 (4.0 min); Flow: 1.0 ml/min; ELSD evaporator temp: 80°C; ELSD nebulizer temp: 80°C; Column temperature 22 °C. LCMS Method C: Waters Symmetry C18 (C18 reverse phase silica; 4.6 x 250 mm, 5 µm); 0.05% TFA (aq) / ACN: 100/0 (5.0 min) --> (1.0 min) --> 10/90 (2.0 min); Flow: 1.0 ml/min; Column temperature: 30 °C; ELSD: 80 °C, Gain: 4. LCMS Method D: Waters XBridge BEH C18 XP column (C18 reversed phase column with Bridged Ethylene Hybrid (BEH) technology; 2.1 x 50 mm, 2.5 µm); Mobile phase A: Ammonium acetate (10 mM), Water/Methanol/Acetonitrile (900:60:40); Mobile phase B: Ammonium acetate (10 mM); Water/Methanol/Acetonitrile (100:540:360); Mobile phase A / Mobile phase B: 80/20 (0.0 min) --> (1.5 min) --> 0/100 (2.5 min); Flow: 0.6 ml/min; ELSD evaporator temp: 40 °C; ELSD nebulizer temp: 40 °C; Column temperature 35 °C.

**Materials.** All chemicals and reagents were purchased from commercial suppliers (ACROS, Sigma Aldrich, Combi Blocks, Chem Impex, LeapChem CO., Hangzhou DayangChem CO.) and used without further purification. Palladium catalyst [(Neocuproine)PdOAc]₂OTf₂ was prepared according to the literature procedure (N. R. Conley et. al., Organometallics 26 (23), 5447 (2007*) and* G.-J. ten Brink, Adv. Synth. Catal. 345, 1341 (2003*)).* Thin-layer chromatography (TLC) was performed with Merck silica gel 60, 0.25 mm plates and visualization was done by UV, ninhydrin spray (0.2% ninhydrin in ethanol), phosphomolybdic acid (PMA) dip (23.4 g phosphomolybdic acid in 300 ml ethanol) and/or potassium permanganate stain (a mixture of KMnO₄ (3 g), K₂CO₃ (10 g), and water (300 mL)). Column chromatography was performed with silica gel (60-120 mesh). Argon, nitrogen and hydrogen were purchased from Air Products and employed as described below.

### 2. Cloning experiments

### Microbial strain: Escherichia coli ATCC 25922.

**Culture media and equipment.** LB Broth (Lennox, Cat. No. L3022, Sigma); LB Broth with agar (Lennox, Cat. No. L2897, Sigma); Kanamycin A (Cat. No. A1493, AppliChem); CloneJET PCR Cloning Kit (Cat. No. K1231, Thermo Scientific); pBluescript II KS(+) (Cat. No. 212207, Agilent); pET-9b(+) (Cat. No. 69432-3, Merck); pSET152 (Cat. No. PVT3395, Life Science Market); FastDigest SacI (Cat. No. FD1133, Thermo Scientific); FastDigest SalI (Cat. No. FD0644, Thermo Scientific); T4 DNA Ligase (Cat. No. EL0014, Thermo Scientific); Agarose MP (Cat. No. 11388991001, Roche); Tris-borate-EDTA buffer (Cat. No. 106177, Merck); illustra GFX PCR DNA and Gel Band Purification Kit (Cat. No. 28903471, GE Healthcare); GenElute^{™} HP Plasmid Miniprep Kit (Cat. No. NA0160, Sigma); Heating block (Eppendorf Thermomixer C); Incubator (Sartorius Certomat BS-1); UV/VIS meter (Eppendorf BioPhotometer Plus); Centrifuge (Beckman Coulter Avanti J-E). **Cloning resistant enzymes.** An Escherichia coli codon optimized gene encoding AAC(3)-III (GenBank acc.nr. X55652.1; SEQ ID NO:1), including a promoter and terminator sequence, was synthesized by Integrated DNA Technologies, Belgium. The synthesized gene and pBluescript II KS(+) plasmid were digested using the restriction enzymes SacI and Sail, and purified from a 1% TBE-agarose gel using the illustra Gel Band purification kit. The purified linear plasmid and T4 DNA ligase were used to ligate *aac(3)-III* under control of the constitutive lipoprotein Ipp promoter and ribosomal RNA rrnC terminator, resulting in pBlue_lpp_aacC3_rrnC.

**Competent cells.** Heat shock competent *E. coli* cells were prepared by culturing *E. coli* ATCC 25922 from a 20% glycerol (40% v/v glycerol in water was diluted with MH2) storage at -80 °C until an optical density (measured by UV/Vis spectroscopy) of 0.6 at 600 nm in LB broth at 37 °C and 200 rpm was achieved. Bacterial cells were spun down for 5 min at 5000 g, and resuspended in 1/10^{th} original volume of ice-cold TSS (10% PEG 8.000, 5% DMSO, 50 mM MgCl₂ in LB). Aliquots of 100 µL were snap-frozen in liquid nitrogen and stored at -80 °C.

**Plasmid transformation.** Approximately 500 ng of the pET-9b(+) (Cat. No. 69432-3, Merck), pAT21-1 (provided by Prof. Dr. Patrice Courvalin, M.D., Pasteur Institute, France; prepared according to Trieu-Cuot et.al, *Mol. Gen. Genet.* 198(2), 348 (1985)), pBluescript::AAC(6')-Ie/APH(2")-Ia (provided by Prof. Dr. Sergei Vakulenko, M.D., University of Notre Dame, Indiana, USA; prepared according to S. B. Vakulenko et.al., J. Biol. Chem. 287 (52), 43262 (2012)), pBlue_lpp_aacC3_rrnC and pSET152 (Cat. No. PVT3395, Life Science Market; prepared according to Bierman et al., Gene 116 (1), 43 (1992)) plasmids, encoding APH(3')-Ia (SEQ ID NO:2), APH(3')-IIIa (SEQ ID NO:3), AAC(6')Ie-APH(2")-Ia (SEQ ID NO:4), AAC(3)-III (SEQ ID NO:1), and AAC(3)-IV (SEQ ID NO:5) respectively, were individually incubated in a vial of competent cells for 15 min on ice. The cells were subsequently heated to 42 °C for 90 s, and cooled on ice for 1 min. After addition of 900 µL LB, the cells were incubated for 1 h at 37 °C and 200 rpm. Cells were plated on LB-agar supplemented with 50 µg/mL KanaA and incubated for 18 h at 37 °C. The next day, a single colony was inoculated in 5 mL LB supplemented with 50 µg/mL and grown for 18 h at 37 °C and 200 rpm. A 200 µL sample of each culture was diluted with 200 µL of 40% v/v glycerol in water, and stored at -80 °C. The plasmid was purified from the remaining culture using the Plasmid Miniprep kit. Plasmid DNA was investigated by Eurofins Genomics (Germany) to confirm the identity of the aminoglycoside resistance genes.

### 3. Minimum Inhibitory Concentration tests.

### 3.1 Against E. coli strains

**Tested reference compounds.** Amikacin sulfate (Cat. No. 01693, Chem-Impex), Gentamicin sulfate (Cat. No. G4918, Sigma Aldrich).

**Microbial strains.** *E. coli* ATCC 25922, *E. coli* ATCC 25922/pET-9b(+), *E. coli* ATCC 25922/pAT21-1, *E. coli* ATCC 25922/pBluescript: :AAC(6')-Ie/APH(2")-Ia, *E. coli* ATCC 25922/pBlue_lpp_aacC3_rrnC, *E. coli* ATCC 25922/pSET152. **Culture media and equipment.** BBL^{™} Mueller Hinton Broth 2 (Lot. BCBW1143, Cat. No. 70192, Sigma); Kanamycin A (Cat. No. A1493, AppliChem); 96-well deep well plate, 1 mL (Cat. No. 0030506.200, Eppendorf); Plate lids (Cat. No. 0030131.525, Eppendorf); 96-well plate, F-bottom (Cat. No. 781602, Brand; Incubator, Infors Multitron Pro); Laminar flow (Heraeus KS12); UV/VIS meter (Eppendorf BioPhotometer Plus); Platereader (Tecan Sunrise).

**Compound preparation.** Compounds were prepared in MH2 media at a concentration of 5 mM and stored at 4 °C. For testing, solutions of 128 µM were prepared in MH2. Out of these working solutions, 400 µL was transferred to wells in the first column of 96-well deep well plates. Each compound was transferred in triplicate. Plates were previously filled with 200 µL of MH2 media in all wells except for the wells in the first column. After compound and antibiotic addition, the compounds were serial two-fold diluted by transferring 200 µL from the first to the second column, then from the second to the third column and so on. Finally, 200 µL was removed from the last column.

**Inoculum preparation.** Microorganisms used were all revived from 20% glycerol (40% v/v glycerol in water was diluted with MH2) storage at -80 °C by inoculating them in 5 mL MH2. inoculums of E. coli containing a plasmid were selected for by adding 50 µg/mL KanaA. The Inoculums were grown to an optical density (measured by UV/Vis spectroscopy) of 0.6-0.8 at 600 nm. Out of these suspensions, actual inoculums were prepared by diluting them to an optical density (OD) of 0.1 in MH2 media. From these inoculums, 200 µL was transferred to each well, resulting in a final compound concentration range of 64 µM - 31.3 nM. All plates were incubated at 900 rpm and 70 % rH for 18 h at 37 °C.

**MIC determination.** After 18 h of growth, 200 µL was transferred to a 96-well F-bottom plate. The optical density was determined by a plate reader at 600 nm, with 10 s shaking before reading. Triplicate measurements were averaged, and a growth curve was plotted in Microsoft Excel. The first column in which the growth reached half the value of maximum growth was determined as the MIC50 value for that particular compound.

### 3.2 Against selected bacteria of the ESKAPE panel and Enterococcus faecalis (DSM strains)

**Tested reference compounds.** Amikacin sulfate (Cat. No. 01693, Chem-Impex), Gentamicin sulfate (Cat. No. G4918, Sigma Aldrich).

**Microbial strains.** *Enterococcus faecalis* DSM 2570, *Klebsiella pneumoniae* DSM 26371, *Acinetobacter baumannii* DSM 105126, *Enterobacter cloacae* 3MRGN (clinical bronchial aspirate isolate).

**Culture media and equipment.** BBL^{™} Mueller Hinton Broth 2 (Lot. BCBW1143, Cat. No. 70192, Sigma); Trypcase Soy Blood-agar plates (Cat. No. 43001, Biomérieux); Cryoinstant (contains 15% glycerol; Cat. No. 822070ZA, VWR International); GasPak^{™} EZ Standard Incubation Container (Cat. No. 260671, Becton Dickinson); GasPak^{™} EZ Campy Container System Sachets (Cat. No. 260680, Becton Dickinson); 96-well deep well plate, 1 mL (Cat. No. 0030506.200, Eppendorf); Plate lid (Cat. No. 0030131.525, Eppendorf); 96-well plate, F-bottom (Cat. No. 260860, Nunc); Incubator (Sartorius Certomat BS-1); Laminar flow (Heraeus HS12); UV/VIS meter (Perkin Elmer Lambda Bio+); Platereader (Tecan Infinite M200 Pro).

**Compound preparation.** Compounds were prepared in MH2 media at a concentration of 5 mM and stored at 4 °C. For testing, solutions of 128 µM were prepared in MH2, for tests against *E. faecalis* a solution of 512 µM was prepared. Out of these working solutions, 400 µL was transferred to wells in the first column of 96-well deep well plates. Each compound was transferred in triplicate. Plates were previously filled with 200 µL of MH2 media in all wells except for the wells in the first column. After compound and antibiotic addition, the compounds were serial two-fold diluted by transferring 200 µL from the first to the second column, then from the second to the third column and so on. Finally, 200 µL was removed from the last column.

**Inoculum preparation.** Microorganisms used were all revived from 15% glycerol storage in Croinstant tubes at -28 °C by plating them on blood agar plates. *A. baumanni,* and *E. cloacae* were grown aerobically at 37 °C, *E. faecalis* was grown microaerophilically at 37 °C, and *K. pneumoniae* was grown aerobically at 37 °C. Plates were stored at 4 °C for up to two weeks, after which new plates were prepared from -28 °C. A single colony was inoculated in MH2 media and grown at 37 °C and 200 rpm to an optical density (measured by UV/Vis spectroscopy) of 0.6-0.8 at 600 nm. Out of these suspensions, actual inoculums were prepared by diluting them to an optical density of 0.1 in MH2 media. From these inoculums, 200 µL was transferred to each well, resulting in a final compound concentration range of 64 µM - 31.3 nM for, *K. pneumoniae, A. baumanni,* and *E. cloacae,* and 256 µM - 125 nM for *E. faecalis.* Plates inoculated with *E. faecalis* were placed inside a GasPak^{™} Container System with GasPak^{™} Campy Container System sachets. All plates were incubated at 200 rpm for 18 h under the microorganism culturing conditions mentioned above.

**MIC determination.** After 18 h of growth, 200 µL was transferred to a 96-well F-bottom plate. The optical density was determined by a plate reader at 600 nm, with 10 s shaking before reading. Triplicate measurements were averaged, and a growth curve was plotted in Excel. The first column in which the growth reached half the value of maximum growth was determined as the MIC50 value for that particular compound.

### 3.3 Protocol for MIC determination (ATCC strains)

**Tested reference compounds.** Amikacin sulfate (Cat. No. 01693, Chem-Impex), Gentamicin sulfate (Cat. No. G4918, Sigma Aldrich), Azithromycin (Cat. No. 1046056, USP), Ceftazidime Pentahydrate (Cat. No. 1098130, USP), Ciprofloxacin (Cat. No. 1134313, USP), Meropenem (Cat. No. 1392454, USP), Rifampicin (Cat. No. R0079, TCI), Tobramycin (Cat. No. 1667508, USP), Vancomycin (Cat. No. 1709007 USP).

**Microbial strains.** *Staphylococcus aureus* ATCC 29213, *Staphylococcus aureus* ATCC BAA-1717, *Escherichia coli* ATCC BAA-1025, *Klebsiella pneumoniae* ATCC BAA-1705, *Klebsiella pneumoniae* ATCC BAA-2524, *Pseudomonas aeruginosa* ATCC 27853, *Pseudomonas aeruginosa* ATCC BAA-2108, *Acinetobacter baumannii* ATCC BAA-1800.

**Culture media and equipment.** BBL^{™} Mueller Hinton Broth (REF 275730, Lot. 7009699, Becton Dickinson), Mueller Hinton Agar 2 (Ref. No. 97580-500G-F, Lot. No. BCBV4646, Sigma), Dulbecco's Modified Eagle's Medium (DMEM) (Cat. No. 41966-029, Gibco), Minimum Essential Medium (MEM) (Cat. No. 42360-024, Gibco), Fetal bovine serum (FBS) (Cat. No. F7524, Sigma), Non-essential amino acids (NEAA) (100x, Cat. No. 11140-035, Gibco), Sodium Pyruvate (100 mM, 100x, Cat. No. 11360-039, Gibco), Staurosporine (Cat. No. BS0188, Biotrend), Cellstar 96-well plates U-bottom (Cat. No. 650180, Lot. No. E14123EB, Greiner Bio-one), CellTiter-Glo (Cat. No. G7573, Promega), 96-well plate V-bottom (Cat. No. 651201, Greiner), 96-well clear plate, U-bottom, (Cat. No. 650180, Greiner), 96-well white plate with clear bottom (Cat. No. 655098, Greiner), 96-well deep well plate 1 mL (Cat. No. 278606, Nunc), Sodium Pyruvate (100 mM, 100x, Gibco, Cat# 11360-039), Incubator (Binder), Laminar flow (Thermo Scientific), BioPhotometer (Eppendorf); DEN-1B (McFarland Densitometer, Biosan); McFarland standards (polymer microparticle suspension, Biosan).

**Compound preparation.** The test compounds and reference antibiotics were prepared according to CLSI guidelines (Clinical and Laboratory Standards Institute (CLSI); Methods for dilution antimicrobial susceptibility tests for bacteria that grow aerobically; approved standard - ninth edition, M07-A9, Vol. 32, No. 2, 2012) as 5 mg/mL stock solutions in DMSO. Out of these DMSO solutions, 43.5 µL was transferred to 1656.5 µL of MH media in a deep well plate. Out of these working solutions 100 µL was transferred to wells in the third column of 96-well plates. Plates were previously filled with 50 µL of MH media in all wells except for the wells in the third column. After compound and antibiotic addition, 50 µL was transferred from the third to the fourth column, then from the fourth to the fifth and so on. In this manner, the compounds and antibiotics were plated in 96-well plate in serial two-fold dilutions giving a final concentration range of 64 - 0.125 µg/mL.

**Preparation of microorganisms.** Designations used for clinical isolates were from the Fidelta strain collection bank. Microorganisms used were all revived from skim milk storage at -70 °C by plating them on MH agar plates. The following day the single colony of each microorganism was again streaked on fresh agar plates. The next day, using the direct colony suspension method, broth solutions that achieve turbidity equivalent to 0.5 McFarland standard for each microorganism were prepared. This resulted in suspensions containing 1-2×10⁸ CFU/mL. Out of these suspensions, actual inoculums were prepared by diluting them 100x with MH media giving a final microorganism count of 2-8×10⁵ CFU/mL. For each strain of microorganisms, 20 mL of these inoculum solutions were prepared. From the second to the twelfth column of 96-well plates, 50 µL of these solutions were transferred per well. To the first column, 50 µL per well of pure growth media was added. In this manner the first column was used as sterility control of media, the second column was used as control of microorganism's growth and the rest of the plate was used for MIC determination. All plates were incubated for 16-24 h at 37 °C.

**MIC determination.** The MIC value was determined by visual inspection of bacterial growth within 96-well plates. The first column in which there was no visible growth of bacteria was determined as the MIC90 value for the test compound or reference antibiotic tested in that particular row. ATCC strains were used as referent strains for which there are determined values of MIC values for reference antibiotics. The assay was considered valid when MIC values for the reference antibiotics were within the CLSI designated range (Clinical and Laboratory Standards Institute (CLSI); Performance standards for antimicrobial susceptibility testing; twenty-third informational supplement, M100-S23, Vol.33, No. 1, 2013) for tested ATCC strains.

### 3.4 Protocol for MIC determination (clinical isolates)

### 3.4.1 MIC determination at Uppsala University (Sweden)

**Tested reference compounds.** Amikacin disulfate (Cat. No. A1774, Sigma Aldrich), Apramycin sulfate (Cat. No. A2024, Sigma Aldrich) and Gentamicin sulfate (Cat. No. G1914, Sigma Aldrich).

**Bacterial strains/isolates.** All assayed laboratory and clinical strains (P. *aeruginosa* PA01, *A. baumanii* ATCC 19606, *A. baumanii* 195N(a), *A. baumanii* 48F, *E. coli* C1162,, *E. coli* C1181, *M. morgannii* S49 and *P. stuartii* B8-1) were from the IMI ND4BB ENABLE consortium strain collection. ATCC laboratory strains were acquired directly from the American Type Culture Collection. Clinical strains used were previously published (M Juhas, et al. J. Antimicrob. Chemother., 74 (4), 944-952 (2019); D. Hughes et al., Antimicrob. Agents Chemother., 47 (10), 3222-3232 (2003)) and were resistance genotyped within the ENABLE consortium.

**Culture media and equipment.** Water (Thermo Scientific, R05819); Mueller-Hinton II broth (BD Ref. 212322); sterile round-bottom 96-well plates (Thermo Scientific, 262162); Sensititre Nephelometer (ThermoFisher Scientific, V3011); 0.5 McFarland Standard (ThermoFisher Scientific, E1041); sterile polyester film (VWR, 391-1251).

**Compound preparation.** MIC assay procedure follows the CLSI M07 standard method (Clinical and Laboratory Standards Institute (CLSI); Methods for dilution antimicrobial susceptibility tests for bacteria that grow aerobically; approved standard - eleventh edition, M07-A10, Vol. 38, No. 2, 2018). Test compounds were received as solids and were dissolved in water to a concentration of 10 mg/mL of active compound. The concentrated compound solutions were diluted to 128 µg/mL by adding 12.8 µL of the concentrated compound to 1 mL of liquid Mueller-Hinton II broth freshly prepared according to manufacturer specifications. This solution was serially diluted in 2-fold steps in the first 10 columns of sterile round-bottom 96-well plates, while the final two columns were filled with Mueller-Hinton II broth without added compound for use as positive and negative growth controls.

**Inoculum preparation.** Inoculums were prepared by suspending several colonies freshly streaked culture of test strains in 5 mL of sterile 0.9% NaCl. The cell density of the inoculum solution was assessed and adjusted using a Sensititre Nephelometer and a 0.5 McFarland Standard. The inoculum was diluted 1:100 in Mueller-Hinton II broth and 50 µL of this inoculum solution was added to 50 µL of the test compound solutions yielding a concentration range of 64 - 0.125 µg/mL. The plates were sealed with sterile polyester film and stationarily incubated at 37° C for 18 hours.

**MIC determination.** The MIC value was determined by visual inspection of bacterial growth within 96-well plates. The lowest concentration completely inhibiting growth was reported as the MIC for each strain and compound combination.

### 3.4.2 MIC determination at Fidelta (Croatia)

**Tested reference compounds.** Amikacin sulfate (Cat. No. 01693, Chem-Impex), Gentamicin sulfate (Cat. No. G4918, Sigma Aldrich), Ceftazidime Pentahydrate (Cat. No. 1098130, USP), Meropenem (Cat. No. 1392454, USP), Tobramycin (Cat. No. 1667508, USP).

**Microbial strains.** *Klebsiella pneumoniae* KP-1919 (abdomen), *Klebsiella pneumoniae* KP-1935 (urine), *Klebsiella pneumoniae* KP-1936 (abscess), *Klebsiella pneumoniae* KP-1937 (blood), *Klebsiella pneumoniae* KP-1942 (urine), *Klebsiella pneumoniae* KP-1944 (blood), *Klebsiella pneumoniae* KP-2027 (tracheal aspirate), *Klebsiella pneumoniae* KP-2029 (urine), *Klebsiella pneumoniae* KP-2030 (trachea), *Klebsiella pneumoniae* KP-2031 (skin), *Klebsiella pneumoniae* KP-2032 (urine), *Klebsiella pneumoniae* KP-2033 (urine), *Klebsiella pneumoniae* KP-2034 (urine), *Klebsiella pneumoniae* KP-2035 (urine), *Klebsiella pneumoniae* KP-2036 (urine), *Klebsiella pneumoniae* KP-2037 (urine), *Klebsiella pneumoniae ATCC 43816, Pseudomonas aeruginosa* PA-1948 (wound), *Pseudomonas aeruginosa* PA-1949 (urine), *Pseudomonas aeruginosa* PA-1950 (sputum), *Pseudomonas aeruginosa* PA-1952 (wound), *Pseudomonas aeruginosa* PA-1953 (urine), *Pseudomonas aeruginosa* PA-1954 (sputum), *Pseudomonas aeruginosa* PA-1967 (blood), *Pseudomonas aeruginosa* ATCC27853, *Acinetobacter baumannii* AB-1931 (urine), *Acinetobacter baumannii* AB-1932 (urine), *Acinetobacter baumannii* AB-2017 (bronchial aspirate), *Acinetobacter baumannii* AB-2018 (skin), *Acinetobacter baumannii* AB-2019 (bronchial aspirate), *Acinetobacter baumannii* AB-1964 (blood), *Acinetobacter baumannii* AB-2025 (skin), *Acinetobacter baumannii* AB-2026 (skin), *Acinetobacter baumannii ATCC17978, methicillin-resistant Staphylococcus aureus MRSA-1995* (liquor), *methicillin-resistant Staphylococcus aureus MRSA-1998* (wound), *methicillin-resistant Staphylococcus aureus MRSA-1999* (skin), *methicillin-resistant Staphylococcus aureus MRSA-2003* (wound), *vancomycin-resistant Enterococcus faecium VRE-2005* (urine), *vancomycin-resistant Enterococcus faecium VRE-2006* (urine), *vancomycin-resistant Enterococcus faecium VRE-2007* (urine), *vancomycin-resistant Enterococcus faecium VRE-2008* (urine), *vancomycin-resistant Enterococcus faecium VRE-2009* (urine), *vancomycin-resistant Enterococcus faecium VRE-2010* (urine), *vancomycin-resistant Enterococcus faecium VRE-2011* (rectum), *vancomycin-resistant Enterococcus faecium VRE-2012* (rectum), *vancomycin-resistant Enterococcus faecium VRE-2013* (rectum).

**Culture media and equipment.** BBL^{™} Mueller Hinton Broth (REF 275730, Lot. 7009699, Becton Dickinson); Mueller Hinton Agar 2 (Ref. No. 97580-500G-F, Lot. No. BCBV4646, Sigma); Cellstar, 96-well plates, U-bottom (Cat. No. 650180, Lot. No. E14123EB, Greiner Bio-one); 96- well deep well plate, 1 mL (Cat. No. 278606, Nunc); Incubator (Binder); Laminar flow (Thermo Scientific); BioPhotometer (Eppendorf); DEN-1B (McFarland Densitometer, Biosan); McFarland standards (polymer microparticle suspension, Biosan). **Compound preparation.** For testing, 5 mg/mL DMSO solutions of the test compounds and reference antibiotics were used. Out of these DMSO solutions, 43.5 µL was transferred to 1656.5 µL of MH media in a deep well plate. Out of these working solutions 100 µL was transferred to wells in the third column of 96-well plates. Plates are previously filled with 50 µL of MH media in all wells except for the wells in the third column. After compound and antibiotic addition, 50 µL was transferred from the third to the fourth column, then from the fourth to the fifth and so on. In this manner, the compounds and antibiotics were plated in 96-well plate in serial two-fold dilutions giving a final concentration range of 64 - 0.125 µg/mL.

**Inoculum preparation.** Designations used for clinical isolates were from Fidelta strain collection bank. Microorganisms used were all revived from skim milk storage at -70 °C by plating them on MH agar plates. The following day the single colony of each microorganism was again streaked on fresh agar plates. The next day, using the direct colony suspension method, broth solutions that achieve turbidity equivalent to 0.5 McFarland standard for each microorganism were prepared. This resulted in suspensions containing 1-2×10⁸ CFU/mL. Out of these suspensions, actual inoculums were prepared by diluting them 100x with MH media giving a final microorganism count of 2-8×10⁵ CFU/mL. For each strain of microorganisms, 20 mL of these inoculum solutions were prepared. From the second to the twelfth column of 96-well plates, 50 µL of these solutions were transferred per well. To the first column, 50 µL per well of pure growth media was added. In this manner the first column was used as sterility control of media used, the second column was used as control of microorganism's growth and the rest of the plate was used for MIC determination. All plates were incubated for 16-24 h at 37 °C.

**MIC determination.** The MIC value was determined by visual inspection of bacterial growth within 96-well plates. The first column in which there was no visible growth of bacteria was determined as the MIC90 value for the compound tested in that particular row.

### 3.4.3 MIC determination at University Medical Center Groningen (The Netherlands)

**Tested reference compounds.** Amikacin sulfate (Cat. No. 01693, Chem-Impex), Gentamicin sulfate (Cat. No. G4918, Sigma Aldrich).

**Bacterial strains/isolates.** *Pseudomonas aeruginosa* ATCC 27853, *Escherichia coli* ATCC 25922, *Klebsiella pneumonia* ATCC43816, *Acinetobacter baumannii ATCC17978,* 9 clinical isolates of *Klebsiella pneumonia* (origin: 4 unknown, 3 urine, 1 sputum, 1 catheter tip), 7 clinical isolates of *Pseudomonas aeruginosa* (origin: 3 unknown, 1 wound, 1 catheter, 1 faeces, bronco alveolar liquid = BAL), 8 clinical isolates of *Escherichia coli* (origin: 2 bile liquid, 2 rectum, 1 throat, 2 blood culture, 1 sputum), 4 clinical isolates of *Acinetobacter baumannii* (origin: 2 blood culture, 1 sputum, 1 bone).

**Culture media and equipment.** As indicated by EUCAST guidelines (European Committee on Antimicrobial Susceptibility Testing (EUCAST); Media preparation for EUCAST disk diffusion testing and for determination of MIC values by the broth microdilution method; Version 5.0, 2017), Mueller Hinton Broth, CM0405, (Oxoid) was used for the broth micro-dilutions; and Mueller Hinton Agar, CM0337, (Oxoid) was used for the plating on agar. The testing assays were conducted in 96-well plates, with U-bottom shape (Greiner Bio-one); All bacteria strains/isolates used were handled in a Laminar flow, Clean Air (Baker). A CO₂ incubator commercialized by Sanyo was used. Finally, spectrophotometer measurements were conducted using a Synergy 2 Multi-detection Microplate Reader (BioTek).

**Compound preparation.** The compounds were weighed and dissolved into MilliQ water. These concentrated stock aliquots were then further diluted to reach the desired testing concentrations. Serial dilutions were made in order to achieve the ten desired final concentrations for the tested compounds, that ranged between 64 - 0.125 µg/mL. The concentrations were calculated in respect to the 150 µL of final volume used for each of the 96-wells. Of these dilutions, 100 µL was added in each testing well of a sterile 96-well plate. All compound dilutions were plated in triplicate, while duplicates were used for the two reference antibiotics.

**Inoculum preparation.** The clinical isolates were revived from Luria Bertani medium storage at -80 °C by plating them overnight on MH agar plates. The following day, a few colonies were picked and inoculated in small glass bottles containing 10 mL MH broth. Growth was subsequently allowed for approx. 3 hours, at 35 °C with 250rpm shaking, until the isolates reached late logarithmic growth phase. At this point culture solutions were prepared with an OD₆₀₀ (optical density measured at 600 nm; measured using Synergy 2 Multi-detection Microplate Reader; BioTek) of 0.05. This resulted in starting bacterial suspensions containing 2-8×10⁵ CFU/mL. 50 µL of culture suspension was added to each of the wells of the 96 well plate previously loaded with the desired antibiotic/medium solution, reaching a final total volume of 150 µL per well. As controls, blanks of the MH medium and of culture were added to the 96-well plate. Plates were incubated in a plate incubator in static conditions at 35 °C for 18 hours.

**MIC determination.** After the incubation time, the MIC value was determined by visual inspection of bacterial growth within 96-well plates. The first column in which there was no visible growth of bacteria was determined as the MIC90 value for the compound tested in that particular row. Additionally, spectrophotometer readouts at 600 nm were recorded for each well.

### 3.5 MIC value reporting

**MIC50.** The MIC50 of a compound was assigned at which the bacterial growth reached half the value of maximum growth.

**MIC90.** The MIC90 of a compound was assigned at which no visible growth of bacteria was determined.

A MIC value given as a concentration range, e.g. 1-2, 2-4, 4-8 etc., which results from two subsequent dilution steps, means that the actual MIC is higher than the lower value of the concentration range but lower than the higher value of this range, i.e. for an MIC range reported as 1-2, this range to be understood to mean "a value between 1 and 2" and thus does not include the explicitly recited end points of 1 and 2.

### 4. In-vivo efficacy

### 4.1 Murine neutropenic thigh model of infection (Klebsiella pneumoniae ATCC 43816)

All animal-related research was conducted in accordance with 2010/63/EU and National legislation regulating the use of laboratory animals in scientific research and for other purposes (Official Gazette **55/13).** An Institutional Committee on Animal Research Ethics (CARE-Zg) oversees that animal-related procedures do not compromise animal welfare.

In vivo studies and CFU determination were performed by Fidelta (Zagreb, Croatia) and all mice were obtained from Charles River Laboratories (Italy).

Four compounds, i.e. ABX5006, ABX5020, ABX5026 and ABX5039 were tested in two separate studies (Table 12 for ABX5006 and ABX5026, Table 13 for ABX5026 and ABX5039) using a mouse model of thigh infection with *Klebsiella pneumoniae* ATCC 43816. A comparator antibiotic (meropenem; Cat. No. 1392454, USP) was used during both studies.

Neutropenic thigh model: Pathogen-free male CD-1 mice (6 mice per dosing group) weighing 36f4 g were used. Prior to infection, neutropenia was induced in mice by two cyclophosphamide doses applied intraperitoneally four days (150 mg/kg body weight) and one day prior infection (100 mg/kg body weight).

Inoculum size and dose of meropenem (broad-spectrum antibiotic) - pharmacological standard were chosen based on previous titration and validation studies that determined the maximum number of CFU that could be injected without subsequent mortality during the course of the experiment. For subcutaneous treatment, pre-weighed test item powder was dissolved in PBS and the pH was adjusted (to reach a near-neutral pH), to reach the desired final concentration.

During the first study ABX5006 and ABX5020 were tested at a dose of 64 mg/kg (group 5 for ABX5006, group 8 for ABX5020), 16 mg/kg (group 4 for ABX5006, group 7 for ABX5020) and of 4 mg/kg (group 3 for ABX5006, group 6for ABX5020).

During a second study ABX5026 and ABX5039 were tested at a dose of 4 mg/kg (group 5 for ABX5026, group 8 for ABX5039), 1 mg/kg (group 4 for ABX5026, group 7 for ABX5039) and of 0.25 mg/kg (group 3 for ABX5026, group 6 for ABX5039).

Each mouse was infected with *Klebsiella pneumoniae* ATCC 43816 (6.0 × 10³/thigh), under light ketamine/xylazine anesthesia, by intramuscular administration of 0.10 mL of a suspension containing bacteria into both thighs. Animals were dosed subcutaneously (10 mL/kg) twice with 8 h interval. Treatment was started 1 h following infection either with Vehicle - PBS (group 1) or meropenem 100 mg/kg (group 2) or test compounds (groups 3-8) as described above. All mice were overdosed with ketamine/xylazine 24 hours post infection. Thighs were aseptically removed and separately placed into sterile Precellys test tubes containing 2 mL of sterile PBS. Precellys test tubes were weighed and the weight was recorded prior and post-sampling. Each thigh was homogenized in sterile PBS using Ultraturax, IKA. After homogenizing, a serial dilution of the homogenates was used for CFU determination. A CFU count of each thigh acted as a single result. A CFU count below limit of detection (LOD=1.0 × 102 CFU/mL) was substituted with the value 0 in order to perform statistical analysis.

**Supplies/Chemicals.** BBL^{™} Mueller Hinton Broth (REF 275730, Becton Dickinson); BBL^{™} Mueller Hinton Agar 2 (REF 211438, Becton Dickinson); PBS (Cat. No. P4417, Sigma); Saline (Serial No. 1422115, HZTM); Xylazine 2% (Alfasan, International B.V.); Narkamon (100mg/mL ketamine chloride, Bioveta); Cyclophosphamide monohydrate (No. C2236, TCI); Sterile, single use needles (BD MICROLANCE Kanuele, 27 G, REF 300220, Becton Dickinson); 5 mL syringes (BD Plastipak, REF 300013, Becton Dickinson).

**Anesthesia.** 1 mL of Narkamon, 0.2 mL of Xylazine and 9 mL of saline; Dose volume: 10 mL/kg body weight.

**Inoculum preparation for intramuscular infection.** The strain was streaked out onto MH agar. On the following day Mueller-Hinton broth was inoculated with several colonies of Klebsiella pneumoniae and incubated at 37 °C in an orbital shaker until log phase of growth was reached (OD₆₀₀ = 0.6). Log bacteria was centrifuged and suspended in equal volume of sterile PBS and subsequently diluted to obtain a bacterial suspension of adequate inoculum size.

**CFU determination.** Thigh samples were homogenized using a Precellys Evolution homogenizer (Bertin Instruments) following the program for hard tissue (Tube: 7 mL; Speed: 6800 RPM; Cycle: 4x15 s; Pause: 5 s). Upon homogenization, 100 µL of homogenates is transferred to 96 well U-bottom plates where serial dilutions in PBS are made by transferring 10 µL of homogenates to 90 µL of PBS. 9 serial dilutions are made in that manner out of which 10 µL were used for inoculation of agar plates in 5 replicates. Agar plates are then incubated overnight at 37°C and CFU counted.

### 4.2 Murine neutropenic thigh model of infection (Klebsiella pneumoniae ATCC BAA-1705)

All animal-related research is conducted in accordance with 2010/63/EU and National legislation regulating the use of laboratory animals in scientific research and for other purposes (Official Gazette **55/13).** An Institutional Committee on Animal Research Ethics (CARE-Zg) oversees that animal-related procedures do not compromise animal welfare.

In vivo studies and CFU determination were performed by Fidelta (Zagreb, Croatia) and all mice were obtained from Charles River Laboratories (Italy). Two compounds, i.e. ABX5006 and ABX5020, and a comparator antibiotic (amikacin sulfate; Cat. No. 01693, Chem-Impex) were tested in a mouse model of thigh infection with *Klebsiella pneumoniae* ATCC BAA-1705. Neutropenic thigh model: Pathogen-free male CD-1 mice (6 mice per dosing group) weighing 36f4 g were used. Prior to infection, neutropenia was induced in mice by two cyclophosphamide doses applied intraperitoneally four days (150 mg/kg body weight) and one day prior to infection (100 mg/kg body weight).

Inoculum size and dose of antibiotic were chosen based on previous titration and validation studies that determined the maximum number of CFU that could be injected without subsequent mortality during the course of the experiment. For subcutaneous treatment, pre-weighed test item powder (ABX5006) was dissolved in PBS and the pH was adjusted (to reach a near-neutral pH), to reach a final concentration of 6.4 mg/mL for a dose of 64 mg/kg (group 3) or 1.6 mg/mL for a dose of 16 mg/kg (group 2). For subcutaneous treatment, pre-weighed test item powder (ABX5020) was dissolved in PBS and the pH was adjusted (to reach a near-neutral pH), to reach a final concentration of 6.4 mg/mL for a dose of 64 mg/kg (group 7) or 1.6 mg/mL for a dose of 16 mg/kg (group 6). For subcutaneous treatment, pre-weighed reference compound (amikacin sulfate) was dissolved in PBS and the pH was adjusted (to reach a near-neutral pH), to reach a final concentration of 6.4 mg/mL for a dose of 64 mg/kg (group 5) or 1.6 mg/mL for a dose of 16 mg/kg (group 4). Each mouse was infected with *Klebsiella pneumoniae* ATCC BAA-1705 (1.18 × 10⁶/thigh), under light ketamine/xylazine anesthesia, by intramuscular administration of 0.10 mL of a suspension containing bacteria into both thighs. Animals were dosed subcutaneously twice with 8 h interval. Treatment was started 1 h following infection either with Vehicle - PBS (group 1) or amikacin sulfate (groups 4-5) or test compounds (groups 2-3 and 6-7) as described above. All Mice were overdosed with ketamine/xylazine 24 hours post infection. Thighs were aseptically removed and separately placed into sterile Precellys test tubes containing 2 mL of sterile PBS. Precellys test tubes were weighed and the weight was recorded prior and post-sampling. Each thigh was homogenized in sterile PBS using Ultraturax, IKA. After homogenizing, a serial dilution of the homogenates was used for CFU determination. A CFU count of each thigh acted as a single result.

**Supplies/Chemicals.** BBL^{™} Mueller Hinton Broth (REF 275730, Becton Dickinson); BBL^{™} Mueller Hinton Agar 2 (REF 211438, Becton Dickinson); PBS (Cat. No. P4417, Sigma); Saline (Serial No. 1422115, HZTM); Xylazine 2% (Alfasan, International B.V.); Narkamon (100mg/mL ketamine chloride, Bioveta); Cyclophosphamide monohydrate (No. C2236, TCI); Sterile, single use needles (BD MICROLANCE Kanuele, 27 G, REF 300220, Becton Dickinson); 5 mL syringes (BD Plastipak, REF 300013, Becton Dickinson).

**Anesthesia.** 1 mL of Narkamon, 0.2 mL of Xylazine and 9 mL of saline; Dose volume: 10 mL/kg body weight.

**Inoculum preparation for intramuscular infection.** The strain was streaked out onto MH agar. On the following day Mueller-Hinton broth was inoculated with several colonies of Klebsiella pneumoniae and incubated at 37 °C in an orbital shaker until log phase of growth was reached (OD₆₀₀ = 0.6). Log bacteria was centrifuged and suspended in equal volume of sterile PBS and subsequently diluted to obtain a bacterial suspension of adequate inoculum size.

**CFU determination.** Thigh samples were homogenized using a Precellys Evolution homogenizer (Bertin Instruments) following the program for hard tissue (Tube: 7 mL; Speed: 6800 RPM; Cycle: 4x15 s; Pause: 5 s). Upon homogenization, 100 µL of homogenates is transferred to 96 well U-bottom plates where serial dilutions in PBS are made by transferring 10 µL of homogenates to 90 µL of PBS. 9 serial dilutions are made in that manner out of which 10 µL were used for inoculation of agar plates in 5 replicates. Agar plates are then incubated overnight at 37°C and CFU counted.

### 4.3 Urinary tract infection model (Escherichia coli ATCC700336)

All animal-related research was conducted in accordance with 2010/63/EU and National legislation regulating the use of laboratory animals in scientific research and for other purposes (Official Gazette **55/13).** An Institutional Committee on Animal Research Ethics (CARE-Zg) oversees that animal-related procedures do not compromise animal welfare.

In vivo studies and CFU determination were performed by Fidelta (Zagreb, Croatia) and all mice were obtained from Janvier Labs (France).

Two compounds, i.e. ABX5006 and ABX5026, and a comparator antibiotic (gentamicin sulfate; Cat. No. G4918, Sigma Aldrich) were tested in a mouse model of urinary tract infection with *Escherichia coli* ATCC700336.

**Urinary tract infection model:** Pathogen-free female C3H/HeNRj mice (8 mice per dosing group) weighing 24±4 g were used.

Inoculum size and dose of antibiotic were chosen based on previous titration and validation studies that determined the maximum number of CFU that could be injected without subsequent mortality during the course of the experiment.

For subcutaneous treatment, pre-weighed test item powder (ABX5006) was dissolved in PBS and the pH was adjusted (to reach a near-neutral pH), to reach a final concentration of 1.5 mg/mL for a dose of 15 mg/kg (group 5) or 3.0 mg/mL for a dose of 30 mg/kg (group 4). For subcutaneous treatment, pre-weighed test item powder (ABX5026) was dissolved in PBS and the pH was adjusted (to reach a near-neutral pH), to reach a final concentration of 1.5 mg/mL for a dose of 15 mg/kg (group 7) or 3.0 mg/mL for a dose of 30 mg/kg (group 6). For subcutaneous treatment, pre-weighed reference compound (gentamicin sulfate) was dissolved in 0.9% sodium chloride solution, to reach a final concentration of 1.5 mg/mL for a dose of 15 mg/kg (group 3) or 3.0 mg/mL for a dose of 30 mg/kg (group 2). Each mouse was infected transurethral with *Escherichia coli* ATCC700336 (5.0 × 10⁹ CFU/100 µL/mouse), under light ketamine/xylazine anesthesia. Mice were water deprived 1.5 hours prior to infection and 1 hour post infection.

Animals were dosed subcutaneously once either with gentamicin (groups 2-3), ABX5006 (groups 4-5), ABX5026 (groups 6-7) or vehicle - PBS (group 1) 24 hours post infection.

All Mice were overdosed with ketamine/xylazine 24 hours post infection. Both kidneys were aseptically removed and placed into pre-weighed sterile Precellys test tube containing 2 mL of sterile PBS. Post-sampling, Precellys test tubes were weighed once again. Kidneys were homogenized in sterile PBS by Precellys Evolution, homogenizer, BERTIN Technologies, and serially diluted for CFU determination. Kidney pairs of one animal will represent one sample. **Supplies/Chemicals.** Tryptic Soy Agar (BD Cat.no. 236950, Becton Dickinson), Trypticase Soy Broth (BD Cat.no. 211768, Becton Dickinson), 2 mL syringes (BD Plastipak, Ref. 300185, Becton Dickinson), 1 mL syringes (BD Plastipak, Ref. 300013, Becton Dickinson), Sterile, single use needles (BD MICROLANCE, 27 G ¾, Nr. 20 ref. 300220, Becton Dickinson), Sterile, single use needles (BD MICROLANCE, 25 G 1, Nr. 18 ref. 300400, Becton Dickinson), Narkamon (100mg/mL ketamine chloride) (Bioveta, a.s., Czech Republic), Xylazine 2% (Alfasan International B.V., Woerden, Netherlands), Saline (HZTM, serial no.: 1422115), PBS (Sigma, cat. no. P4417, Germany), Precellys lysing kit (ref. no. KT03961-1-302.7, Bertin technologies, France). **Anesthesia.** 1 mL of Narkamon, 0.2 mL of Xylazine and 9 mL of saline; Dose volume: 10 mL/kg body weight.

**Inoculum preparation for intramuscular infection.** *Escherichia coli ATCC 700336* was grown on Tryptic Soy Agar plate and incubated for 18-20h at 37°C. Bacterial agar growth was suspended to 1.0 × 10¹⁰ colony-forming units (CFU)/mL in adequate volume of tryptic soy broth (TSB), and the inoculum was held at 4°C until it was used for infection.

**CFU determination.** Kidney pairs were homogenized using a Precellys Evolution homogenizer (Bertin Instruments). Upon homogenization, 100 µL of homogenates is transferred to 96 well U-bottom plates where serial dilutions in PBS are made by transferring 10 µL of homogenates to 90 µL of PBS. 9 serial dilutions are made in that manner out of which 10 µL were used for inoculation of agar plates in 5 replicates. Agar plates are then incubated overnight at 37°C and CFU counted.

### 4.4 Maximum tolerated dose (MTD) study in male CD (Sprague Dawley) rats.

The study was performed in an AAALAC I - approved Facility. The standard study plan relating to this study was reviewed by the Ethical Committee (CARE - Zagreb) as required by International Laws/Regulations and Croatian Low on Animal Welfare ("The Animal Protection Act", Official Gazette, NN 37/13) and Animal Welfare Officer.

In vivo studies were performed by Fidelta (Zagreb, Croatia) and all rats (Sprague Dawley) were obtained from Charles River Laboratories (Italy). Three compounds, i.e. ABX5006, ABX5020and ABX5039 were tested to determine the MTD of the test articles in male CD (Sprague Dawley) rats following a one-time dose (IV).

**MTD study:** Pathogen-free male CD (Sprague Dawley) rats (3 rats per dosing group) weighing 225-260 g were used. Five different dosing groups (50 mg/kg, 75 mg/kg, 100 mg/kg, 150 mg/kg and 200 mg/kg) were used and the test article was administered intravenous over 15 min (slow injection, 5 mL/kg).

For intravenous administration, pre-weighed test item powder was dissolved in PBS and the pH was adjusted (to reach a near-neutral pH), to reach a final concentration of 10.0 mg/mL for a dose of 50 mg/kg (group 2), 15.0 mg/mL for a dose of 75 mg/kg (group 3), 20.0 mg/mL for a dose of 100 mg/kg (group 4), 30.0 mg/mL for a dose of 150 mg/kg (group 3) or 40.0 mg/mL for a dose of 200 mg/kg (group 6). All rats were observed for 30 min postadministration and again after 1 hour, 4 hours and 8 hours, and twice daily for the remaining duration of the study (4 days). Clinical signs, body weight, food consumption and mortality was recorded during the in-life phase of the study. All animals surviving to the end of the 4 day monitoring period were euthanized on day 4. Terminal studies included gross necropsy and organ weight recording of all animals.

**Supplies/Chemicals.** Sterile, single used needles (25 G Microlance, REF300400, Becton Dickinson); 2 mL syringes (REF300185, Becton Dickinson); Phosphate buffered saline (P3813, Sigma Aldrich); isoflurane anesthesia (Abbott, Netherlands)

**Anesthesia.** Animals were deeply anaesthetized using isoflurane and subsequently exsangunated for full post mortem examination.

### B. Synthesis

As referred to herein, "Class 1" compounds are aminoglycoside compounds which exhibit neither a fluoro-substituent at the C-5 position nor a guanidine group at the C-3" position. "Class 2" aminoglycoside compounds exhibit a fluoro-substituent at the C-5 position but no guanidine group at the C-3" position. "Class 3" aminoglycoside compounds exhibit no fluoro-substituent at the C-5 position but do exhibit a guanidine group at the C-3" position. "Class 4" compounds are aminoglycoside compounds which exhibit both a fluoro-substituent at the C-5 position and a guanidine group at the C-3" position, i.e. compounds of the invention.

### B.1 Class 1 compounds (comparative examples)

### B.1.1 Tobra-AHB (ABX5004) / KanaB-AHB (ABX4001)

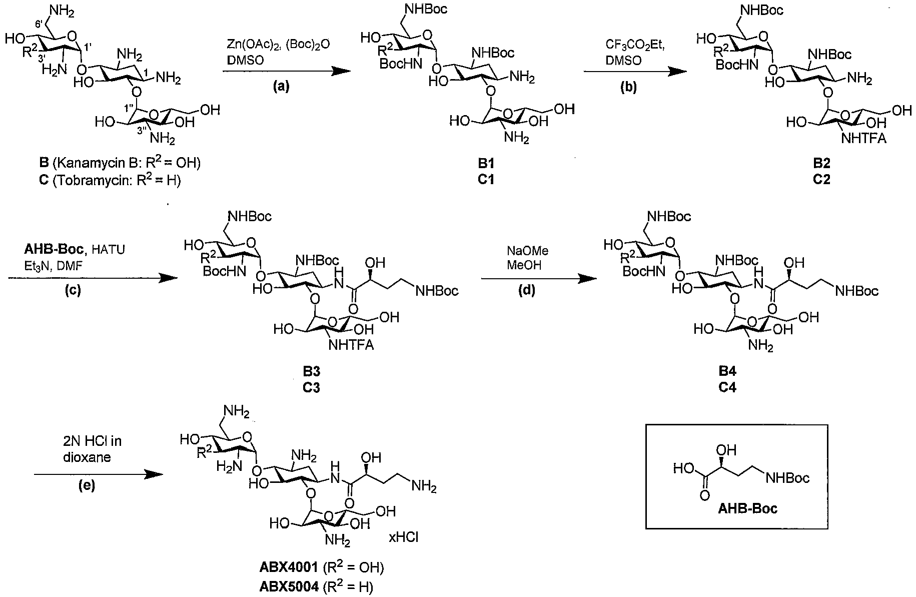

**Step (a).** To a suspension of Tobramycin (30.8 g, 65.88 mmol) in dimethyl sulfoxide (1.8 L), zinc acetate dihydrate (59.14 g, 269.42 mmol, 4.1 equiv.) was added. The mixture was stirred at room temperature overnight. Di-*tert-*butyl dicarbonate (48.88 g, 223.96 mmol, 3.4 equiv.) was added and the reaction mixture was stirred at room temperature for 5 hours. The reaction mixture was poured into water (3 L) and applied on a plug of Amberlite CG50 (H⁺ form), which was washed with water (1 L), methanol (1 L), and water (1 L) prior to use. Dimethyl sulfoxide was eluted with water (6 L). The product was eluted with a mixture of 25% NH₃ in water / methanol / water (1:2:1; 6 L). The solvents were removed *in vacuo.* The residue was dissolved in methanol and filtered over cotton. Concentration of the solution and drying via co-evaporation with toluene resulted in desired product **C1** (26.74 g, 34.84 mmol, 53% yield) as a yellowish solid. LC/MS (System 2, method A): t_{R} (min) = 5.89; MS (m/z) = 768.2 (M+H⁺) observed, 768.42 (M+H⁺) calculated. **Step (b).** Tobramycin derivative **C1** (26.30 g, 34.27 mmol) was dissolved in dimethyl sulfoxide (50 mL) and ethyl trifluoroacetate (4.47 mL, 5.34 g, 37.56 mmol, 1.1 equiv.) was added dropwise. The reaction mixture was stirred at room temperature for 5 hours and poured into brine (1.8 L). The precipitate (white solid) was filtered off, washed with water (2.5 L) and dried *in vacuo* by co-evaporation of the remaining water with toluene (3x) giving the product **C2** (22.31 g, 25.83 mmol, 75% yield) as white solid. LC/MS (System 2, method A): t_{R} (min) = 6.48; MS (m/z) = 864.2 (M+H⁺) observed, 864.41 (M+H⁺) calculated.

**Step (c).** To a solution of (2S)-Boc-4-amino-2-hydroxybutyric acid (5.13 g, 23.41 mmol, 1.2 equiv.) in dimethyl formamide (160 mL), triethylamine (3.25 mL, 23.31 mmol, 1.2 equiv.) was added and the mixture was stirred for 10 minutes at room temperature. A solution of compounds **C2** (16.86 g, 19.52 mmol, 1 equiv.) in dimethyl formamide (160 mL) was added followed by an immediate addition of HATU (8.89 g, 23.38 mmol, 1.2 equiv.) and the reaction mixture was stirred at room temperature overnight. After dimethyl formamide was evaporated *in vacuo* water (500 mL) was added to the residue and the mixture was shaken until white solid was formed and no residual oil was remaining. The white solid was filtered off, washed with sufficient amount of water (1L) and dried *in vacuo* by co-evaporation of the remaining water with toluene (3x). The resulting product **C3** (18.58 g, 17.44 mmol, 89% yield) was isolated as a white solid. LC/MS (System 2, method A): t_{R} (min) = 8.11; MS (m/z) = 965.2 (M-Boc+H⁺) observed, 965.45 (M-Boc+H⁺) calculated.

**Step (d).** To a solution of aminoglycoside derivative **C3** (18.58 g, 17.4 mmol) in methanol (170 mL) was added sodium methanolate (2.31 g, 42.8 mmol, 2.5 equiv.) and the reaction mixture was allowed to stir at 30 °C overnight. Next day, another portion of sodium methanolate (1.39 g, 25.7 mmol, 1.5 equiv.) was added and the reaction mixture was stirred for another 24 hours. Amberlite CG50 (H⁺ from) was added portion wise until a pH of 7 was reached. After removal of the Amberlite via filtration, the remaining solution was concentrated until dryness resulting in the crude product **C4** (16.02 g) which was used in the subsequent step without further purification. LC/MS (System 2, method A): t_{R} (min) = 6.98; MS (m/z) = 969.2 (M+H⁺) observed, 969.52 (M+H⁺) calculated.

**Step (e).** To a solution of crude product **C4** (50 mg) in 1,4-dioxane (1 mL) a 4N hydrogen chloride solution in 1,4-dioxane (1mL) was added and the reaction mixture was allowed to stir at room temperature overnight. Removal of the volatiles *in vacuo* resulted in a white solid, which was purified by recrystallization/precipitation from methanol/tetrahydrofuran yielding the desired product **ABX5004** as the penta-hydrogen chloride salt (750.93 g/mol, 25 mg, 0.033 mmol, 61% yield over two steps) as a white solid. LC/MS (System 2, method C): t_{R} (min) = 2.16; MS (m/z) = 569.0 (M+H⁺) observed, 569.31 (M+H⁺) calculated. ¹H NMR (300 MHz, D2O) δ = 5.78 (1H), 5.20 (1H), 4.31 (1H), 4.23 - 4.10 (1H), 4.10 - 3.63 (10H), 3.63 - 3.51 (1H), 3.51 - 3.40 (2H), 3.40 - 3.25 (1H), 3.25 - 3.11 (3H), 2.41 - 2.11 (3H), 2.11 - 1.79 (3H).

The penta-hydrogen chloride salt of **ABX4001** was synthesized starting from Kanamycin B using the same sequence of reactions as for **ABX5004. ABX4001** (766.92 g/mol, penta-hydrogen chloride salt): white solid; LC/MS (System 2, method C): t_{R} (min) = 2.16; MS (m/z) = 585.0 (M+H⁺) observed, 585.31 (M+H⁺) calculated. ¹H NMR (300 MHz, D2O) δ = 5.95 (1H), 5.20 (1H), 4.30 (1H), 4.22 - 4.10 (1H), 4.10 - 3.63 (10H), 3.63 - 3.44 (5H), 3.44 - 3.26 (1H), 3.20 (2H), 2.35 - 2.12 (2H), 2.12 - 1.72 (2H).

### B.1.2 3'epi-KanaB-AHB (ABX4002) / 3'epi-KanaA-AHB (ABX3002)

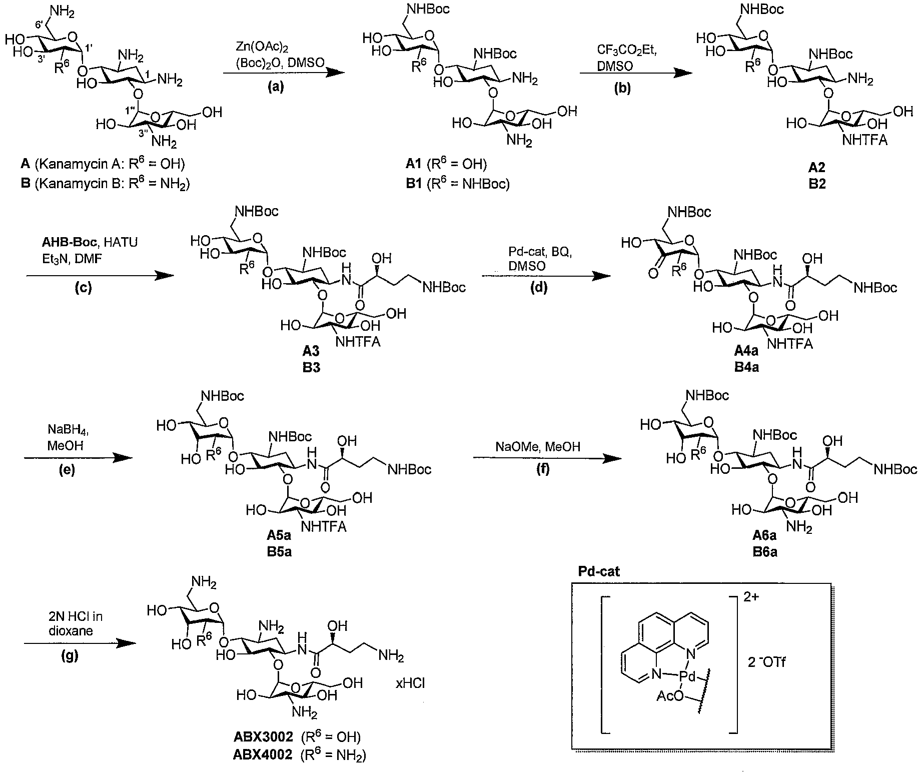

**Step (a).** To a suspension of Kanamycin B (30 g, 62.05 mmol) in dimethyl sulfoxide (900 mL), zinc acetate dihydrate (55.84 g, 254.40 mmol, 4.1 equiv.) was added. The mixture was stirred at room temperature overnight. *Di-tert-*butyl dicarbonate (46.04 g, 210.96 mmol, 3.4 equiv.) was added and the reaction mixture was stirred at room temperature for 5 hours. In the next step, the entire reaction mixture was poured into water (3 L) and applied on a plug of Amberlite CG50 (H⁺ from) (approx. 250 g), which was washed with water (1 L), methanol (1 L), and water (1 L) prior to use. Dimethyl sulfoxide was eluted with water (6 L). The product was eluted with a mixture of 25% NH₃ in water / methanol / water (1:2:1; 6 L). The solvents were removed *in vacuo.* The residue was dissolved in methanol and filtered over cotton. Concentration of the solution and drying via co-evaporation with toluene resulted in desired product **B1** (32.3 g, 41.24 mmol, 66% yield) as a yellowish solid. LC/MS (System 2, method A): t_{R} (min) = 5.24; MS (m/z) = 784.2 (M+H⁺) observed, 784.42 (M+H⁺) calculated.

**Step (b).** Kanamycin B derivative **B1** (57.45 g, 73.29 mmol) was dissolved in dimethyl sulfoxide (120 mL) and ethyl trifluoroacetate (9.56 mL, 80.6 mmol, 1.1 equiv.) was added dropwise. The reaction mixture was stirred at room temperature for 5 hours and poured into brine (3 L). The precipitate (white solid) was filtered off, washed with water (3 L) and dried *in vacuo* by co-evaporation of the remaining water with toluene (3x) yielding the product **B2** (40.57 g, 46.13 mmol, 63% yield) as white solid. LC/MS (System 2, method A): t_{R} (min) = 5.88; MS (m/z) = 880.2 (M+H⁺) observed, 880.40 (M+H⁺) calculated.

**Step (c).** To a solution of (2S)-Boc-4-amino-2-hydroxybutyric acid (12.49 g, 56.97 mmol, 1.2 equiv.) in dimethyl formamide (150 mL), triethylamine (7.96 mL, 5.78 g, 57.12 mmol, 1.2 equiv.) was added and the mixture was stirred for 10 minutes at room temperature. A solution of compound **B2** (40.57 g, 46.13 mmol, 1 equiv.) in dimethyl formamide (150 mL) was added followed by immediate addition of HATU (21.68 g, 57.02 mmol, 1.2 equiv.). After stirring the reaction mixture at room temperature overnight dimethyl formamide was removed *in vacuo* and water (1 L) was added to the oily residue. The mixture was shaken until a white solid was formed and no residual oil remained. The white solid was filtered off, washed with sufficient amount of water (2L) and dried *in vacuo* by co-evaporation of the remaining water with toluene (3x) yielding product **B3** (37.23 g, 34.44 mmol, 75% yield) as a white solid. LC/MS (System 2, method A): t_{R} (min) = 7.51; MS (m/z) = 981.2 (M-Boc+H⁺) observed, 981.45 (M-Boc+H⁺) calculated.

**Step (d).** Compound **B3** (23.28 g, 21.53 mmol) was dissolved in dimethyl sulfoxide (450 mL), and benzoquinone (7.0 g, 64.75 mmol, 3 equiv.) and palladium catalyst [(2,9-dimethyl-1,10-phenanthroline)-Pd(µ-OAc)]₂(OTf)₂ (1.63 g, 1.56 mmol, 7.3 mol%; synthesized according to the procedure published by N. R. Conley et. al., Organometallics 26 (23), 5447 (2007) and G.-J. ten Brink, Adv. Synth. Catal. 345, 1341 (2003)) were added. The reaction mixture was allowed to stir at room temperature overnight before it was poured into brine (2.5 L). The precipitate was filtered off, washed with water (2-3L), and taken up in methanol (500 mL). Insoluble material was filtered off over cotton and the solution was concentered *in vacuo.* Drying the residue by co-evaporation with toluene (3x) yielded the desired product **B4a** (20.29 g, 18.80 mmol, 87% yield) as an off-white solid. LC/MS (System 2, method A): t_{R} (min) = 8.01; MS (m/z) = 979.4 (M-Boc+H⁺) observed, 979.43 (M-Boc+H⁺) calculated.

**Step (e).** To a solution of aminoglycoside derivative **B4a** (17.7 g, 16.4 mmol) in methanol (320 mL) was added portion wise sodium borohydride (3.1 g, 82 mmol, 5 equiv.) at a temperature between -5 °C and 0 °C. After the reaction mixture had been stirred for 4 hours at 0 °C Amberlite CG50 (H⁺ from) was added until a pH of 7 was reached. Amberlite was filtered off and washed with methanol, and the filtrate was concentrated yielding product **B5a** (16.6 g, 15.4 mmol, 94% yield) as white solid. LC/MS (System 2, method A): t_{R} (min) = 7.72; MS (m/z) = 981.2 (M-Boc+H⁺) observed, 981.45 (M-Boc+H⁺) calculated.

**Step (f).** To a solution of aminoglycoside derivative **B5a** (10 g, 9.24 mmol) in methanol (90 mL) was added sodium methanolate (2.5 g, 46.28 mmol, 5 equiv.) and the reaction mixture was allowed to stir at 30 °C for 7 days. Amberlite CG50 (H⁺ from) was added portion wise until a pH of 7 was reached. After removal of the Amberlite via filtration, the remaining solution was concentrated until dryness resulting in the crude product **B6a** (9.1 g), which was used in the subsequent step without further purification. LC/MS (System 2, method A): t_{R} (min) = 6.49; MS (m/z) = 985.2 (M+H⁺) observed, 985.47 (M+H⁺) calculated.

**Step (g).** To a solution of crude product **B6a** (50 mg) in 1,4-dioxane (1 mL) a 4N hydrogen chloride solution in 1,4-dioxane (1 mL) was added and the reaction mixture was allowed to stir at room temperature overnight. Removal of the volatiles *in vacuo* resulted in a white solid, which was purified by recrystallization/precipitation from methanol/dichloromethane to yield the product **ABX4002** as penta-hydrogen chloride salt (766.92 g/mol, 32.7 mg, 0.043 mmol, 84% yield over two steps) as white solid. LC/MS (System 2, method C): t_{R} (min) = 2.14; MS (m/z) = 585.0 (M+H⁺) observed, 585.31 (M+H⁺) calculated. ¹H NMR (300 MHz, D2O) δ = 5.80 (1H), 5.16 (1H), 4.40 - 4.19 (2H), 4.19 - 3.97 (2H), 3.97 - 3.62 (8H), 3.62 - 3.38 (3H), 3.38 - 3.35 (3H), 3.25 - 3.05 (2H), 2.30 - 1.14 (2H), 2.01 - 1.72 (2H).

The tetra-hydrogen chloride salt of **ABX3002** was synthesized starting from Kanamycin A using the same sequence of reactions as for **ABX4002:**
**ABX3002** (731.45 g/mol, tetra-hydrogen chloride salt): white solid; LC/MS (System 2, method C): t_{R} (min) = 2.16; MS (m/z) = 586.0 (M+H⁺) observed, 586.29 (M+H⁺) calculated. ¹H NMR (300 MHz, D2O) δ = 5.49 (1H), 5.15 (1H), 4.26 (1H), 4.20 - 3.99 (3H), 3.96 - 3.30 (13H), 3.26 - 3.09 (3H), 2.27 - 2.09 (2H), 2.09 - 1.87 (1H), 1.87 - 1.63 (1H).

### Synthesis of (2S)-Boc-4-amino-2-hydroxybutyric acid (AHB-Boc)

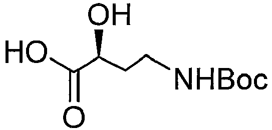

Into a solution of (2S)-4-amino-2-hydroxybutyric acid (16 g, 134.3 mmol) in water (140 mL) was added a solution of di-*tert*-butyl dicarbonate (58.4 g, 267.5 mmol, 2 equiv.) and triethylamine (37.6 mL, 269.8 mmol, 2 equiv.) in tetrahydrofuran (800 mL) and the reaction mixture was allowed to stir at room temperature overnight. After removal of tetrahydrofuran *in vacuo* the aqueous solution was acidified to a pH of 1-2 with an aq. 1N hydrogen chloride solution and extracted with dichloromethane (3x 150 mL). The combined organic layers were dried over anhydrous sodium sulfate and concentrated. The residue was taken up in EtOAc (75 mL) and shaken in heptane (800 ml). The organic solution was decanted leaving an oil sticking to the glass wall. The oil was dissolved in EtOAc, and the solution was transferred into a 1-neck flask. Concentration *in vacuo* until dryness resulted in the desired product (2S)-Boc-4-amino-2-hydroxybutyric acid (18.97 g, 86.58 mmol, 64% yield) as a very viscous oil. LC/MS (System 1, method D): t_{R} (min) = 0.37; MS (m/z) = 218.1 (M-H⁺) observed, 218.10 (M-H⁺) calculated.

### B.2 Class 2 compounds (comparative examples)

### B.2.1 Tobra-AHB-F (ABX5024) / KanaB-AHB-F (ABX4007)

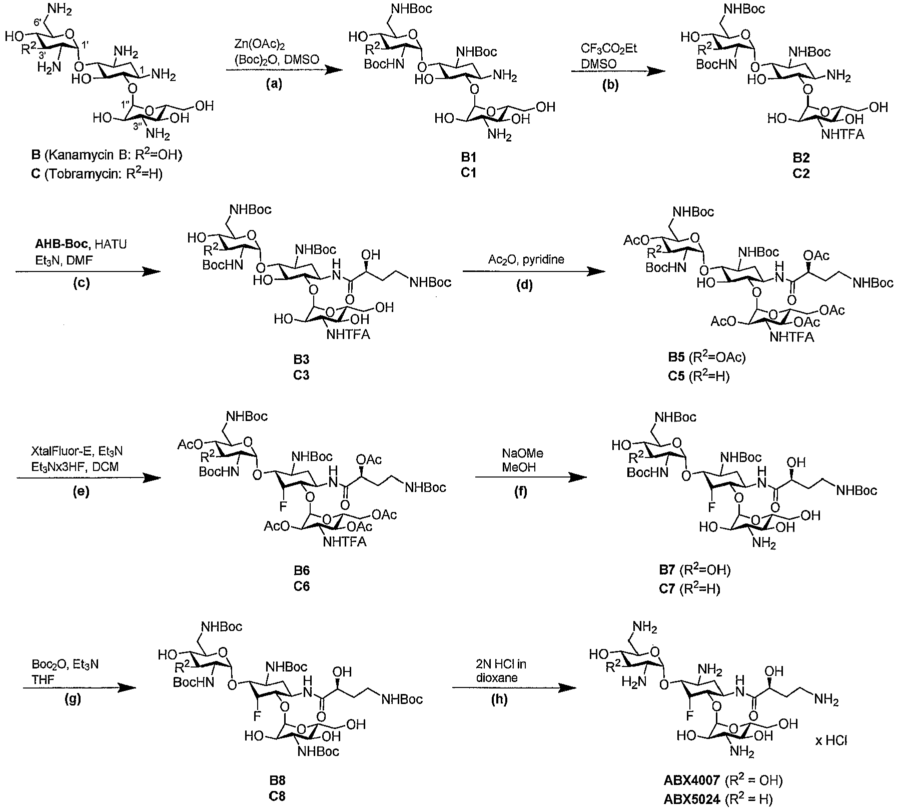

Compound **C3** was synthesized as described above under B.1.1 for the class 1 compounds.

**Step (d).** Tobramycin derivative **C3** (34.4 g, 32.3 mmol) was dissolved in pyridine (320 mL) and acetic anhydride (30.35 mL, 321.1 mmol, 10 equiv.) was added at room temperature. After stirring at room temperature overnight another portion of acetic anhydride (6.07 mL, 63.6 mmol, 2 equiv.) was added and the reaction mixture was stirred for another day. The crude mixture was poured into heptane (500 mL) and the suspension was filtrated over a plug of silica. Product **C5** was eluted with heptane containing ethyl acetate (50-70%) (R_{f} = 0.29 using 70% ethyl acetate in heptane). After removal of the volatiles product **C5** was obtained as white solid (38.3 g, 30.0 mmol, 93% yield). LC/MS (System 2, method A): t_{R} (min) = 10.49; MS (m/z) = 1175.4 (M-Boc+H⁺) observed, 1175.51 (M-Boc+H⁺) calculated.

**Step (e).** Tobramycin derivative **C5** (38.3 g, 30 mmol) was dissolved in dichloromethane (300 mL) and triethylamine (8.36 mL, 60 mmol, 2 equiv.), triethylamine trihydrofluoride (39.1 mL, 240 mmol, 4 equiv.) and X-TalFluor-E (41.22 g, 180 mmol, 3 equiv.) were added in the exact order. After stirring at room temperature overnight, the reaction mixture was cooled to 0 °C and a saturated aqueous solution of sodium bicarbonate (150 mL) was added carefully. The crude mixture was allowed to warm up to room temperature under stirring, and the aqueous and organic layers were separated. The aqueous solution was extracted with dichloromethane (3x 300 mL) and all combined organic layers were dried over anhydrous sodium sulfate, concentrated *in vacuo* and the residue was purified by column chromatography on silica using ethyl acetate (30% to 70%) in heptane (R_{f} = 0.37 using 70% ethyl acetate in heptane) as eluent yielding the product **C6** as white solid (20.5 g, 16 mmol, 54% yield). LC/MS (System 2, method A): t_{R} (min) = 10.70; MS (m/z) = 1177.4 (M-Boc+H⁺) observed, 1177.50 (M-Boc+H⁺) calculated.

**Step (f).** To a solution of aminoglycoside derivative **C6** (20.5 g, 16 mmol) in methanol (160 mL) was added sodium methanolate (8.64 g, 160 mmol, 10 equiv.) and the reaction mixture was allowed to stir at 30 °C for three days. Amberlite CG50 (H⁺ from) was added portion wise until a pH of 7 was reached. After removal of the Amberlite via filtration, the remaining solution was concentrated until dryness resulting in the crude product **C7** (22.5 g) as white solid, which was used in the subsequent step without further purification. LC/MS (System 1, method A): t_{R} (min) = 7.33; MS (m/z) = 971.4 (M+H⁺) observed, 971.52 (M+H⁺) calculated.

**Step (g).** To a solution of the crude product **C7** (200 mg) in tetrahydrofuran (2 mL) triethylamine (85 µL, 0.6 mmol, approx. 3 equiv.) and di-tert-butyl dicarbonate (65 mg, 0.3 mmol, approx. 1.5 equiv.) were added and the reaction mixture was allowed to stir at room temperature overnight. After concentration *in vacuo* the residue was shaken in water (10 mL) and the resulting white precipitate was filtered off, washed with water (2x 10 mL) and dried via co-evaporation with toluene (3x) yielding product **C8** (153 mg, 0.14 mmol, 99% yield over two steps). LC/MS (System 1, method B): t_{R} (min) = 6.31; MS (m/z) = 971.4 (M-Boc+H⁺) observed, 971.52 (M-Boc+H⁺) calculated.

**Step (h).** To a solution of compounds **C8** (100 mg, 0.094 mmol) in 1,4-dioxane (2 mL) a 4N hydrogen chloride solution in 1,4-dioxane (2 mL) was added and the reaction mixture was allowed to stir at room temperature overnight. Removal of the volatiles *in vacuo* resulted in a white solid, which was purified by recrystallization/precipitation from methanol/tetrahydrofuran yielding product **ABX5024** as penta-hydrogen chloride salt (752.92 g/mol, 59 mg, 0.078 mmol, 83% yield) as white solid. LC/MS (System 2, method B): t_{R} (min) = 1.68; MS (m/z) = 571.2 (M+H⁺) observed, 571.31 (M+H⁺) calculated. ¹H NMR (300 MHz, D2O) δ = 5.62 (d, *J* = 51.9 Hz, 1H), 5.49 (1H), 5.19 (1H), 4.45 - 4.17 (3H), 4.17 - 4.01 (1H), 4.01 - 3.67 (8H), 3.61 (1H), 3.54 - 3.36 (2H), 3.31 - 3.23 (1H), 3.23 - 3.09 (2H), 2.47 - 2.27 (2H), 2.28 - 2.11 (1H), 2.11 - 1.75 (3H). ¹⁹F NMR (282 MHz, D₂O) δ = δ -216.09 (dt, ²*J*_{HF} = 52 Hz, ³*J*_{HF} = 28 Hz).

The penta-hydrogen chloride salt of **ABX4007** was synthesized starting from Kanamycin B using the same sequence of reactions as for **ABX5024: ABX4007** (768.92 g/mol, penta-hydrogen chloride salt): white solid; LC/MS (System 2, method B): t_{R} (min) = 1.62; MS (m/z) = 587.2 (M+H⁺) observed, 587.30 (M+H⁺) calculated. ¹H NMR (300 MHz, D2O) δ = 5.63 (1H), 5.62 (d, *J* = 51.5 Hz, 1H), 5.19 (1H), 4.44 - 4.16 (3H), 4.09 (1H), 4.03 - 3.91 (3H), 3.91 - 3.61 (5H), 3.61 - 3.37 (4H), 3.37 - 3.27 (1H), 3.20 (2H), 2.47 - 2.30 (1H), 2.30 - 2.13 (1H), 2.10 - 1.77 (2H). ¹⁹F NMR (282 MHz, D₂O) δ = δ - 216.09 (dt, ²*J*_{HF} = 52 Hz, ³*J*_{HF} = 28 Hz).

### B.2.2 3'epi-KanaB-AHB-F (ABX4003) / 3'epi-KanaA-AHB-F (ABX3005)

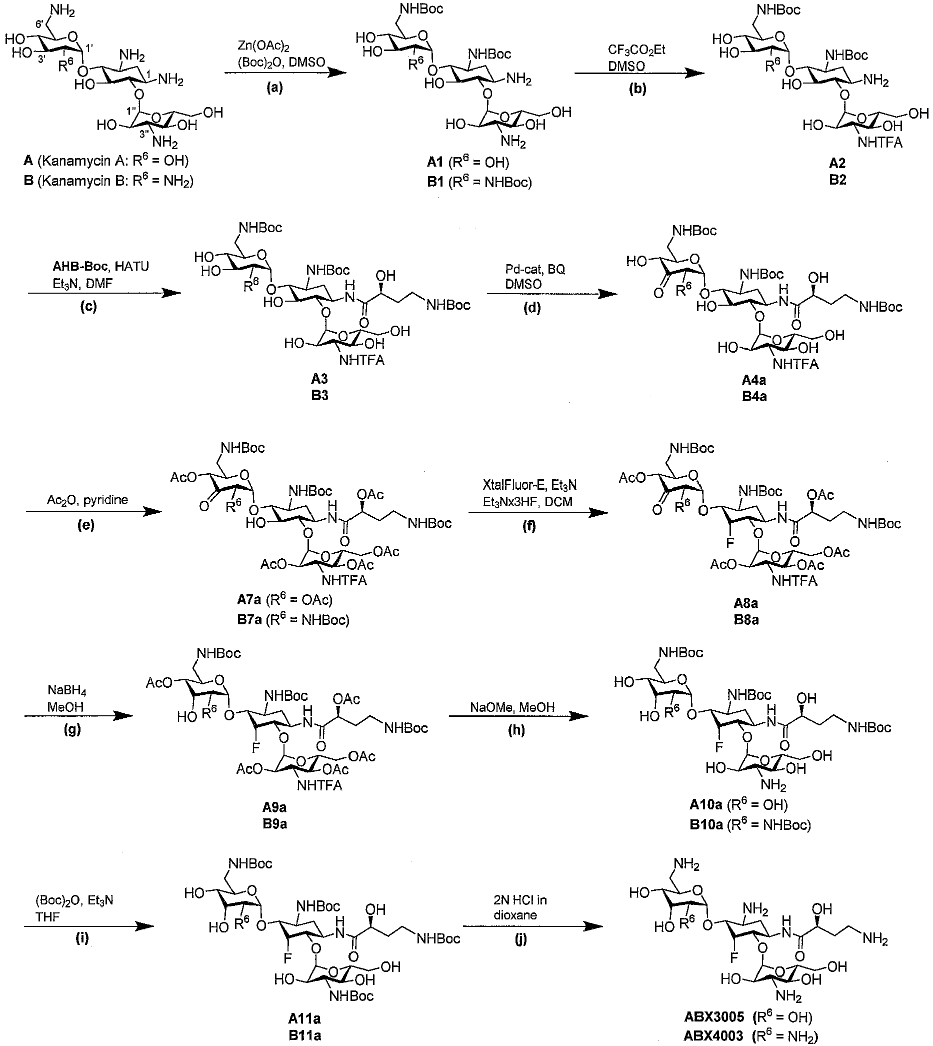

Compound **A4a** was synthesized as described above under B.1.2 for the class 1 compounds.

**Step (e).** Kanamycin A derivative **A4a** (12 g, 12.2 mmol) was dissolved in pyridine (120 ml) and acetic anhydride (17.34 mL, 183.3 mmol, 15 equiv.) was added at room temperature. After stirring at room temperature overnight another portion of acetic anhydride (2.88 mL, 30.4 mmol, 2.5 equiv.) was added and the reaction mixture was stirred for another day. The crude mixture was poured into heptane (200 mL) and the suspension was filtrated over a plug of silica. Product **A7a** was eluted with ethyl acetate (100%) (R_{f} = 0.2 using 70% EtOAc in heptane). The solvent was evaporated yielding product **A7a** in form of a white solid (14.4 g, 11.7 mmol, 96% yield). LC/MS (System 2, method A): t_{R} (min) = 9.72; MS (m/z) = 1132.0 (M-Boc+H⁺) observed, 1132.43 (M-Boc+H⁺) calculated.

**Step (f).** Compound **A7a** (14 g, 11.37 mmol) was dissolved in dichloromethane (120 mL) and triethylamine (3.16 mL, 2.29 g, 22.67 mmol, 2 equiv.), triethylamine trihydrofluoride (7.42 mL, 7.34 g, 45.52 mmol, 4 equiv.) and X-TalFluor-E (7.81 g, 34.16 mmol, 3 equiv.) were added in the exact order. After stirring at room temperature overnight, the reaction mixture was cooled to 0 °C and an aqueous saturated solution of sodium bicarbonate (120 mL) was added carefully. The crude mixture was allowed to warm up to room temperature under stirring, and the aqueous and organic layers were separated. The aqueous solution was extracted with dichloromethane (3x 150 mL) and all combined organic layers were dried over anhydrous sodium sulfate, concentrated and the residue was purified by column chromatography on silica using a solution of methanol (0% to 4%) in dichloromethane (R_{f} = 0.25 using 4% MeOH in DCM) as eluent yielding product **A8a** as a beige solid (3.84 g, 3.11 mmol, 27% yield). LC/MS (System 2, method A): t_{R} (min) = 9.83; MS (m/z) = 1134.0 (M-Boc+H⁺) observed, 1134.42 (M-Boc+H⁺) calculated.

**Step (g).** To a solution of aminoglycoside derivative **A8a** (3.45 g, 2.80 mmol) in methanol (60 mL) was added sodium borohydride (535 mg, 14.02 mmol, 5 equiv.) portion wise at a temperature between -10 °C and 0 °C. After the reaction mixture has been stirred for 4 hours at 0 °C Amberlite CG50 (H⁺ from) was added until a pH of 7 was reached. The solution was filtered over cotton and the Amberlite was washed with methanol. Removal of the solvent *in vacuo* yielded the product **A9a** (3.09 g, 2.50 mmol, 89% yield) as a white solid. LC/MS (System 2, method A): t_{R} (min) = 9.70; MS (m/z) = 1136.4 (M-Boc+H⁺) observed, 1136.44 (M-Boc+H⁺) calculated.

**Step (h).** To a solution of aminoglycoside **A9a** (3.05 g, 2.47 mmol) in methanol (25 mL) was added sodium methanolate (1.33 g, 24.7 mmol, 10 equiv.) and the reaction mixture was allowed to stir at 30 °C for three days. Amberlite CG50 (H⁺ from) was added portion wise until a pH of 7 was reached. After removal of the Amberlite via filtration, the remaining solution was concentrated until dryness resulting in the crude product **AlOa** (2.96 g) as white solid, which was used in the subsequent step without further purification. LC/MS (System 2, method A): t_{R} (min) = 6.00; MS (m/z) = 888.4 (M+H⁺) observed, 888.45 (M+H⁺) calculated.

**Step (i).** To a solution of the crude product **A10a** (200 mg) in tetrahydrofuran (2 mL) triethylamine (94 µL, 0.68 mmol, approx. 3 equiv.) and di-*tert*-butyl dicarbonate (74 mg, 0.34 mmol, approx. 1.5 equiv.) were added and the reaction mixture was allowed to stir at room temperature for two days. After concentration *in vacuo* the residue was shacked in water (10 mL). The white precipitate formed was filtered off, washed with water (2x 10 mL) and dried via co-evaporation with toluene (3x) resulting in product **A11a** (143 mg, 0.15 mmol, 87% yield over two steps) as white solid. LC/MS (System 1, method B): t_{R} (min) = 5.83; MS (m/z) = 888.2 (M-Boc+H⁺) observed, 888.45 (M-Boc+H⁺) calculated.

**Step (j).** To a solution of compound **A11a** (100 mg, 0.10 mmol) in 1,4-dioxane (5 mL) a 4N hydrogen chloride solution in 1,4-dioxane (2 mL) was added and the reaction mixture was allowed to stir overnight. Removal of the volatiles *in vacuo* resulted in a white solid, which was purified by recrystallization/precipitation from methanol/tetrahydrofuran yielding product **ABX3005** as tetra-hydrogen chloride salt (733.44 g/mol, 55 mg, 0.075 mmol, 75% yield) as white solid. LC/MS (System 2, method B): t_{R} (min) = 1.61; MS (m/z) = 588.2 (M+H⁺) observed, 588.29 (M+H⁺) calculated. ¹H NMR (300 MHz, D2O) δ = 5.47 (d, *J* = 51.4 Hz, 1H), 5.22 (1H), 5.14 (1H), 4.39 - 4.21 (2H), 4.21 - 3.96 (4H), 3.96 - 3.49 (8H), 3.49 - 3.33 (2H), 3.26 - 3.08 (3H), 2.39 - 2.26 (1H), 2.24 - 2.06 (1H), 2.05 - 1.63 (2H). ¹⁹F NMR (282 MHz, D₂O) δ = δ -215.60 (dt, ²*J*_{HF} = 51.7 Hz, ³*J*_{HF} = 28 Hz).

The penta-hydrogen chloride salt of **ABX4003** was synthesized starting from Kanamycin B using the same sequence of reactions as for **ABX3005: ABX4003** (768.91 g/mol, penta-hydrogen chloride salt): white solid; LC/MS (System 2, method C): t_{R} (min) = 2.15; MS (m/z) = 587.0 (M+H⁺) observed, 587.30 (M+H⁺) calculated. ¹H NMR (300 MHz, D2O) δ = 5.56 (d, *J* = 50 Hz, 1H), 5.47 (1H), 5.16 (1H), 4.43 - 4.02 (4H), 4.02 - 3.88 (2H), 3.88 - 3.67 (6H), 3.67 - 3.53 (1H), 3.53 - 3.22 (3H), 3.22 - 2.99 (3H), 2.32 (1H), 2.18 (1H), 2.09 - 1.81 (2H). ¹⁹F NMR (282 MHz, D₂O) δ = δ -215.95 (dt, ²*J*_{HF} = 51.8 Hz, ³*J*_{HF} = 28.4 Hz).

### B.3. Class 3 compounds (comparative examples)

### B.3.1 KanaB-AHB-Gua (ABX4009) / Tobra-AHB-Gua (ABX5005) / Tobra-AHB-Gua ethyl-p-aniline (ABX5014) / Tobra-AHB-Gua 2-tBu-ethyl (ABX5015)

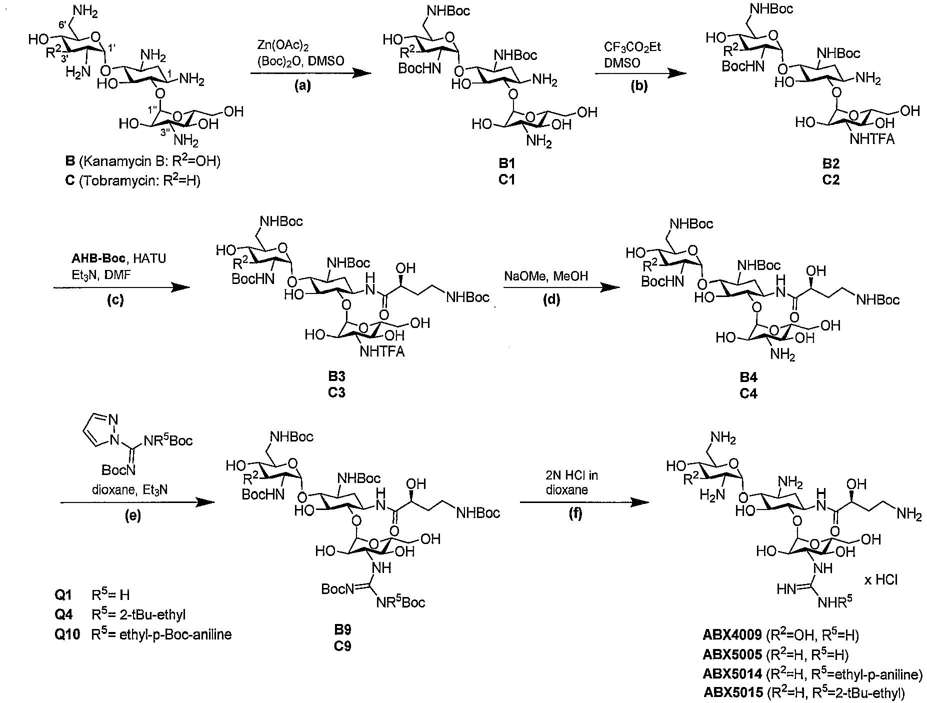

Compound **C4** was synthesized as described above under B.1.1 for the class 1 compounds.

**Step (e).** Tobramycin derivative **C4** (1.9 g crude material) was dissolved in 1,4-dioxane (70 mL), and triethylamine (2.2 mL, 1.60 g, 15.77 mmol, approx. 8.2 equiv.) and **Q1** (2.4 g, 7.73 mmol, approx. 4 equiv.) were added and the reaction mixture was allowed to stir at 50 °C for three days. Another portion of triethylamine (2.2 mL, 1.60 g, 15.77 mmol, approx. 8.2 equiv.) and **Q1** (2.4 g, 7.73 mmol, approx. 4 equiv.) were added and the reaction mixture was stirred for another three days at room temperature. After removal of all volatiles the crude mixture was purified by column chromatography on silica using ethyl acetate (50-80%) in heptane (R_{f} = 0.42 in EtOAc) yielding product **C9.1** as white solid (1.06 g, 0.88 mmol, 46% yield over two steps). LC/MS (System 2, method A): t_{R} (min) = 8.90; MS (m/z) = 1211.2 (M+H⁺) observed, 1211.65 (M+H⁺) calculated.

**Step (f).** To a solution of compound **C9.1** (1.06 g, 0.88 mmol) in 1,4-dioxane (20 mL) a 4N hydrogen chloride solution in 1,4-dioxane (20 mL) was added and the reaction mixture was allowed to stir at room temperature overnight. Removal of the volatiles *in vacuo* resulted in a white solid, which was purified by recrystallization/precipitation from methanol/tetrahydrofuran to yield product **ABX5005** as penta-hydrogen chloride salt (792.97 g/mol, 550 mg, 0.69 mmol, 79% yield) as white solid. LC/MS (System 2, method C): t_{R} (min) = 2.19; MS (m/z) = 611.0 (M+H⁺) observed, 611.34 (M+H⁺) calculated. ¹H NMR (300 MHz, D2O) δ = 5.79 (1H), 5.19 (1H), 4.29 (1H), 4.23 - 4.12 (1H), 4.12 (11H), 3.64 - 3.40 (3H), 3.40 - 3.24 (1H), 3.24 - 3.13 (2H), 2.42 - 2.11 (3H), 2.11 - 1.74 (3H).

The penta-hydrogen chloride salt of **ABX4009** was synthesized starting from Kanamycin B using the same sequence of reactions as for **ABX5005: ABX4009** (808.96 g/mol, penta-hydrogen chloride salt): white solid; LC/MS (System 2, method B): t_{R} (min) = 1.62; MS (m/z) = 627.2 (M+H⁺) observed, 627.33 (M+H⁺) calculated. ¹H NMR (300 MHz, D2O) δ = 5.89 (1H), 5.14 (1H), 4.25 (1H), 4.12 (1H), 4.08 - 3.73 (7H), 3.73 - 3.60 (2H), 3.60 - 3.39 (6H), 3.30 (1H), 3.22 - 3.08 (2H), 2.36 - 2.06 (2H), 2.06 - 1.68 (2H).

The hydrogen chloride salts of **ABX5014** and **ABX5015** were synthesized accordingly using the corresponding guanidine-introducing reagents **Q4** and **Q10** respectively:
**ABX5014** (945.26 g/mol, hexa-hydrogen chloride salt): white solid; LC/MS (System 2, method C): t_{R} (min) = 3.399; MS (m/z) = 730.2 (M+H⁺) observed, 730.41 (M+H⁺) calculated. ¹H NMR (300 MHz, D₂O) δ = 7.42 (4H), 5.77 (1H), 5.18 (1H), 4.29 (1H), 4.16 - 3.93 (3H), 3.93 - 3.64 (9H), 3.64 - 3.36 (5H), 3.36 - 3.24 (1H), 3.19 (2H), 2.99 (2H), 2.41 - 2.13 (3H), 2.13 - 1.82 (3H).

**ABX5015** (877.12 g/mol, penta-hydrogen chloride salt): white solid; LC/MS (System 2, method B): t_{R} (min) = 4.703; MS (m/z) = 695.4 (M+H⁺) observed, 695.43 (M+H⁺) calculated. ¹H NMR (300 MHz, D₂O) δ = 5.78 (1H), 5.19 (1H), 4.28 (1H), 4.14 (1H), 4.11 - 3.63 (11H), 3.63 - 3.37 (3H), 3.37 - 3.23 (3H), 3.23 - 3.10 (2H), 2.39 - 2.10 (3H), 2.10 - 1.77 (3H), 1.54 (2H), 0.94 (9H).

The corresponding derivatives based on the Kanamycin A scaffold (KanaA-AHB-Gua-R⁵) carrying residue R⁵ at the guanidine moiety can be obtained following the same sequence of reactions as shown above for ABX5005 but starting from Kanamycin A.

### B.3.2 3'epi-KanaB-AHB-Gua (ABX4005) / 3'epi-KanaA-AHB-Gua (ABX3004)

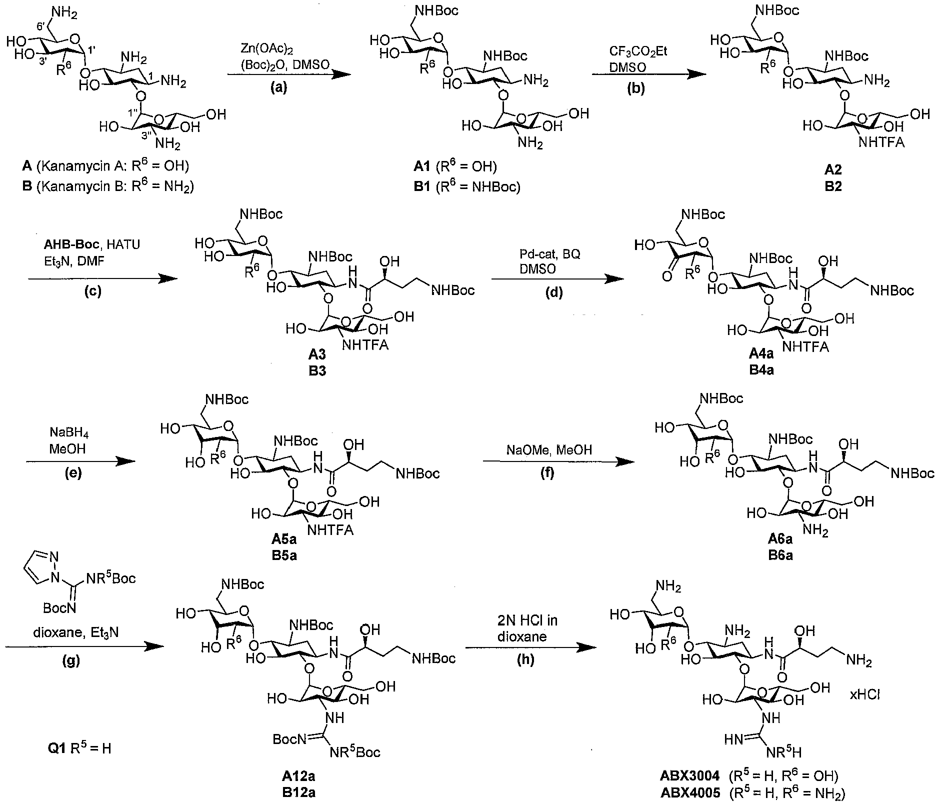

Compound **B6a** was synthesized as described above under B.1.2 for the class 1 compounds.

**Step (g).** Kanamycin B derivative **B6a** (1.5 g, crude mixture) was dissolved in 1,4-dioxane (90 mL), and triethylamine (2.54 mL, 1.84 g, 18.22 mmol, approx. 13.5 equiv.) and N,N'-Bis-boc-1-guanylpyrazole (**Q1**) (1.47 g, 4.74 mmol, approx. 3.5 equiv.) were added and the reaction mixture was allowed to stir at 50 °C for one day. Another portion of triethylamine (1.1 mL, 0.80 g, 7.89 mmol, approx. 5.8 equiv.) and **Q1** (1.16 g, 3.74 mmol, approx. 2.8 equiv.) were added and the reaction mixture was stirred for another day at 50 °C. After removal of all volatiles the crude mixture was purified by column chromatography on silica using ethyl acetate (50-80%) in heptane yielding product **B12a** as white solid (613 mg, 0.50 mmol, 37% yield over two steps). LC/MS (System 2, method A): t_{R} (min) = 8.43; MS (m/z) = 1227.3 (M+H⁺) observed, 1227.65 (M+H⁺) calculated.

**Step (h).** To a solution of compound **B12a** (233 mg, 0.19 mmol) in 1,4-dioxane (2 mL) a 4N hydrogen chloride solution in 1,4-dioxane (2 mL) was added and the reaction mixture was allowed to stir at room temperature overnight. Removal of the volatiles *in vacuo* resulted in a white solid, which was purified by recrystallization/precipitation from methanol/diethyl ether to yield product **ABX4005** as penta-hydrogen chloride salt (808.96 g/mol, 152 mg, 0.19 mmol, 99% yield) as white solid. LC/MS (System 2, method C): t_{R} (min) = 2.32; MS (m/z) = 627.0 (M+H⁺) observed, 627.33(M+H⁺) calculated. ¹H NMR (300 MHz, D2O) δ = 5.85 (1H), 5.20 (1H), 4.42 - 4.24 (2H), 4.24 - 4.02 (2H), 4.02 - 3.64 (9H), 3.64 - 3.44 (4H), 3.41 - 3.27 (1H), 3.27 - 3.11 (2H), 2.38 - 2.12 (2H), 2.12 - 1.73 (2H).

The tetra-hydrogen chloride salt of **ABX3004** was synthesized starting from Kanamycin A using the same sequence of reactions as for **ABX4005: ABX3004** (774.49 g/mol, tetra-hydrogen chloride salt): white solid; LC/MS (System 2, method C): t_{R} (min) = 2.21; MS (m/z) = 627.9 (M+H⁺) observed, 628.31 (M+H⁺) calculated. ¹H NMR (300 MHz, D2O) δ = 5.50 (1H), 5.12 (1H), 4.25 (1H), 4.19 - 3.99 (3H), 3.99 - 3.49 (12H), 3.49 - 3.33 (1H), 3.30 - 3.08 (3H), 2.29 - 2.07 (2H), 2.07 - 1.84 (1H), 1.84 - 1.58 (1H).

### B.3.3 Tobra-AHB-Gua-2-amino-ethyl (ABX5007) / Tobra-AHB-Gua-3-amino-propyl (ABX5013)

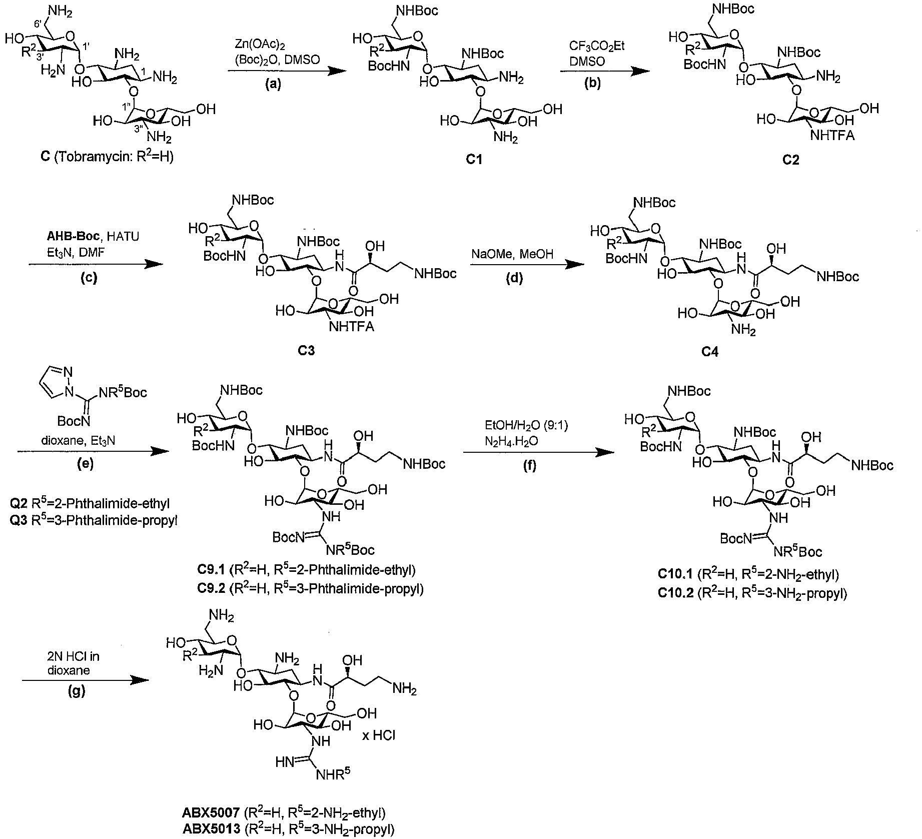

Compound **C4** was synthesized as described above under B.1.1 for class 1 compounds.

**Step (e).** Tobramycin derivative **C4** (2.67 g, crude compound) was dissolved in 1,4-dioxane (135 mL), and triethylamine (2.3 mL, 1.67 g, 16.5 mmol, approx. 8.2 equiv.) and reagent **Q2** (4 g, 8.27 mmol, approx. 4 equiv.) were added, and the reaction mixture was allowed to stir at room temperature for three days. Another portion of triethylamine (1.15 mL, 0.83 g, 8.25 mmol, approx. 4.1 equiv.) and reagent **Q2** (2 g, 4.14 mmol, approx. 2 equiv.) were added and the mixture was stirred at 50 °C for another three days. After removal of all volatiles the crude mixture was purified by column chromatography on silica using up to 5% methanol in ethyl acetate (R_{f} = 0.27 in ethyl acetate) yielding product **C9.4** as white solid (1.42 g, 1.03 mmol, 50% yield over two steps). LC/MS (System 2, method A): t_{R} (min) = 9.61; MS (m/z) = 1384.4 (M+H⁺) observed, 1384.70 (M+H⁺) calculated.

**Step (f).** Compound **C9.4** (130 mg, 0.09 mmol) was dissolved in a 9:1 mixture of ethanol and water (7 mL) and a solution of hydrazine monohydrate (45 µL, 0.90 mmol, 10 equiv.) in water (1 mL) was added. After stirring at room temperature for one day the volatiles were removed *in vacuo* and the residue was purified by column chromatography on silica using up to 5% methanol in ethyl acetate (R_{f} = 0.69 using 5% methanol in ethyl acetate) yielding product **C10.4** as white solid (70 mg, 0.056 mmol, 62% yield). LC/MS (System 2, method A): t_{R} (min) = 8.29; MS (m/z) = 1254.4 (M+H⁺) observed, 1254.69 (M+H⁺) calculated.

**Step (g).** To a solution of compound **C10.4** (65 mg, 0.052 mmol) in 1,4-dioxane (1 mL) a 4N hydrogen chloride solution in 1,4-dioxane (1 mL) was added and the reaction mixture was allowed to stir at room temperature overnight. Removal of the volatiles *in vacuo* resulted in a white solid, which was purified by recrystallization/precipitation from methanol/tetrahydrofuran yielding the product **ABX5007** as hexa-hydrogen chloride salt (872.48 g/mol, 36 mg, 0.041 mmol, 79% yield) as white solid. LC/MS (System 2, method A): t_{R} (min) = 0.95; MS (m/z) = 654.2 (M+H⁺) observed, 654.38 (M+H⁺) calculated. ¹H NMR (300 MHz, D2O) δ = 5.79 (1H), 5.20 (1H), 4.36 - 4.26 (1H), 4.26 - 4.14 (1H), 4.14 - 3.52 (15H), 3.52 - 3.42 (1H), 3.37 - 3.25 (3H), 3.25 - 3.13 (2H), 2.42 - 2.11 (3H), 2.11 - 1.75 (3H).

The hexa-hydrogen chloride salt of **ABX5013** was synthesized accordingly using the corresponding guanidine-introducing reagent **Q3.**

**ABX5013** (886.51 g/mol, hexa-hydrogen chloride salt): white solid; LC/MS (System 2, method C): t_{R} (min) = 2.043; MS (m/z) = 668.2 (M+H⁺) observed, 668.39 (M+H⁺) calculated. ¹H NMR (300 MHz, D₂O) δ = 5.78 (1H), 5.19 (1H), 4.29 (1H), 4.16 (1H), 4.11 - 3.63 (11H), 3.63 - 3.51 (2H), 3.51 - 3.23 (4H), 3.19 (2H), 3.10 (2H), 3.39 - 2.11 (3H), 2.11 - 1.75 (5H).

The corresponding derivatives based on the Kanamycin A scaffold (KanaA-AHB-Gua-R⁵) or the Kanamycin B scaffold (KanaB-AHB-Gua-R⁵) carrying residue R⁵ at the guanidine moiety can be obtained following the same sequence of reactions as shown above for **ABX5007** but starting from Kanamycin A or Kanamycin B, respectively.

### B.4 Class 4 compounds

**B.4.1 KanaB-AHB-F-Gua (ABX4006)** / **Tobra-AHB-F-Gua (ABX5006)** / **Tobra-AHB-F-Gua 2-tBu-ethyl (ABX5025)** / **Tobra-AHB-F-Gua (S) pyrrolidine-2-yl-methyl (ABX5026)** / **Tobra-AHB-F-Gua cyclopropylmethyl (ABX5027) / Tobra-AHB-F-Gua 2-hydroxy-ethyl (ABX5029)** / **Tobra-AHB-F-Gua methyl (ABX5031)** / **Tobra-AHB-F-Gua** ethyl **(ABX5032) / Tobra-AHB-F-Gua ethyl-p-aniline (ABX5036)** / **Tobra-AHB-F-Gua 2-(methylamino)ethyl (ABX5038)** / **Tobra-AHB-F-Gua (R) pyrrolidine-3-ylmethyl (ABX5039) / Tobra-AHB-F-Gua (S) morpholin-2-yl methyl (ABX5040)** / **Tobra-AHB-F-Gua 3-hydroxy-propyl (ABX5041)** / **Tobra-AHB-F-Gua (S) pyrrolidine-3-ylmethyl (ABX5042)** / **Tobra-AHB-F-Gua (R) morpholin-2-yl methyl (ABX5043) / Tobra-AHB-F-Gua (S) piperidin-3-yl methyl (ABX5044) / Tobra-AHB-F-Gua tetrahydrofuran-2-yl methyl (ABX5046) / Tobra-AHB-F-Gua pyridin-4-yl methyl (ABX5048) / Tobra-AHB-F-Gua imidazol-5-yl methyl (ABX5050) / Tobra-AHB-F-Gua tetrahydrofuran-3-yl methyl (ABX5051)**

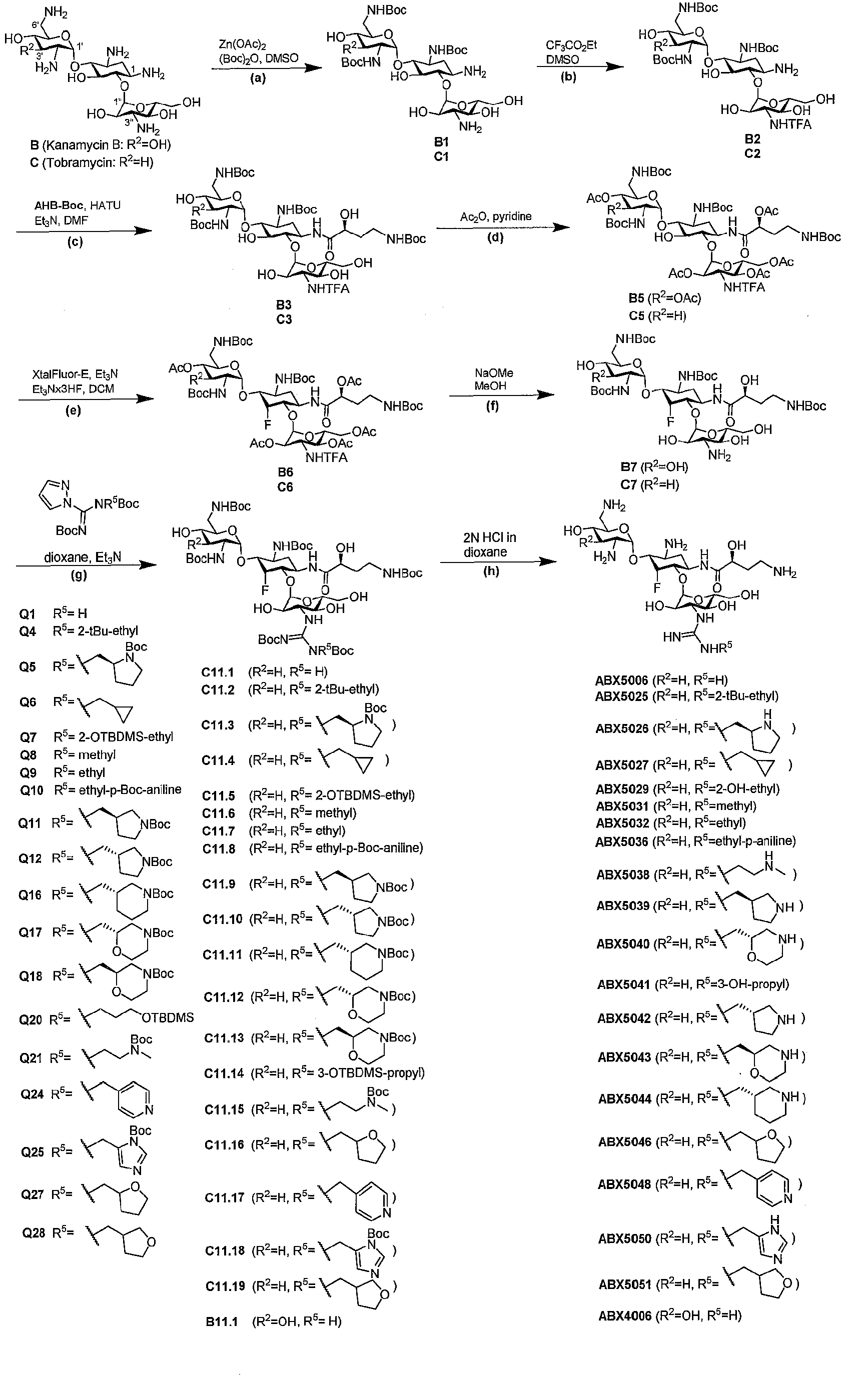

Compound **C7** was synthesized as described above under B.2.1 for the class 2 compounds.

**Step (g).** Tobramycin derivative **C7** (1 g, crude mixture) was dissolved in 1,4-dioxane (50 mL), and triethylamine (1.42 mL, 1.04 g, 10.18 mmol, approx. 13.7 equiv.) and **Q1** (0.82 g, 2.64 mmol, approx. 3.6 equiv.) were added and the reaction mixture was allowed to stir at 45 °C for three days. Another portion of triethylamine (0.6 mL, 0.44 g, 4.30 mmol, approx. 5.8 equiv.) and **Q1** (0.65 g, 2.09 mmol, approx. 2.8 equiv.) were added and the reaction mixture was stirred at 45 °C overnight. After removal of all volatiles the crude mixture was purified by column chromatography on silica using ethyl acetate (30-100%) in heptane (R_{f} = 0.48 in EtOAc) yielding product **C11.1** as white solid (520 mg, 0.43 mmol, 58% yield over two steps). LC/MS (System 2, method A): t_{R} (min) = 9.43; MS (m/z) = 1213.2 (M+H⁺) observed, 1213.65 (M+H⁺) calculated.

**Step (h).** To a solution of compound **C11.1** (500 mg, 0.41 mmol) in 1,4-dioxane (6 mL) a 4N hydrogen chloride solution in 1,4-dioxane (6 mL) was added and the reaction mixture was allowed to stir at room temperature overnight. Removal of the volatiles *in vacuo* resulted in a white solid, which was purified by recrystallization/precipitation from methanol/DCM yielding product **ABX5006** as penta-hydrogen chloride salt (794.96 g/mol, 267 mg, 0.34 mmol, 82%) yield as white solid. LC/MS (System 2, method A): t_{R} (min) = 0.96; MS (m/z) = 613.0 (M+H⁺) observed, 613.33 (M+H⁺) calculated.

1H NMR (400 MHz, D2O): δ = 5.64 (d, *J* = 52 Hz, 1H, H-5), 5.49 (1H, H-1'), 5.17 (1H, H-1"), 4.44 - 4.24 (3H, H-1, H-4, H-8), 4.14 (1H, H-6), 4.00 - 3.93 (2H, H-2", H-6"), 3.90 - 3.80 (2H, H-3, H-5'), 3.79 - 3.67 (5H, H-2', H-4', H-4", H-3", H-6"), 3.53 - 3.42 (2H, H-6', H-5"), 3.25 (1H, H-6'), 3.19 (2H, H-10), 2.42 - 2.33 (2H, H-2eq, H-3'eq), 2.24 - 2.13 (1H, H-9), 2.13 - 2.00 (1H, H-3'ax), 2.00 - 1.92 (1H, H-9), 1.92 - 1.83 (1H, H-2ax).

¹³C NMR (100 MHz, D₂O containing MeOH as internal standard): δ = 175.97 (C=O, C-7), 158.36 (C=NH, C-11), 100.35 (CH, C-1"), 89.53 (CH, C-1'), 87.45 (d, *J*_{CF} = 181 Hz, CH, C-5), 78.03 (d, *J*_{CF} = 16 Hz, CH, C-6), 72.91 (CH, C-2"), 72.43 (d, *J*_{CF} = 17 Hz, CH, C-4), 69.74 (3 CH, C-8, C-5', C-4"), 68.52 (CH, C-5"), 64.61 (CH, C-4'), 61.11 (CH₂, C-6"), 57.28 (CH, C-3"), 47.61 (CH, C-2'), 47.37 (d, *J*_{CF} = 5 Hz, CH, C-3), 46.16 (d, *J*_{CF} = 4 Hz, CH, C-1), 40.10 (CH₂, C-6'), 37.13 (CH₂, C-10), 30.89 (CH₂, C-9), 29.87 (CH₂, C-3'), 29.83 (CH₂, C-2).

¹⁹F NMR (282 MHz, D2O): δ = -215.9 (dt, ²*J*_{HF} = 52 Hz, ³*J*_{HF} = 27 Hz).

All signals were assigned using 1D- and 2D-NMR spectroscopy. ¹H, ¹³C, APT, COSY and HSQC were recorded and analyzed.

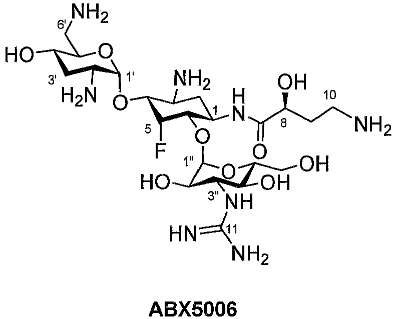

| number | ¹H-NMR (ppm) | ¹³C-NMR (ppm) |
|---|---|---|
| 1 | 4.39 | 46.16 (³*J*_{CF} = 4 Hz) |
| 2 | 1.87 and 2.36 | 29.83 |
| 3 | 3.85 | 47.37 (³*J*_{CF} = 5 Hz) |
| 4 | 4.30 (³*J*_{HF} = 28 Hz) | 72.43 (²*J*_{CF} = 17 Hz) |
| 5 | 5.65 (²*J*_{HF} = 52 Hz) | 87.45 (¹*J*_{CF} = 181 Hz) |
| 6 | 4.14 (³*J*_{HF} = 28 Hz) | 78.03 (²*J*_{CF} = 16 Hz) |
| 7 | - | 175.97 |
| 8 | 4.30 | 69.74 |
| 9 | 1.95 and 2.16 | 30.89 |
| 10 | 3.19 | 37.13 |
| 11 | - | 158.36 |
| 1' | 5.50 | 89.53 |
| 2, | 3.75 | 47.61 |
| 3' | 2.04 and 2.36 | 29.87 |
| 4' | 3.75 | 64.61 |
| 5' | 3.87 | 69.74 |
| 6' | 3.25 and 3.50 | 40.10 |
| 1" | 5.17 | 100.35 |
| 2" | 3.96 | 72.91 |
| 3" | 3.70 | 57.28 |
| 4" | 3.70 | 69.74 |
| 5" | 3.45 | 68.52 |
| 6" | 3.71 and 3.96 | 61.11 |

The penta-hydrogen chloride salt of **ABX4006** was synthesized starting from Kanamycin B using the same sequence of reactions as for **ABX5006: ABX4006** (810.96 g/mol, penta-hydrogen chloride salt): white solid; LC/MS (System 1, method B): t_{R} (min) = 1.63; MS (m/z) = 629.2 (M+H⁺) observed, 629.33 (M+H⁺) calculated. ¹H NMR (300 MHz, D₂O) δ = 5.63 (d, *J* = 52 Hz, 1H), 5.63 (1H), 5.17 (1H), 4.47 - 4.13 (3H), 4.13 - 4.02 (1H), 4.02 - 3.91 (2H), 3.91 - 3.61 (6H), 3.61 - 3.28 (4H), 3.38 - 3.26 (1H), 3.26 - 3.07 (2H), 2.46 - 2.26 (1H), 2.26 - 2.09 (1H), 2.07 - 1.74 (2H). ¹⁹F NMR (282 MHz, D₂O) δ = -215.90 (dt, ²*J*_{HF} = 53 Hz, ³*J*_{HF} = 27 Hz).

The hydrogen chloride salts of **ABX5025, ABX5026, ABX5027, ABX5029, ABX5031, ABX5032, ABX5036, ABX5038, ABX5039, ABX5040, ABX5041, ABX5042, ABX5043, ABX5044, ABX5046, ABX5048, ABX5050** and **ABX5051** were synthesized accordingly using the corresponding guanidine-introducing reagents **Q4, Q5, Q6, Q7, Q8, Q9, Q10, Q21, Q11, Q17, Q20, Q12, Q18, Q16, Q27, Q24, Q25** and **Q28** respectively:
**ABX5025** (879.12 g/mol, penta-hydrogen chloride salt): white solid; LC/MS (System 1, method B): t_{R} (min) = 2.34; MS (m/z) = 697.4 (M+H⁺) observed, 697.43 (M+H⁺) calculated. ¹H NMR (300 MHz, D₂O) δ = 5.63 (d, *J* = 53.2 Hz, 1H), 5.49 (1H), 5.17 (1H), 4.52 - 4.04 (4H), 4.06 - 3.59 (9H), 3.50 (2H), 3.35 - 3.07 (5H), 2.44 - 2.26 (2H), 2.27 - 1.75 (4H), 1.54 (2H), 0.94 (9H). ¹⁹F NMR (282 MHz, D₂O) δ = -215.92 (dt, ²*J*_{HF} = 52 Hz, ³*J*_{HF} = 26 Hz).
**ABX5026** (914.55 g/mol, hexa-hydrogen chloride salt): white solid; LC/MS (System 2, method B): t_{R} (min) = 3.81; MS (m/z) = 696.3 (M+H⁺) observed, 696.41 (M+H⁺) calculated. ¹H NMR (300 MHz, D₂O) δ = 5.63 (d, *J* = 52.5 Hz, 1H), 5.50 (1H), 5.18 (1H), 4.51 - 4.04 (4H), 4.05 - 3.55 (12H), 3.58 - 3.33 (4H), 3.34 - 3.08 (3H), 2.53 - 1.67 (10H). ¹⁹F NMR (282 MHz, D₂O) δ = - 215.85 (dt, ²*J*_{HF} = 52 Hz, ³*J*_{HF} = 27 Hz).
**ABX5027** (849.05 g/mol, penta-hydrogen chloride salt): white solid; LC/MS (System 1, method B): t_{R} (min) = 4.99; MS (m/z) = 667.2 (M+H⁺) observed, 667.38 (M+H⁺) calculated. ¹H NMR (300 MHz, D₂O) δ = 5.64 (d, *J* = 52.5 Hz, 1H), 5.49 (1H), 5.18 (1H), 4.47 - 4.06 (4H), 4.02 - 3.62 (9H), 3.54 - 3.40 (2H), 3.32 - 3.03 (5H), 2.46 - 2.26 (2H), 2.24 - 1.75 (4H), 1.15 - 0.96 (1H), 0.58 (2H), 0.26 (2H). ¹⁹F NMR (282 MHz, D₂O) δ = δ -215.85 (dt, ²*J*_{HF} = 52 Hz, ³*J*_{HF} = 27 Hz).
**ABX5029** (839.00 g/mol, penta-hydrogen chloride salt): white solid; LC/MS (System 1, method B): t_{R} (min) = 1.64; MS (m/z) = 657.2 (M+H⁺) observed, 657.36 (M+H⁺) calculated. ¹H NMR (300 MHz, D₂O) δ = 5.62 (d, *J* = 53.9 Hz, 1H), 5.49 (1H), 5.18 (1H), 4.47 - 4.05 (4H), 4.05 - 3.57 (11H), 3.57 - 3.34 (4H), 3.33 - 3.11 (3H), 2.51 - 2.28 (2H), 2.28 - 1.75 (4H). ¹⁹F NMR (282 MHz, D₂O) δ = δ -215.85 (dt, ²*J*_{HF} = 54 Hz, ³*J*_{HF} = 29 Hz).
**ABX5031** (808.98 g/mol, penta-hydrogen chloride salt): white solid; LC/MS (System 1, method B): t_{R} (min) = 1.65; MS (m/z) = 627.2 (M+H⁺) observed, 627.35 (M+H⁺) calculated. ¹H NMR (300 MHz, D₂O) δ = 5.64 (d, *J* = 52.6 Hz, 1H), 5.50 (1H), 5.18 (1H), 4.47 - 4.05 (4H), 4.05 - 3.63 (9H), 3.56 - 3.41 (2H), 3.33 - 3.10 (3H), 2.88 (3H), 2.48 - 2.26 (2H), 2.26 - 1.78 (4H). ¹⁹F NMR (282 MHz, D₂O) δ = δ -215.85 (dt, ²*J*_{HF} = 53 Hz, ³*J*_{HF} = 28 Hz).
**ABX5032** (823.01 g/mol, penta-hydrogen chloride salt): white solid; LC/MS (System 1, method B): t_{R} (min) = 2.20; MS (m/z) = 641.3 (M+H⁺) observed, 641.36 (M+H⁺) calculated. ¹H NMR (300 MHz, D₂O) δ = δ 5.62 (d, *J* = 52.4 Hz, 1H), 5.48 (1H), 5.18 (1H), 4.47 - 4.04 (4H), 4.053- 3.64 (9H), 3.56 - 3.42 (2H), 3.34 - 3.08 (5H), 2.48 - 2.29 (2H), 2.28 - 1.71 (4H), 1.21 (3H). ¹⁹F NMR (282 MHz, D₂O) δ = -215.80 (dt, ²*J*_{HF} = 51 Hz, ³*J*_{HF} = 29 Hz).
**ABX5036** (950.57 g/mol, hexa-hydrogen chloride salt): white solid; LC/MS (System 1, method B): t_{R} (min) = 4.57; MS (m/z) = 732.2 (M+H⁺) observed, 732.41 (M+H⁺) calculated. ¹H NMR (300 MHz, D₂O) δ = 7.42 (4H), 5.64 (d, *J* = 52.2 Hz, 1H), 5.50 (1H), 5.18 (1H), 4.51 - 4.04 (4H), 4.04 - 3.62 (9H), 3.62 - 3.38 (4H), 3.35 - 3.11 (3H), 2.99 (2H), 2.48 - 2.29 (2H), 2.27 - 1.81 (4H). ¹⁹F NMR (282 MHz, D₂O) δ = -215.91 (dt, ²*J*_{HF} = 53 Hz, ³*J*_{HF} = 28 Hz).
**ABX5038** (888.50 g/mol, hexa-hydrogen chloride salt): white solid; LC/MS (System 2, method B): t_{R} (min) = 1.65; MS (m/z) = 670.4 (M+H⁺) observed, 670.39 (M+H⁺) calculated.
**ABX5039** (914.54 g/mol, hexa-hydrogen chloride salt): white solid; LC/MS (System 2, method B): t_{R} (min) = 1.64; MS (m/z) = 696.4 (M+H⁺) observed, 696.41 (M+H⁺) calculated.
**ABX5040** (930.54 g/mol, hexa-hydrogen chloride salt): white solid; LC/MS (System 2, method B): t_{R} (min) = 1.63; MS (m/z) = 712.4 (M+H⁺) observed, 712.40 (M+H⁺) calculated.
**ABX5041** (853.03 g/mol, penta-hydrogen chloride salt): white solid; LC/MS (System 2, method B): t_{R} (min) = 2.04; MS (m/z) = 671.4 (M+H⁺) observed, 671.37 (M+H⁺) calculated.
**ABX5042** (914.54 g/mol, hexa-hydrogen chloride salt): white solid; LC/MS (System 1, method B): t_{R} (min) = 2.10; MS (m/z) = 696.3 (M+H⁺) observed, 696.41 (M+H⁺) calculated.
**ABX5043** (930.54 g/mol, hexa-hydrogen chloride salt): white solid; LC/MS (System 2, method B): t_{R} (min) = 1.95; MS (m/z) = 712.4 (M+H⁺) observed, 712.40 (M+H⁺) calculated.
**ABX5044** (928.57 g/mol, hexa-hydrogen chloride salt): white solid; LC/MS (System 2, method B): t_{R} (min) = 3.04; MS (m/z) = 710.4 (M+H⁺) observed, 710.42 (M+H⁺) calculated.
**ABX5046** (879.07 g/mol, penta-hydrogen chloride salt): white solid; LC/MS (System 2, method B): t_{R} (min) = 4.53; MS (m/z) = 697.4 (M+H⁺) observed, 697.39 (M+H⁺) calculated.
**ABX5048** (922.52 g/mol, hexa-hydrogen chloride salt): white solid; LC/MS (System 1, method B): t_{R} (min) = 2.08; MS (m/z) = 704.3 (M+H⁺) observed, 704.37 (M+H⁺) calculated.
**ABX5050** (911.50 g/mol, hexa-hydrogen chloride salt): white solid; LC/MS (System 1, method B): t_{R} (min) = 2.11; MS (m/z) = 693.3 (M+H⁺) observed, 693.37 (M+H⁺) calculated.
**ABX5051** (879.07 g/mol, penta-hydrogen chloride salt): white solid; LC/MS (System 1, method B): t_{R} (min) = 5.57; MS (m/z) = 697.4 (M+H⁺) observed, 697.39 (M+H⁺) calculated.

The corresponding derivatives based on the Kanamycin A scaffold (KanaA-AHB-F-Gua-R⁵) carrying residue R⁵ at the guanidine moiety can be obtained following the same sequence of reactions as shown above for **ABX5006** but starting from Kanamycin A.

### B.4.2 3'epi-KanaB-AHB-F-Gua (ABX4004) / 3'epi-KanaA-AHB-F-Gua (ABX3003)

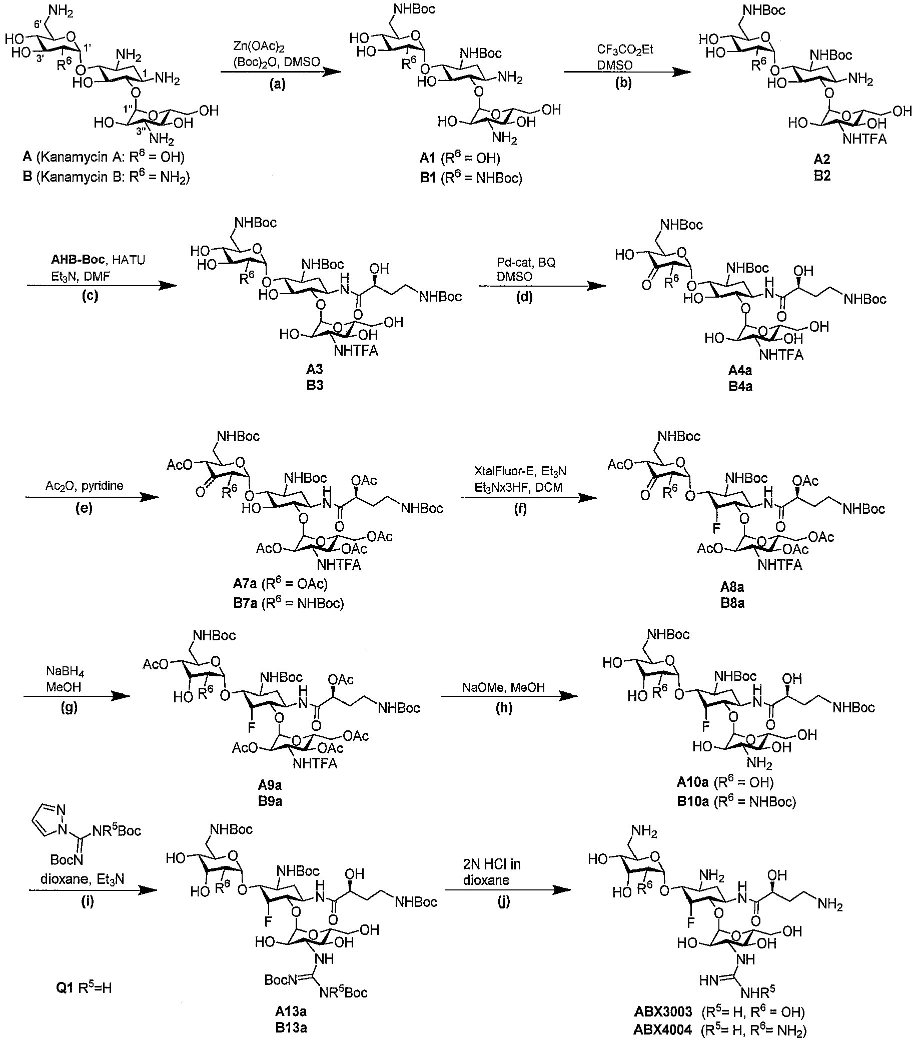

Compound **B10a** was synthesized as described above under B.2.2 for the class 2 compounds.

**Step (i).** Kanamycin B derivative **B10a** (435 mg, crude compound) was dissolved in 1,4-dioxane (25 mL), and triethylamine (0.37 mL, 0.27 g, 2.65 mmol, approx. 8 equiv.) and N,N'-Bis-boc-1-guanylpyrazole (**Q1**) (0.41 g, 1.32 mmol, approx. 4 equiv.) were added and the reaction mixture was allowed to stir at 45 °C for one day. Another portion of triethylamine (0.62 mL, 0.45 g, 4.44 mmol, approx. 13.5 equiv.) and N,N'-Bis-boc-1-guanylpyrazole (**Q1**) (0.68 g, 2.19 mmol, approx. 6.6 equiv.) were added and the reaction mixture was stirred for another day at 45°C. After removal of all volatiles the crude mixture was purified by column chromatography on silica using ethyl acetate (10-80%) in heptane (R_{f} = 0.41 in EtOAc) yielding product **B13a** as white solid (220 mg, 0.18 mmol, 41% yield over two steps). LC/MS (System 2, method A): t_{R} (min) = 8.77; MS (m/z) = 1229.2 (M+H⁺) observed, 1229.64 (M+H⁺) calculated.

**Step (j).** To a solution of compound **B13a** (200 mg, 0.16 mmol) in 1,4-dioxane (2 mL) a 4N hydrogen chloride solution in 1,4-dioxane (2 mL) was added and the reaction mixture was allowed to stir overnight. Removal of the volatiles *in vacuo* resulted in a white solid, which was purified by recrystallization/precipitation from methanol/DCM yielding product **ABX4004** as penta-hydrogen chloride salt (810.96 g/mol, 115 mg, 0.14 mmol, 89% yield) as white solid. LC/MS (System 2, method C): t_{R} (min) = 2.06; MS (m/z) = 629.0 (M+H⁺) observed, 629.33 (M+H⁺) calculated. ¹H NMR (300 MHz, D₂O) δ = 5.60 (d, J = 52 Hz, 1H), 5.50 (1H), 5.17 (1H), 4.47 - 4.21 (3H), 4.22 - 4.04 (2H), 3.96 (2H), 3.92 - 3.62 (7H), 3.62 - 3.41 (2H), 3.31 (1H), 3.19 (2H), 2.49 - 2.28 (1H), 2.29 - 2.10 (1H), 2.06 - 1.76 (2H). ¹⁹F NMR (300 MHz, D₂O) δ = -215.77 (dt, ²*J*_{HF} = 50 Hz, ³*J*_{HF} = 27 Hz).

The tetra-hydrogen chloride salt of **ABX3003** was synthesized starting from Kanamycin A using the same sequence of reactions as for **ABX4004: ABX3003** (775.47 g/mol, tetra-hydrogen chloride salt): white solid; LC/MS (System 2, method C): t_{R} (min) = 2.23; MS (m/z) = 630.0 (M+H⁺) observed, 630.31 (M+H⁺) calculated. ¹H NMR (300 MHz, D2O) δ = 5.51 (d, J= 51.7, 1H), 5.26 (1H), 5.15 (1H), 4.50 - 4.24 (2H), 4.24 - 3.99 (4H), 3.99 - 3.53 (8H), 3.53 - 3.39 (2H), 3.33 - 3.12 (3H), 2.33 (1H), 2.15 (1H), 2.03 - 1.88 (1H), 1.79 (1H). ¹⁹F NMR (300 MHz, D2O) δ = -215.32 (dt, ²*J*_{HF} = 53 Hz, ³*J*_{HF} = 27 Hz).

### B.4.3. Tobra-AHB-F-Gua-2-amino-ethyl (ABX5020) / Tobra-AHB-F-Gua-3-amino-propyl (ABX5030) / Tobra-AHB-F-Gua (R) 3-amino-butyl (ABX5047)

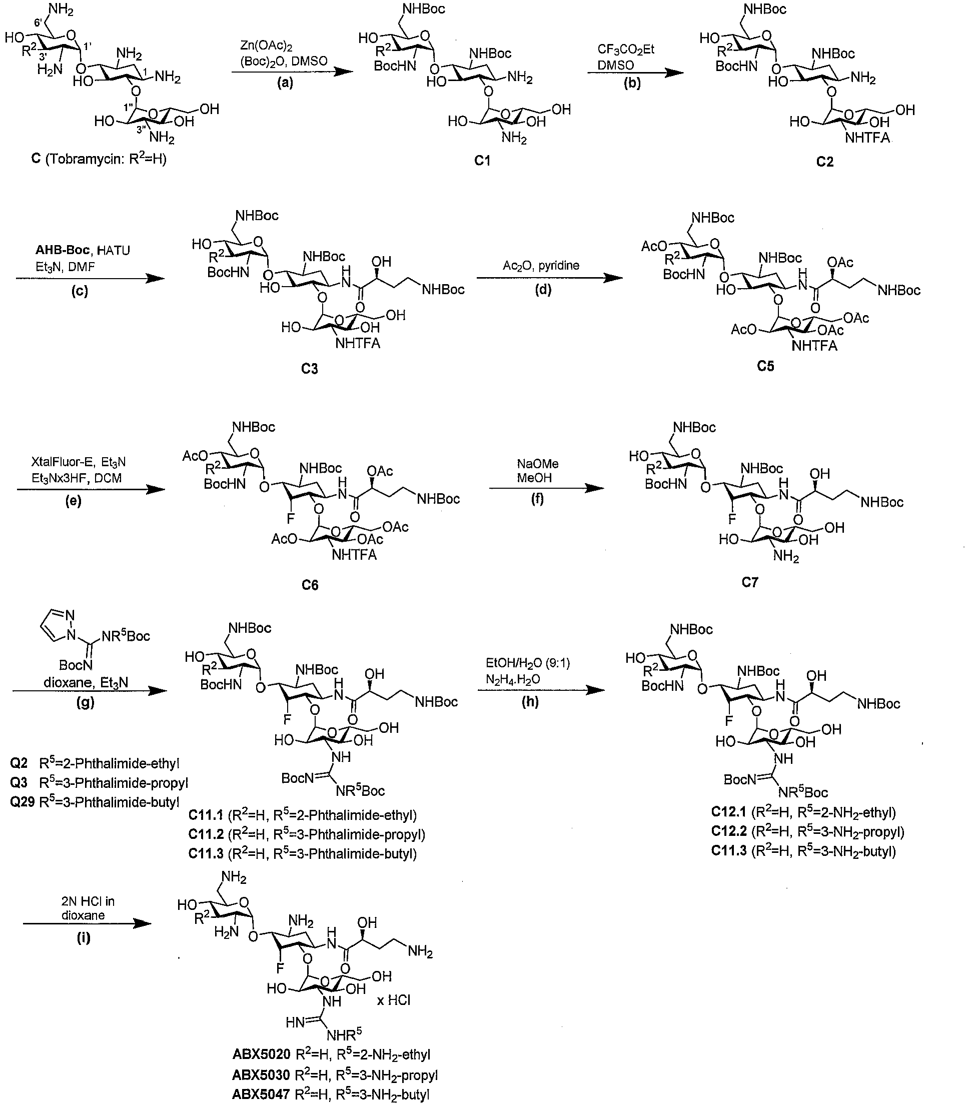

Compound **C7** was synthesized as described above under B.2.1 for the class 2 compounds.

**Step (g).** Tobramycin derivative **C7** (1 g, crude mixture) was dissolved in 1,4-dioxane (50 mL), triethylamine (1.12 mL, 0.81 g, 8.0 mmol, approx. 10.8 equiv.) and reagent **Q2** (2 g, 4.14 mmol, approx. 5.6 equiv.) were added, before the reaction mixture was allowed to stir at 45 °C overnight. Another portion of triethylamine (0.56 mL, 0.41 g, 4.0 mmol, approx. 5.4 equiv.) and reagent **Q2** (1 g, 2.07 mmol, approx. 2.7 equiv.) were added and the mixture was stirred for another 4 days at 45 °C. After removal of all volatiles the crude mixture was purified by column chromatography on silica using up to 100% EtOAc in heptane (R_{f} = 0.58 in EtOAc) yielding product **C11.16** as white solid (424 mg, 0.31 mmol, 42% yield over two steps). LC/MS (System 2, method A): t_{R} (min) = 9.43; MS (m/z) = 1386.6 (M+H⁺) observed, 1386.69 (M+H⁺) calculated.

**Step (h).** Compound **C11.16** (400 mg, 0.29 mmol) was dissolved in a 9:1 mixture of ethanol and water (25 mL) and a solution of hydrazine hydrate (145 µL, 2.9 mmol, 10 equiv.) in water (7.5 mL) was added. After stirring at room temperature for one day the volatiles were removed *in vacuo* and the residue was purified by column chromatography on silica using up to 5% methanol in EtOAc (R_{f} = 0.29 using 5% MeOH in EtOAc) yielding product **C12.16** as white solid (207 mg, 0.16 mmol, 57% yield). LC/MS (System 2, method A): t_{R} (min) = 8.22; MS (m/z) = 1256.6 (M+H⁺) observed, 1256.69 (M+H⁺) calculated.

**Step (i).** To a solution of compound **C12.16** (163 mg, 0.13 mmol) in 1,4-dioxane (2 mL) a 4N hydrogen chloride solution in 1,4-dioxane (2 mL) was added and the reaction mixture was allowed to stir at room temperature overnight. Removal of the volatiles *in vacuo* resulted in a white solid, which was purified by recrystallization/precipitation from methanol/DCM yielding product **ABX5020** as hexa-hydrogen chloride salt (874.49 g/mol, 80 mg, 0.091 mmol, 70% yield) as white solid. LC/MS (System 1, method B): t_{R} (min) = 1.61; MS (m/z) = 656.4 (M+H⁺) observed, 656.37 (M+H⁺) calculated. ¹H NMR (400 MHz, D2O): δ = 5.65 (d, *J* = 52 Hz, 1H, H-5), 5.50 (1H, H-1'), 5.19 (1H, H-1"), 4.43 - 4.25 (3H, H-1, H-4, H-8), 4.23-4.09 (1H, H-6), 4.02 - 3.93 (2H, H-2", H-6"), 3.91 - 3.81 (2H, H-3, H-5'), 3.81 - 3.68 (5H, H-2', H-4', H-3", H-4", H-6"), 3.68 - 3.61 (2H, H-12), 3.55 - 3.47 (2H, H-6', H-5"), 3.33 - 3.23 (3H, H-13, H-6'), 3.23 - 3.17 (2H, H-10), 2.43 - 2.35 (2H, H-2eq, H-3'eq), 2.25 - 2.14 (1H, H-9), 2.14 - 2.02 (1H, H-3'ax), 2.02 - 1.84 (2H, H-9, H-2ax).

¹³C NMR (100 MHz, D₂O containing MeOH as internal standard): δ = 175.90 (C=O, C-7), 157.65 (C=NH, C-11), 100.44 (CH, C-1"), 89.53 (CH, C-1'), 87.35 (d, *J*_{CF} = 181 Hz, CH, C-5), 77.88 (d, *J*_{CF} = 17 Hz, CH, C-6), 72.94 (CH, C-2"), 72.40 (d, *J*_{CF} = 17 Hz, CH, C-4), 69.74 (2 CH, C-8, C-5'), 69.60 (CH, C-4"), 68.47 (CH, C-5"), 64.60 (CH, C-4'), 61.09 (CH₂, C-6"), 57.47 (CH, C-3"), 47.61 (CH, C-2'), 47.37 (d, *J*_{CF} = 5 Hz, CH, C-3), 46.21 (d, *J*_{CF} = 5 Hz, CH, C-1), 40.10 (CH₂, C-6'), 38.95 (CH₂, C-12), 38.16 (CH₂, C-13), 37.13 (CH₂, C-10), 30.93 (CH₂, C-9), 29.86 (CH₂, C-3'), 29.84 (CH₂, C-2).

¹⁹F NMR (282 MHz, D2O): δ = -215.9 (dt, ²*J*_{HF} = 53 Hz, ³*J*_{HF} = 27 Hz).

All signals were assigned using 1D- and 2D-NMR spectroscopy. ¹H, ¹³C, APT, COSY and HSQC were recorded and analyzed.

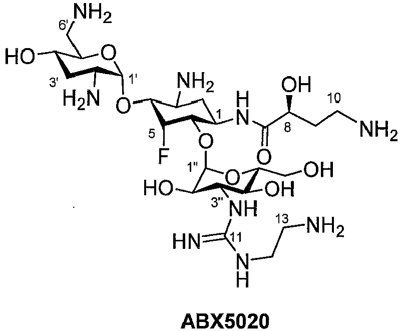

| number | ¹H-NMR (ppm) | ¹³C-NMR (ppm) |
|---|---|---|
| 1 | 4.40 | 46.21 (³*J*_{CF} = 5 Hz) |
| 2 | 1.88 and 2.38 | 29.84 |
| 3 | 3.86 | 47.37 (³*J*_{CF} = 5 Hz) |
| 4 | 4.32 (³*J*_{HF} = 28 Hz) | 72.40 (²*J*_{CF} = 17 Hz) |
| 5 | 5.65 (²*J*_{HF} = 52 Hz) | 87.35 (¹*J*_{CF} = 181 Hz) |
| 6 | 4.16 (³*J*_{HF} = 28 Hz) | 77.88 (²*J*_{CF} = 17 Hz) |
| 7 | - | 175.90 |
| 8 | 4.31 | 69.74 |
| 9 | 1.98 and 2.20 | 30.93 |
| 10 | 3.20 | 37.13 |
| 11 | - | 157.65 |
| 12 | 3.65 | 38.95 |
| 13 | 3.29 | 38.16 |
| 1' | 5.50 | 89.53 |
| 2, | 3.75 | 47.61 |
| 3' | 2.06 and 2.38 | 29.86 |
| 4' | 3.76 | 64.60 |
| 5' | 3.87 | 69.74 |
| 6' | 3.28 and 3.50 | 40.10 |
| 1" | 5.19 | 100.44 |
| 2" | 3.98 | 72.94 |
| 3" | 3.79 | 57.47 |
| 4" | 3.76 | 69.60 |
| 5" | 3.50 | 68.47 |
| 6" | 3.72 and 3.97 | 61.09 |

The hexa-hydrogen chloride salts of **ABX5030** and **ABX5047** were synthesized accordingly using the corresponding guanidine-introducing reagent **Q3** and **Q29** respectively:
**ABX5030** (888.50 g/mol, hexa-hydrogen chloride salt): white solid; LC/MS (System 2, method B): t_{R} (min) = 1.93; MS (m/z) = 670.2 (M+H⁺) observed, 670.39 (M+H⁺) calculated. ¹H NMR (300 MHz, D₂O) δ = 5.63 (d, *J* = 52.3 Hz, 1H), 5.49 (1H), 5.18 (1H), 4.48 - 4.05 (4H), 4.05 - 3.61 (9H), 3.57 - 3.42 (2H), 3.43 - 3.32 (2H), 3.32 - 3.14 (3H), 3.13 - 3.02 (2H), 2.47 - 2.27 (2H), 2.27 - 2.10 (1H), 2.12 - 1.75 (5H). ¹⁹F NMR (282 MHz, D₂O) δ = -215.9 (dt, ²*J*_{HF} = 52 Hz, ³*J*_{HF} = 27 Hz).
**ABX5047** (902.53 g/mol, hexa-hydrogen chloride salt): white solid; LC/MS (System 1, method B): t_{R} (min) = 2.18; MS (m/z) = 684.4 (M+H⁺) observed, 684.41 (M+H⁺) calculated.

The penta-hydrogen chloride salt of 3'epi-KanaB-AHB-F-Gua-2-amino-ethyl **(ABX4008)** and tetra-hydrogen chloride salt of 3'epi-KanaA-AHB-F-Gua-2-amino-ethyl **(ABX3006)** were synthesized from the corresponding precursors **B10a** and **A10a** (see class 2 compounds under B.2.2) respectively, following the same sequence of reactions (steps (g) to (i)) as shown above for **ABX5020.**

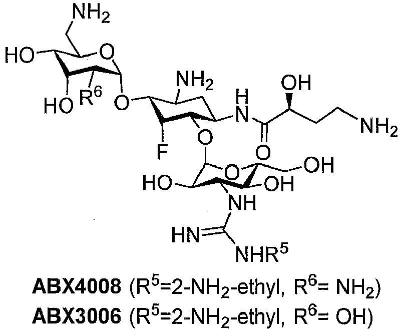

**ABX4008** (890.47 g/mol, hexa-hydrogen chloride salt): white solid; LC/MS (System 2, method B): t_{R} (min) = 1.63; MS (m/z) = 672.2 (M+H⁺) observed, 672.37 (M+H⁺) calculated. ¹H NMR (300 MHz, D₂O) δ = 5.60 (d, J = 52.1 Hz, 1H), 5.50 (1H), 5.18 (1H), 4.45 - 4.24 (3H), 4.24 - 4.03 (2H), 4.03-3.90 (2H), 3.90 - 3.58 (9H), 3.56 - 3.43 (2H), 3.37 - 3.24 (3H), 3.24 - 3.13 (2H), 2.46 - 2.27 (1H), 2.27- 2.08 (1H), 2.04 - 1.79 (2H). ¹⁹F NMR (282 MHz, D₂O) δ = -215.72 (dt, ²*J*_{HF} = 52 Hz, ³*J*_{HF} = 27 Hz).

**ABX3006** (855.00 g/mol, penta-hydrogen chloride salt): white solid; LC/MS (System 2, method B): t_{R} (min) = 1.63; MS (m/z) = 673.2 (M+H⁺) observed, 673.35 (M+H⁺) calculated. ¹H NMR (300 MHz, D₂O) δ = 5.50 (d, J = 51.3 Hz, 1H), 5.26 (1H), 5.16 (1H), 4.46 - 4.24 (2H), 4.24 - 4.11 (2H), 4.11 - 3.99 (2H), 3.99- 3.59 (10H), 3.57 - 3.40 (2H), 3.33 - 3.23 (3H), 3.22 - 3.11 (2H), 2.43 - 2.25 (1H), 2.26 - 2.10 (1H), 2.03 - 1.70 (2H). ¹⁹F NMR (282 MHz, D₂O) δ = -215.30 (dt, ²*J*_{HF} = 52 Hz, ³*J*_{HF} = 28 Hz).

The corresponding derivatives based on the Kanamycin A scaffold (KanaA-AHB-F-Gua-R⁵) or Kanamycin B scaffold (KanaB-AHB-F-Gua-R⁵) carrying residue R⁵ at the guanidine moiety can be obtained following the same sequence of reactions as shown above for **ABX5020** but starting from Kanamycin A or Kanamycin B, respectively.

### B.4.4 3'-Me-3'epi-KanaB-AHB-F-Gua (ABX4011)

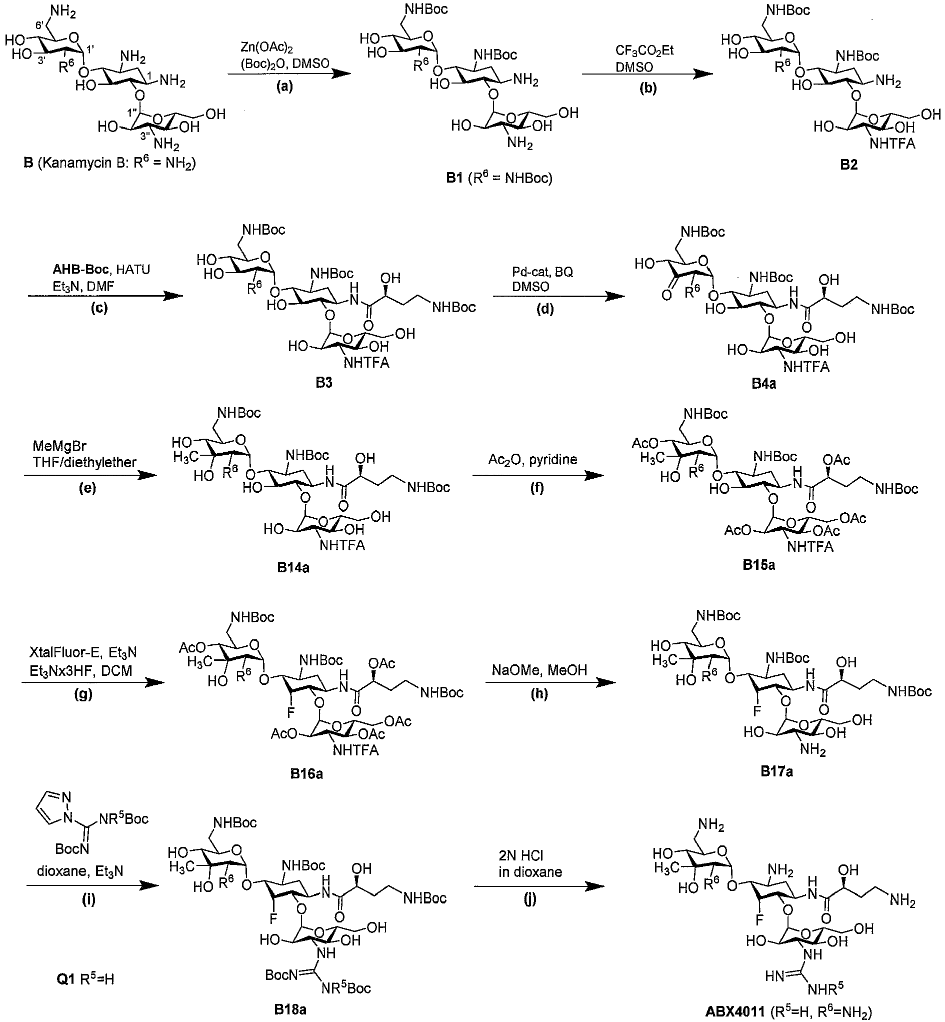

Compound **B4a** was synthesized as described above under B.1.2 for the class 1 compounds.

**Step (e).** 3'-Oxo-kanamycin B derivative **B4a** (13 g, 12.04 mmol) was dissolved in tetrahydrofuran (700 mL) and cooled to -15 °C. Methylmagnesium bromide (3M in diethyl ether, 96.25 mL, 288.75 mmol, 24 equiv.) was added dropwise and the reaction mixture was stirred at -10 °C for one hour, at 0 °C for 2 hours, before it was allowed to warm up slowly to 15 °C over four to five hours. After storing the reaction mixture in a freezer at - 20 °C for overnight, a pH of 6 was adjusted by addition of an aqueous 3N hydrogen chloride solution under cooling (0 °C) and the solution was extracted with ethyl acetate (3x). The combined organic layers were dried over anhydrous sodium sulfate and concentrated. The crude product was purified by column chromatography on silica using a solution of methanol (0-5%) in ethyl acetate (R_{f} = 0.74 using 5% methanol in ethyl acetate) resulting in product **B14a** as white solid (2.2 g, 2.01 mmol, 17% yield). LC/MS (System 2, method A): t_{R} (min) = 7.81; MS (m/z) = 995.4 (M-Boc+H⁺) observed, 995.46 (M-Boc+H⁺) calculated.

**Step (f).** Compound **B14a** (2 g, 1.9 mmol) was dissolved in pyridine (20 ml) and acetic anhydride (2.15 mL, 22.8 mmol, 12 equiv.) was added and stirred at room temperature for five days. The crude mixture was diluted with heptane (200 mL) and the suspension was filtrated over a plug of silica and washed with heptane (500 mL). Product **B15a** was eluted with a mixture of heptane and ethyl acetate (1:1) (R_{f} = 0.29 using 70% ethyl acetate in heptane). The solvent was evaporated yielding product **B15a** in form of a white solid (2.0 g, 1.53 mmol, 81% yield). LC/MS (System 1, method A): t_{R} (min) = 11.06; MS (m/z) = 1205.2 (M-Boc+H⁺) observed, 1205.52 (M-Boc+H⁺) calculated.

**Step (g).** Kanamycin B derivative **B15a** (2 g, 1.53 mmol) was dissolved in dichloromethane (15 mL) and triethylamine (0.43 mL, 3.09 mmol, 2 equiv.), triethylamine trihydrofluoride (1.0 mL, 6.13 mmol, 4 equiv.) and X-TalFluor-E (1.05 g, 4.6 mmol, 3 equiv.) were added in the exact order. After stirring at room temperature overnight, the reaction mixture was cooled to 0 °C and an aqueous saturated solution of sodium bicarbonate (20 mL) was added carefully. The crude mixture was allowed to warm up to room temperature under stirring, and the aqueous and organic layers were separated. The aqueous solution was extracted with dichloromethane (3x) and all combined organic layers were dried over anhydrous sodium sulfate, concentrated *in vacuo* and the residue was purified by column chromatography on silica using ethyl acetate (0% to 50%) in heptane (R_{f} = 0.52 using 70% ethyl acetate in heptane) as eluent yielding product **B16a** as white solid (600 mg, 0.46 mmol, 30% yield). LC/MS (System 2, method A): t_{R} (min) = 10.46; MS (m/z) = 1207.4 (M-Boc+H⁺) observed, 1207.51 (M-Boc+H⁺) calculated.

**Step (h).** To a solution of aminoglycoside **B16a** (600 mg, 0.46 mmol) in methanol (5 mL) was added sodium methanolate (248 mg, 4.6 mmol, 10 equiv.) and the reaction mixture was allowed to stir at room temperature for four days. Amberlite CG50 (H⁺ from) was added portion wise until a pH of 5-6 was reached. After removal of the Amberlite via filtration, the remaining solution was concentrated until dryness resulting in the crude product **B17a** (700 mg) as white solid, which was used in the subsequent step without further purification. LC/MS (System 2, method A): t_{R} (min) = 6.77; MS (m/z) = 1023.5 (M+Na⁺) observed, 1023.51 (M+Na⁺) calculated.

**Step (i).** The crude product **B17a** (700 mg) was dissolved in 1,4-dioxane (35 mL), and triethylamine (0.50 mL, 7.8 mmol, approx. 11 equiv.) and **Q1** (1.1 g, 7.8 mmol, approx. 11 equiv.) were added and the reaction mixture was allowed to stir at 50 °C for three days. After removal of all volatiles the crude mixture was purified by column chromatography on silica using ethyl acetate (30-100%) in heptane (R_{f} = 0.58 in ethyl acetate) yielding product **B18a** as white solid (63 mg, 0.051 mmol, 11% yield over two steps). LC/MS (System 1, method A): t_{R} (min) = 9.23; MS (m/z) = 1243.4 (M+H⁺) observed, 1243.66 (M+H⁺) calculated.

**Step (j).** To a solution of compound **B18a** (50 mg, 0.040 mmol) in 1,4-dioxane (5 mL) and a 4N hydrogen chloride solution in 1,4-dioxane (5 mL) was added and the reaction mixture was allowed to stir overnight. Removal of the volatiles *in vacuo* resulted in a white solid, which was purified by recrystallization/precipitation from methanol/dichloromethane yielding product **ABX4011** as penta-hydrogen chloride salt (824.97 g/mol, 31 mg, 0.038 mmol, 94% yield) as white solid. LC/MS (System 1, method B): t_{R} (min) = 1.63; MS (m/z) = 643.3 (M+H⁺) observed, 643.34 (M+H⁺) calculated. ¹H NMR (300 MHz, D₂O) δ = 5.63 (1H), 5.62 (d, *J* = 52 Hz, 1H), 5.17 (1H), 4.46 - 4.03 (4H), 4.03 - 3.61 (8H), 3.61 - 3.42 (3H), 3.42 - 3.25 (1H), 3.25 - 3.11 (2H), 2.43 - 2.27 (1H), 2.27 - 2.08 (1H), 2.02 - 1.74 (2H), 1.53 (3H). ¹⁹F NMR (282 MHz, D₂O) δ = -215.70 (dt, ²*J*_{HF} = 52 Hz, ³*J*_{HF} = 28 Hz).

The corresponding derivatives based on the 3'epi-Kanamycin A scaffold (3'-Me-3'epi-KanaA-AHB-F-Gua) can be obtained following the same sequence of reactions as shown above for **ABX4011** but starting from Kanamycin A.

### B.4.5 6N-2-amino-ethyl-Tobra-AHB-F-Gua (ABX5037)

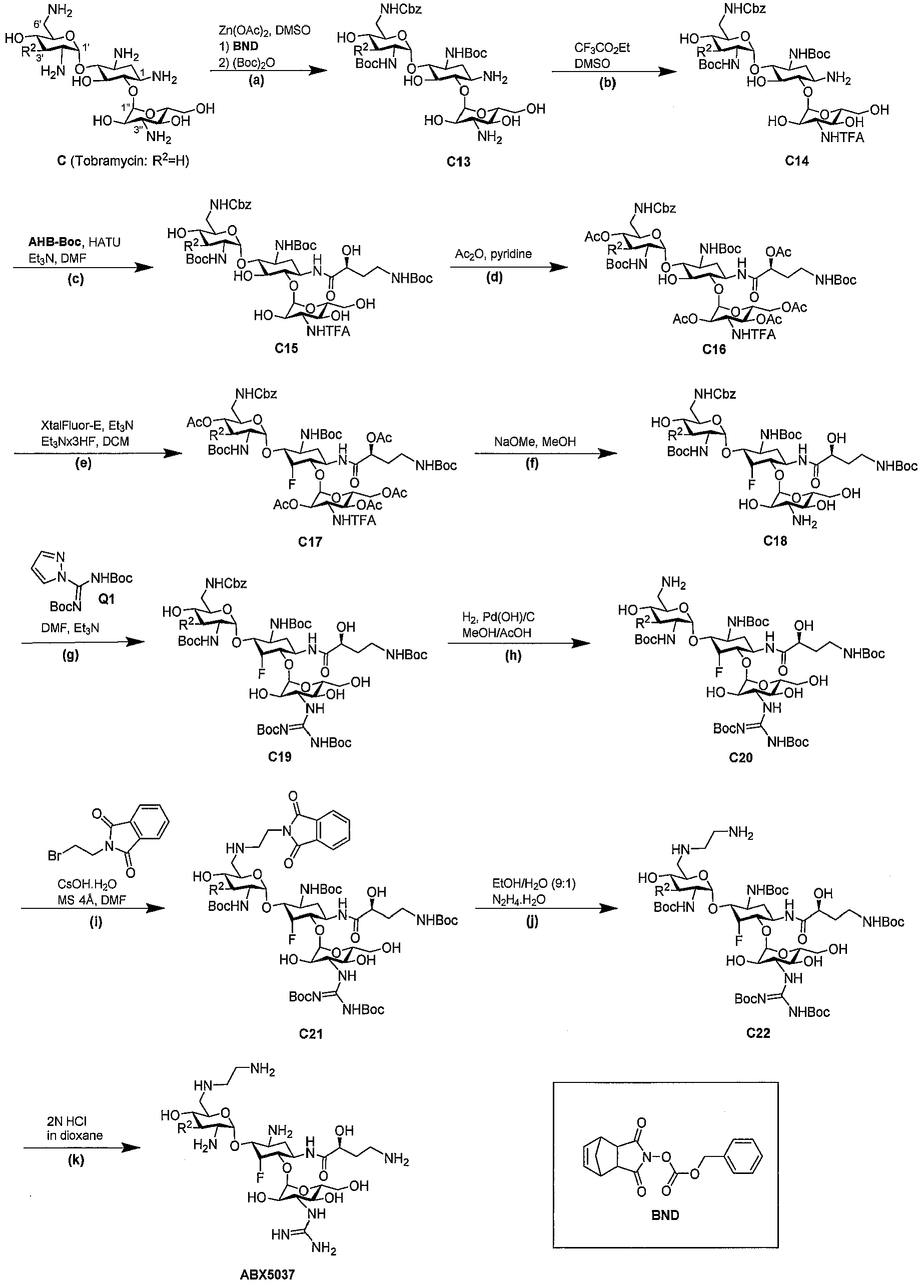

**ABX5037** was synthesized according to the procedures described for **ABX5020** (section B.4.3 above) with the exception of a modified version of step (a) and additional steps (step (h) and (i)), each of which is described below.

**Step (a).** To a suspension of Tobramycin (30.0 g, 64 mmol) in dimethyl sulfoxide (1.3 L), ZnOAc₂×2H₂O (42.3 g, 192.7 mmol, 3 equiv.) was added and the mixture was stirred at room temperature overnight. After addition of BND (20.1 g, 64.2 mmol, 1equiv.) the reaction was allowed to stir for another day at room temperature. Di-tert-butyl dicarbonate (30.8 g, 141.1 mmol, 2.2 equiv.) was added and the reaction was stirred at room temperature for another 4 hours before it was poured into water (4.2 L) and applied on a plug of Amberlite CG50 (H⁺ form), which was washed with water (1 L), methanol (1 L), and water (1 L) prior to use. Dimethyl sulfoxide was eluted with water (6 L). The product **C13** was eluted with a mixture of 25% NH₃ in water / methanol / water (1:2:1; 6 L). The solvents were removed *in vacuo* and the residue was dissolved in methanol. Insoluble material was filtered off over cotton. Concentration of the filtrate *in vacuo* and drying via co-evaporation with toluene (3x) yielded product **C13** (29.1 g, 36.3 mmol, 57%) as a white solid. LC/MS (System 2, method A): t_{R} (min) = 5.84; MS (m/z) = 802.2 (M+H⁺) observed, 802.41 (M+H⁺) calculated.

**Step (h).** Compounds **C19** (3.3 g, 2.65 mmol) was dissolved in methanol (70 mL) and palladium hydroxide on carbon (20 wt. %) (70 mg, 0.13 mmol, 5 mol%) and acetic acid (0.66 mL, 0.69 g, 11.5 mmol, 4.3 equiv.) were added. After flushing the reaction vessel with hydrogen, the reaction was allowed to stir under hydrogen atmosphere (1 atm) at room temperature. After two days, the reaction was filtered over celite and concentrated until dryness to obtain the product **C20** as white solid (2.3 g, 2.06 mmol, 78%). LC/MS (System 2, method A): t_{R} (min) = 7.31; MS (m/z) = 1113.4 (M+H⁺) observed, 1113.59 (M+H⁺) calculated.

**Step (i).** Cesium(II)hydroxide monohydrate (302 mg, 1.80 mmol, 1 equiv.) and molecular sieves (4 A, 600 mg) were taken up in DMF (20 mL) and the suspension was stirred at 35 °C for 10 minutes before compounds **C20** (2 g, 1.80 mmol, 1 equiv.) was added. After 2h stirring at 35 °C N-(2-Bromoethyl)phthalimide (3.01 g, 11.85 mmol, 6.6 equiv.) was added and the reaction mixture was stirred at 35 °C for 24 hours. The molecular sieves were filtered off over a glass filter and the solution was concentrated. The crude was purified by column chromatography on silica using first 50-100% ethyl acetate in heptane to remove impurities and then 1-3% methanol in ethyl acetate to elute the product **C21** (259 mg, 0.2 mmol, 11%). LC/MS (System 2, method A): t_{R} (min) = 8.13; MS (m/z) = 1286.6 (M+H⁺) observed, 1286.64 (M+H⁺) calculated.

Subsequent to step (i) in which product **C21** was furnished, steps (j) and (k) were performed under the conditions described for steps (h) and (i) in the synthesis of **ABX5020** (section B.4.3 above), thus furnishing the end product **ABX5037.**

### N-Benzyloxycarbonyloxy-5-norbornene-2,3-dicarboximide (BND)

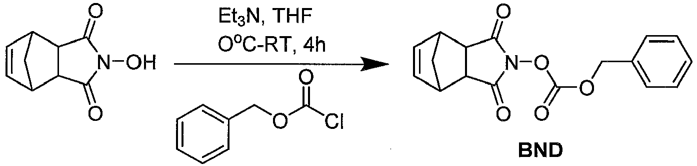

A solution of 4-Benzylchloroformate (39 mL, 46.61 g, 0.273 mol) in tetrahydrofuran (900 mL) was cooled to 0 °C and N-hydroxy-dicarboximide (47.5 g, 0.265 mol) was added. After subsequent addition of triethylamine (36.9 mL, 26.77 g, 0.265 mol) at 0 °C, the reaction mixture was allowed to warm up to room temperature and stir for another 4 hours. The reaction was cooled to -5 °C (ice salt bath) and the solid was filtered off. The Filtrate was concentrated and the residue was taken up in methanol (900 mL). The formed solid was filtered on a glass filter and dried at air yielding N-Benzyloxycarbonyloxy-5-norbornene-2,3-dicarboximide as white solid (48.5 g, 0.15 mol, 58%). LC/MS (System 1, method D): t_{R} (min) = 1.89; MS (m/z) = 352.1 (M+K⁺) observed, 352.06 (M+K⁺) calculated. ¹H NMR (300 MHz, CDCl₃) δ = 7.38 (5H), 6.17 (2H), 5.27 (2H), 3.45 (2H), 3.31 (2H), 1.78 (1H), 1.54 (1H).

The corresponding derivatives based on the Kanamycin A scaffold (6N-2-amino-ethyl-KanaA-AHB-F-Gua) or Kanamycin B scaffold (6N-2-amino-ethyl-KanaB-AHB-F-Gua) carrying 2-amino-ethyl at the 6N position can be obtained following the same sequence of reactions as shown above for **ABX5037** but starting from Kanamycin A or Kanamycin B, respectively.

### B.4.6 Tobra-(AHB-Gua)-F-Gua (ABX5033) / Tobra-(AFB)-F-Gua (ABX5034)

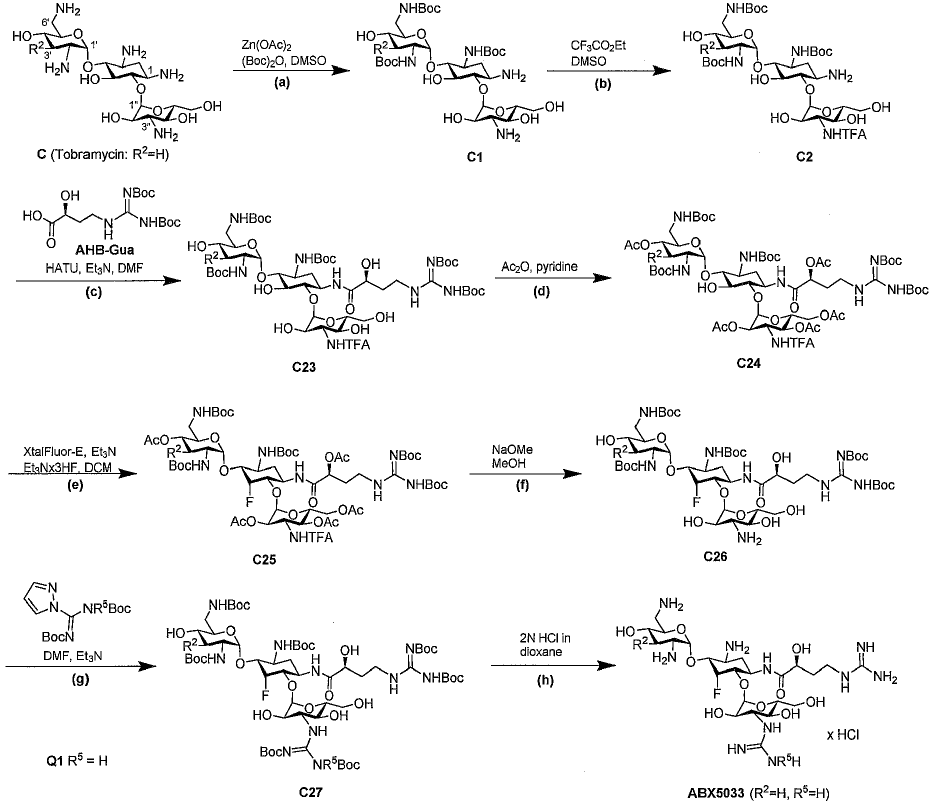

Compound **C2** was synthesized as described above under B.1.1 for the class 1 compounds.

**Step (c).** To a solution of **AHB-Gua** (1.7 g, 4.7 mmol, 1.2 equiv.) in dimethyl formamide (19 mL), triethylamine (670 µL, 4.8 mmol, 1.2 equiv.) was added and the mixture was stirred for 10 minutes at room temperature. A solution of Tobramycin derivative **C2** (3.45 g, 4 mmol, 1 equiv.) in dimethyl formamide (19 mL) was added followed by an immediate addition of HATU (1.54 g, 4 mmol, 1 equiv.) and the reaction mixture was stirred at room temperature overnight. After dimethyl formamide was evaporated *in vacuo* water (60 mL) was added to the residue and the mixture was shaken until white solid was formed and no residual oil was remaining. The white solid was filtered off, washed with sufficient amount of water (120 mL) and dried *in vacuo* by co-evaporation of the remaining water with toluene (3x). The resulting product **C23** (4.14 g, 3.43 mmol, 86% yield) was isolated as a white solid. LC/MS (System 2, method A): t_{R}(min) = 8.35; MS (m/z): 1207.45 (M+H⁺) observed, 1207.57 (M+H⁺) calculated.

**Step (d).** Compound **C23** (4 g, 3.31 mmol) was dissolved in pyridine (33 mL) and acetic anhydride (3.15 mL, 33.1 mmol, 10 equiv.) was added at room temperature and stirred for two days. The crude mixture was poured into heptane (100 mL) and the suspension was filtrated over a plug of silica. Product **C24** was eluted with heptane containing ethyl acetate (50-70%) (R_{f} = 0.41 using 70% ethyl acetate in heptane). After removal of the volatiles *in vacuo* product **C24** was obtained as white solid (2.05 g, 1.45 mmol, 44% yield). LC/MS (System 2, method A): t_{R} (min) = 10.78; MS (m/z): 1417.6 (M+H⁺) observed, 1417.57 (M+H⁺) calculated.

**Step (e).** Tobramycin derivative **C24** (1.95 g, 1.37 mmol) was dissolved in dichloromethane (14 mL), and triethylamine (382 µL, 2.74 mmol, 2 equiv.), triethylamine trihydrofluoride (893 µL, 5.48 mmol, 4 equiv.) and XtalFluor-E (941 mg, 4.11 mmol, 2 equiv.) were added in the exact order. After stirring at room temperature overnight, the reaction mixture was cooled to 0 °C and a saturated aqueous solution of sodium bicarbonate (50 mL) was added carefully. The crude mixture was allowed to warm up to room temperature under stirring, and the aqueous and organic layers were separated. The aqueous solution was extracted with dichloromethane (3x 50 mL) and all combined organic layers were dried over anhydrous sodium sulfate. After removal of the volatiles *in vacuo* the obtained residue was purified by column chromatography on silica using ethyl acetate (60%) in heptane (R_{f} = 0.57 using 70% ethyl acetate in heptane) as eluent yielding the product **C25** as pale yellow solid (1.02 g, 0.72 mmol, 52% yield). LC/MS (System 1, method A): t_{R} (min) = 10.74; MS (m/z): 1419.2 (M+H⁺) observed, 1419.62 (M+H⁺) calculated.

**Step (f).** To a solution of aminoglycoside derivative **C25** (985.6 mg, 0.69 mmol) in methanol (6.9 mL) was added sodium methanolate (373 mg, 6.9 mmol, 10 equiv.) and the reaction mixture was allowed to stir at room temperature for two days. Amberlite CG50 (H⁺ from) was added portion wise until a pH of 7 was reached. After removal of the Amberlite via filtration, the remaining solution was concentrated until dryness resulting in the crude product **C26** (1g) as white solid, which was used in the subsequent step without further purification. LC/MS (System 2, method A): t_{R} (min) = 6.31; MS (m/z): 1013.4 (M-Boc+H⁺) observed, 1013.53 (M-Boc+H⁺) calculated.

**Step (g).** The crude product **C26** (1g) was dissolved in DMF (50 mL), and triethylamine (1 mL, 7.17 mmol, approx. 4.5 equiv.) and reagent **Q1** (1.2 g, 3.87 mmol, approx. 2.4 equiv.) were added. The reaction mixture was allowed to stir at room temperature for two days. After removal of all volatiles the crude mixture was purified by column chromatography on silica using a 5% methanol solution in ethyl acetate (R_{f} = 0.23 in 100% EtOAc) yielding product **C27** as white solid (169 mg, 0.13 mmol, 19% yield over two steps). LC/MS (System 1, method A): t_{R} (min) = 8.24; MS (m/z): 1255.4 (M+H⁺) observed, 1255.67 (M+H⁺) calculated.

**Step (h).** To a solution of compound **C27** (118.6 mg, 0.094 mmol) in 1,4-dioxane (12 mL) a 4N hydrogen chloride solution in 1,4-dioxane (12 mL) was added and the reaction mixture was allowed to stir at room temperature overnight. Removal of the volatiles *in vacuo* resulted in a white solid, which was purified by recrystallization/precipitation from methanol/tetrahydrofuran yielding product **ABX5033** as penta-hydrogen chloride salt (836.99 g/mol, 55 mg, 0.066 mmol, 70% yield) as white solid. LC/MS (System 2, method B): t_{R} (min) = 2.02; MS (m/z): 655.2 (M+H⁺) observed, 655.35 (M+H⁺) calculated.

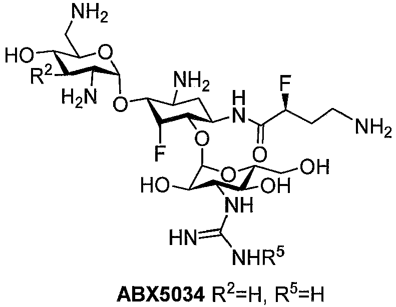

The penta-hydrogen chloride salt of **ABX5034** was synthesized as shown for **ABX5033** using the (2S)-Boc-4-amino-2-fluoro-butyric acid **(AFB-Boc)** as the reagent in step (c).

**ABX5034** (796.94 g/mol, penta-hydrogen chloride salt): white solid; LC/MS (System 1, method B): t_{R} (min) = 1.77; MS (m/z) = 615.2 (M+H⁺) observed, 615.33 (M+H⁺) calculated. ¹H NMR (300 MHz, D₂O) δ = 5.64 (d, *J* = 52 Hz, 1H), 5.49 (1H), 5.18 (ddd, *J* = 3.3 Hz, *J* = 9.1 Hz, *J* = 48.2 Hz, 1H), 5.16 (1H), 4.51 - 4.03 (3H), 4.03 - 3.62 (10H), 3.55 - 3.40 (2H), 3.34 - 3.16 (3H), 2.49 - 2.16 (3H), 2.16 - 1.98 (1H), 1.98 - 1.78 (1H). ¹⁹F NMR (282 MHz, D₂O) δ = -191.37 (ddd, ²*J*_{HF} = 48.6 Hz, ³*J*_{HF} = 33.8 Hz, ³*J*_{HF} = 18.8 Hz), - 215.93 (dt, ²*J*_{HF} = 52.5 Hz, ³*J*_{HF} = 26.2 Hz).

The corresponding derivatives based on the Kanamycin A scaffold (KanaA-(1N-R⁴)-F-Gua) or Kanamycin B scaffold (KanaB-(1N-R⁴)-F-Gua) carrying AHB-Gua or AFB in 1N-position can be obtained following the same sequence of reactions as shown above for **ABX5033** and **ABX5034** but starting from Kanamycin A or Kanamycin B, respectively.

### Synthesis of reagents AHB-Gua and AFB-Boc

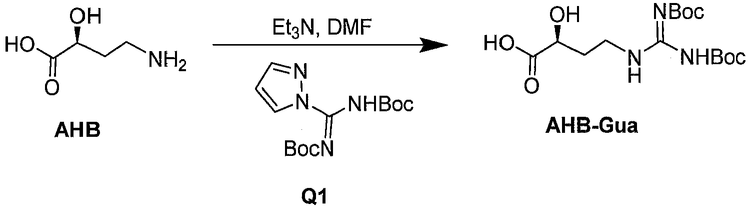

To a suspension of (2S)-4-amino-2-hydroxybutyric acid **(AHB)** (16.8 mmol, 2 g, 1 equiv.) in dimethyl formamide (20 ml), **Q1** (4.72 g, 15.2 mmol, 0.9 equiv.) was added at room temperature followed by triethylamine (25.7 ml, 184 mmol, 11 equiv.). After 5 days stirring at room temperature, volatiles were removed *in vacuo* and the residue was purified by column chromatography (9:1; ethyl acetate/heptane) affording the desired product **AHB-Gua** as pale-yellow oil (3.82 g, 10.58 mmol, 63% yield). LC/MS (System 1, method D): t_{R} (min) =1.89; MS (m/z) = 362.4 (M+H⁺) observed, 362.19 (M+H⁺) calculated. ¹H NMR (300 MHz, CD₃OD) δ = 4.22 (1H), 3.57 (2H), 2.11 (1H), 1.91 (1H), 1.57 (9H), 1.52 (9H).

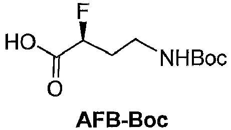

Reagent (2S)-Boc-4-amino-2-fluoro-butyric acid **(AFB-Boc)** was synthesized according to published procedures (M. E. Farkas, B. C. Li, C. Dose, P. B. Dervan, Bioorg. & Med. Chem. Lett. 2009, 19, 3919-3923).

**AFB-Boc** (221.11 g/mol): colorless oil; LC/MS (System 1, method D): t_{R} (min) = 0.55; MS (m/z) = 244.4 (M+Na⁺) observed, 244.10 (M+Na⁺) calculated. ¹H NMR (300 MHz, DMSO-*d₆*) δ = 13.27 (1H), 6.89 (1H), 4.96 (ddd, ²*J* = 49.0 Hz, ³*J* = 8.3 Hz, ³*J* = 3.7 Hz, 1H), 3.04 (2H), 2.08 - 1.65 (2H), 1.37 (9H).

### B.4.7 Tobra-(AHCA)-F-Gua (ABX5035)

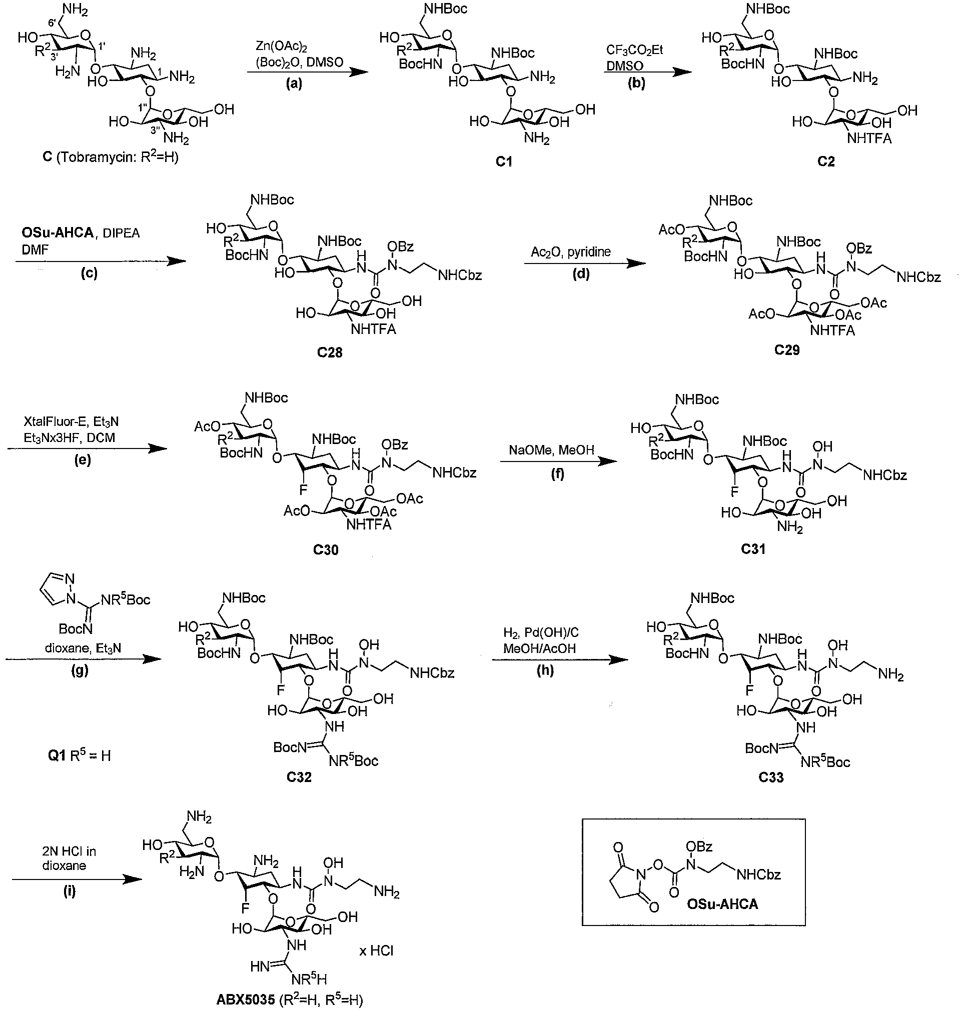

Compound **C2** was synthesized as described above under B.1.1 for the class 1 compounds.

**Step (c).** To a solution of Tobramycin derivative **C2** (72 mg, 0.084 mmol, 1 eqiuv.) in DMF (0.5 ml) was added a solution of DIPEA (60 µl, 0.34 mmol, 4 equiv.) and OSu-AHCA (98 mg, 0.21 mmol, 2.5 equiv.) in DMF (1.5 ml) dropwise at -40 °C. After 1 hour the reaction mixture was warmed to room temperature and stirred for 16 hours. Volatiles were removed *in vacuo* and the sticky residue was suspended in water and stirred for few hours in order to obtain a fine powder. The suspension was filtered over a glass funnel and the remaining product was dried by co-evaporation with toluene (3x). The desire product **C28** was obtained as a beige powder. (72.4 mg, 0.06 mmol, 71%) LC/MS (System 1, method A): t_{R} (min) = 9.23; MS (m/z) = 1104.2 (M-Boc+H⁺) observed, 1104.1 (M-Boc+H⁺) calculated.

The final compound **ABX5035** is accessible from intermediate **C28** following the sequence of reactions as shown in the reaction scheme using the procedures described above under B.4.5 (steps (d) to (h) and (k) under B.4.5) for 6N-substituted class 4 compounds.

The corresponding derivatives based on the Kanamycin A scaffold (KanaA-(1N-R⁴)-F-Gua) or Kanamycin B scaffold (KanaB-(1N-R⁴)-F-Gua) carrying AHCA in 1N-position can be obtained following the same sequence of reactions as shown above for **ABX5035** but starting from Kanamycin A or Kanamycin B, respectively.

### Synthesis of reagent OSu-AHCA

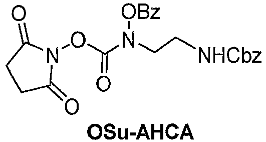

For the synthesis of the reagent **OSu-AHCA** the published procedures (P. Dozzo, A. A. Goldblum, J. B. Aggen, M. Sheringham Linsell, WO2010132768A9, 2011) were adapted as followed (3 steps):
**Step 1.** Benzyl chloroformate (7.8 mL, 55 mmol, 1 equiv.) in anhydrous dichloromethane (180 mL) was added dropwise over a period of 7 hours to a well-stirred solution of 1,2-diaminoethane (36 mL, 539 mmol, 10 equiv.) in anhydrous dichloromethane (540 mL) at -78 °C under nitrogen atmosphere. The solution was allowed to slowly warm up to 0 °C and was stirred at the same temperature overnight. The dicarbamate by-product was removed by filtration and the filtrate was washed with water (3 × 500 mL), dried over Na₂SO₄ and concentrated to give a mixture of benzyl (2-aminoethyl)carbamate and bis-Cbz-protected ethylenediamine. The mixture was solubilized in dichloromethane and washed with a 1M solution of hydrochloric acid. The aqueous phase was basified to pH = 12 and extracted again with dichloromethane. The organic layer was dried over Na₂SO₄ and volatiles were removed *in vacuo* affording the desired product as white solid (8.7 g, 44.8 mmol, 82%). LC/MS (System 1, method D): t_{R} (min) = 0.68; MS (m/z) = 195.4 (M+H⁺) observed, 195.11 (M+H⁺) calculated.
**Step 2.** To a solution of benzyl-N-(2-aminoethyl)carbamate chloride salt (7.9 g, 34.25 mmol, 1 equiv.) in sat. aq. NaHCO₃ (656 mL) was added 1 M NaOH (219 mL) and the reaction was stirred vigorously. Dichloromethane (438 mL) was added, followed by benzoylperoxide (containing 25% water, 22.05 g, 68.5 mmol, 2 equiv.) and the reaction was stirred overnight at room temperature. The organic layer was separated and washed with brine, dried over Na₂SO₄, filtered and concentrated *in vacuo* until approximately 100 ml of solution were left. The solution was poured into an excess of heptane (400 ml) over a silica column and washed abundantly with heptane. The product was eluted with a 35% solution of ethyl acetate in heptane (R_{f} = 0.48 using 40% ethyl acetate in heptane) and volatiles were removed *in vacuo* to yield the product as colorless oil (5.63 g, 17.9 mmol, 53%). LC/MS (System 1, method D): t_{R} (min) = 1.98; MS (m/z) = 315.4 (M+H⁺) observed, 315.13 (M+H⁺) calculated. **Step 3.** To a stirring solution of disuccinimidyl carbonate (461 mg, 1.8 mmol, 1.1 equiv.) in acetonitrile (25 mL) was added dropwise a solution of benzyl-2-(benzoyloxyamino)ethyl carbamate (252 mg, 1.6 mmol, 1 equiv.) as a solution in acetonitrile (25 mL). The reaction mixture was stirred overnight at 60 °C before all volatiles were removed *in vacuo.* The crude product was purified on a small pad of silica gel (1:1; heptane/EtOAc) affording the desired product as a yellow oil (98 mg, 0.21 mmol, 13%). LC/MS (System 1, method D): t_{R} (min) = 1.971; MS (m/z) = 456.3 (M+H⁺) observed, 456.14 (M+H⁺) calculated. ¹H NMR (300 MHz, CDCl₃) δ = 8.08 (2H), 7.71 - 7.61 (1H), 7.55 - 7.42 (2H), 7.41 - 7.28 (5H), 5.57 (1H), 5.09 (2H), 4.02 (2H), 3.52 (2H), 2.77 (4H).

### B.4.8 3'-epi-3'-O-alkyl KanaB-AHB-F-Gua (ABX4012)

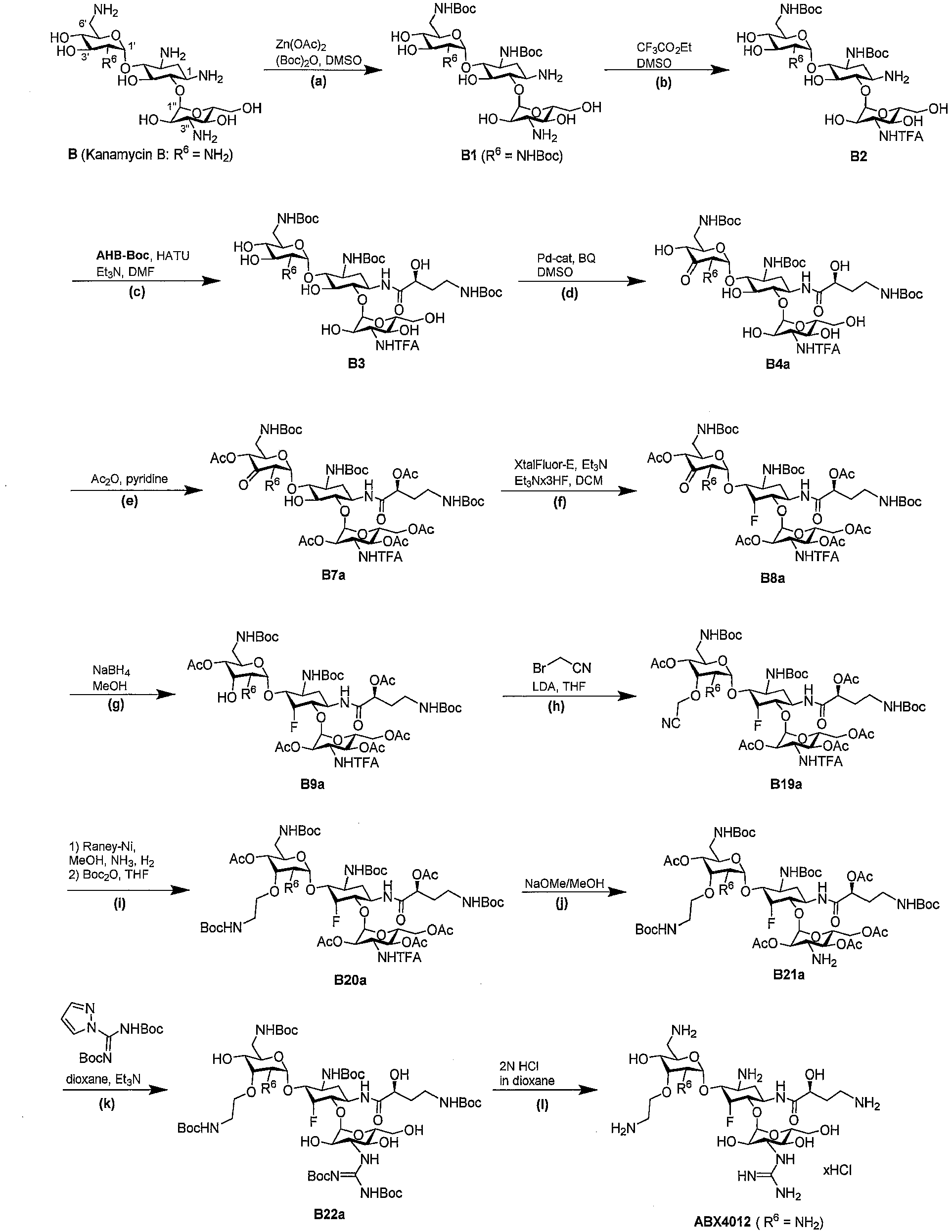

Compound **B9a** was synthesized as described above under B.2.2 for the class 2 compounds.

**Step (h).** A solution of LDA (2M in THF/heptane/ethylbenzene, 1.2 equiv.) was added dropwise to a pre-cooled solution of the Kanamycin B derivative **B9a** (1 equiv.) in tetrahydrofuran (20 mL/mmol) at -35 °C. After stirring at this temperature for 30 minutes the reaction was warmed up to -20 °C and a solution of bromoacetonitrile (2 equiv.) in tetrahydrofuran (1.5 mL/mmol of bromoacetonitrile) was added dropwise. The reaction was stirred for 4 hours at -10 °C before it was allowed to stir over night at room temperature. A saturated aqueous ammonium chloride solution was added and the aqueous layer was extracted with DCM (3x). The combined organic layers were dried over Na₂SO₄ and concentrated to yield the desired product **B19a.**

**Step (i).** A solution of aminoglycoside derivative **B19a** (1 equiv.) in methanolic ammonia (7N NH₃ in methanol, 20 mL/mol) was added to Raney-Ni (50% slurry, in H₂O, 1 mL/mmol) under inert atmosphere (N₂) at room temperature. The reaction vessel was flushed with H₂ and the reaction was stirred over night at room temperature. The reaction mixture was filtered through celite and concentrated *in vacuo* to yield the desired crude product. To a solution of the crude product (1 equiv.) in tetrahydrofuran (10 mL/mmol) triethylamine (3 equiv.) and di-tert-butyl dicarbonate (1.5 equiv.) were added and the reaction mixture was allowed to stir at room temperature for two days. After concentration *in vacuo* the residue was shaken in water. The precipitate formed was filtered off, washed with water (2x) and dried via co-evaporation with toluene (3x) resulting in product **B20a.**

**Step (j).** To a solution of aminoglycoside derivative **B20a** (1 equiv.) in methanol (10 mL/mmol) was added sodium methanolate (10 equiv.) and the reaction mixture was allowed to stir at room temperature for two days. Amberlite CG50 (H⁺ from) was added portion wise until a pH of 7 was reached. After removal of the Amberlite via filtration, the remaining solution was concentrated until dryness resulting in the crude product **B21a.**

**Step (k).** Kanamycin B derivative **B21a** (1 equiv.) was dissolved in 1,4-dioxane (70 mL/mmol), and triethylamine (10 equiv.) and N,N'-Bis-boc-1-guanylpyrazole **(Q1)** (9 equiv.) were added and the reaction mixture was allowed to stir at 45 °C for three days. After removal of all volatiles the crude mixture was purified by column chromatography on silica using ethyl acetate in heptane yielding product **B22a.**

**Step (I).** To a solution of compound **B22a** (1 equiv.) in 1,4-dioxane (20 mL/mmol) a 4N hydrogen chloride solution in 1,4-dioxane (20 mL/mmol) was added and the reaction mixture was allowed to stir overnight. Removal of the volatiles *in vacuo* resulted in the crude product, which was purified by recrystallization/precipitation from methanol/DCM yielding product **ABX4012** as hexa-hydrogen chloride salt.

The corresponding derivative based on the Kanamycin A scaffold (3'-epi-3'-O-alkyl KanaA-AHB-F-Gua) carrying a residue on the epimerized hydroxy group in 3'-position can be obtained following the same sequence of reactions as shown above for **ABX4012.**

### B.4.9 3'-Me-3'-epi-3'-O-alkyl Kanamycin B-AHB-F-Gua (ABX4013)

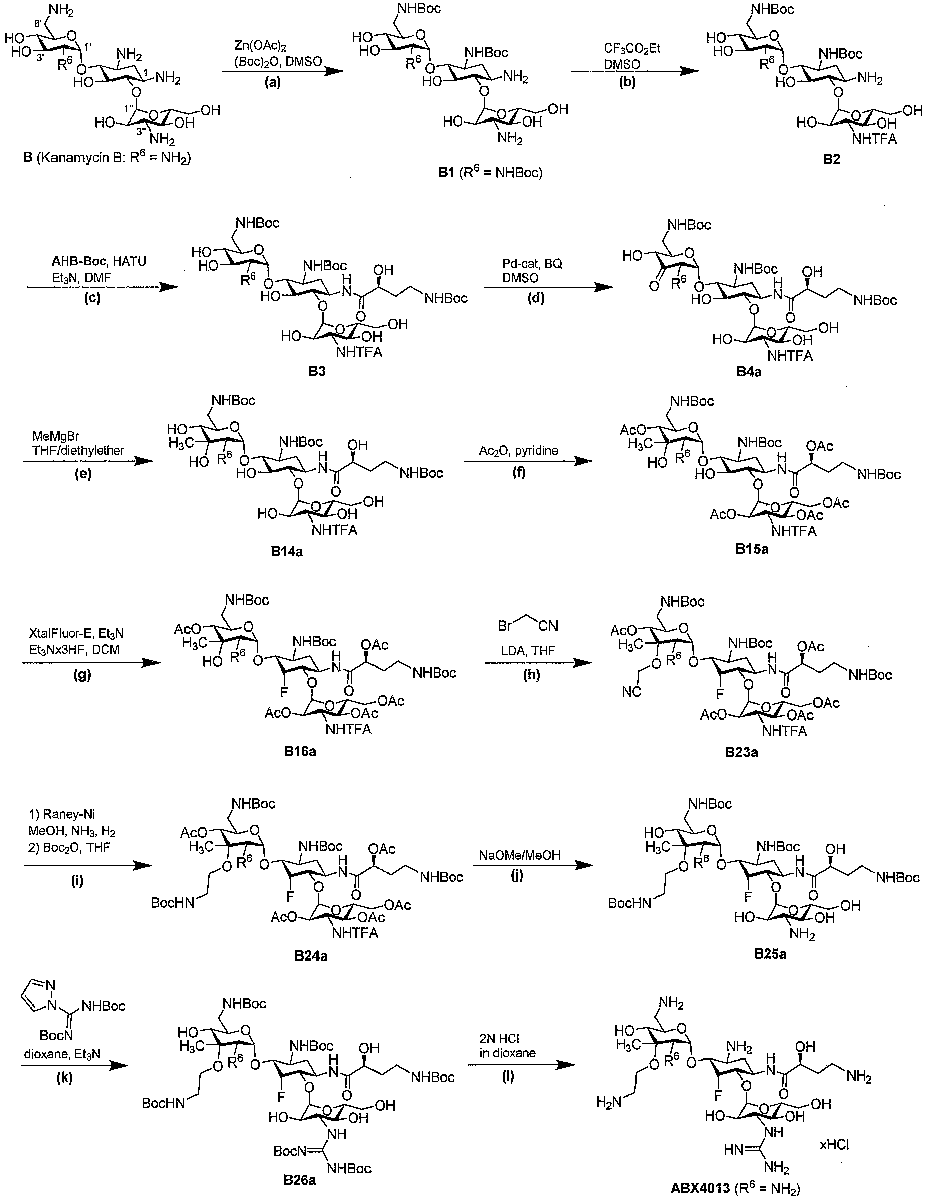

Compound **B16a** was synthesized as described above under B.4.4 for the class 4 compounds (whereby, compound **B4a** can be obtained using the under B.1.2 described sequence of reactions for the class 1 compounds).

**Step (h).** A solution of LDA (2M, 1.2 equiv.) was added dropwise to a pre-cooled solution of the Kanamycin B derivative **B16a** (1 equiv.) in tetrahydrofuran (20 mL/mmol) at -35 °C. After stirring at this temperature for 30 minutes the reaction was warmed up to -20 °C and a solution of bromoacetonitrile (2 equiv.) in tetrahydrofuran (1.5 mL/mmol of bromoacetonitrile) was added dropwise. The reaction was stirred for 4 hours at -10 °C before it was allowed to stir over night at room temperature. A saturated aqueous ammonium chloride solution was added and the aqueous layer was extracted with DCM (3x). The combined organic layers were dried over Na₂SO₄ and concentrated to yield the desired product **B23a.**

**Step (i).** A solution of aminoglycoside derivative **B23a** (1 equiv.) in methanolic ammonia (7N NH₃ in methanol, 20 mL/mol) was added to Raney-Ni (50% slurry, in H₂O, 1 mL/mmol) under inert atmosphere (N₂) at room temperature. The reaction vessel was flushed with H₂ and the reaction was stirred over night at room temperature. The reaction mixture was filtered through celite and concentrated *in vacuo* to yield the desired crude product. To a solution of the crude product (1 equiv.) in tetrahydrofuran (10 mL/mmol) triethylamine (3 equiv.) and di-tert-butyl dicarbonate (1.5 equiv.) were added and the reaction mixture was allowed to stir at room temperature for two days. After concentration *in vacuo* the residue was shaken in water. The precipitate formed was filtered off, washed with water (2x) and dried via co-evaporation with toluene (3x) resulting in product **B24a.**

**Step (j).** To a solution of aminoglycoside derivative **B24a** (1 equiv.) in methanol (10 mL/mmol) was added sodium methanolate (10 equiv.) and the reaction mixture was allowed to stir at room temperature for two days. Amberlite CG50 (H⁺ from) was added portion wise until a pH of 7 was reached. After removal of the Amberlite via filtration, the remaining solution was concentrated until dryness resulting in the crude product **B25a.**

**Step (k).** Kanamycin B derivative **B25a** (1 equiv.) was dissolved in 1,4-dioxane (70 mL/mmol), and triethylamine (10 equiv.) and N,N'-Bis-boc-1-guanylpyrazole **(Q1)** (9 equiv.) were added and the reaction mixture was allowed to stir at 45 °C for three days. After removal of all volatiles the crude mixture was purified by column chromatography on silica using ethyl acetate in heptane yielding product **B26a.**

**Step (I).** To a solution of compound **B26a** (1 equiv.) in 1,4-dioxane (20 mL/mmol) a 4N hydrogen chloride solution in 1,4-dioxane (20 mL/mmol) was added and the reaction mixture was allowed to stir overnight. Removal of the volatiles *in vacuo* resulted in the crude product, which was purified by recrystallization/precipitation from methanol/DCM yielding product **ABX4013** as the hexa-hydrogen chloride salt.

The corresponding derivative based on the Kanamycin A scaffold (3'-Me-3'-epi-3'-O-alkyl KanaA-AHB-F-Gua) carrying a residue on the epimerized hydroxy group in 3'-position can be obtained following the same sequence of reactions as shown above for **ABX4013.**

### B.4.10 6N-2-hydroxy-ethyl-Tobra-AHB-F-Gua (ABX5045)

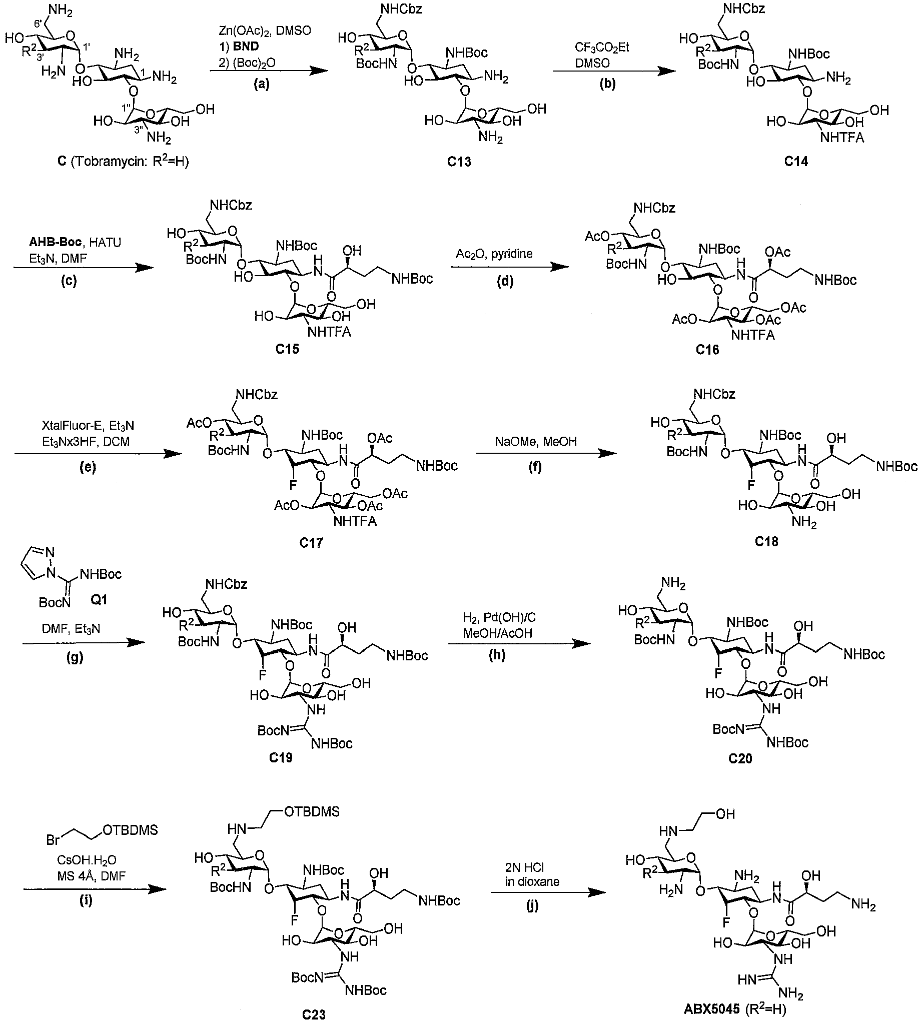

Compound **C20** was synthesized as described above under B.4.5 for the class 4 compounds.

**Step (i).** Cesium(II)hydroxide monohydrate (1 equiv.) and molecular sieves (4 A) were taken up in DMF (10 mL/mmol) and the suspension was stirred at 35 °C for 10 minutes before compounds **C20** (1 equiv.) was added. After 2h stirring at 35 °C (2-bromoethoxy)-tert-butyldimethylsilane (2 equiv.) was added and the reaction mixture was stirred at 35 °C for 24 hours. The molecular sieves were filtered off over a glass filter and the solution was concentrated. The crude was purified by column chromatography on silica using methanol in ethyl acetate to elute the product **C23.**

**Step (j).** To a solution of compound **C23** (1 equiv.) in 1,4-dioxane (20 mL/mmol) a 4N hydrogen chloride solution in 1,4-dioxane (20 mL/mmol) was added and the reaction mixture was allowed to stir overnight. Removal of the volatiles *in vacuo* resulted in the crude product, which was purified by recrystallization/precipitation from methanol/DCM yielding product **ABX5045** as hydrogen chloride salt.

**ABX5045** as penta-hydrogen chloride salt (839.00 g/mol) as white solid. LC/MS (System 2, method B): t_{R} (min) = 2.13; MS (m/z): 657.4 (M+H⁺) observed, 657.36 (M+H⁺) calculated.

The corresponding derivatives based on the Kanamycin A scaffold (6N-2-hydroxy-ethyl-KanaA-AHB-F-Gua) or Kanamycin B scaffold (6N-2-hydroxy-ethyl-KanaB-AHB-F-Gua) carrying 2-hydroxy-ethyl at the 6N position can be obtained following the same sequence of reactions as shown above for **ABX5045** but starting from Kanamycin A or Kanamycin B, respectively.

### B.5 Guanidination reagents

| 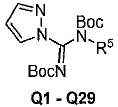 | | | | | | | | |
|---|---|---|---|---|---|---|---|---|
| **Q1** | R⁵ = | 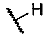 | **Q11** | R⁵ = | 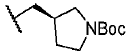 | **Q21** | R⁵ = | 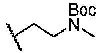 |
| **Q2** | R⁵ = | 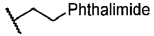 | **Q12** | R⁵ = | 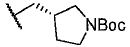 | **Q22** | R⁵ = | 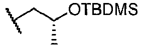 |
| **Q3** | R⁵ = | 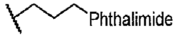 | **Q13** | R⁵ = | 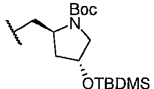 | **Q23** | R⁵ = | 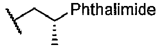 |
| **Q4** | R⁵ = | 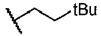 | **Q14** | R⁵ = | 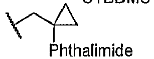 | **Q24** | R⁵ = | 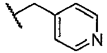 |
| **Q5** | R⁵ = | 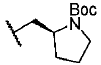 | **Q15** | R⁵ = | 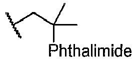 | **Q25** | R⁵ = | 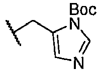 |
| **Q6** | R⁵ = | 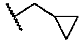 | **Q16** | R⁵ = | 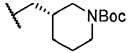 | **Q26** | R⁵ = | 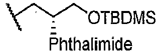 |
| **Q7** | R⁵ = | 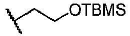 | **Q17** | R⁵ = | 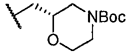 | **Q27** | R⁵ = | 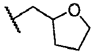 |
| **Q8** | R⁵ = | 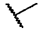 | **Q18** | R⁵ = | 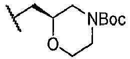 | **Q28** | R⁵ = | 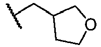 |
| **Q9** | R⁵ = | 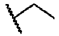 | **Q19** | R⁵ = | 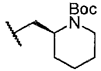 | **Q29** | R⁵ = | 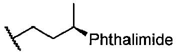 |
| **Q10** | R⁵ = | 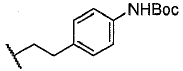 | **Q20** | R⁵ = | 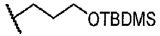 | | | |

General procedure for the synthesis of reagents **Q2** - **Q6** and **Q8** - **Q29** starting from **Q1** and an alcohol carrying R⁵:

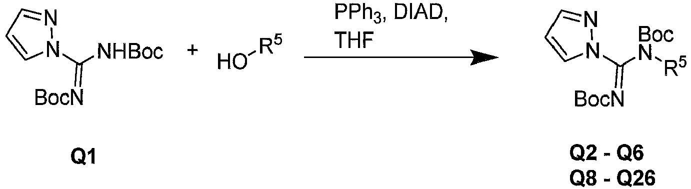

### Example Q2:

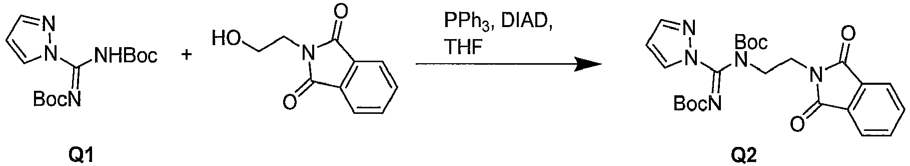

N,N'-Bis-boc-1-guanylpyrazole **Q1** (10.09 g, 32.5 mmol) and N-(2-hydroxyethyl)phthalimide (9.32 g, 48.75 mmol, 1.5 equiv.) were dissolved in tetrahydrofuran (140 mL) and triphenyl phosphine (12.79 g, 48.75 mmol, 1.5 equiv.) was added at room temperature. The reaction mixture was cooled to 0 °C and Di-isopropyl-azodicarboxylate (15.21 g, 14.81 mL, 75.22 mmol, 2.3 equiv.) was added dropwise over a period of 30 minutes. The reaction mixture was allowed to warm up to room temperature and to stir for 2 days. After removal of the volatiles the entire crude residue was purified by column chromatography using up to 30% ethyl acetate in heptane (R_{f} = 0.38 using 30% ethyl acetate in heptane) yielding product **Q2** as white solid (10.33 g, 21.36 mmol, 66% yield). LC/MS (System 1, method D): t_{R} (min) = 2.19; MS (m/z) = 484.2 (M+H⁺) observed, 484.22 (M+H⁺) calculated. ¹H NMR (300 MHz, CDCl₃) δ = 7.95 (1H), 7.83 (2H), 7.69 (2H), 7.57 (1H), 6.37 (1H), 4.05 (4H), 1.40 (9H), 1.24 (9H).

General procedure for the synthesis of reagent **Q7** starting from **Q1** and a bromide carrying R⁵:

### Example Q7:

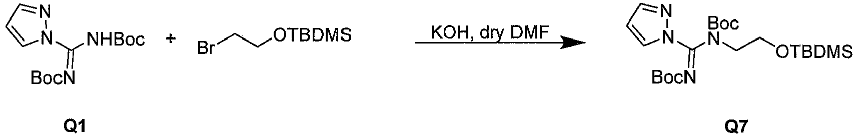

To a solution of potassium hydroxide (85% pure, 768 mg, 11.63 mmol, 1 equiv.) and N,N'-Bis-boc-1-guanylpyrazole **Q1** (3.61 g, 11.63 mmol, 1 equiv.) in dry DMF (16 mL), (2-bromoethoxy)(tert-butyl)dimethylsilane (2.5 ml, 11.63 mmol, 1 equiv.) in dry DMF (20 mL) was added dropwise. After stirring at 30 °C for 10 days the reaction mixture was partitioned between water and ethyl acetate. The organic phase was separated, dried over anhydrous sodium sulfate and concentrated. The residue was purified by the use of column chromatography on silica gel using up to 10% of ethyl acetate in heptane (R_{f} = 0.76 using 30% ethyl acetate in heptane) yielding the product **Q7** as colorless oil (1.85 g, 3.95 mmol, 34% yield). LC/MS (System 1, method D): t_{R} (min) = 2.91; MS (m/z) = 469.4 (M+H⁺) observed, 469.28 (M+H⁺) calculated. ¹H NMR (300 MHz, CDCl₃) δ = 7.97 (1H), 7.67 (1H), 6.37 (1H), 3.85 (4H), 1.49 (9H), 1.26 (9H), 0.83 (9H), 0.01 (6H).

### Biological Data and Results

*Escherichia coli* is, *inter alia,* among the most prominent Gram-negative bacteria found in hospital-treated infections and hospital-acquired infections. The diversity of diseases caused by *Escherichia coli* results from the acquisition of specific virulence factors that are harbored on transmissible genetic elements (e.g. plasmids) or within distinct DNA segments, called pathogenicity islands, that are absent from nonpathogenic strains. As a result, many strains of *Escherichia coli* exist, and some are more virulent than others. This highly adaptable bacterial species is usually opportunistic when encountered in the hospital setting and is commonly identified in, *inter alia,* urinary tract infections, intra-abdominal wound infections, and bacteremia. The provision of novel compounds exhibiting activity against *Escherichia coli,* including *Escherichia coli* strains harboring plasmids encoding different aminoglycoside modifying enzymes (AMEs), which are known to be responsible for bacterial resistance against aminoglycoside (AG) antibiotics, would thus constitute a valuable contribution to the art and thus constitutes an object underlying the present invention.

As alluded to herein above, the compounds of the present invention exhibit not only excellent activity against a range of strains of *Escherichia coli (E. coli)* including Wild Type *E. coli* and *E. coli* strains expressing a series of different aminoglycoside modifying enzymes (AMEs) known to be responsible for bacterial resistance against aminoglycoside (AG) antibiotics but also a synergistic activity profile relative to the analogous compounds which bear either the 5-Fluoro or 3"-Guanidyl substituent alone. As such, the specific combination of both the 5-Fluoro and 3"-Guanidyl substituents leads to a level of activity against the respective *E. coli* strains which would have been entirely unpredictable based on the known prior art. In this regard, to the best of the knowledge of the applicant there is no teaching whatsoever in the prior art which would lead to the expectation that both a 5-Fluoro (i.e. 5-epi-5-Fluoro) and a 3"-Guanidyl substituent would be simultaneously tolerated in a single molecule from the perspective of antibacterial activity, let alone in such a wide range of aminoglycoside structures as demonstrated for the compounds of the present invention. That such a combination of substituents not only gives rise to compounds, which continue to demonstrate activity against the respective bacterial strain but, in fact, for which a synergistic (not merely additive) increase in potency is observed is entirely surprising and unexpected.

In this regard, reference is made to the data presented in Table 1 below. Four Amikacin-like (4,6-disubstituted-2-DOS AGs bearing a AHB group at the N1-position; 2-DOS=2-desoxystreptamine, AHB = (2S)-4-amino-2-hydroxybutyrate) antibiotics bearing neither the 5-Fluoro nor the 3"-Guanidyl substituents were selected as the scaffolds on which the effect of the substituents was to be tested. These compounds were, specifically, ABX4001 (1N-AHB-kanamycin B =amikacin B), ABX5004 (1N-AHB-torbramycin), ABX3002 (1N-AHB-3'-epi-kanamycin A =3'-epi-amikacin A), and ABX4002 (1N-AHB-3'-epi-kanamycin B =3'-epi-amikacin B), the structures of which are shown both herein above and in Figure 1 below. Analogous compounds of each bearing the 5-Fluoro, 3"-Guanidyl and both the 5-Fluoro and 3"-Guanidyl substituents were synthesized as outlined herein above. The antibacterial activities of each were investigated using an antimicrobial susceptibility test using the method set forth under A.3.1 of the Examples section above, during which the minimal inhibitory concentrations (MICs) for each compound were determined against the various bacterial strains set forth in Table 1. Further tested against these bacterial strains was a series of compounds bearing additional substitution on the 3"-Guanidyl group. Table 1 shows the MIC values of said compounds against American Type Culture Collection (ATCC) strains of *Escherichia coli.* All compounds have been tested against the *Escherichia coli* wild type strain (i.e. ATCC25922), which is also used for quality control, and *Escherichia coli strains* harboring plasmids encoding different AMEs.

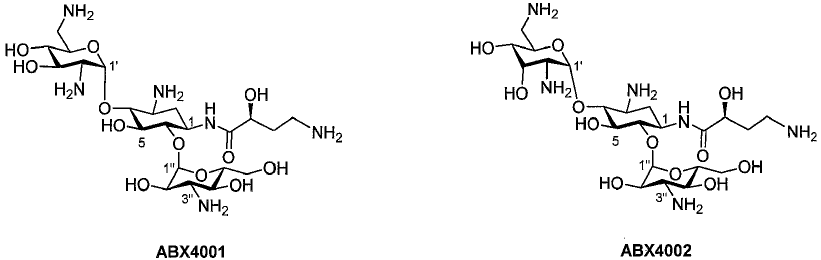

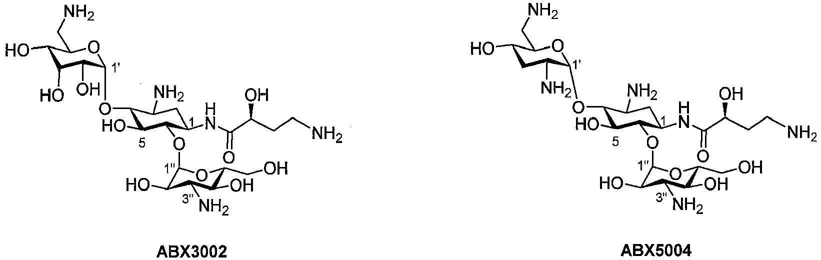

### Figure 1 - Aminoglycoside (AG) Scaffolds

As can be seen from Table 1, irrespective of which *E. coli* strain (be it wild-type or those expressing the various AMEs) is tested against, the compound exhibiting simultaneously both the 5-Fluoro and 3"-Guanidyl groups (i.e. ABX4006, ABX4004, ABX3003, ABX5006) not only demonstrates improved activity against the respective strain relative to the "parent compound" bearing neither of said substituents (i.e. ABX4001, ABX4002, ABX3002, ABX5004) (which itself was entirely unexpected), but also a level of activity which is improved when compared to the introduction of either of these substituents alone. Furthermore and most surprising of all, the combination of each of these substituents in a single molecule is seen to have a synergistic effect on activity which goes beyond what would be expected from the data shown in Table 1 when considering the respective effects on activity for the analogous compounds bearing either a 5-Fluoro or 3"-Guanidyl group relative to the respective "parent compounds". In fact, in several cases, it is even observed that the introduction of the 3"-Guanidyl substituent alone leads to a loss of activity yet, when this substituent is introduced into the corresponding molecule bearing the 5-Fluoro substituent, not only is the expected loss of activity not seen but, remarkably, an improvement in potency is observed. In this regard, reference is made, in particular, to the data of compound ABX4009 versus ABX4006, ABX4005 versus ABX4004, and ABX3004 versus ABX3003 against *E. coli* expressing APH3'(IIIa); ABX4005 versus ABX4004, and ABX3004 versus ABX3003 against *E. coli* expressing APH3'(Ia); ABX4005 versus ABX4004, and ABX3004 versus ABX3003 against *E. coli* expressing AAC(6')Ie-APH(2")Ia.

As such, the data in Table 1 further clearly demonstrate that compounds bearing both the 5-Fluoro- and 3"-Guanidyl group, i.e. ABX4006, ABX4004, ABX3003 and ABX5006, overcome bacterial resistance mediated by all AMEs tested. While *Escherichia coli* strains expressing the AME APH(3')IIIa are resistant to amikacin (Breakpoint for amikacin at MIC >16 according to EUCAST - "The European Committee on Antimicrobial Susceptibility Testing. Breakpoint tables for interpretation of MICs and zone diameters, Version 8.1, 2018. http://www.eucast.org.") and strains harboring resistant-causing enzymes AAC(3)III, AAC(6')Ie-APH(2")Ia or AAC(3')IV are resistant to gentamicin (Breakpoint for gentamicin at MIC >4 according to EUCAST Breakpoint tables for interpretation of MICs and zone diameters. Version 8.1, 2018. http://www.eucast.org."), all bacterial strains tested show increased susceptibility to the compounds of the invention. Furthermore, the compounds of the invention exhibit, in almost all cases, potencies substantially higher against all pathogens tested than that of the clinically administered antibiotic Amikacin (and never show lower potency) whilst, against *E. coli* expressing AAC(3)III, AAC(6')Ie-APH(2")Ia or AAC(3')IV, all compounds of the invention exhibit substantially improved potency compared to gentamicin. Even in the case of compounds for which the potency against a given strain is equal to and thus not improved relative to that of Amikacin (or Gentamicin), the compounds of the invention are seen to have a broader spectrum of antibacterial activity against the strains tested compared to the clinical comparator(s). Assuming that compounds of the invention have a breaking point similar or equal to Amikacin, each compound bearing a 5-Fluoro- and 3"-Guanidyl group overcomes class-related bacterial resistance mediated by AMEs.

Not only do the data in Table 1 serve to demonstrate the above-outlined effects and advantages of the compounds of the invention which bear a free guanidine group at the 3"-C position (also referred to as the 3"-position), but, moreover, the surprising activity of hitherto unknown substituted (derivatized) guanidines at this position is also demonstrated (compounds ABX5020, ABX5025, ABX5026, ABX5027, ABX5029, ABX5030, ABX5034, ABX5038, ABX5039, ABX5040, ABX5041, ABX5042, ABX5043, ABX5044, ABX5045, ABX5046, ABX5047, ABX5048, ABX5050 and ABX5051). Such additional structural alternations are not only of interest as a tool to tackle bacterial resistance or to increase a drug's general potency or affinity to the target molecule, but are also an important strategy for modifying the physical (e.g. solubility, melting point, lipophilicity, hygroscopicity) and/or pharmacological (e.g. volume of distribution, plasma protein binding, toxicology, metabolism profile, CYP inhibition, hERG activity, etc.) properties of a molecule, properties which play an extremely important role in drug development. Not only do the data in Table 1 show that such substitution on the guanidine group is tolerated in terms of activity/potency against all pathogens tested but compounds bearing these substituents continue to exhibit significantly improved activity relative to their respective parent compound (ABX5004). Furthermore, in many cases (for example, against *E. coli* and *E. coli APH(3')IIIa*) these compounds continue to show levels of activity which are synergistic relative to those which would be expected based on the individual data for ABX5024 (5-F) and ABX5005 (3"-guanidiyl). Moreover, all compounds bearing such a substituted guanidine moiety at the 3"-C position show significantly improved activity against all pathogens tested when compared to the clinically administered antibiotic Amikacin. In addition, each of those derivatives possesses an improved or at least equal antibacterial activity when compared to Gentamicin.

In addition, the results in Table 1 demonstrate that the simultaneous introduction of the 5-Fluoro and 3"-Guanidyl substituents can also be used to regain potency in AG derivatives, which have lost some of their activity due to previous structural modifications. For example, epimerization of the hydroxyl group at the 3'-C position was introduced in 4,6-disubstituted-2-DOS AGs (Jaeger, M. Selective oxidation of glycosides, Ph.D. thesis, ISBN 978-90-367-7965-4 (digital version), 2015, 83-130) in order to tackle bacterial resistance mediated by APH(3') enzymes. However, this modification results in significant loss of antibacterial activity against the wild type E. coli strain. As shown in Table 1, derivatives ABX3002 and ABX4002 are the direct analogues of amikacin and ABX4001 (=amikacin B), respectively in which the hydroxyl group at the 3'-C position has been epimerized. The MIC values in Table 1 show that epimerization at the 3'-C position results in a 4-fold and between 8- and 16-fold decreased antibacterial activity for the amikacin and the amikacin B (ABX4001) scaffold, respectively, leaving both AG derivatives ABX4002 and ABX3002 with MIC values against *E. coli* wild-type of 8 and 16-32, respectively. As demonstrated in Table 1, however, through introduction of the 5-Fluoro and 3"-Guanidyl substituents in accordance with the present invention (resulting in ABX3003 and ABX4004 respectively), the potencies of the parent compounds Amikacin and ABX4001 are once more realized despite the epimerization at the 3'-C position (Table 1). Moreover, the compounds of the invention show not only regained antibacterial activity against all *Escherichia coli* strains tested, but are seen to also overcome bacterial resistance mediated by all AMEs tested.

In the following Table (Table 1) and all further Tables presented herein below, values expressed using commas (standard expression in, *inter alia,* Germany for a "decimal point") are to be understood to correspond to the same value which would be expressed using a decimal point in, for example, the United Kingdom or the United States of America. For example, a value expressed in a Table herein below as being 0,25 corresponds to the value which would be expressed in, for example, the United Kingdom or the United States of America as 0.25 and is equal to one quarter (1/4). Furthermore, the values reported in the Tables herein are often expressed in terms of ranges, e.g. 1-2, 4-8, 16-32, etc. Such ranges are to be understood to include all values between the explicitly recited end points but not including said end points. As such, a range of 1-2 is to be understood to refer to a value between 1 and 2 but not including 1 or 2, i.e. greater than 1 but less than 2. Consequently, a reported activity value of 1 represents an improvement over a reported activity value of 1-2 and there is no overlap of said activities at the value of 1. Likewise, a reported activity value of 1-2 represents an improvement over a reported activity value of 2 and there is no overlap of said activities at the value of 2. The same applies for all further activity ranges and values expressed in this manner in the Tables herein below. Finally, in the interests of clarity it should be noted that the manner and form used herein for expressing such ranges as, for example, 1-2 or 4-8 may commonly be expressed in the art using "slashes" as opposed to hyphens, i.e. as 1/2 and 4/8 respectively. Each such manner of expressing said ranges is standard in the art.

**Table 1. Antimicrobial susceptibility testing with a wild-type Escherichia coli and Escherichia coli strains expressing different AMEs.**

| **¹MIC 50 (µmol/L)** | **Derivatization** | ***²E. coli*** | ***³E. coli* (APH(3')IIIa)** | ***³E. coli* (APH(3')la)** | ***³E. coli* (AAC(6')le-APH(2")la)** | **³*E. coli* (AAC(3)III)** | **³*E. coli* (AAC(3)IV)** |
|---|---|---|---|---|---|---|---|
| **Amikacin** | | 2 | 16-32 | 8 | 4-8 | 2 | 8 |
| **Genatmicin** | | 1 | 1 | 2 | 16 | >64 | >64 |
| **ABX4001** | | 2 | 8-16 | 8 | 4-8 | 1-2 | 4 |
| **ABX4007** | **5C-F** | 1 | 16 | 4 | 2-4 | 1 | 4-8 |
| **ABX4009** | **3"C-Gua** | 2 | 64 | 8 | 2-4 | 1-2 | 8 |
| **ABX4006** | **5C-F, 3"C-Gua** | 0,25-0,5 | 4-8 | 1-2 | 1 | 0,25 | 2 |
| **ABX4002** | | 8 | 8 | 8-16 | 4-8 | 4 | 8-16 |
| **ABX4003** | **5C-F** | 4-8 | 8 | 8-16 | 8 | 2 | 8-16 |
| **ABX4005** | **3"C-Gua** | 4 | 8-16 | 16 | 8 | 4 | 16 |
| **ABX4004** | **5C-F,3"C-Gua** | 1-2 | 1-2 | 4 | 2 | 0,5 | 4 |
| **ABX3002** | | 16-32 | 16-32 | 32 | 16 | 16 | 32 |
| **ABX3005** | **5C-F** | 4 | 4-8 | 8-16 | 4 | 4 | 8-16 |
| **ABX3004** | **3"C-Gua** | 16-32 | 64 | 32-64 | 32 | 16 | 64 |
| **ABX3003** | **5C-F, 3"C-Gua** | 2 | 2 | 8 | 2 | 1 | 4 |
| **ABX5004** | | 2 | 4-8 | 4-8 | 4 | 1 | 4 |
| **ABX5024** | **5C-F** | 1 | 1 | 2-4 | 1 | 1 | 2 |
| **ABX5005** | **3"C-Gua** | 1-2 | 2-4 | 2-4 | 2 | 0,5 | 1-2 |
| **ABX5006** | **5C-F,3"C-Gua** | <0,25 | <0,25 | 0,5 | <0,25 | 0,25 | 0,5-1 |
| **ABX5020** | **5C-F, 3"C-Gua-R⁵** | 0,5 | 0,5 | 1-2 | 0,5 | 0,25-0,5 | 1 |
| **ABX5025** | **5C-F, 3"C-Gua-R⁵** | 0,25-0,5 | 0,25-0,5 | 1 | 0,5 | 0,5 | 2 |
| **ABX5026** | **5C-F, 3"C-Gua-R⁵** | 0,25 | 0,25 | 1 | 0,25-0,5 | 0,25 | 0,5 |
| **ABX5027** | **5C-F, 3"C-Gua-R⁵** | 0,5 | 0,25-0,5 | 1 | 0,5 | 0,25 | 2 |
| **ABX5029** | **5C-F, 3"C-Gua-R⁵** | 0,5 | 0,5 | 2 | 0,5-1 | 0,5 | 2 |
| **ABX5030** | **5C-F, 3"C-Gua-R⁵** | 0,25 | 0,25 | 2 | 0,25-0,5 | 0,25 | 0.5 |
| **ABX5034** | **5C-F, 3"C-Gua-R⁵** | 1 | 0,5-1 | 1 | 1 | 0,5-1 | 2-4 |
| **ABX5038** | **5C-F, 3"C-Gua-R⁵** | 0,25 | 0,25 | 0,5 | 0,25 | 0,25 | 1 |
| **ABX5039** | **5C-F, 3"C-Gua-R⁵** | 0,25 | 0,25 | 0,25 | 0,25 | 0,25 | 1 |
| **ABX5040** | **5C-F, 3"C-Gua-R⁵** | 0,5 | 0,25 | 1 | 0,25 | 0,25 | 1 |
| **ABX5041** | **5C-F, 3"C-Gua-R⁵** | 0,5 | 0,5 | 1 | 0,5 | 0,5 | 2 |
| **ABX5042** | **5C-F, 3"C-Gua-R⁵** | 0,25 | 0,25 | 0,25 | 0,25 | 0,125 | 1 |
| **ABX5043** | **5C-F, 3"C-Gua-R⁵** | 0,25 | 0,25 | 0,5-1 | 0,25 | 0,25 | 1 |
| **ABX5044** | **5C-F, 3"C-Gua-R⁵** | 0,5 | 0,5 | 0,5-1 | 0,5 | 0,5 | 2 |
| **ABX5045** | **5C-F, 3"C-Gua-R⁵** | 1 | 1 | 1 | 0,5 | 0,5-1 | 2 |
| **ABX5046** | **5C-F, 3"C-Gua-R⁵** | 0,5 | 0,5 | 1 | 0,5 | 0,5 | 1-2 |
| **ABX5047** | **5C-F, 3"C-Gua-R⁵** | 0,25 | 0,25 | 0,5 | 0,25 | 0,25 | 1-2 |
| **ABX5048** | **5C-F, 3"C-Gua-R⁵** | 1 | 0,25 | 1 | 0,5 | 0,5-1 | 1-2 |
| **ABX5050** | **5C-F, 3"C-Gua-R⁵** | 1 | 1-2 | 2 | 1 | 1 | 2-4 |
| **ABX5051** | **5C-F, 3"C-Gua-R⁵** | 0,5 | 0,5 | 1 | 0,5 | 0,5-1 | 2 |

| | | | | | | | |
|---|---|---|---|---|---|---|---|
| *¹The method used for antimicrobial testing was broth micro-dilution method performed according to CLSI guidelines as outlined herein above in the Materials and Methods Section; ²ATCC25922 strain, ³ATCC25922 strain harboring plasmids encoding AMEs.* | | | | | | | |

In order to determine whether the compounds of the invention additionally show potency against further bacterial species and, in particular, the aforementioned selected ESKAPE family bacteria, said compounds were initially tested for *in vitro* activity against four Gram-negative and one Gram-positive pathogen of the ESKAPE panel, namely *Staphylococcus aureus* (Gram+), *Klebsiella pneunomiae* (Gram-), *Acinetobacter baumannii* (Gram-), *Pseudomonas aeruginosa* (Gram-), and *Enterobacter cloacae* (Gram-). In addition, all compounds were tested against the Gram-positive pathogen *Enterococcus faecalis.* The results are presented in Tables 2 and 3.

With regards to the data presented in Table 2, the latter bacterial strain (*Enterobacter cloacae*) is a clinical isolate whilst the other bacterial species are laboratory strains of "the Deutsche Sammlung von Mikroorganismen" (DSM). This susceptibility test confirms not only that the combination of fluorination at the 5-C position (also referred to as the 5-position) and guanidine introduction (functionalized or not) at the 3"-C position surprisingly results in AG derivatives with excellent potency against all bacterial strains tested but that, entirely unexpectedly, the same synergistic effect as observed against the *E. coli* strains (see Table 1) is again seen. In this regard, attention is drawn, for example, to a comparison of the data against *Enterococcus faecalis, Klebsiella pneumoniae* and *Enterobacter cloacae* where the introduction of both the 5-fluoro and 3"-guanidiyl substituents is seen to give rise not merely to additivity (which in itself would nonetheless be unexpected based on the teaching of the known prior art) but to a synergistic effect in terms of activity for all compounds of the invention (ABX4006, ABX4004, ABX3003 and ABX5006) relative to that seen upon the incorporation of either of said substituents alone. This is all the more remarkable given the fact that, in many cases, the introduction of the 3"-guanidyl substituent alone is seen to lead to a loss of activity (see, for example, the activity data for ABX4009, ABX4005, and ABX3004 against *Enterococcus faecalis,* ABX4009 and ABX4005 against *Klebsiella pneumoniae,* ABX4005 and ABX3004 against *Enterobacter cloacae,* and ABX3004 against *Acinetobacter baumannii*)*.* Furthermore, derivatization of the 3"-guanidyl group (ABX5020, ABX5025, ABX5026, ABX5027, ABX5029, ABX5030, ABX5038, ABX5039, ABX5040, ABX5041, ABX5042, ABX5043, ABX5045, ABX5046, ABX5047, ABX5048, ABX5050 and ABX5051) is seen to give rise to compounds which demonstrate excellent levels of activity against all pathogens tested, in almost all cases demonstrating improved activity than their respective parent compound (ABX5004) and in all cases exhibiting improved activity in comparison to the clinical comparator Amikacin. In the case of *Klebsiella pneumoniae, Acinetobacter baumannii* and *Enterobacter cloacae,* all compounds of the invention show a level of potency which is at least equal to and in most cases far greater than that shown by each of the clinically relevant aminoglycosides Amikacin and Gentamicin. The result of this is not only compounds exhibiting an entirely surprising level of activity against *individual* ESKAPE panel pathogens and *Enterococcus faecalis* but also an entirely unexpected and substantially improved breadth of spectrum of activity (cross-panel activity) against the entire panel of pathogens tested relative to Amikacin and Gentamicin.

**Table 2. Antimicrobial susceptibility testing with selected pathogens of the bacterial ESKAPE panel and Enterococcus faecalis.**

| ¹MIC 50 (µmol/L) | Derivatization | *E. faecalis DSM 2570* | *K. pneumoniae DSM 26372* | *A. baumannii DSM 105126* | *²E. Cloacae* |
|---|---|---|---|---|---|
| **Amikacin** | | >64 | 0,5 | 0,5 | 2 |
| **Gentamicin** | | 16 | 8 | 0,25-0,5 | 8 |
| **ABX4001** | | 32-64 | 0,5 | 0,5 | 2 |
| **ABX4007** | **5C-F** | 32-64 | 0,5 | 0,5 | 1-2 |
| **ABX4009** | **3"C-Gua** | 64-128 | 1 | 0,5 | 2 |
| **ABX4006** | **5C-F, 3"C-Gua** | 16-32 | 0,125 | 0,125 | 0,25-0,5 |
| **ABX4002** | | 16-32 | 1 | 0,5 | 4 |
| **ABX4003** | **5C-F** | 64 | 0.5 | 0,5 | 4 |
| **ABX4005** | **3"C-Gua** | 32-64 | 1-2 | 0,5 | 4-8 |
| **ABX4004** | **5C-F, 3"C-Gua** | 16-32 | 0,25 | 0,125 | 1 |
| **ABX3002** | | 128 | 8 | 2 | 16 |
| **ABX3005** | **5C-F** | 64 | 1 | 1 | 4 |
| **ABX3004** | **3"C-Gua** | >256 | 8 | 2-4 | 32 |
| **ABX3003** | **5C-F, 3"C-Gua** | 64 | 0,5 | 0,0625-0,125 | 1-2 |
| **ABX5004** | | 32 | 1 | 0,5 | 1 |
| **ABX5024** | **5C-F** | 16-32 | 1 | 0,125 | 1 |
| **ABX5005** | **3"C-Gua** | 16 | 0,25 | 0,25 | 1 |
| **ABX5006** | **5C-F, 3"C-Gua** | 4-8 | <0,25 | 0,0625 | 0,25-0,5 |
| **ABX5020** | **5C-F, 3"C-Gua-R⁵** | 4-8 | 0,5 | 0,0625-0,125 | 0,5 |
| **ABX5025** | **5C-F, 3"C-Gua-R⁵** | 16 | 0,5 | 0,25 | 0,5-1 |
| **ABX5026** | **5C-F, 3"C-Gua-R⁵** | 32 | 0,125 | 0,03125-0,0625 | 0,25 |
| **ABXS027** | **5C-F, 3"C-Gua-R⁵** | 32 | 0,125 | 0,125 | 0,5 |
| **ABX5029** | **5C-F, 3"C-Gua-R⁵** | 64 | 0,125-0,25 | 0,0625 | 1 |
| **ABX5030** | **5C-F, 3"C-Gua-R⁵** | 32 | 0,125 | 0,03125-0,0625 | 1 |
| **ABX5038** | **5C-F, 3"C-Gua-R⁵** | 16 | 0,5 | 0,0625-0,125 | 0,5 |
| **ABX5039** | **5C-F, 3"C-Gua-R⁵** | 16 | 0,5 | 0,0625 | 0,25 |
| **ABX5040** | **5C-F, 3"C-Gua-R⁵** | 16 | 0,5 | 0,0625-0,125 | 0,25 |
| **ABX5042** | **5C-F, 3"C-Gua-R⁵** | 8 | 0,5 | 0,0625 | 0,25 |
| **ABXS043** | **5C-F, 3"C-Gua-R⁵** | 16 | 0,5 | 0,0625-0,125 | 0,25 |
| **ABX5041** | **5C-F, 3"C-Gua-R⁵** | 4-8 | 0,5 | 0,25 | 1 |
| **ABX5045** | **5C-F, 3"C-Gua-R⁵** | 8-16 | 0,5 | 0,5 | 1 |
| **ABX5046** | **5C-F, 3"C-Gua-R⁵** | 8-16 | 0,5 | 0,25 | 1 |
| **ABX5047** | **5C-F, 3"C-Gua-R⁵** | 2-4 | 0,25 | 0,125 | 0,25-0,5 |
| **ABX5048** | **5C-F, 3"C-Gua-R⁵** | 8 | 0,5 | 0,5 | 1 |
| **ABX5050** | **5C-F, 3"C-Gua-R⁵** | 8-16 | 0,5 | 0,5 | 1-2 |
| **ABX5051** | **5C-F, 3"C-Gua-R⁵** | 8 | 0,5 | 0,5 | 1 |

| | | | | | |
|---|---|---|---|---|---|
| *¹The method used for antimicrobial testing was broth micro-dilution method performed according to CLSI guidelines as outlined in the Materials and Methods section herein above; ²clinical isolate.* | | | | | |

In addition to the bacterial strains against which data is presented in Table 2 above, compounds of the invention (based on scaffolds ABX4002, ABX3002 and ABX5004) were further tested against laboratory ATCC strains of *Pseudomonas aeruginosa* (ATCC 29212) and *Staphylococcus aureus* (ATCC 29213) as well as against an ATCC strain of *Escherichia coli* (ATCC BAA-1025 = BL1). In contrast to the susceptibility tests discussed above (Tables 1 and 2), in this case MIC90-values were determined instead of MIC50-values (these values indicate the MIC at a level of inhibition of bacterial growth of 90% and 50%, respectively). The results are presented in Table 3.

All compounds of the invention (ABX4004, ABX3003, ABX5006, ABX5030, ABX5020, ABX5026) are seen to have overall superior antibacterial potency relative to their respective parent compounds (ABX4002, ABX3002, ABX5004). Moreover, the unexpected synergistic effect on activity upon introducing both the 5-fluoro and 3"-guanidyl substituents relative to either alone is again observed against these targets. Once again, this is seen to be all the more remarkable due to the fact that in the case of, for example, ABX3004, the introduction of the guanidine group at the 3"-C position alone results in decreased antibacterial activity. Nonetheless, when this substituent is introduced into the compound bearing the 5-fluoro group (ABX3005) resulting in derivative ABX3003, the negative effect on activity of the 3"-guanidyl group is entirely negated and, in the case of the ATCC strains of *Escherichia coli* and *Staphylococcus aureus* strains, is even seen to lead to an increase in activity. Accordingly, ABX3003 is seen to possess 4/8-fold increased activity against all three bacterial strains in comparison to the parent compound ABX3002 lacking both structural changes.

**Table 3. Antimicrobial susceptibility testing with selected pathogens of the bacterial ESKAPE panel and an Escherichia coli strain.**

| ¹MIC 90 (µmol/L) | Derivatization | *E. coli* ATCC BAA-1025 (=BL 21) | *P. aeruginosa* ATCC 27853 | *S*. *aureus* ATCC 29213 |
|---|---|---|---|---|
| **Amikacin** | | 2 | 1 | 4 |
| **Gentamicin** | | 0,5 | 2 | 0,5 |
| **ABX4002** | - | 8 | 4 | 2 |
| **ABX4003** | **5C-F** | 4 | 8 | 4 |
| **ABX4005** | **3"C-Gua** | 8 | 4 | 2 |
| **ABX4004** | **5C-F, 3"C-Gua** | 2 | 4 | 2 |
| **ABX3002** | - | 32 | 32 | 16 |
| **ABX3005** | **5C-F** | 8 | 8 | 4 |
| **ABX3004** | **3"C-Gua** | 64 | 64 | 16 |
| **ABX3003** | **5C-F, 3"C-Gua** | 4 | 8 | 2 |
| **ABX5004** | - | 2 | 4 | 1 |
| **ABX5024** | **5C-F** | 1 | 2 | 1 |
| **ABX5005** | **3"C-Gua** | 2 | 4 | 1 |
| **ABX5006** | **5C-F, 3"C-Gua** | 0,5 | 0,25 | 0,5 |
| **ABX5030** | **5C-F, 3"C-Gua-R⁵** | 1 | 0,5 | 0,5 |
| **ABX5020** | **5C-F, 3"C-Gua-R⁵** | 1 | 0,25 | 0,25 |
| **ABX5026** | **5C-F, 3"C-Gua-R⁵** | 1 | 1 | 0,5 |

| | | | | |
|---|---|---|---|---|
| *¹The method used for antimicrobial testing was broth micro-dilution method performed according to CLSI guidelines as outlined in the Materials and Methods section herein above.* | | | | |

Further remarkable based on the data presented in Tables 2 and 3 is the improvement in potency observed against *Enterococcus faecalis* and *Staphylococcus aureus.* These species are Gram-positive which is of particular importance as AG antibiotics are currently not used as a mono-therapy for infections caused by Gram-positive bacteria. One reason for this is the low antibacterial activity of AGs as shown for Amikacin (Tables 2 and 3). In contrast, the compounds of the invention exhibit a substantially increased level of activity against *Enterococcus faecalis* and *Staphylococcus aureus,* respectively, thus making them potential candidates for an aminoglycoside (AG) mono-therapy against these Gram-positive targets or for a potentially more effective combination therapy combined when used together with other regulatory approved antibiotics, in particular with (regulatory approved) betalactam antibiotics.

### Antibacterial Activity against selected wild-type bacterial strains and clinical isolates expressing multiple aminoglycoside-modifying enzymes

Compounds of invention ABX5006, ABX5026 and ABX5039 were tested against two wild-type bacterial strains of the ESKAPE panel, namely *Pseudomonas aeruginosa* (PAO1) and *Acinetobacter baumannii* (ATCC19606), as well as against a series of clinical isolates, namely *Acinetobacter baumannii* (195N(a) and 48F), *Escherichia coli* (C1162, and C1181), *Morganella morganii* (S49) and *Providencia stuartii* (B8-1), harboring various AMES. While the *Escherichia coli* isolate C1162 harbored a single AME, namely APH(3')Ia, the remaining isolates expressed multiple and different subclasses of AME enzymes, namely AACs, ANTs and APHs. Table 4 shows the MIC values for the compounds of invention against the said bacterial isolates in comparison to the aminoglycosides Amikacin, Apramycin and Gentamicin. Remarkably, all compounds of invention exhibit a substantially increased level of activity compared to all comparator aminoglycosides (Table 4). It must also be emphasized that all bacterial strains tested are susceptible to the compounds of invention. In contrast, five strains are resistant to Gentamicin (MIC>4) and three resistant to Amikacin according to *defined clinical breaking points by EUCAST (The European Committee on Antimicrobial Susceptibility Testing. Breakpoint tables for interpretation of MICs and zone diameters. Version 8.1, 2018.* *http:*//*www.eucast.org*)*.* Since, Apramycin is still in development for human use, the breaking points for this aminoglycoside have not yet been defined. However, the MIC values of apramycin are two- to sixteen-fold higher compared to those of the compounds of invention.

**Table 4. Antimicrobial susceptibility testing with selected wild-type pathogens and bacterial strains expressing multiple AMEs**

| **¹MIC 90 (ug/mL)** | **Strain Number** | **Amikacin** | **Apramycin** | **Gentamicin** | **ABX5006** | **ABX5026** | **ABX5039** | **AMEs** |
|---|---|---|---|---|---|---|---|---|
| *P. aeruginosa* | PA01 | 4 | 8 | 2 | 1 | 1 | 1 | WT |
| *A. baumanii* | ATCC 19606 | 16 | 32 | 64 | 8 | 8 | 8 | WT |
| *A. baumanil* | 195N (a) | >64 | 32 | >64 | 4 | 8 | 4 | AAC(3)Ia, ANT(3")Ia, APH(3")Ib, APH(3')VI, APH(6)Id |
| *A. baumanii* | 48F | >64 | 32 | >64 | 4 | 4 | 4 | ANT(2")Ia, APH(3")-Ib, APH(3')-VIa, APH(6)-Id |
| *E. coli* | C1162 | 4 | 8 | 2 | 1 | 1 | 1 | APH(3')-Ia |
| *E. coli* | C1181 | 4 | 8 | 2 | 1 | 0.5 | 0.5 | ANT(3")-Ia, APH(3')-Ia |
| *M. morgannii* | 549 | 4 | 8 | >64 | 1 | 2 | 1 | AAC(3)-IId, ANT(3"), APH(3")-Ib, APH(3')-Ia, APH(6)-Id |
| *P. stuartii* | B8-1 | >64 | 8 | >64 | 4 | 2 | 2 | AAC(3)-Ic, ANT(3")Ia, ANT(3"), APH(3')-IIa, APH(3')-IIb, APH(3')-VI, APH(6)-Ic |

| | | | | | | | | |
|---|---|---|---|---|---|---|---|---|
| *¹The method used for antimicrobial testing was broth micro-dilution method performed according to CLSI guidelines as outlined in the Materials and Methods section herein above.* | | | | | | | | |

### Antibacterial Activity against ESKAPE Panel Strains exhibiting Resistance

The provision of novel compounds capable of treating bacterial strains which exhibit resistance against clinically relevant antibiotics and, in particular, aminoglycoside antibiotics such as, *inter alia,* Amikacin would constitute a major contribution to the art (and thus constitutes an object underlying he present invention). With this in mind, a series of compounds of the invention was further evaluated to test their respective potencies against selected resistant bacterial strains of representative Gram-negative and Gram-positive pathogens of the ESKAPE panel (Table 5). In particular, employed in this susceptibility study were ATCC strains of *Staphylococcus aureus* (ATCC BAA-1717), *Klebsiella pneumoniae* (ATCC BAA-1705, ATCC BAA-2524), *Pseudomonas aeruginosa* (ATCC BAA-2108) and *Acinetobacter baumannii* (ATCC BAA-1800), which harbor genes for various clinically relevant resistance mechanisms against antibiotics belonging to different antibiotic classes.

As shown in Table 5, all compounds of invention show an overall increased potency against Gram-positive and Gram-negative pathogens compared to the clinical comparator Amikacin. All compounds of the invention are more potent against the Amikacin-resistant pathogens of *Klebsiella pneumoniae* (ATCC BAA-1705) and *Acinetobacter baumannii* (ATCC BAA-1800), and have up to 16-fold amplified antibacterial activity against the Methicillin-resistant *Staphylococcus aureus* (MRSA) strain ATCC BAA-1717 compared to Amikacin.

### Antibacterial Activity against Clinical isolates of Gram-negative pathogens Pseudomonas aeruginosa, Acinetobacter baumannii and Klebsiella pneumoniae and Gram-positive pathogens Enterococcus faecium and Staphylococcus aureus.

*Pseudomonas aeruginosa, Acinetobacter baumannii and Klebsiella pneumoniae* are prominent nosocomial Gram-negative bacterial strains in hospital settings responsible for the majority of hospital-acquired infections, such as urinary tract infections, nosocomial pneumonia, ventilator-associated pneumonia, blood stream infections, intra-abdominal infections, and skin and skin structure infections. Clinicians have very limited treatment options for patients infected by those pathogens.

*Pseudomonas aeruginosa* is the most frequently isolated pathogen in hospitals and has been found in ice machines, pharmacy preparations, plaster, mouthwash, nebulizers, whirlpools, mattresses, sinks, potted plants, and many other locations and materials. It is a versatile pathogen with the ability to cause diverse infection types. Predominately, this pathogen causes hospital-acquired pneumonia and neonates and infects wounds after surgery. Due to the intensive use of broad-spectrum antibiotics, *Pseudomonas aeruginosa* became the most prominent pathogen in hospitalized patients resistant to most available antibiotics. Toxic drugs, such as polymyxins, are often the last therapy option but are associated with significant adverse effects. Moreover, most *Pseudomonas aeruginosa* infections are endemic and sporadic. Heavily contaminated fluids or medical equipment and particularly virulent *Pseudomonas aeruginosa* strains cause epidemics.

*Acinetobacter baumannii* is responsible for hospital-acquired blood stream infections, pneumonia, soft-tissue infections, urinary tract infections, abdominal infections, meningitis, and endocarditis. This pathogen is prominent in patients in intensive care units and is one of the main causes of ventilator-associated bacterial pneumonia. Its ability to rapidly develop resistance against major antibiotic classes is the reason for the emergence of multidrug-resistant *Acinetobacter baumannii.* In fact, this pathogen has become one of the key Gram-negative bacterial strains which needs to be tackled in hospitals. While in the past carbapenems were still effective against multidrug-resistant infections cause by *Acinetobacter baumannii,* over the past decade carbapenem-resistant *Acinetobacter baumannii* (CRAB) species have emerged. Nowadays, CRAB infections can often be treated only with toxic antibiotics such as colistin and polymyxin B.

Due to outbreaks associated with multidrug-resistant strains, *Klebsiella pneumoniae* (KP) became one of the most prominent Gram-negative bacteria isolated from patients in intensive care units. In fact, *Klebsiella pneumoniae* is the most clinically important member of the *Klebsiella* genus of *Enterobacteriaceae* and is responsible for various hospital-acquired infections, such as nosocomial pneumonia, urinary tract infections, wound/burn infections and blood stream infections, particularly in patients with a weakened immune system. Moreover, this opportunistic pathogen has developed a resistance to third-generation cephalosporin antibacterials. This resistance is mediated by the expression of extended-spectrum beta-lactamases (ESBLs). In particular, infections caused by Ceftazidime-resistant *Klebsiella pneumoniae* has become a real challenge in hospital settings, since they are associated with adverse clinical outcomes. For instance, sepsis-related mortality is more than 30% higher for patients infected by those third-generation cephalosporin-resistant pathogens compared to cephalosporin-susceptible *Klebsiella pneumoniae* strains. While in 2006, 6-33% of clinical isolates in Europe were resistant to third-generation cephalosporins, in 2013, 14-55% of clinical isolates in Europe were resistant to third-generation cephalosporins (European Center for Disease Control and Prevention, Antimicrobial Surveillance Report, 2008 and 2013).

*Staphylococcus aureus* and *Enterococcus faecium* are prominent nosocomial Gram-positive bacterial strains responsible for various hospital-acquired infections, such as endocarditis, urinary tract infections, pulmonary infections, bacteremia, and skin and skin-structure infections. Clinicians have only very limited treatment options for patients infected by those pathogens, especially when infected by resistant isolates of **V**ancomycin-**r**esistant ***E**nterococcus faecium* (VRE) and **M**ethicillin-**r**esistant ***S**taphylococcus **a**ureus* (MRSA).

*Staphylococcus aureus* is a major bacterial human pathogen responsible for infections both in community-acquired as well as hospital-acquired settings. The multi-drug resistant strain MRSA is the leading cause of bacteremia, bone and joint infections, skin and soft tissue infections, endocarditis, gastroenteritis, meningitis, toxic shock syndrome, and urinary tract infections. MRSA infections are common in hospitalized patients with open wounds, invasive devices such as catheters, and weakened immune systems. Hospital-acquired MRSA infections result in longer hospital stays and higher economic costs. Moreover, characterized by the serial emergence of epidemic strains, MRSA is a dangerous clinical threat causing infections of persistently high morbidity and mortality. Undoubtedly, novel antimicrobials and adjunctive aspects of care, such as infectious disease consultation, echocardiography and source control, are urgently needed for as successful treatment of hospitalized patients infected by MRSA.

Since 1990, *Enterococcus faecium* has emerged as one of the major causes of multidrug-resistant enterococcal infection. Approximately 50% of pathogenic isolates of *Enterococcus faecium* are resistant to Vancomycin, Ampicillin, and aminoglycosides. Nowadays, treatment of infections caused by *Enterococcus faecium* pathogens is very challenging with the consequence of increased mortality rates. VRE and ampicillin-resistant *Enterococcus faecium* cause 80% and 90.4% of hospital-acquired infections, respectively, which are associated with the use of devices, such as ventilators and catheters. Due to its colonization strategy, persistence in the environment, and genome plasticity, VRE has become a major nosocomial pathogen worldwide. In immunosuppressed patients it is responsible for a wide range of infections, such as bacteremia, infective endocarditis, intra-abdominal and pelvic infections, urinary tract infections, central nervous system infections and skin and skin-structure infections. New agents, improved dosing regimens and combination therapies are urgently needed for the treatment of VRE infections in intensive care units.

In light of the above, the provision of novel compounds capable of treating infections caused by these bacterial strains (in particular strains of these bacteria which exhibit resistance) would constitute a further major contribution to the art and thus constitutes an object underlying the present invention. As such, the present studies were extended to test compounds of the invention against clinical isolates of *Pseudomonas aeruginosa, Acinetobacter baumannii, Klebsiella pneumoniae, Staphylococcus aureus* and *Enterococcus faecium.*

To this end, a panel of clinical isolates of *Pseudomonas aeruginosa* (PA), all originating from the University Hospital Zagreb in Croatia, was assembled and tested as outlined in the Materials and Methods section herein above for their susceptibility to a wide variety of antibiotic classes including aminoglycoside, carbapenems, third-generation cephalosporins, macrolides and quinolones. According to the clinical breaking points defined by EUCAST on May 16 in 2018 *(Breakpoint tables for interpretation of MICs and zone diameters, Version 8.1, 2018.* *http:*//*www.eucast.org*)*,* the clinical isolates tested are multidrug-resistant and not susceptible to most available antibiotics. The panel of the six clinical isolates includes five pathogens with intermediate resistance and one with a high level of resistance against aminoglycosides. The corresponding non-resistant ATCC27853 strain of *Pseudomonas aeruginosa* was used for quality control. The results in Table 6 demonstrate the superior performance of the compounds of the invention (ABX5006, ABX5020, ABX5026 and ABX5039) in comparison to other aminoglycoside antibiotics (Amikacin and Gentamicin) as well as representatives of other classes of antibiotics, i.e. third-generation cephalosporins (Ceftazidime) and carbapenems (Meropenem).

Furthermore, a panel of clinical isolates of *Acinetobacter baumannii* (AB), which also originate from the University Hospital Zagreb in Croatia, was also assembled and tested as outlined in the Materials and Methods section herein above for their susceptibility to a wide variety of antibiotic classes including aminoglycoside, carbapenems, third-generation cephalosporins, macrolides and quinolones. According to the defined clinical breaking points defined by EUCAST *(Breakpoint tables for interpretation of MICs and zone diameters. Version 8.1, 2018.* *http:*//*www.eucast.org;* dated May 16 in 2018), the selected clinical isolates are multidrug-resistant and not susceptible to most available antibiotics. All tested isolates are CRAB strains, and are hence resistant to carbapenems, drugs of last resort. The panel of the five clinical isolates includes one strain with intermediate resistance and four with high level of resistance against aminoglycosides. The corresponding non-resistant ATCC17978 strain of *Acinetobacter baumannii* was used for quality control. The MIC values in Table 7 demonstrate that the compounds of the invention (ABX5006, ABX5020, ABX5026 and ABX5039) possess by far the highest potency and are effective against the whole pathogen panel, including isolates resistant to the comparator compounds.

Further, a panel of clinical isolates of *Klebsiella pneumonia* (KP), all originating from the University Hospital Zagreb in Croatia, was assembled and tested as outlined in the Materials and Methods section herein above for their susceptibility to a wide variety of antibiotic classes including aminoglycoside, carbapenems, third-generation cephalosporins, macrolides and quinolones. The pathogens were isolated from hospitalized patients suffering from urinary tract infections, nosocomial pneumonia, blood stream infections, and intra-abdominal infections. According to the defined clinical breaking points defined by EUCAST *(Breakpoint tables for interpretation of MICs and zone diameters. Version 8.1, 2018.* *http:*//*www.eucast.org;* dated May 16 in 2018), the clinical isolates tested are multidrug-resistant and not susceptible to most available antibiotics. Those bacterial isolates express OXA-, NDM-, or VIM-type carbapenemases and extended-spectrum beta-lactamases (ESBL). In addition, the selected panel of seven clinical isolates includes six strains with intermediate resistance and one with high level of resistance against aminoglycosides. The corresponding non-resistant ATCC43816 strain of *Klebsiella pneumoniae* was used for quality control. The results in Table 8 show the antibacterial activity profile of selected compounds of the invention (ABX5006, ABX5020, ABX5026, ABX5039 and ABX4006). Remarkably, all compounds of the invention show not only an improved activity profile relative to Amikacin, but are also potent against the multidrug-resistant *Klebsiella pneumoniae* strain, which is resistant to the last-resort aminoglycoside Amikacin.

Furthermore, a panel of clinical isolates of Methicillin-resistant *Staphylococcus aureus* (MRSA), all originating from the University Hospital Zagreb in Croatia, was assembled and tested as outlined in the Materials and Methods section herein above for their susceptibility to a wide variety of antibiotic classes including aminoglycosides, carbapenems, and third-generation cephalosporins. According to the clinical breaking points defined by EUCAST on May 16 in 2018 *(Breakpoint tables for interpretation of MICs and zone diameters, Version 8.1, 2018.* *http:*//*www.eucast.org*)*,* the clinical isolates tested are multidrug-resistant and not susceptible to most carbapenems and third-generation cephalosporins. The results in Table 9 demonstrate the superior performance of all three compounds of the invention ABX5006, ABX5026 and ABX5039 in comparison to other aminoglycoside antibiotics (Amikacin and Gentamicin) as well as representatives of other classes of antibiotics, namely third-generation cephalosporins (Ceftazidime) and carbapenems (Meropenem).

In a further study, a panel of clinical isolates of Vancomycin-resistant *Enterococcus faecium* (VRE), all originating from the University Hospital Zagreb in Croatia, was assembled and tested as outlined in the Materials and Methods section herein above for their susceptibility to a wide variety of antibiotic classes including aminoglycosides, carbapenems, and third-generation cephalosporins. According to the clinical breaking points defined by EUCAST on May 16 in 2018 *(Breakpoint tables for interpretation of MICs and zone diameters, Version 8.1, 2018.* *http*://*www.eucast.org*)*,* the clinical isolates tested are multidrug-resistant and not susceptible to most aminoglycosides, carbapenems and third-generation cephalosporins. Of particular note (as seen in Table 10) is that the entire panel of the nine clinical isolates has high level of resistance against aminoglycosides. The results in Table 10 demonstrate the superior performance of all three compounds of the invention ABX5006, ABX5026 and ABX5039 in comparison to other aminoglycoside antibiotics (Amikacin and Gentamicin) as well as representatives of other classes of antibiotics, namely third-generation cephalosporins (Ceftazidime) and carbapenems (Meropenem).

**Table 5. Antibacterial activities of compounds of the invention and Amikacin against selected resistant bacterial strains of the ESKAPE panel.**

| **¹MIC 90 (ug/mL)** | **Strain** | **Resistance** | **ABX 5006** | **ABX 5020** | **ABX 5026** | **ABX 5029** | **ABX 5038** | **ABX 5039** | **ABX 5040** | **ABX 5042** | **ABX 4006** | **Amikacin** |
|---|---|---|---|---|---|---|---|---|---|---|---|---|
| *S. aureus* | *ATCC BAA-1717* | MRSA | 0,5 | 0,25 | 0,5 | 0,5 | 0,5 | 0,5 | 0,5 | 0,5 | 4 | 4 |
| *K. pneumoniae* | *ATCC BAA-1705* | KPC | 2 | 8 | 4 | 4 | 8 | 4 | 8 | 4 | 2 | 32* |
| *K. pneumoniae* | *ATCC BAA-2524* | OXA-48 | <0,125 | 0,5 | 0,5 | 0,25 | 0,5 | 0,5 | 0,5 | 0,5 | <0,125 | 0,5 |
| *P*. *aeruginosa* | ATCC BAA-2108 | MDR | 4 | 2 | 8 | 4 | 4 | 8 | 8 | 8 | 16 | 16 |
| *A. baumannii* | ATCC BAA-1800 | MDR | 16 | 32 | 32 | 16 | 32 | 16 | 32 | 32 | 16 | 64* |

| | | | | | | | | | | | | |
|---|---|---|---|---|---|---|---|---|---|---|---|---|
| *¹The method used for antimicrobial testing was broth micro-dilution method performed according to CLSI guidelines as outlined in the Materials and Methods section herein above; *Resistant to amikacin according to defined clinical breaking points by EUCAST (The European Committee on Antimicrobial Susceptibility Testing. Breakpoint tables for interpretation* of *MICs and zone diameters. Version 8.1*, *2018.* *http:*//*www.eucast.org.); OXA-48 is a carbapenemase; KPC means Klebsiella pneumoniae carbapenemase; MDR means multidrug-resistant; MRSA mean methicillin-resistant Staphylococcus aureus.* | | | | | | | | | | | | |

**Table 6. Antibacterial activities of compounds of the invention and clinical comparators against clinical isolates of multidrug-resistant Pseudomonas aeruginosa.**

| **¹MIC 90 (ug/mL)** | **Origin/ Resistance** | **ABX5006** | **ABX5020** | **ABX5026** | **ABX5039** | **Amikacin** | **Gentamicin** | **Ceftazidime** | **Meropenem** |
|---|---|---|---|---|---|---|---|---|---|
| ***²P. aeruginosa*** | | 0,25 | 0,25 | 1 | 0.5 | 1 | 2 | 1 | 1 |
| *PA-1948* | Wound/OXA-2 | 0,5 | 1 | 1 | 1 | 2 | 64* | 32* | 64* |
| *PA-1949* | Urine/OXA-2 | 8 | 8 | 16 | 8 | 16 | 16* | >64* | 32* |
| *PA-1950* | Sputum/OXA-2 | 1 | 1 | 1 | 1 | 2 | 16* | 64* | >64* |
| *PA-1952* | Wound/OXA-2 | 4 | 8 | 8 | 4 | 16 | >64* | >64* | >64* |
| *PA-1953* | Urine/MDR | 8 | 16 | 32 | 32 | 32* | >64* | >64* | >64* |
| *PA-1954* | Sputum/OXA-2 | 0,5 | 0,5 | 1 | 1 | 1 | 16* | 8 | 64* |
| *PA-1967* | Blood/VIM | 4 | n.a. | 8 | 4 | 32 | >64 | 64 | 32 |

| | | | | | | | | | |
|---|---|---|---|---|---|---|---|---|---|
| *¹The method used for antimicrobial testing was broth micro-dilution method performed according to CLSI guidelines as outlined in the Materials and Methods section herein above; ²ATCC27853 strain; *Resistant to comparators according to defined clinical breaking points by EUCAST (The European Committee on Antimicrobial Susceptibility Testing. Breakpoint tables for interpretation of MICs and zone diameters. Version 8.1, 2018.* *http:*//*www.eucast.org : for Amikacin (MIC >16, Gentamicin (MIC>4), Ceftazidime (MIC>8) and Meropenem (MIC>8)); OXA-2 is a carbapenemase; MDR means multidrug-resistant.* | | | | | | | | | |

**Table 7. Antibacterial activities of compounds of the invention and clinical comparators against clinical isolates of multidrug-resistant Acinetobacter baumannii.**

| **¹MIC 90 (ug/mL)** | **Origin/ Resistance** | **ABX5006** | **ABX5020** | **ABX5026** | **ABX5039** | **Amikacin** | **Gentamicin** | **Ceftazidime** | **Meropenem** |
|---|---|---|---|---|---|---|---|---|---|
| ***²A. baumannii*** | | <0,125 | 0,5 | 0,5 | 0,5 | 1 | 0,25 | 4 | 1 |
| *AB-1931* | Urine/CRAB | 0,5 | 1 | 1 | 1 | >64* | >64* | >64 | >64* |
| *AB-1932* | Urine (catheter)/CRAB | 1 | 1 | 1 | 1 | >64* | >64* | 64 | >64* |
| *AB-2017* | Bronchial aspirate/CRAB | 0,5 | 1 | 1 | 1 | >64* | >64* | >64 | >64* |
| *AB-2018* | Skin/CRAB | 1 | 2 | 1 | 1 | 4 | >64* | >64 | >64* |
| *AB-2019* | Bronchial aspirate/CRAB | 1 | 2 | 1 | 1 | >64* | >64* | >64 | >64* |
| *AB-1964* | Blood | 1 | n.a. | 2 | 1 | 4 | >64 | >64 | >64 |
| *AB-2025* | Skin | 0,5 | n.a. | 1 | 1 | 2 | 4 | >64 | >64 |
| *AB-2026* | Skin | 1 | n.a. | 2 | 2 | 4 | 8 | >64 | 64 |

| | | | | | | | | | |
|---|---|---|---|---|---|---|---|---|---|
| *¹The method used for antimicrobial testing was broth micro-dilution method performed according to CLSI guidelines* as *outlined in the Materials and Methods section herein above; ²ATCC17978 strain; *Resistant to comparators according to defined clinical breaking points by EUCAST (The European Committee on Antimicrobial Susceptibility Testing. Breakpoint tables for interpretation of MICs and zone diameters. Version 8.1, 2018.* *http:*//*www.eucast.org : for Amikacin (MIC >16, gentamicin (MIC>4), and Meropenem (MIC>8)); CRAB means carbapenem-resistant Acinetobacter baumannii.* | | | | | | | | | |

**Table 8, Antibacterial activities of compounds of the invention and clinical comparators against clinical isolates of multidrug-resistant Klebsiella pneumoniae.**

| **¹MIC 90 (ug/mL)** | **Origin/Resistance** | **ABX5006** | **ABX5020** | **ABX5026** | **ABX5039** | **ABX4006** | **Amikacin** | **Gentamicin** | **Tobramycin** | **Ceftazidime** | **Meropenem** |
|---|---|---|---|---|---|---|---|---|---|---|---|
| ***²K. pneumoniae*** | | 0,25 | 1 | 1 | 1 | 0,25 | 0,5 | 0,25 | 0,125 | <0,125 | <0,125 |
| *KP-1919* | Abdomen/VIM, ESBL | 0,5 | 4 | 1 | 1 | 1 | 8 | >64* | 32* | 64* | 4 |
| *KP-1935* | Urine/ESBL, NDM | 0,5 | 4 | 1 | 2 | 0,5 | 8 | >64* | 32* | >64* | 32* |
| *KP-1936* | Abscess/NDM | 0,5 | 2 | 1 | 1 | 0,5 | 4 | 8* | 16* | >64* | >64* |
| *KP-1937* | Blood/VIM, ESBL | 2 | 8 | 2 | 2 | 4 | 32* | 32* | >64* | >64* | 32* |
| *KP-1942* | Urine (catheter)/VIM | 0,25 | 1 | 1 | 1 | 0,25 | 2 | >64* | 16* | >64* | 64* |
| *KP-1944* | Blood/NDM | 1 | 4 | 2 | 2 | 1 | 8 | 64* | 32* | >64* | >64* |
| *KP-2027* | Tracheal aspirate/ESBL, OXA-48, ColR | 0,5 | 2 | 1 | 1 | 0,5 | 4 | >64* | 16* | >64* | 4 |
| *KP-2029* | Urine/ESBL, OXA-48 | 0,25 | n.a. | 0,5 | 0,5 | n.a. | 8 | >64 | 32 | >64 | 4 |
| *KP-2030* | Trachea/ESBL, OXA-48 | 0,5 | n.a. | 1 | 1 | n.a. | 16 | >64 | 64 | >64 | 4 |
| *KP-2031* | Skin/ESBL, OXA-48 | 0,5 | n.a. | 0,5 | 0,5 | n.a. | 8 | >64 | 32 | >64 | 2 |
| *KP-2032* | Urine/ESBL, OXA-48 | 0,25 | n.a. | 0,5 | 0,5 | n.a. | 4 | 64 | 16 | >64 | 2 |
| *KP-2033* | Urine/OXA-48 | 0,25 | n.a. | 0,5 | 0,5 | n.a. | 2 | 64 | >64 | >64 | 16* |
| *KP-2034* | Urine/ESBL, OXA-48 | 0,5 | n.a. | 1 | 1 | n.a. | 16 | >64 | 32 | >64 | 16* |
| *KP-2035* | Urine/ESBL, OXA-48 | 0,5 | n.a. | 0,5 | 1 | n.a. | 8 | >64 | 64 | >64 | 2 |
| *KP-2036* | Urine/ESBL | 0,25 | n.a. | 0,5 | 1 | n.a. | 8 | 0,5 | 16 | >64 | 16* |
| *KP-2037* | Urine/ESBL, OXA-48 | 0,5 | n.a. | 0,5 | 0,5 | n.a. | 4 | >64 | 16 | >64 | 2 |

| | | | | | | | | | | | |
|---|---|---|---|---|---|---|---|---|---|---|---|
| *¹The method used for antimicrobial testing was broth micro-dilution method performed according to CLSI guidelines as outlined in the Materials and Methods section herein above; ²ATCC43816 strain; Resistant to comparators according to defined clinical breaking points by EUCAST (The European Committee on Antimicrobial Susceptibility Testing. Breakpoint tables for interpretation of MICs and zone diameters. Version 8. 1, 2018.* *http:*//*www.eucast.org : for Amikacin (MIC >16), Gentamicin (MIC>4), Ceftazidime (MIC>4) and Meropenem (MIC>8)); VIM, NDM, OXA-48 are carbapenemases, ESBL means extended-spectrum beta-lactamases; CoIR means colistin resistant.* | | | | | | | | | | | |

**Table 9. Antibacterial activities of compounds of invention and clinical comparators against clinical isolates of methicillin-resistant Staphylococcus aureus (MRSA).**

| **¹MIC 90 (ug/mL)** | **Origin/ Resistance** | **ABX5006** | **ABX5026** | **ABX5039** | **Amikacin** | **Gentamicin** | **Ceftazidime** | **Meropenem** |
|---|---|---|---|---|---|---|---|---|
| *MRSA-1995* | Liquor | 0,5 | 0,5 | 0,5 | 4 | 1 | >64* | 64* |
| *MRSA-1998* | Wound | 0,25 | 0,25 | 0,25 | 4 | 0,5 | >64* | 8 |
| *MRSA-1999* | Skin | 0,5 | 0,5 | 0,25 | 8 | 1 | >64* | 64* |
| *MRSA-2003* | Wound | 0,5 | 0,5 | 0,5 | 4 | 1 | >64* | 8 |

| | | | | | | | | |
|---|---|---|---|---|---|---|---|---|
| *¹The method used for antimicrobial testing was broth micro-dilution method performed according to CLSI guidelines as outlined in the Materials and Methods section herein above; Resistant to comparators according to defined clinical breaking points by EUCAST* *(The European Committee on Antimicrobial Susceptibility Testing. Breakpoint tables for interpretation of MICs and zone diameters. Version 8. 1, 2018.* *http:*//*www.eucast.org.* | | | | | | | | |

**Table 10. Antibacterial activities of compounds of the invention and clinical comparators against clinical isolates of Vancomycin-resistant Enterococcus faecium (VRE).**

| **¹MIC 90 (ug/mL)** | **Origin/ Resistance** | **ABX5006** | **ABX5026** | **ABX5039** | **Amikacin** | **Gentamicin** | **Ceftazidime** | **Meropenem** |
|---|---|---|---|---|---|---|---|---|
| *VRE-2005* | Urine | 4 | 4 | 4 | >64* | >64* | >64* | >64* |
| *VRE-2006* | Urine | 32 | 8 | 16 | >64* | >64* | >64* | >64* |
| *VRE-2007* | Urine | 4 | 4 | 4 | >64* | 8* | >64* | >64* |
| *VRE-2008* | Urine | 8 | 4 | 4 | >64* | >64* | >64* | >64* |
| *VRE-2009* | Urine | 16 | 8 | 4 | >64* | >64* | >64* | >64* |
| *VRE-2010* | Urine | 8 | 8 | 8 | >64* | >64* | >64* | >64* |
| *VRE-2011* | Rectum | 16 | 8 | 8 | >64* | >64* | >64* | >64* |
| *VRE-2012* | Rectum | 4 | 4 | 4 | >64* | >64* | >64* | >64* |
| *VRE-2013* | Rectum | 4 | 4 | 4 | >64* | >64* | >64* | >64* |

| | | | | | | | | |
|---|---|---|---|---|---|---|---|---|
| *¹The method used for antimicrobial testing was broth micro-dilution method performed according to CLSI guidelines as outlined in the Materials and Methods section herein above; Resistant to comparators according to defined clinical breaking points by EUCAST* *(The European Committee on Antimicrobial Susceptibility Testing. Breakpoint tables for interpretation of MICs and zone diameters. Version 8. 1, 2018.* *http:*//*www.eucast.org.* | | | | | | | | |

In order to demonstrate the potential of the compounds of the invention for clinical application, ABX5006 was further tested against clinical isolates originating from the University Medical Center Groningen (UMCG) in the Netherlands. Eight AG-resistant strains of *Escherichia coli* (MIC>breaking point for amikacin, tobramycin and gentamicin as defined by EUCAST (*Breakpoint tables for interpretation of MICs and zone diameters. Version 8.1, 2018.* *http:*//*www.eucast.org;* dated May 16 in 2018)), a pandrug-resistant *Pseudomonas aeruginosa* strain, six aminoglycoside-susceptible *Pseudomonas aeruginosa* strains, nine pandrug-resistant *Klebsiella pneumoniae* carbapenemase (KPC)-producing bacterial isolates, and four aminoglycoside-susceptible *Acinetobacter baumannii* strains were selected and tested as outlined herein above in the Materials and Methods section. Pandrug-resistant bacteria are a group of emerging highly drug-resistant Gram-negative bacilli that cause infections associated with significant morbidity and mortality. Selected pandrug-resistant pathogens were isolated from patients with infections resistant or non-susceptible to all available antibiotics. The corresponding non-resistant ATCC strains of the corresponding bacterial species were used for quality control in this experiment. The MIC values shown for ABX5006 in Table 11 demonstrate that, remarkably, all isolates including the highly aminoglycoside-resistant and pandrug-resistant bacteria tested are susceptible to the compound of invention.

**Table 11. Antibacterial activities of ABX5006 and ABX5020 against multidrug-resistant clinical isolates.**

| **¹MIC 90 (ug/mL)** | **Resistance** | **Origin** | **ABX5006** |
|---|---|---|---|
| *²E. coli* | | | 0,5 |
| *EC-11* | Multidrug-resistant | Throat | 0,5 |
| *EC-12* | Multidrug-resistant | Rectum | 2 |
| *EC-22* | Multidrug-resistant | Sputum | 4 |
| *CC-25* | Multidrug-resistant | Bile liquid | 4 |
| *EC-26* | Multidrug-resistant | Bile liquid | 4 |
| *EC-40* | Multidrug-resistant | Blood culture | 2 |
| *EC-43* | Multidrug-resistant | Rectum | 4 |
| *EC-46* | susceptible | Blood culture | 2 |
| ***³P. aeruginosa*** | | | 0,25 |
| *PA-171* | Pandrug-resistant | BAL | 8 |
| PA-179 | Multidrug-resistant | Wound | 2-4 |
| PA-195 | Multidrug-resistant | Catheter | 0,5-1 |
| PA-196 | Multidrug-resistant | unkown | 0,5-1 |
| PA-198 | Multidrug-resistant | unkown | 1-2 |
| PA-201 | Multidrug-resistant | unkown | 0,5-1 |
| PA-202 | Multidrug-resistant | Faeces | 0,25-0,5 |
| ***⁴K. pneumoniae*** | | | 0,25 |
| *KP-106* | KPC/Pandrug-resistant | unknown | 8 |
| *KP-107* | KPC/Pandrug-resistant | Urine | 8 |
| *KP-108* | KPC/Pandrug-resistant | Urine | 8 |
| *KP-109* | KPC/Pandrug-resistant | Catheter tip | 16 |
| *KP-110* | KPC/Pandrug-resistant | *Sputum* | 8 |
| *KP-112* | KPC/Pandrug-resistant | Urine | 8 |
| *KP-112* | KPC/Pandrug-resistant | unkown | 16 |
| *KP-113* | KPC/Pandrug-resistant | unkown | 4 |
| *KP-114* | KPC/Pandrug-resistant | unkown | 8 |
| ***⁵A. baumannii*** | | | <0,125 |
| AP-2 | Multidrug-resistant | Sputum | 0,5-1 |
| AB-10 | Multidrug-resistant | Blood culture | 0,5-1 |
| AB-14 | Multidrug-resistant | Blood culture | 0,5-1 |
| AB-18 | Multidrug-resistant | Bone | 2-4 |

| | | | |
|---|---|---|---|
| *¹The method used for antimicrobial testing was broth micro-dilution method performed according to CLSI guidelines as outlined in the Materials and* *Methods section herein above; ²ATCC25922, ³ATCC27853 strain;⁴ATCC43816 strain; ⁵ATCC17978 strain.* | | | |

### In Vivo Efficacy Testing

Compounds of the invention ABX5006, ABX5020, ABX5026 and ABX5039 were further tested *in vivo.* The *in vivo* potency of the compounds was evaluated using the neutropenic mouse thigh model of infection as outlined above in section the Materials and Methods section. Pathogen-free male CD-1 (outbred) mice (6 animals per group) and the non-resistant ATCC strain of *Klebsiella pneumoniae* (ATCC 43816) were employed. The MIC 90 (µg/ml) value for ABX5006 against this ATCC strain is 0.25 and the MIC 90 (µg/ml) value for each of ABX5020, ABX5026 and ABX5039 is 1. The *in vivo* efficacy study was performed using meropenem (a broad-spectrum antibiotic) as a reference compound (for quality control) and each drug was delivered subcutaneously (SC) twise a day (BID) at one and nine hours post infection.

During this study one dose of meropenem, i.e. 100 mg/kg, and three different doses per novel aminoglycoside were tested. Compounds ABX5006 and ABX5020 were tested first using doses of 4, 16 or 64 mg/kg (Table 12). Both thighs were infected resulting in a total of 12 samples for the determination of the colony-forming units (CFUs) per gram of thigh. However, the control group (vehicle) had a 50% mortality rate resulting in only 6 samples for the read out. The survival rate for the group treated with meropenem and both compounds of the invention was 100%. All mice were overdosed with ketamine+xylazine 24 hours post infection. Each thigh was homogenized in sterile PBS using Ultraturax, IKA. After homogenizing, a serial dilution of the homogenates was used for CFU determination. A CFU count of each thigh will act as a single result.

The results of the *in vivo* efficacy study are summarized in Table 12. ABX5006 and ABX5020 show excellent *in vivo* activity, already reaching a significant CFU count reduction of a 7-log10 and >7-log10 in comparison to the vehicle control group, respectively at the lowest total dose of 4 mg/kg. Both compounds reach a CFU count reduction of >7-log10 in comparison to the vehicle control group at the highest dose tested, i.e. 64 mg/kg. In fact, multiple samples of the group treated with ABX5006 and ABX5020 resulted in CFU counts below the limit of detection (LOD = 1,0×10² CFU/mL), demonstrating high *in vivo* activity of both compounds. Also surprising is that compound ABX5020 has similar *in vivo* activity compared to ABX5006, when the same dose of each is applied. As reported above, ABX5020 has a 4-fold lower antibacterial activity *in vitro* compared to ABX5006 against the *Klebsiella pneumoniae* strain, which was used also in the *in vivo* study (Table 12). Without being bound to any theory, it would appear that the R⁵ residue on the 3"-Guanidyl group in ABX5020 has an influence on the *in vivo* performance compensating the reduced in vitro activity compared to ABX5006.

**Table 12. Results of in vivo neutropenic mouse thigh model of infection with Klesiella pneumoniae (ATCC43816).**

| **Group** | **drug** | **Dose (mg/kg)** | **Avg. Log CFU/g thigh** | **St. Dev.** | **Change from vehicle group** |
|---|---|---|---|---|---|
| **1** | - | | 9,51 | 0,22 | |
| **2** | meropenem | 100 | 4,95 | 1,03 | -4,56 |
| **3** | ABX5006 | 4 | 2,47 | 0,18 | -7,05 |
| **4** | ABX5006 | 16 | 1,15 | 1,20 | -8,36 |
| **5** | ABX5006 | 64 | 0,38 | 0,90 | -9,13 |
| **6** | ABX5020 | 4 | 1,47 | 1,30 | -8,04 |
| **7** | ABX5020 | 16 | 1,37 | 1,21 | -8,14 |
| **8** | ABX5020 | 64 | 0,19 | 0,66 | -9.32 |

Due to the high *in vivo* efficacy of ABX5006 and ABX5020, in the subsequent study with compounds ABX5026 and AXB5039 4-fold lower doses were administered, i.e. 0.25, 1 or 4 mg/kg (Table 13). The exact same protocol was used for this study as described above for ABX5006 and ABX5020. The results of the *in vivo* efficacy study are summarized in Table 13. Remarkably, ABX5026 and ABX5039 show excellent *in vivo* activity, reaching a significant CFU count reduction of a >6-log10 and >4-log10 in comparison to the vehicle control group, respectively already at a dose of 1 mg/kg. Both compounds reach a CFU count reduction of >7-log10 in comparison to the vehicle control group at the highest dose tested, i.e. 4 mg/kg.

**Table 13. Results of in vivo neutropenic mouse thigh model of infection with Klesiella pneumoniae (ATCC43816).**

| **Group** | **drug** | **Dose (mg/kg)** | **Avg. Log CFU/g** | **St. Dev.** | **Change from vehicle group** |
|---|---|---|---|---|---|
| **1** | - | | 9,78 | 0,40 | - |
| **2** | meropenem | 100 | 1,61 | 2,02 | 8,17 |
| **3** | ABX5026 | 0,25 | 7,98 | 1,61 | 1,80 |
| **4** | ABX5026 | 1 | 3,34 | 1,15 | 6,44 |
| **5** | ABX5026 | 4 | 2,22 | 1,35 | 7,56 |
| **6** | ABX5039 | 0,25 | 9,08 | 0,5 | 0,70 |
| **7** | ABX5039 | 1 | 5,07 | 1,06 | 4,71 |
| **8** | ABX5039 | 4 | 2,23 | 1,36 | 7,55 |

Compounds of the invention ABX5006 and ABX020 were further tested against the resistant *Klebsiella pneumoniae* strain ATCC BAA-1705. This is a KPC-carrying *Klebsiella pneumoniae* strain which is also resistant or non-susceptible to the aminoglycosides Tobramycin and Amikacin. MIC values against this strain are shown in Table 5. The detailed experimental procedure is described in the experimental and method section above. Pathogen-free male CD-1 (outbred) mice (6 animals per group) and amikacin as a reference compound were used in this study. Amikacin (a clinical comparator, which was also used for quality control) and each compound of the invention were delivered subcutaneously (SC) twice a day (BID) at one and nine hours post infection. Two different doses (16 or 64 mg/kg) for each compound (Table 10) was investigated. Both thighs were infected resulting in total in 12 samples for the determination of the CFUs per gram of thigh. Two animals of the group treated with ABX5020 were euthanized prior to second dose due to weak clinical condition resulting in eight samples for the CFU determination. The survival rate for all other animal groups was 100%. All mice were overdosed with ketamine+xylazine 24 hours post infection. Each thigh was homogenized in sterile PBS using Ultraturax, IKA. After homogenizing, a serial dilution of the homogenates was used for CFU determination. A CFU count of each thigh will act as a single result.

The results of the *in vivo* efficacy study are summarized in Table 14. Again, ABX5006 and ABX5020 show excellent *in vivo* activity against the resistant infection compared to Amikacin. Treatment with ABX5006 at doses of 16 and 64 mg/kg (SC, BID) resulted in a statistically significant decrease in CFU number in thighs (~2,8-3,2 log10 reduction) in comparison to the vehicle control group. Furthermore, treatment with ABX5020 at doses of 16 and 64 mg/kg (SC, BID) resulted in a statistically significant decrease in CFU number in thighs (~2,7-3,6 log10 reduction) in comparison to the vehicle control group. In contrast, Amikacin treatment did not significantly decrease CFU counts in thighs when compared to the vehicle control at either dose applied.

The same effect as that seen above in the case of *Klebsiella pnuemoniae* was also observed in this study when comparing the *in vitro* and *in vivo* activity of each compound of the invention. Again, when the same doses are applied, compound ABX5020 surprisingly shows similar *in vivo* activity to ABX5006. As shown in Table 5, ABX5020 has a 4-fold lower antibacterial activity *in vitro* compared to ABX5006 against the *Klebsiella pnuemoniae* strain tested in this *in vivo* study (Table 14). Without being bound to any theory, it would appear that the R⁵ residue on the 3"-Guanidyl group in ABX5020 has an influence on the *in vivo* performance compensating the reduced in vitro activity compared to ABX5006.

**Table 14. Results of in vivo neutropenic mouse thigh model of infection with Amikacin-resistant Klebsiella pneumoniae (ATCC BAA-1705).**

| **Group** | **drug** | **Dose (mg/kg)** | **Avg. Log CFU/g thigh** | **St. Dev.** | **Change from vehicle group** |
|---|---|---|---|---|---|
| **1** | - | | 8,89 | 0,26 | |
| **2** | Amikacin | 16 | 8,19 | 1,01 | 0,70 |
| **3** | Amikacin | 64 | 7,24 | 0,63 | 1,65 |
| **4** | ABX5006 | 16 | 6,08 | 0,49 | 2,81 |
| **5** | ABX5006 | 64 | 5,69 | 0,35 | 3,20 |
| **6** | ABX5020 | 16 | 6,18 | 0,29 | 2,71 |
| **7** | ABX5020 | 64 | 5,29 | 0,35 | 3,60 |

To further evaluate the in vivo efficacy of the compounds of the invention, ABX5006 and ABX5026 were also tested *in vivo* employing the murine model for urinary tract of infection. Urinary tract infections (UTIs) are among the most common bacterial infections in humans. Annually, approx. 7 million hospital-treated UTI infections worldwide are caused by Gram-negative pathogens alone. Therefore, a model for *Escherichia coli* infection has been used to evaluate the efficacy of the compounds of the invention in comparison to Gentamicin (a broad-spectrum antibiotic). Gentamicin is, of all aminoglycosides, the most frequently used aminoglycoside against Gram-negative hospital-treated infections. The detailed protocol for this study is outlined above in the Materials and Methods section. Pathogen-free female C3H/HeNRj (inbred) mice (8 animals per group) and the non-resistant ATCC strain of *Escherichia coli* (ATCC 700336) were employed. The MIC (µg/ml) values for ABX5006 and ABX5026 against this ATCC strain are 0,25 and 1, respectively. The MIC (µg/ml) value of Gentamicin against the same *Escherichia coli* is 1.

Comparator Gentamicin and each of the compounds of the invention ABX5006 and ABX5020 were tested at doses of 15 and 30 mg/kg (Table 15). Each drug was delivered subcutaneously (SC) once daily (QD) 24 hours post infection. Each mouse was infected by transurethral administration of 100 µL/mouse of bacterial suspension under light ketamine+xylazine anaesthesia. Mice were water deprived 90 minutes prior to infection and one hour post infection. Colony-forming units (CFUs) were determined in kidneys 24 hours post treatment. The survival rate in the vehicle group and treated groups was 100%. All mice were overdosed with ketamine+xylazine 24 hours post infection. Kidneys were homogenized in sterile PBS using Ultraturax, IKA. After homogenizing, a serial dilution of the homogenates was used for CFU determination. A CFU count of each kidney pair will act as a single result.

The results of the *in vivo* efficacy study are summarized in Table 15. A potent and significant reduction in CFU counts in kidneys was observed following subcutaneous treatment with ABX5006 and ABX5026 at both doses, i.e. 30 and 15 mg/kg, when compared to the vehicle control group. ABX5006 and ABX5026 show excellent *in vivo* activity, reaching a significant CFU count reduction of 1.53-log10 and 1.72-log10, respectively, at a dose of 30 mg/kg, and a CFU count reduction of 1.38-log10 and 1.58-log10, respectively, at a dose of 15 mg/kg in comparison to the vehicle control group. A significant reduction in CFU counts in kidneys was observed for ABX5006 at a dose of 30 mg/kg in comparison to the Gentamicin-treated group at the same dose. Remarkably, a significant reduction in CFU counts in kidneys was observed for ABX5026 at both doses in comparison to the Gentamicin-treated group at the same dose. In fact, five out of eight samples of the group treated with ABX5026 resulted in CFU counts below the limit of detection (LOD = 2,0×10² CFU/mL), demonstrating high *in vivo* efficacy. Of particular note is that the *in vivo* efficacy of ABX5026 is significantly higher than that of Gentamicin, despite ABX5026 having the same MIC value as this reference antibiotic against the *Escherichia coli* strain used in this study. Also surprising is that compound ABX5026 has slightly higher *in vivo* activity compared to ABX5006, when the same dose of each is applied. As mentioned above, ABX5026 has a 4-fold lower antibacterial activity *in vitro* compared to ABX5006 (i.e. 1 vs. 0,25) against the *Escherichia coli* strain, which was used also in the *in vivo* study (Table 15). Without being bound by any theory, it would appear that the R⁵ residue on the 3"-Guanidyl group in ABX5026 has an influence on the *in vivo* performance which compensates for the reduced *in vitro* activity compared to ABX5006. This result confirms also the observation made during the *in vivo* study with ABX5006 and ABX5026 employing the murine thigh model of infection (Table 12).

**Table 15. Results of in vivo mouse model of urinary tract infection with Escherichia coli (ATCC700336).**

| **Group** | **drug** | **Dose (mg/kg)** | **Avg. Log CFU/g (kidney pair)** | **St. Dev.** | **Change from vehicle group** |
|---|---|---|---|---|---|
| **1** | - | | 3,98 | 1,27 | - |
| **2** | Gentamicin | 15 | 2,73 | 0,57 | 1.26 |
| **3** | Gentamicin | 30 | 3,05 | 0,28 | 0,93 |
| **4** | ABX5006 | 15 | 2,60 | 0,46 | 1.38 |
| **5** | ABX5006 | 30 | 2,45 | 0,24 | 1.53 |
| **6** | ABX5026 | 15 | 2.40 | 0.43 | 1,58 |
| **7** | ABX5026 | 30 | 2,26 | 0.54 | 1.72 |

### In Vivo Toxicity Testing

The compounds of the invention ABX5006, ABX5026 and ABX5039 were further tested for their *in vivo* acute toxicity in rats. During this study the **m**aximum **t**olerated **d**ose (MTD) and median **l**ethal **d**ose (LD50) were determined for all compounds in male CD (Spraque Dawley) rats by employing the intravenous (IV) route. Three animals per group were used and the groups were treated once daily with as dose of 50, 75, 100, 150 or 200 mg/kg/day. Each compound was delivered intravenously via a slow injection over 15 minutes and the dose volume was 5 mL/kg. During the duration of the study (4 days) mortality was recorded twice a day, i.e. in the morning and at the end of the working day. Animals were examined clinically before administration, post-administration for 30 minutes, again one hour, four hours and eight hours later, then twice daily for the duration of the study. On day 1 between 30 and 90 minutes after administration and on day 4 the animals were submitted to a full clinical examination outside the housing cage, including complete external examination, posture and movements observations as well as behavioral examination. All animals surviving the end of day 4 were euthanized. All animals were subjected to gross necropsy, and organ weights (adrenal glands, brain, heart, kidneys, liver, spleen, epididymites, thymus, testes) were recorded for all animals of each group and compared with those of the vehicle group. Table 16 shows the results of the *in vivo* acute toxicity studies. Remarkably, the pathology resulted in no macroscopic findings in the treated groups. Also, the absolute and relative organ weights were in the same range as in the vehicle group (data not shown). ABX5026 and ABX5049 have the same MTD of 75, their LD50 value has been determined to be higher than 75mg/kg (no death occurred), but lower than 100mg/kg (all animals died). In contrast, ABX5006 stands out with a significantly (at least 2-fold) higher MTD and LD50 value (Table 16) compared to the ABX5026 and ABX5039. Nevertheless, all compounds of invention have significantly lower acute toxicity when compared to Gentamicin. A previous study (Robbins et al. 1971) has reported a LD50 value for Gentamicin in rats to be 67 mg/kg. In contrast, ABX5006 has a LD50 value of >200 mg/kg (only one out of three rats died), approx. 3-fold higher than the one reported for Gentamicin. Also, compounds of invention ABX5026 and AXB5039 have with a LD50 of >75 mg/kg, i.e. a significantly lower acute toxic compared to the reference antibiotic.

**Table 16. Results of in vivo acute toxicity studies in rats.**

| | **MTD (mg/kg)** | **LD50 (mg/kg)** |
|---|---|---|
| ABX5006 | 150 | >200 |
| ABX5026 | 75 | 75-100 |
| ABX5039 | 75 | 75-100 |
| Gentamicin | n.a. | ¹67 |

| | | |
|---|---|---|
| *1Robbins et al., 1971* | | |

## Claims

1. A compound of formula (I) or a pharmaceutically acceptable salt thereof, 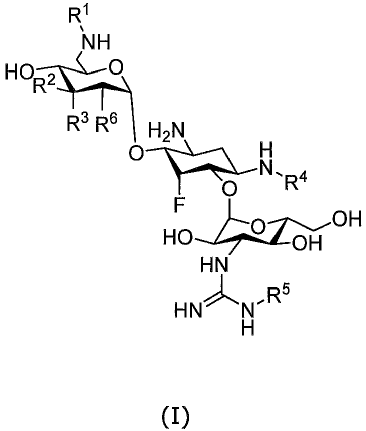 wherein
(i) R¹ is selected from the group consisting of H, methyl, ethyl, straight chain or branched C₃₋₆alkyl, C₃₋₆cycloalkyl, 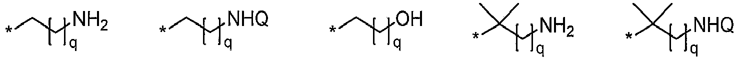 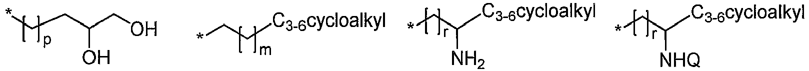 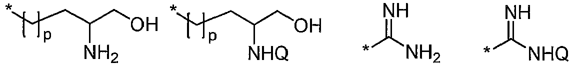 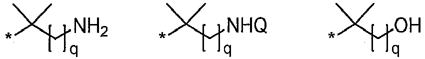 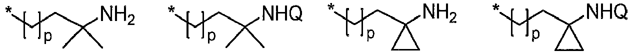 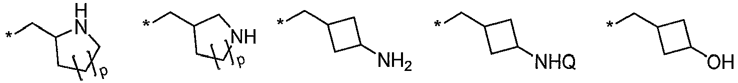 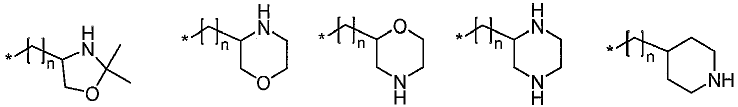 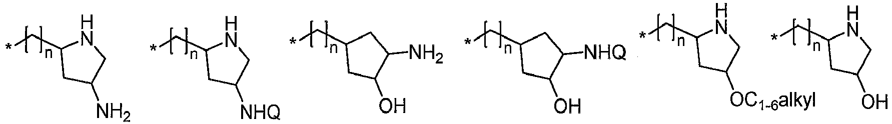 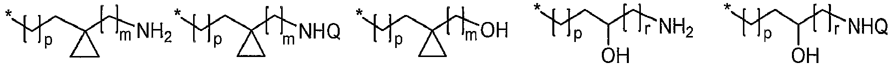 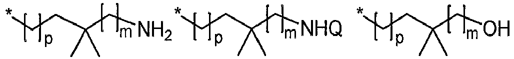 where * is the point of connection to the N atom to which R¹ is attached in formula (I);
(ii) R² is selected from the group consisting of H, methyl, -CH₂F, -CF₃, ethyl, n-propyl, iso-propyl, cyclopropyl, halogen, hydroxyl, -OCH₃, -OEt, -OCH₂F, -OCF₃, -NH₂, -NHCH₃, -NHEt, -N(CH₃)₂, -N(Et)₂, -NHCH₂F, -NHCF₃, and -NHQ;
wherein when R² is ethyl, n-propyl, iso-propyl, cyclopropyl, -OEt, -NHEt, or -N(Et)₂, the alkyl and cycloalkyl moieties in said R² groups may optionally be substituted with one or more substituents independently selected from the group consisting of halogen, hydroxyl, -OCH₃, -OEt, -NH₂, -NHCH₃, -NHEt, -N(CH₃)₂, -N(Et)₂, and -NHQ; with the proviso that when R² is -OEt, -NHEt, or -N(Et)₂, the carbon atom joining the Et group of said OEt, -NHEt, or -N(Et)₂ group to the O or N atom of said OEt, -NHEt, or -N(Et)₂ group may only be substituted with one or more substituents independently selected from halogen;
(iii) R³ is selected from the group consisting of H, halogen, hydroxyl, -OCH₃, -OCH₂F, -OCF₃, -OEt, -OC₃₋₈alkyl, -OC₃₋₆cycloalkyl, -OCH₂C₃₋₆cycloalkyl, -NH₂, -NHCH₃, -NHCH₂F, -NHCF₃, -NHEt, -NHC₃₋₈alkyl, -N(CH₃)₂, -N(Et)₂, -N(C₃-₈alkyl)₂, and -NHQ;
wherein when R³ is -OEt, -OC₃₋₈alkyl, -OC₃₋₆cycloalkyl, -OCH₂C₃₋₆cycloalkyl, -NHEt, -NHC₃₋₈alkyl, -N(Et)₂, -N(C₃₋₈alkyl)₂, the alkyl and cycloalkyl moieties in said R³ groups may optionally be substituted with one or more substituents independently selected from the group consisting of halogen, hydroxyl, -OCH₃, -OCH₂F, -OCF₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom in each of said R³ groups which is directly bonded to the O or N atom in each of said R³ groups may only be substituted with one or more substituents independently selected from halogen; and
wherein when R³ is -OCH₃ or -NHCH₃ it may be optionally substituted on the CH₃ moiety of said -OCH₃ or -NHCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S;
(iv) R⁴ is selected from the group consisting of H, methyl, ethyl, -CH₂F, -CF₃, straight chain or branched C₃₋₆alkyl, substituted straight chain C₂₋₆alkyl, substituted branched C₃₋₆alkyl, optionally substituted C₃₋₆cycloalkyl, optionally substituted -CH₂C₃₋₆cycloalkyl, formyl, optionally substituted phenyl, optionally substituted 5- or 6-membered heteroaryl, 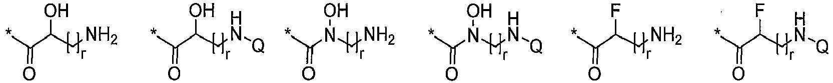 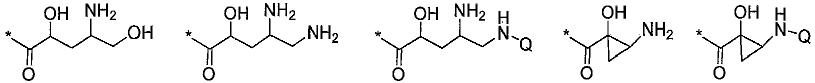 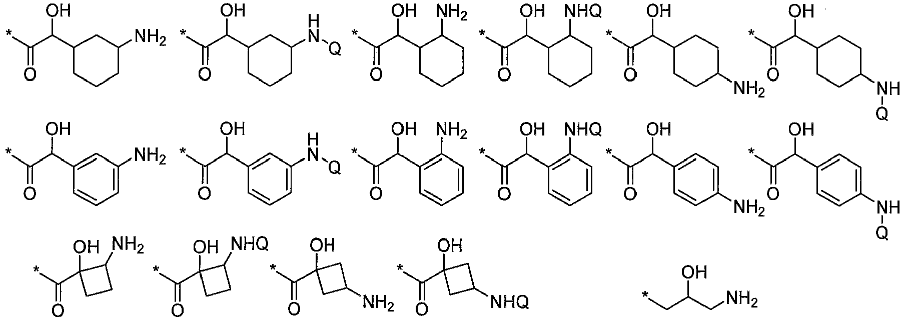 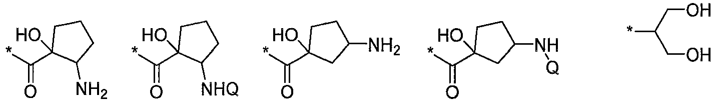 where * is the point of connection to the N atom to which R⁴ is attached in formula (I), and wherein
when R⁴ is substituted straight chain C₂₋₆alkyl, substituted branched C₃₋₆alkyl, substituted C₃₋₆cycloalkyl, or substituted -CH₂C₃₋₆cycloalkyl, it is substituted with one or more substituents independently selected from the group consisting of halogen, hydroxyl, -OCH₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom of said R⁴ group which is directly bonded to the N atom to which R⁴ is connected in the structure of formula (I) may only be substituted with one or more substituents independently selected from halogen;
(v) R⁵ is selected from the group consisting of H; methyl; -CH₂F; -CF₃; ethyl; straight chain or branched C₃₋₈alkyl; substituted straight chain C₂₋₈alkyl; substituted branched C₃₋₈alkyl; optionally substituted C₃₋₆cycloalkyl; optionally substituted -CH₂C₃₋₆cycloalkyl; optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; optionally substituted 6-membered non-aromatic heterocycloalkyl containing 1, 2 or 3 heteroatoms selected from O, N, and S; -C(=NH)NH₂; -C(=NR⁷)NH₂; -C(=NH)NHR⁸_{;} -C(=NR⁷)NHR⁸; -C(=NR⁷)NR⁸R⁹; and
-X-Z, wherein
X is selected from the group consisting of methylene, ethylene, straight chain or branched C₃₋₈alkylene; each of which may in addition to being attached to Z be optionally substituted with one or more substituents independently selected from the group consisting of methyl, -CH₂F, -CF₃, ethyl, straight chain or branched C₃₋₆alkyl, halogen, -OH, -OCH₃, -OCH₂F, -OCF₃, -OC₂₋₆alkyl, -NH₂, -NHQ, -NHR¹⁰, -NR¹⁰R¹¹, -CO₂H, -CO₂CH₃, -CO₂C₂₋₆alkyl, -OCOCH₃, -OCOC₂₋₆alkyl, -CN, -CONHR¹², -CONR¹²R¹³, -NHCOCH₃, -NHCOC₂₋₆alkyl, -NR¹⁴COCH₃, and -NR¹⁵COC₂₋₆alkyl; with the proviso that the atom of said X group which connects it to the N atom to which R⁵ is connected in the structure of formula (I) cannot be directly connected to a further O or N atom;
and
Z is selected from the group consisting of optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; optionally substituted 6-membered non-aromatic heterocycloalkyl containing 1, 2 or 3 heteroatoms selected from O, N, and S; optionally substituted C₃₋₆cycloalkyl;
wherein each of R⁷ to R¹⁵ is independently selected from the group consisting of H, methyl, ethyl, C₃₋₆alkyl, and C₃-₆cycloalkyl;
(vi) R⁶ is OH or NH₂;
(vii) with the proviso for all compounds of formula (I) or pharmaceutically acceptable salts thereof that when the atom of R² which connects it to the tetrahydropyran ring to which both it and R³ are connected in the structure of formula (I) is an O or N atom, the atom of R³ which connects it to said tetrahydropyran ring cannot be an O or N atom; and the proviso that when the atom of R³ which connects it to the tetrahydropyran ring to which both it and R² are connected in the structure of formula (I) is an O or N atom, the atom of R² which connects it to said tetrahydropyran ring cannot be an O or N atom; and
(viii) wherein for each of the groups disclosed
m = 0, 1, 2, 3, 4, or 5;
n = 1 or 2;
p = 0, 1, or 2;
q = 1, 2, 3, 4, or 5;
r = 1, 2, 3, or 4; and
(ix) wherein in respect of all of the above substituents and groups containing the moiety Q,
Q is 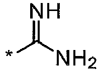
where * is the point of connection to the N atom to which Q is attached.

2. The compound or pharmaceutically acceptable salt thereof according to claim 1, wherein R² is selected from the group consisting of H, methyl, -CH₂F, -CF₃, ethyl, n-propyl, iso-propyl, cyclopropyl, halogen, hydroxyl, -OCH₃, -OEt, -OCH₂F, -OCF₃, -NH₂, -NHCH₃, -NHEt, -N(CH₃)₂, -N(Et)₂, -NHCH₂F, -NHCF₃, and -NHQ.

3. The compound or pharmaceutically acceptable salt thereof according to any of the preceding claims, wherein R² is selected from the group consisting of H, methyl, ethyl, n-propyl, iso-propyl, cyclopropyl, -F, hydroxyl, -OCH₃, -OEt, -OCH₂F, and -OCF₃.

4. The compound or pharmaceutically acceptable salt thereof according to any of the preceding claims, wherein R³ is selected from the group consisting of H, halogen, hydroxyl, -OCH₃, -OCH₂F, -OCF₃, -OEt, -OC₃₋₈alkyl, -OC₃₋₆cycloalkyl, and -OCH₂C₃₋₆cycloalkyl,
wherein when R³ is -OEt, -OC₃₋₈alkyl, -OC₃₋₆cycloalkyl, or -OCH₂C₃₋₆cycloalkyl, the alkyl and cycloalkyl moieties in said R³ groups may be optionally substituted with one or more substituents independently selected from the group consisting of halogen, hydroxyl, -OCH₃, -OCH₂F, -OCF₃, -OC₂₋₄alkyl, -NH₂, -NHCH₃, -NHC₂₋₄alkyl, -N(CH₃)₂, -N(C₂₋₄alkyl)₂, and -NHQ; with the proviso that the carbon atom in each of said R³ groups which is directly bonded to the O atom in each of said R³ groups may only be substituted with one or more substituents independently selected from halogen; and
wherein when R³ is -OCH₃ it may be optionally substituted on the CH₃ moiety of said -OCH₃ group with optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S.

5. The compound or pharmaceutically acceptable salt thereof according to any of the preceding claims, wherein R³ is selected from the group consisting of H, halogen, hydroxyl, -OCH₃, -OCH₂F, -OCF₃, -OEt, -OC₃-₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); and -OCH₂(optionally substituted 6-membered non-aromatic heterocycloalkyl containing 1, 2 or 3 heteroatoms selected from O, N, and S).

6. The compound or pharmaceutically acceptable salt thereof according to any of the preceding claims, wherein the compound of formula (I) is selected from the compounds of formula (Ia) to (If) 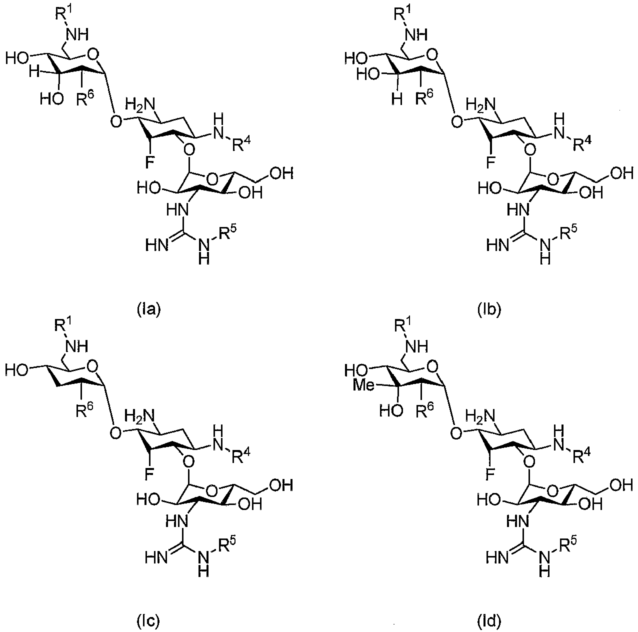 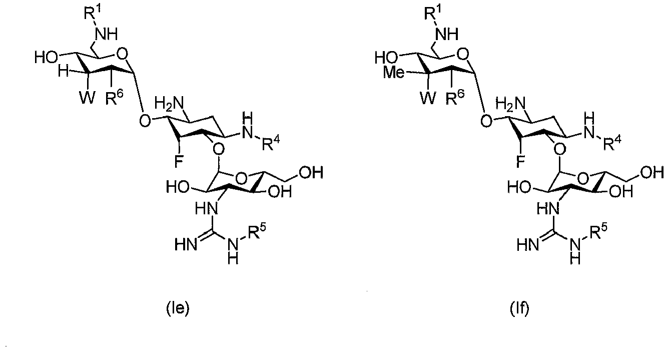 wherein independently for each of the compounds of formula (Ia) to (If) R¹, R⁴, R⁵ and R⁶ are as defined in any of the preceding claims, and wherein W is independently selected for each of the compounds of formula (Ie) to (If) from the group consisting of -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S); and -OCH₂(optionally substituted 6-membered non-aromatic heterocycloalkyl containing 1, 2 or 3 heteroatoms selected from O, N, and S).

7. The compound or pharmaceutically acceptable salt thereof according to any of the preceding claims, wherein the compound of formula (I) is selected from the compounds of formula (Ig) to (Ip) 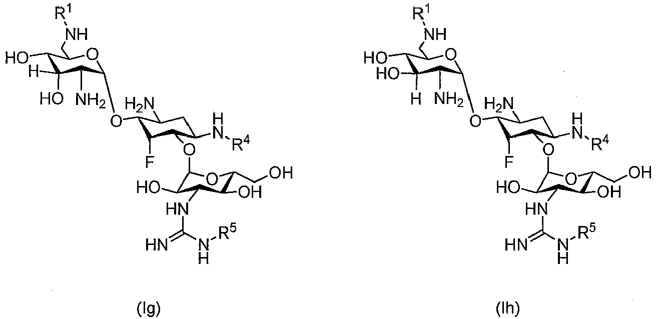 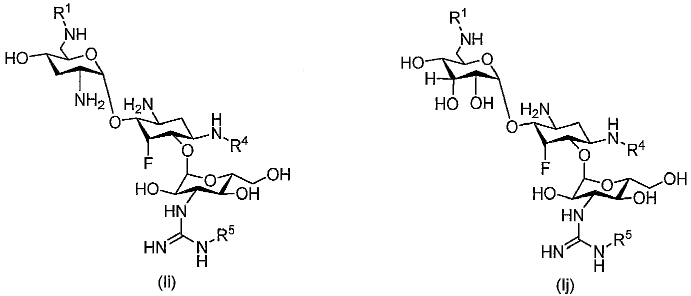 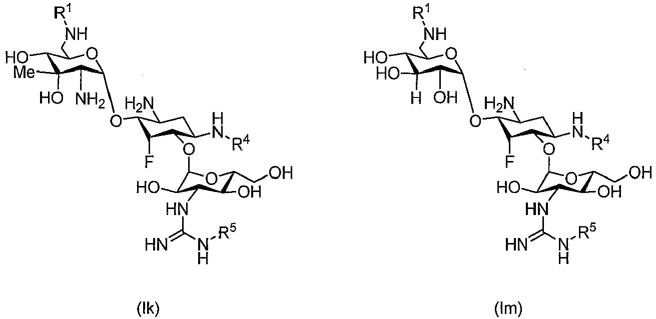 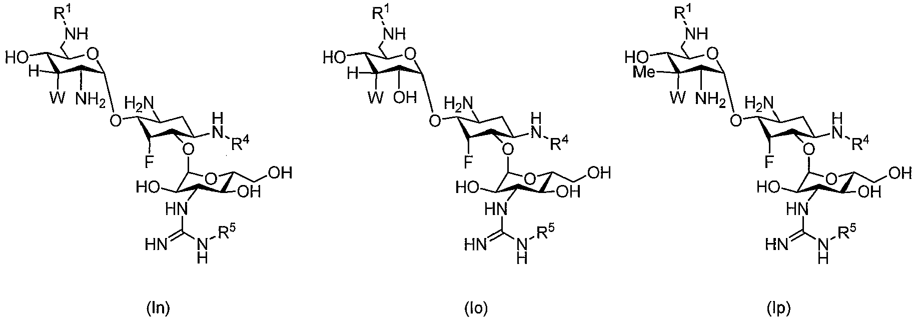 wherein independently for each of the compounds of formula (Ig) to (Ip) R¹, R⁴, and R⁵ are as defined in any of the preceding claims, and wherein W is independently selected for each of the compounds of formula (In) to (Ip) from the group consisting of -OCH₃, -OCH₂F, -OCF₃, -OEt, -OC₃₋₈alkyl, -OC₃₋₆cycloalkyl, -OCH₂C₃₋₆cycloalkyl, -OCH₂(optionally substituted phenyl), -OCH₂(optionally substituted 5-membered heteroaryl), -OCH₂(optionally substituted 6-membered heteroaryl), -OCH₂(optionally substituted 4-membered non-aromatic heterocycloalkyl containing 1 heteroatom selected from O, N, and S); -OCH₂(optionally substituted 5-membered non-aromatic heterocycloalkyl containing 1 or 2 heteroatoms selected from O, N, and S; and -OCH₂(optionally substituted 6-membered non-aromatic heterocycloalkyl containing 1, 2 or 3 heteroatoms selected from O, N, and S).

8. The compound or pharmaceutically acceptable salt thereof according to any of the preceding claims, wherein R¹ is selected from the group consisting of H, methyl, ethyl, straight chain or branched C₃₋₆alkyl, C₃₋₆cycloalkyl, 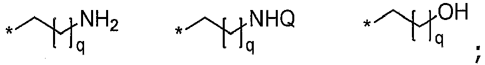 preferably from the group consisting of H, 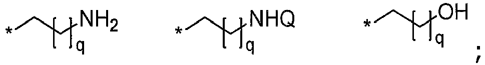 wherein in said R¹ moieties q and Q are as defined in claim 1.

9. The compound or pharmaceutically acceptable salt thereof according to any of the preceding claims, wherein R⁴ is selected from the group consisting of H, 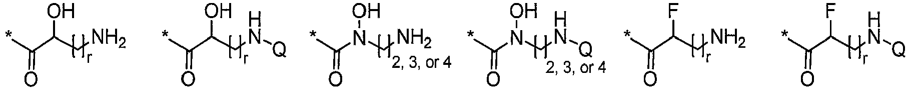 and wherein in said R⁴ moieties r and Q are as defined in claim 1.

10. The compound or pharmaceutically acceptable salt thereof according to any of the preceding claims, wherein R⁴ is selected from the group consisting of H,

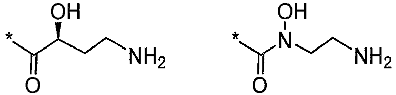

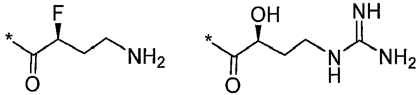

11. The compound or pharmaceutically acceptable salt thereof according to any of the preceding claims, wherein R⁵ is selected from the group consisting of H, methyl, ethyl, straight chain or branched C₃₋₈alkyl, substituted straight chain C₂₋₈alkyl, substituted branched chain C₃₋₈alkyl, optionally substituted C₃₋₆cycloalkyl, 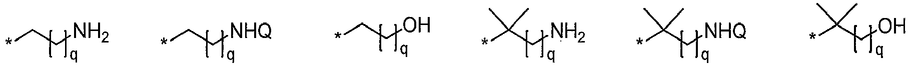 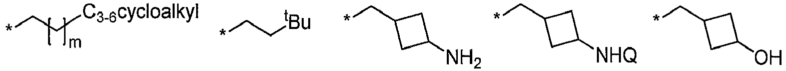 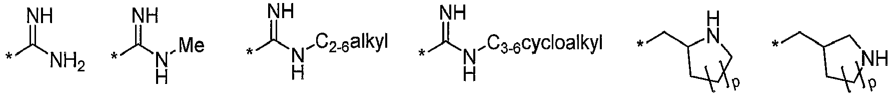 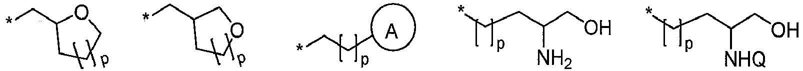 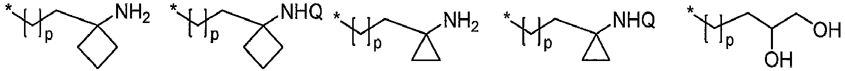 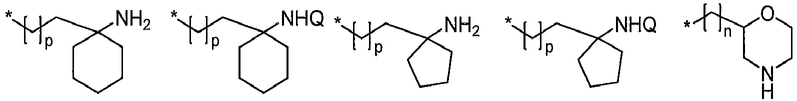 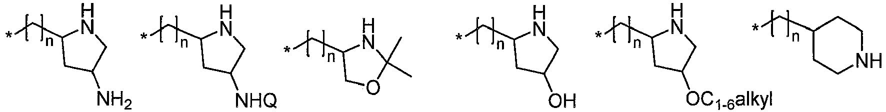 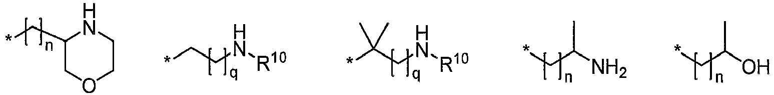 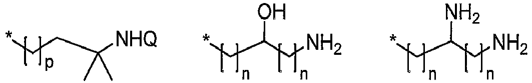
where * is the point of connection to the N atom to which R⁵ is attached in formula (I), and
wherein A is selected from the group consisting of optionally substituted phenyl; optionally substituted 5-membered heteroaryl; optionally substituted 6-membered heteroaryl; optionally substituted 4-membered non-aromatic heterocycloalkyl containing 1 heteroatom selected from O, N, and S; optionally substituted 5-membered non-aromatic heterocycloalkyl containing 1 or 2 heteroatoms selected from O, N, and S; or optionally substituted 6-membered non-aromatic heterocycloalkyl containing 1, 2 or 3 heteroatoms selected from O, N, and S; and
wherein m, n, q, p, and R¹⁰ in said R⁵ moieties are as defined in any of the previous claims.

12. The compound or pharmaceutically acceptable salt thereof according to any of the preceding claims, wherein R⁵ is selected from the group consisting of H, methyl, ethyl, straight chain or branched C₃₋₆alkyl, 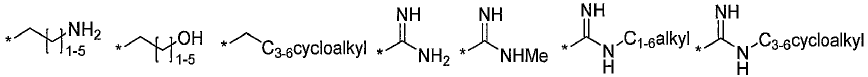 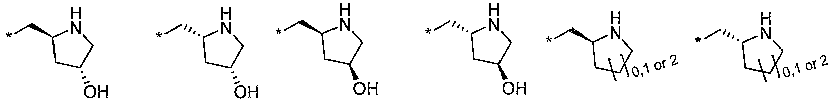 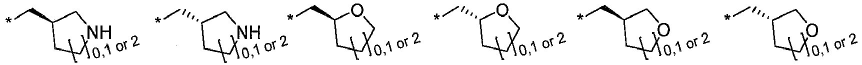 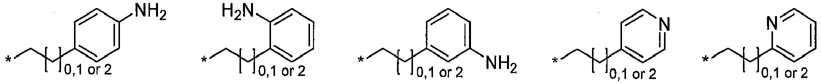 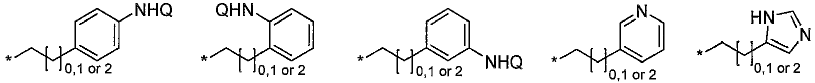 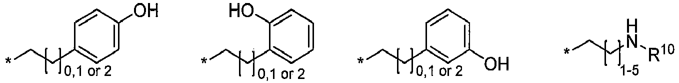 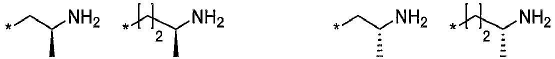 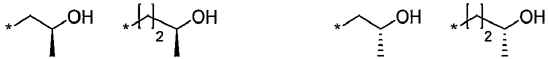 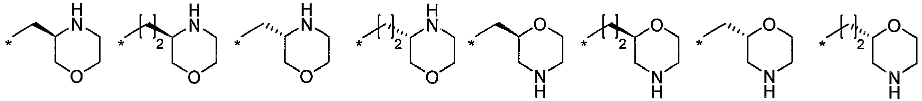 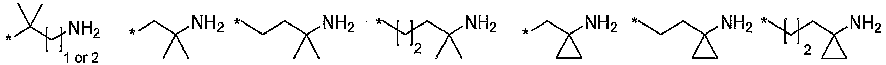 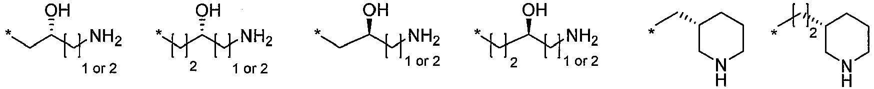 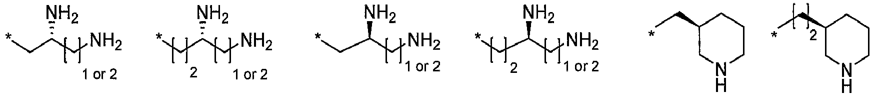 wherein Q and R¹⁰ in said R⁵ moieties are as defined in any of the previous claims.

13. A pharmaceutical composition comprising the compound or pharmaceutically acceptable salt thereof according to any of the preceding claims and a pharmaceutically acceptable carrier.

14. A composition comprising at least one compound or pharmaceutically acceptable salt thereof according to any of claims 1 to 12 and at least one further antibacterial agent, wherein said at least one further antibacterial agent is different to the at least one compound or pharmaceutically acceptable salt thereof according to any of claims 1 to 12.

15. A compound or pharmaceutically acceptable salt thereof according to any of claims 1 to 12 or a composition according to any of claims 13 to 14 for use as a medicament.

## Patentansprüche

1. Verbindung der Formel (I) oder pharmazeutisch annehmbares Salz davon: 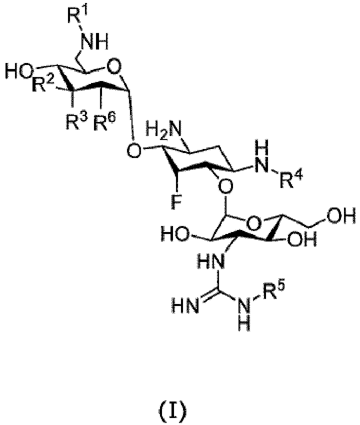 wobei
(i) R¹ aus der Gruppe ausgewählt ist, die aus H, Methyl, Ethyl, geradkettigem oder verzweigtem C₃₋₆-Alkyl, C₃₋₆-Cycloalkyl besteht, 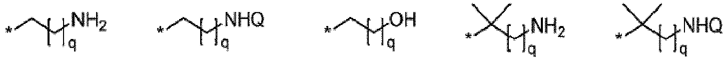 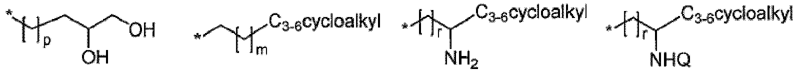 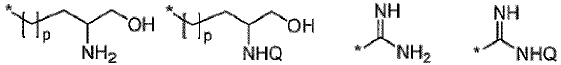 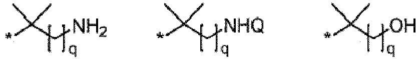 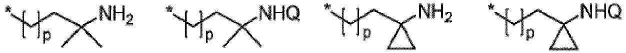 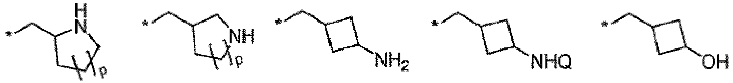 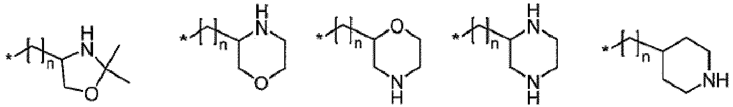 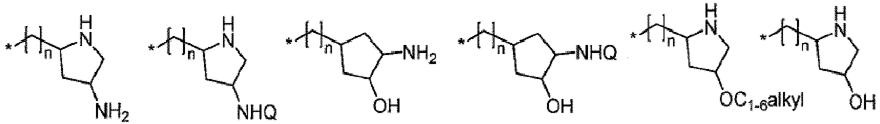 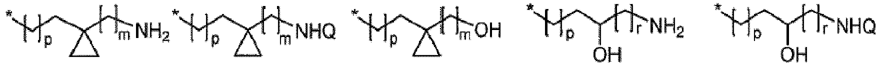 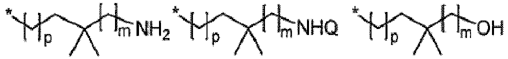 wobei * der Verbindungspunkt zum N-Atom ist, an das R¹ in Formel (I) gebunden ist;
(ii) R² aus der Gruppe ausgewählt ist, die aus H, Methyl, -CH₂F, -CF₃, Ethyl, n-Propyl, Isopropyl, Cyclopropyl, Halogen, Hydroxy, -OCH₃, -OEt, -OCH₂F, -OCF₃, -NH₂, -NHCH₃, -NHEt, -N(CH₃)₂, -N(Et)₂, -NHCH₂F, -NHCF₃ und -NHQ besteht;
wobei, wenn R² Ethyl, n-Propyl, Isopropyl, Cyclopropyl, -OEt, -NHEt oder -N(Et)₂ ist, die Alkyl- und Cycloalkyl-Struktureinheiten in den R²-Gruppen gegebenenfalls mit einem oder mehreren Substituenten substituiert sein können, die unabhängig aus der Gruppe ausgewählt sind, die aus Halogen, Hydroxy, -OCH₃, -OEt, -NH₂, -NHCH₃, -NHEt, -N(CH₃)₂, -N(Et)₂ und -NHQ besteht; mit der Maßgabe, dass dann, wenn R² = -OEt, -NHEt oder -N(Et)₂ ist, das Kohlenstoffatom, das die Et-Gruppe der OEt-, -NHEt- oder -N(Et)₂-Gruppe mit dem O- oder N-Atom der OEt-, -NHEt- oder -N(Et)₂-Gruppe verbindet, nur mit einem oder mehreren Substituenten substituiert sein kann, die unabhängig aus Halogen ausgewählt sind;
(iii) R³ aus der Gruppe ausgewählt ist, die aus H, Halogen, Hydroxy, -OCH₃, -OCH₂F, -OCF₃, -OEt, -OC₃₋₈-Alkyl, -OC₃₋₆-Cycloalkyl, -OCH₂C₃₋₆-Cycloalkyl, -NH₂, -NHCH₃, -NHCH₂F, -NHCF₃, -NHEt, -NHC₃₋₈-Alkyl, -N(CH₃)₂, -N(Et)₂, -N(C₃₋₈-Alkyl)₂ und -NHQ besteht;
wobei, wenn R³ = -OEt, -OC₃₋₈-Alkyl, -OC₃₋₆-Cycloalkyl, -OCH₂C₃₋₆-Cycloalkyl, -NHEt, -NHC₃₋₈-Alkyl, -N(Et)₂, -N(C₃₋₈-Alkyl)₂ ist, die Alkyl- und Cycloalkyl-Struktureinheiten in den R³-Gruppen gegebenenfalls mit einem oder mehreren Substituenten substituiert sein können, die unabhängig aus der Gruppe ausgewählt sind, die aus Halogen, Hydroxy, -OCH₃, -OCH₂F, -OCF₃, -OC₂₋₄-Alkyl, -NH₂, -NHCH₃, -NHC₂₋₄-Alkyl, -N(CH₃)₂, -N(C₂₋₄-Alkyl)₂ und -NHQ besteht; mit der Maßgabe, dass das Kohlenstoffatom in jeder der R³-Gruppen, das direkt an das O- oder N-Atom in jeder der R³-Gruppen gebunden ist, nur mit einem oder mehreren Substituenten substituiert sein kann, die unabhängig aus Halogen ausgewählt sind; und
wobei dann, wenn R³ = -OCH₃ oder -NHCH₃ ist, es an der CH₃-Struktureinheit der -OCH₃- oder -NHCH₃-Gruppe gegebenenfalls substituiert sein kann mit gegebenenfalls substituiertem Phenyl; gegebenenfalls substituiertem fünfgliedrigen Heteroaryl; gegebenenfalls substituiertem sechsgliedrigen Heteroaryl; gegebenenfalls substituiertem viergliedrigen nichtaromatischem Heterocycloalkyl, das 1 Heteroatom enthält, welches aus O, N und S ausgewählt ist; gegebenenfalls substituiertem fünfgliedrigen nichtaromatischem Heterocycloalkyl, das 1 oder 2 Heteroatome enthält, die aus O, N und S ausgewählt sind; oder gegebenenfalls substituiertem sechsgliedrigen nichtaromatischem Heterocycloalkyl, das 1, 2 oder 3 Heteroatome enthält, die aus O, N und S ausgewählt sind;
(iv) R⁴ aus der Gruppe ausgewählt ist, die aus H, Methyl, Ethyl, -CH₂F, -CF₃, geradkettigem oder verzweigtem C₃₋₆-Alkyl, substituiertem geradkettigen C₂₋₆-Alkyl, substituiertem verzweigten C₃₋₆-Alkyl, gegebenenfalls substituiertem C₃₋₆-Cycloalkyl, gegebenenfalls substituiertem -CH₂C₃₋₆-Cycloalkyl, Formyl, gegebenenfalls substituiertem Phenyl, gegebenenfalls substituiertem fünf- oder sechsgliedrigen Heteroaryl besteht, 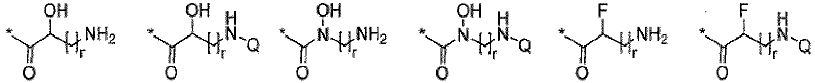 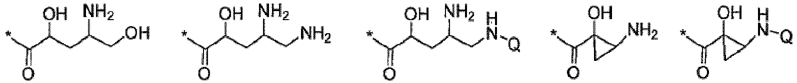 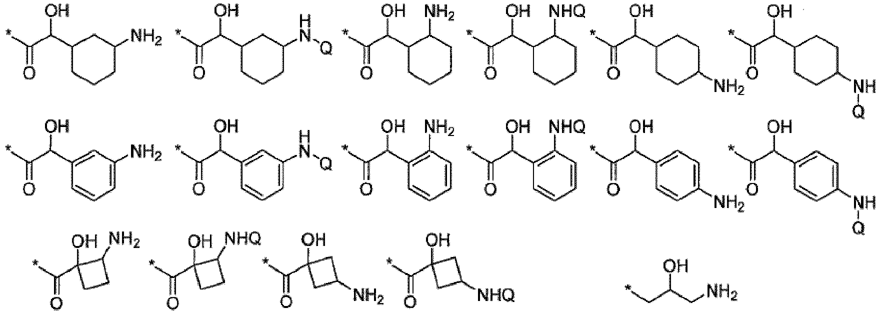 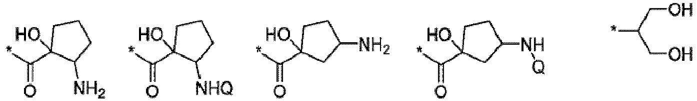 wobei * der Verbindungspunkt zum N-Atom ist, an das R⁴ in Formel (I) gebunden ist, und wobei
dann, wenn R⁴ substituiertes geradkettiges C₂₋₆-Alkyl, substituiertes verzweigtes C₃₋₆-Alkyl, substituiertes C₃₋₆-Cycloalkyl oder substituiertes -CH₂C₃₋₆-Cycloalkyl ist, es mit einem oder mehreren Substituenten substituiert ist, die unabhängig aus der Gruppe ausgewählt sind, welche aus Halogen, Hydroxy, -OCH₃, -OC₂₋₄-Alkyl, -NH₂, -NHCH₃, -NHC₂₋₄-Alkyl, -N(CH₃)₂, -N(C₂₋₄-Alkyl)₂ und -NHQ besteht; mit der Maßgabe, dass das Kohlenstoffatom der R⁴-Gruppe, das direkt an das N-Atom gebunden ist, mit dem R⁴ in der Struktur von Formel (I) verknüpft ist, nur mit einem oder mehreren Substituenten substituiert sein kann, die unabhängig aus Halogen ausgewählt sind;
(v) R⁵ aus der Gruppe ausgewählt ist, die aus H; Methyl; -CH₂F; -CF₃; Ethyl; geradkettigem oder verzweigtem C₃₋₈-Alkyl; substituiertem geradkettigen C₂₋₈-Alkyl; substituiertem verzweigten C₃₋₈-Alkyl; gegebenenfalls substituiertem C₃₋₆-Cycloalkyl; gegebenenfalls substituiertem -CH₂C₃₋₆-Cycloalkyl; gegebenenfalls substituiertem Phenyl; gegebenenfalls substituiertem fünfgliedrigen Heteroaryl; gegebenenfalls substituiertem sechsgliedrigen Heteroaryl; gegebenenfalls substituiertem viergliedrigen nichtaromatischem Heterocycloalkyl, das 1 Heteroatom enthält, welches aus O, N und S ausgewählt ist; gegebenenfalls substituiertem fünfgliedrigen nichtaromatischem Heterocycloalkyl, das 1 oder 2 Heteroatome enthält, die aus O, N und S ausgewählt sind; oder gegebenenfalls substituiertem sechsgliedrigen nichtaromatischem Heterocycloalkyl, das 1, 2 oder 3 Heteroatome enthält, die aus O, N und S ausgewählt sind; -C(=NH)NH₂; -C(=NR⁷)NH₂; -C(=NH)NHR⁸; -C(=NR⁷)NHR⁸; -C(=NR⁷)NR⁸R⁹ und -X-Z besteht;
wobei
X aus der Gruppe ausgewählt ist, die aus Methylen, Ethylen, geradkettigem oder verzweigtem C₃₋₈-Alkylen besteht; von denen jedes, außer dass es an Z gebunden ist, auch gegebenenfalls mit einem oder mehreren Substituenten substituiert ist, die unabhängig aus der Gruppe ausgewählt sind, die aus Methyl, -CH₂F, -CF₃, Ethyl, geradkettigem oder verzweigtem C₃₋₆-Alkyl, Halogen, -OH, -OCH₃, -OCH₂F, -OCF₃, -OC₂₋₆-Alkyl, -NH₂, -NHQ, -NHR¹⁰, -NR¹⁰R¹¹, -CO₂H, -CO₂CH₃, -CO₂C₂₋₆-Alkyl, -OCOCH₃, -OCOC₂₋₆-Alkyl, -CN, -CONHR¹², -CONR¹²R¹³, -NHCOCH₃, -NHCOC₂₋₆-Alkyl, -NR¹⁴COCH₃ und -NR¹⁵COC₂₋₆-Alkyl besteht; mit der Maßgabe, dass das Atom der Gruppe X, das diese mit dem N-Atom, an das R⁵ in der Struktur der Formel (I) verbunden ist, verbindet, nicht direkt mit einem weiteren O- oder N-Atom verbunden sein kann;
und
Z aus der Gruppe ausgewählt ist, die aus gegebenenfalls substituiertem Phenyl; gegebenenfalls substituiertem fünfgliedrigen Heteroaryl; gegebenenfalls substituiertem sechsgliedrigen Heteroaryl; gegebenenfalls substituiertem viergliedrigen nichtaromatischem Heterocycloalkyl, das 1 Heteroatom enthält, welches aus O, N und S ausgewählt ist; gegebenenfalls substituiertem fünfgliedrigen nichtaromatischem Heterocycloalkyl, das 1 oder 2 Heteroatome enthält, die aus O, N und S ausgewählt sind; gegebenenfalls substituiertem sechsgliedrigen nichtaromatischem Heterocycloalkyl, das 1, 2 oder 3 Heteroatome enthält, die aus O, N und S ausgewählt sind; gegebenenfalls substituiertem C₃₋₆-Cycloalkyl; besteht,
wobei R⁷ bis R¹⁵ jeweils unabhängig aus der Gruppe ausgewählt ist, die aus H, Methyl, Ethyl, C₃₋₆-Alkyl und C₃₋₆-Cycloalkyl besteht;
(vi) R⁶ = OH oder NH₂ ist;
(vii) mit der Maßgabe für alle Verbindungen der Formel (I) oder ihre pharmazeutisch annehmbaren Salze, dass dann, wenn das Atom von R², das dieses an den Tetrahydropyranring, mit dem sowohl es als auch R³ in der Struktur von Formel (I) verbunden sind, bindet, ein O- oder N-Atom ist, das Atom von R³, das dieses an den Tetrahydropyranring bindet, kein O- oder N-Atom sein kann; und mit der Maßgabe, dass dann, wenn das Atom von R³, das dieses an den Tetrahydropyranring, mit dem sowohl es als auch R² in der Struktur von Formel (I) verbunden sind, bindet, ein O- oder N-Atom ist, das Atom von R², das dieses an den Tetrahydropyranring bindet, kein O- oder N-Atom sein kann; und
(viii) wobei für jede der offenbarten Gruppen Folgendes gilt:
m = 0, 1, 2, 3, 4 oder 5;
n = 1 oder 2;
p = 0, 1 oder 2;
q = 1, 2, 3, 4 oder 5;
r = 1, 2, 3 oder 4; und
(ix) wobei in Bezug auf alle die obigen Substituenten und Gruppen, die die Struktureinheit Q enthalten, 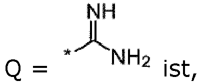 wobei * der Verbindungspunkt zum N-Atom ist, an das Q gebunden ist.

2. Verbindung oder pharmazeutisch annehmbares Salz davon gemäß Anspruch 1, wobei R² aus der Gruppe ausgewählt ist, die aus H, Methyl, -CH₂F, -CF₃, Ethyl, n-Propyl, Isopropyl, Cyclopropyl, Halogen, Hydroxy, -OCH₃, -OEt, -OCH₂F, -OCF₃, -NH₂, -NHCH₃, -NHEt, -N(CH₃)₂, -N(Et)₂, -NHCH₂F, -NHCF₃ und -NHQ besteht.

3. Verbindung oder pharmazeutisch annehmbares Salz davon gemäß einem der vorstehenden Ansprüche, wobei R² aus der Gruppe ausgewählt ist, die aus H, Methyl, Ethyl, n-Propyl, Isopropyl, Cyclopropyl, -F, Hydroxy, -OCH₃, -OEt, -OCH₂F und -OCF₃ besteht.

4. Verbindung oder pharmazeutisch annehmbares Salz davon gemäß einem der vorstehenden Ansprüche, wobei R³ aus der Gruppe ausgewählt ist, die aus H, Halogen, Hydroxy, -OCH₃, -OCH₂F, -OCF₃, -OEt, -OC₃₋₈-Alkyl, -OC₃₋₆-Cycloalkyl und -OCH₂C₃₋₆-Cycloalkyl besteht,
wobei dann, wenn R³ = -OEt, -OC₃₋₈-Alkyl, -OC₃₋₆-Cycloalkyl oder -OCH₂C₃₋₆-Cycloalkyl ist, die Alkyl- und Cycloalkyl-Struktureinheiten in den R³-Gruppen gegebenenfalls mit einem oder mehreren Substituenten substituiert sein können, die unabhängig aus der Gruppe ausgewählt sind, die aus Halogen, Hydroxy, -OCH₃, -OCH₂F, -OCF₃, -OC₂₋₄-Alkyl, -NH₂, -NHCH₃, -NHC₂₋₄-Alkyl, -N(CH₃)₂, -N(C₂₋₄-Alkyl)₂ und -NHQ besteht; mit der Maßgabe, dass das Kohlenstoffatom in jeder der R³-Gruppen, das direkt an das O-Atom in jeder der R³-Gruppen gebunden ist, nur mit einem oder mehreren Substituenten substituiert sein kann, die unabhängig aus Halogen ausgewählt sind; und
wobei dann, wenn R³ = -OCH₃ ist, es an der CH₃-Struktureinheit der -OCH₃-Gruppe gegebenenfalls substituiert sein kann mit gegebenenfalls substituiertem Phenyl; gegebenenfalls substituiertem fünfgliedrigen Heteroaryl; gegebenenfalls substituiertem sechsgliedrigen Heteroaryl; gegebenenfalls substituiertem viergliedrigen nichtaromatischem Heterocycloalkyl, das 1 Heteroatom enthält, welches aus O, N und S ausgewählt ist; gegebenenfalls substituiertem fünfgliedrigen nichtaromatischem Heterocycloalkyl, das 1 oder 2 Heteroatome enthält, die aus O, N und S ausgewählt sind; oder gegebenenfalls substituiertem sechsgliedrigen nichtaromatischem Heterocycloalkyl, das 1, 2 oder 3 Heteroatome enthält, die aus O, N und S ausgewählt sind.

5. Verbindung oder pharmazeutisch annehmbares Salz davon gemäß einem der vorstehenden Ansprüche, wobei R³ aus der Gruppe ausgewählt ist, die aus H, Halogen, Hydroxy, -OCH₃, -OCH₂F, -OCF₃, -OEt, -OC₃₋₈-Alkyl, -OC₃₋₆-Cycloalkyl, -OCH₂C₃₋₆-Cycloalkyl, -OCH₂-(gegebenenfalls substituiertes Phenyl), -OCH₂-(gegebenenfalls substituiertes fünfgliedriges Heteroaryl), -OCH₂-(gegebenenfalls substituiertes sechsgliedriges Heteroaryl), -OCH₂-(gegebenenfalls substituiertes viergliedriges nichtaromatisches Heterocycloalkyl, das 1 Heteroatom enthält, welches aus O, N und S ausgewählt ist); -OCH₂-(gegebenenfalls substituiertes fünfgliedriges nichtaromatisches Heterocycloalkyl, das 1 oder 2 Heteroatome enthält, die aus O, N und S ausgewählt sind); und -OCH₂-(gegebenenfalls substituiertes sechsgliedriges nichtaromatisches Heterocycloalkyl, das 1, 2 oder 3 Heteroatome enthält, die aus O, N und S ausgewählt sind) besteht.

6. Verbindung oder pharmazeutisch annehmbares Salz davon gemäß einem der vorstehenden Ansprüche, wobei die Verbindung der Formel (I) aus den Verbindungen der Formel (Ia) bis (If) ausgewählt ist, 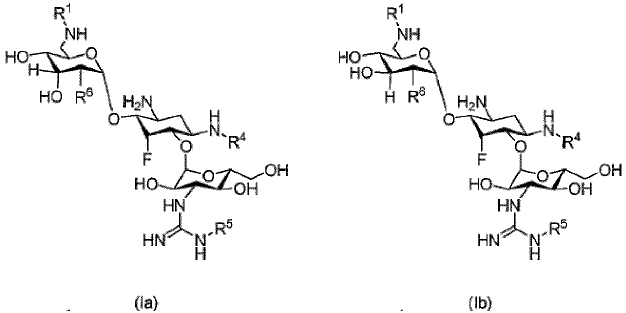 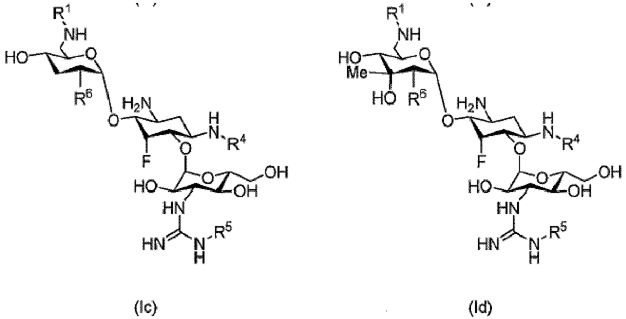 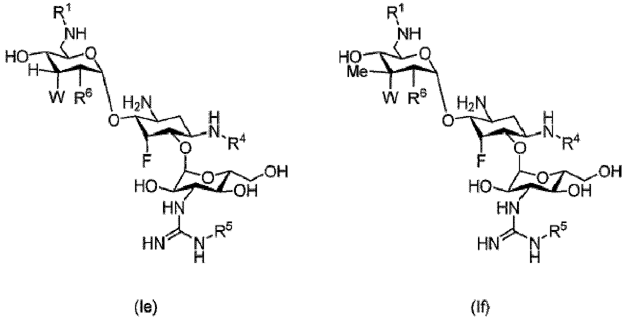 wobei unabhängig für jede der Verbindungen der Formel (Ia) bis (If) R¹, R⁴, R⁵ und R⁶ wie in einem der vorstehenden Ansprüche definiert sind, und wobei W unabhängig für jede der Verbindungen der Formel (Ie) bis (If) aus der Gruppe ausgewählt ist, die aus -OCH₃, -OCH₂F, -OCF₃, -OEt, -OC₃₋₈-Alkyl, -OC₃₋₆-Cycloalkyl, -OCH₂C₃₋₆-Cycloalkyl, -OCH₂-(gegebenenfalls substituiertes Phenyl), -OCH₂-(gegebenenfalls substituiertes fünfgliedriges Heteroaryl), -OCH₂-(gegebenenfalls substituiertes sechsgliedriges Heteroaryl), -OCH₂-(gegebenenfalls substituiertes viergliedriges nichtaromatisches Heterocycloalkyl, das 1 Heteroatom enthält, welches aus O, N und S ausgewählt ist); -OCH₂-(gegebenenfalls substituiertes fünfgliedriges nichtaromatisches Heterocycloalkyl, das 1 oder 2 Heteroatome enthält, die aus O, N und S ausgewählt sind); und -OCH₂-(gegebenenfalls substituiertes sechsgliedriges nichtaromatisches Heterocycloalkyl, das 1, 2 oder 3 Heteroatome enthält, die aus O, N und S ausgewählt sind) besteht.

7. Verbindung oder pharmazeutisch annehmbares Salz davon gemäß einem der vorstehenden Ansprüche, wobei die Verbindung der Formel (I) aus den Verbindungen der Formel (Ig) bis (Ip) ausgewählt ist, 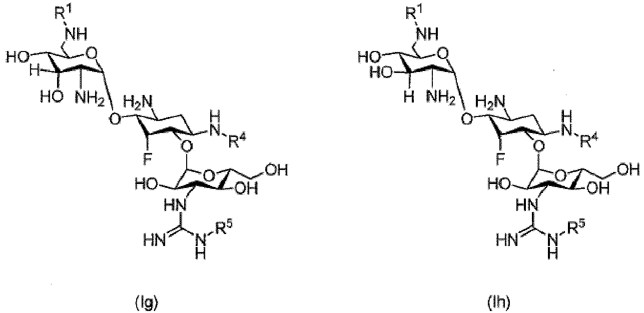 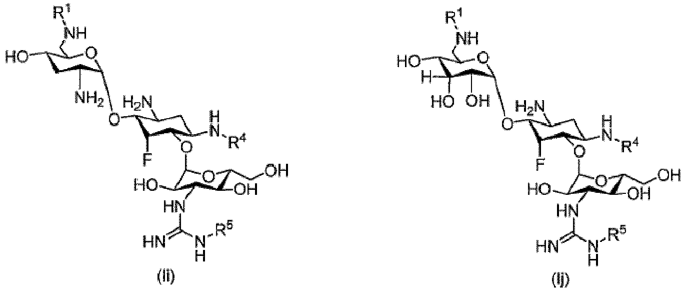 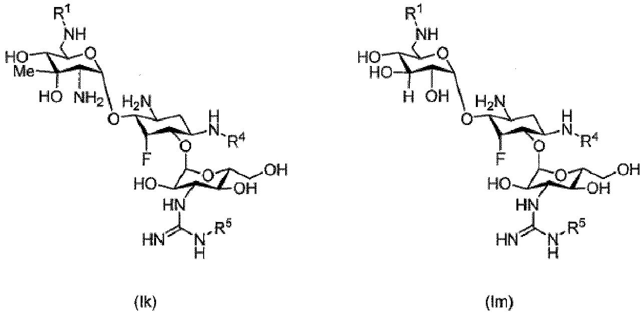 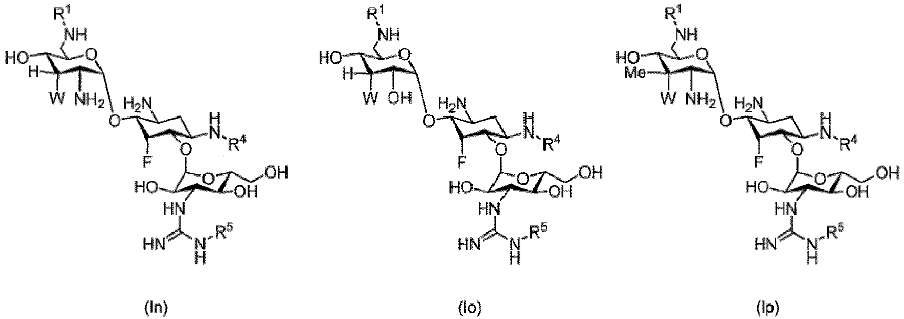 wobei unabhängig für jede der Verbindungen der Formel (Ig) bis (Ip) R¹, R⁴ und R⁵ wie in einem der vorstehenden Ansprüche definiert sind, und wobei W unabhängig für jede der Verbindungen der Formel (In) bis (Ip) aus der Gruppe ausgewählt ist, die aus -OCH₃, -OCH₂F, -OCF₃, -OEt, -OC₃₋₈-Alkyl, -OC₃₋₆-Cycloalkyl, -OCH₂C₃₋₆-Cycloalkyl, -OCH₂-(gegebenenfalls substituiertes Phenyl), -OCH₂-(gegebenenfalls substituiertes fünfgliedriges Heteroaryl), -OCH₂-(gegebenenfalls substituiertes sechsgliedriges Heteroaryl), -OCH₂-(gegebenenfalls substituiertes viergliedriges nichtaromatisches Heterocycloalkyl, das 1 Heteroatom enthält, welches aus O, N und S ausgewählt ist); -OCH₂-(gegebenenfalls substituiertes fünfgliedriges nichtaromatisches Heterocycloalkyl, das 1 oder 2 Heteroatome enthält, die aus O, N und S ausgewählt sind); und -OCH₂-(gegebenenfalls substituiertes sechsgliedriges nichtaromatisches Heterocycloalkyl, das 1, 2 oder 3 Heteroatome enthält, die aus O, N und S ausgewählt sind) besteht.

8. Verbindung oder pharmazeutisch annehmbares Salz davon gemäß einem der vorstehenden Ansprüche, wobei R¹ aus der Gruppe, die aus H, Methyl, Ethyl, geradkettigem oder verzweigtem C₃₋₆-Alkyl, C₃₋₆-Cycloalkyl 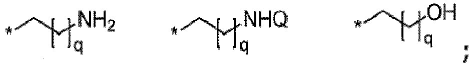 besteht, vorzugsweise aus der Gruppe, die aus H, 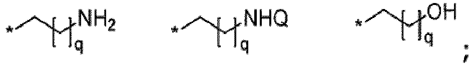 besteht, ausgewählt ist, wobei in den R¹-Struktureinheiten q und Q wie in Anspruch 1 definiert sind.

9. Verbindung oder pharmazeutisch annehmbares Salz davon gemäß einem der vorstehenden Ansprüche, wobei R⁴ aus der Gruppe ausgewählt ist, die aus H, 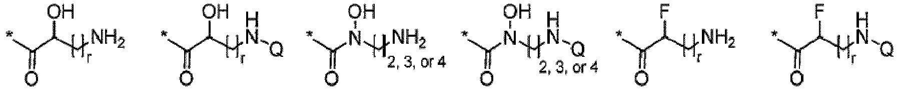 besteht, und wobei in den R⁴-Struktureinheiten r und Q wie in Anspruch 1 definiert sind.

10. Verbindung oder pharmazeutisch annehmbares Salz davon gemäß einem der vorstehenden Ansprüche, wobei R⁴ aus der Gruppe ausgewählt ist, die aus H 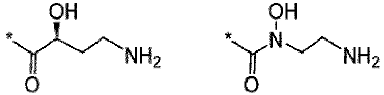 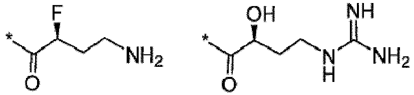 besteht.

11. Verbindung oder pharmazeutisch annehmbares Salz davon gemäß einem der vorstehenden Ansprüche, wobei R⁵ aus der Gruppe ausgewählt ist, die aus H, Methyl, Ethyl, geradkettigem oder verzweigtem C₃₋₈-Alkyl, substituiertem geradkettigem C₂₋₈-Alkyl, substituiertem verzweigtkettigem C₃₋₈-Alkyl, gegebenenfalls substituiertem C₃₋₆-Cycloalkyl besteht, 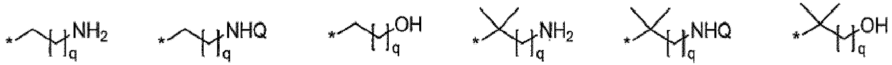 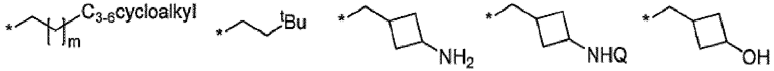 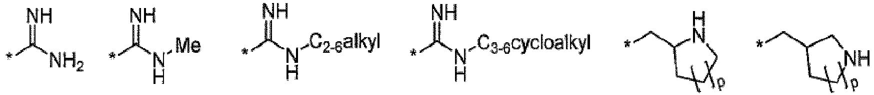 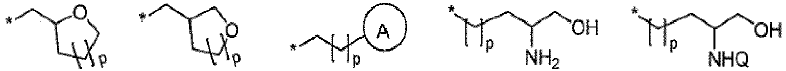 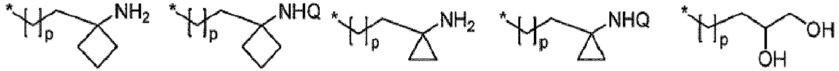 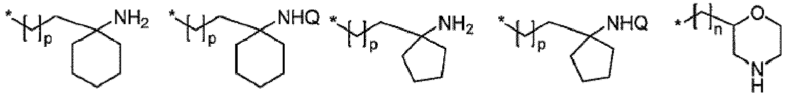 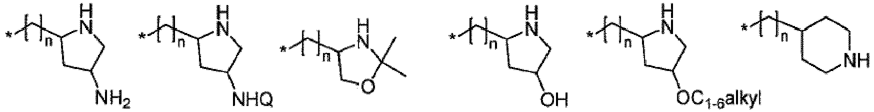 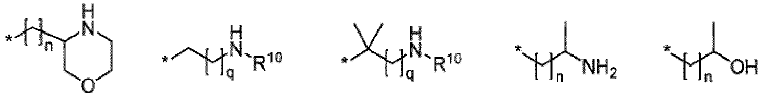 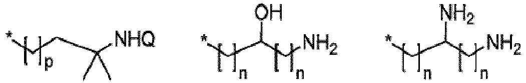
wobei * der Verbindungspunkt zum N-Atom ist, an das R⁵ in Formel (I) gebunden ist, und
wobei A aus der Gruppe ausgewählt ist, die aus gegebenenfalls substituiertem Phenyl; gegebenenfalls substituiertem fünfgliedrigen Heteroaryl; gegebenenfalls substituiertem sechsgliedrigen Heteroaryl; gegebenenfalls substituiertem viergliedrigen nichtaromatischem Heterocycloalkyl, das 1 Heteroatom enthält, welches aus O, N und S ausgewählt ist; gegebenenfalls substituiertem fünfgliedrigen nichtaromatischem Heterocycloalkyl, das 1 oder 2 Heteroatome enthält, die aus O, N und S ausgewählt sind; gegebenenfalls substituiertem sechsgliedrigen nichtaromatischem Heterocycloalkyl, das 1, 2 oder 3 Heteroatome enthält, die aus O, N und S ausgewählt sind, besteht, und
wobei m, n, q, p und R¹⁰ in den R⁵-Struktureinheiten wie in einem der vorstehenden Ansprüche definiert sind.

12. Verbindung oder pharmazeutisch annehmbares Salz davon gemäß einem der vorstehenden Ansprüche, wobei R⁵ aus der Gruppe ausgewählt ist, die aus H, Methyl, Ethyl, geradkettigem oder verzweigtem C₃₋₆-Alkyl 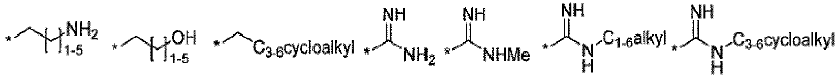 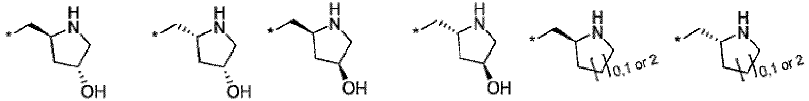 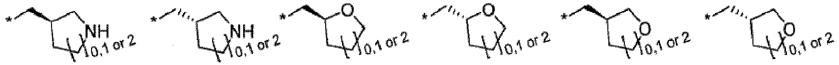 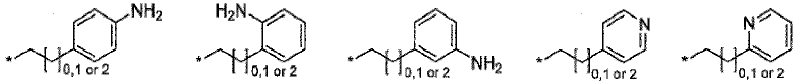 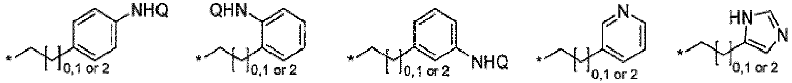 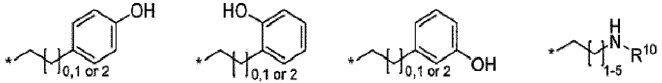 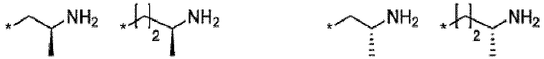 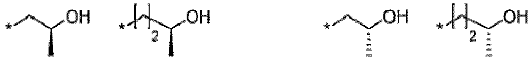 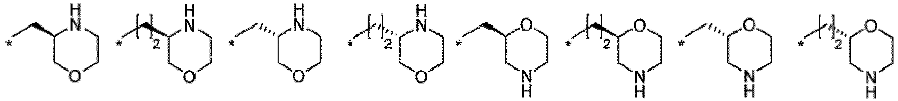 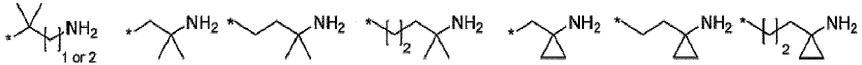 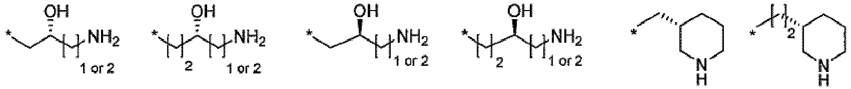 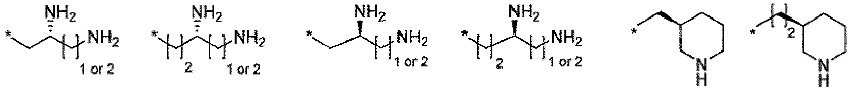 besteht, wobei Q und R¹⁰ in den R⁵-Struktureinheiten wie in einem der vorstehenden Ansprüche definiert sind.

13. Pharmazeutische Zusammensetzung, die die Verbindung oder ihr pharmazeutisch annehmbares Salz gemäß einem der vorstehenden Ansprüche und einen pharmazeutisch annehmbaren Träger umfasst.

14. Zusammensetzung, umfassend wenigstens eine Verbindung oder ein pharmazeutisch annehmbares Salz davon gemäß einem der Ansprüche 1 bis 12 und wenigstens ein weiteres antibakterielles Mittel, wobei das wenigstens eine weitere antibakterielle Mittel von der wenigstens einen Verbindung oder ihrem pharmazeutisch annehmbaren Salz gemäß einem der Ansprüche 1 bis 12 verschieden ist.

15. Verbindung oder pharmazeutisch annehmbares Salz davon gemäß einem der Ansprüche 1 bis 12 oder Zusammensetzung gemäß einem der Ansprüche 13 bis 14 zur Verwendung als Medikament.

## Revendications

1. Composé de formule (I) ou sel pharmaceutiquement acceptable de celui-ci 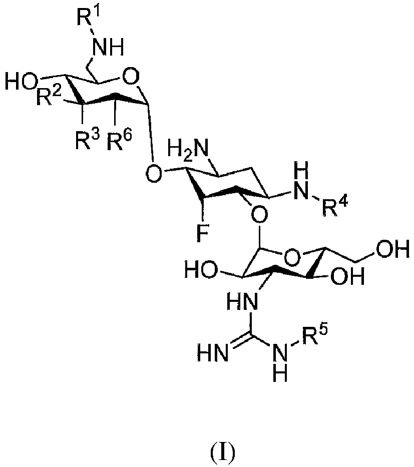 dans laquelle
(i) R¹ est choisi dans le groupe constitué par H, un groupe méthyle, éthyle, alkyle en C₃₋₆ à chaîne linéaire ou ramifié, cycloalkyle en C₃₋₆, 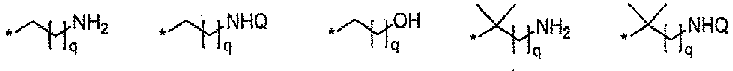 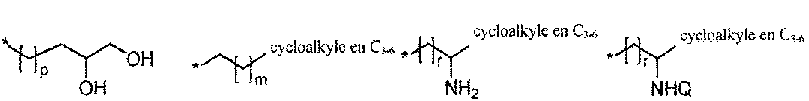 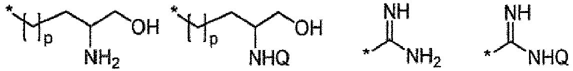 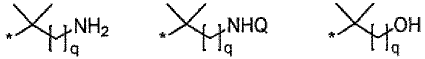 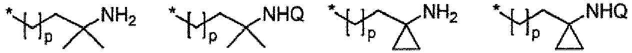 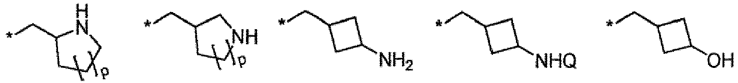 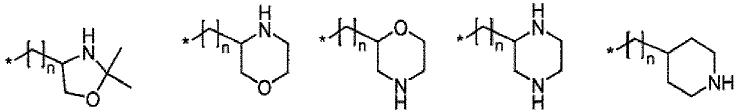 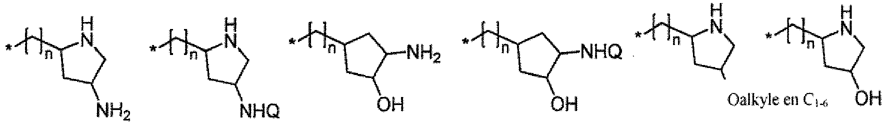 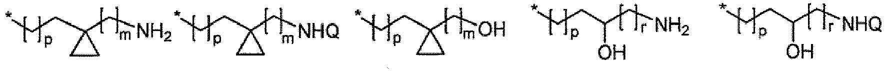 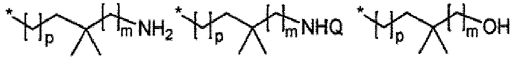 où * représente le point de liaison avec l'atome de N auquel R¹ est lié dans la formule (1) ;
(ii) R² est choisi dans le groupe constitué par H, les groupes méthyle, -CH₂F, -CF₃, éthyle, n-propyle, isopropyle, cyclopropyle, halogène, hydroxyle, -OCH₃, -OEt, -OCH₂F, -OCF₃, -NH₂, -NHCH₃, -NHEt, -N(CH₃)₂, -N(Et)₂, -NHCH₂F, -NHCF₃ et -NHQ ; dans laquelle lorsque R² est un groupe éthyle, n-propyle, isopropyle, cyclopropyle, -OEt, -NHEt ou -N(Et)₂, les groupements alkyle et cycloalkyle dans lesdits groupes R² peuvent être éventuellement substitués par un ou plusieurs substituants indépendamment choisis dans le groupe constitué par les substituants halogène, hydroxyle, -OCH₃, -OEt, -NH₂, -NHCH₃, -NHEt, -N(CH₃)₂, -N(Et)₂ et -NHQ ; sous réserve que lorsque R² représente -OEt, -NHEt ou -N(Et)₂, l'atome de carbone reliant le groupe Et dudit groupe -OEt, -NHEt ou -N(Et)₂ à l'atome de O ou N dudit groupe -OEt, -NHEt ou -N(Et)₂ ne puisse être substitué que par un ou plusieurs substituants indépendamment choisis parmi les substituants halogène ;
(iii) R³ est choisi dans le groupe constitué par H, les groupes halogène, hydroxyle, -OCH₃, -OCH₂F, -OCF₃, -OEt, -Oalkyle en C₃₋₈, -Ocycloalkyle en C₃₋₆, -OCH₂(cycloalkyle en C₃₋₆), -NH₂, -NHCH₃, -NHCH₂F, -NHCF₃, -NHEt, -NHalkyle en C₃₋₈, -N(CH₃)₂, -N(Et)₂, -N(alkyle en C₃₋₈)₂ et -NHQ ;
dans laquelle R³ est un groupe -OEt, -Oalkyle en C₃₋₈, -Ocycloalkyle en C₃₋₆, -OCH₂(cycloalkyle en C₃₋₆), -NHEt, -NHalkyle en C₃₋₈, -N(Et)₂, -N(alkyle en C₃₋₈)₂, les groupements alkyle et cycloalkyle dans lesdits groupes R³ peuvent être éventuellement substitués par un ou plusieurs substituants indépendamment choisis dans le groupe constitué par les substituants halogène, hydroxyle, -OCH₃, -OCH₂F, -OCF₃, -Oalkyle en C₂₋₄, -NH₂, -NHCH₃, -NHalkyle en C₂₋₄, -N(CH₃)₂, -N(alkyle en C₂₋₄)₂ et -NHQ ; sous réserve que l'atome de carbone dans chacun desdits groupes R³ qui est directement lié à l'atome de O ou N dans chacun desdits groupes R³ ne puisse être substitué que par un ou plusieurs substituants indépendamment choisis parmi les substituants halogène ; et
dans laquelle lorsque R³ est un groupe -OCH₃ ou -NHCH₃, il peut être éventuellement substitué sur le groupement CH₃ dudit groupe -OCH₃ ou - NHCH₃ par un groupe phényle facultativement substitué, hétéroaryle à 5 chaînons facultativement substitué, hétéroaryle à 6 chaînons facultativement substitué, hétérocycloalkyle non aromatique à 4 chaînons facultativement substitué contenant 1 hétéroatome choisi parmi O, N et S, hétérocycloalkyle non aromatique à 5 chaînons facultativement substitué contenant 1 ou 2 hétéroatomes choisis parmi O, N et S, ou hétérocycloalkyle non aromatique à 6 chaînons facultativement substitué contenant 1, 2 ou 3 hétéroatomes choisis parmi O, N et S ;
(iv) R⁴ est choisi dans le groupe constitué par H, les groupes méthyle, éthyle, -CH₂F, -CF₃, alkyle en C₃₋₆ à chaîne linéaire ou ramifié, alkyle en C₂₋₆ à chaîne linéaire substitué, alkyle en C₃₋₆ ramifié substitué, cycloalkyle en C₃₋₆ facultativement substitué, -CH₂(cycloalkyle en C₃₋₆) facultativement substitué, formyle, phényle facultativement substitué, hétéroaryle à 5 ou 6 chaînons facultativement substitué, 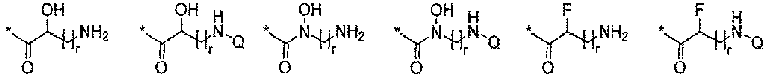 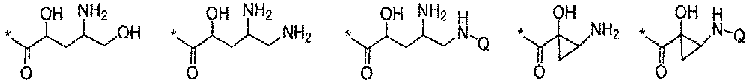 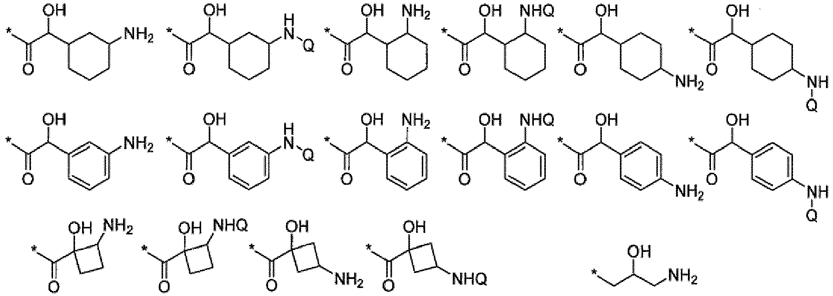 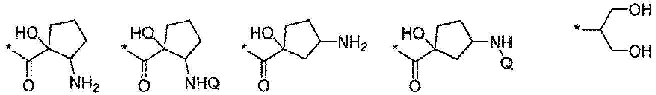 où * représente le point de liaison avec l'atome de N auquel R⁴ est lié dans la formule (I), et dans laquelle
lorsque R⁴ est un groupe alkyle en C₂₋₆ à chaîne linéaire substitué, alkyle en C₃₋₆ ramifié substitué, cycloalkyle en C₃₋₆ substitué, ou - CH₂(cycloalkyle en C₃₋₆) substitué, il est substitué par un ou plusieurs substituants indépendamment choisis dans le groupe constitué par les substituants halogène, hydroxyle, -OCH₃, -Oalkyle en C₂₋₄, -NH₂, - NHCH₃, -NHalkyle en C₂₋₄, -N(CH₃)₂, -N(alkyle en C₂₋₄)₂ et -NHQ, sous réserve que l'atome de carbone dudit groupe R⁴ qui est directement lié à l'atome de N auquel R⁴ est lié dans la structure de formule (I) ne puisse être substitué que par un ou plusieurs substituants indépendamment choisis parmi les substituants halogène ;
(v) R⁵ est choisi dans le groupe constitué par H, les groupes méthyle, -CH₂F, -CF₃, éthyle, alkyle en C₃₋₈ à chaîne linéaire ou ramifié, alkyle en C₂₋₈ à chaîne linéaire substitué, alkyle en C₃₋₈ ramifié substitué, cycloalkyle en C₃₋₆ facultativement substitué, -CH₂(cycloalkyle en C₃₋₆) facultativement substitué, phényle facultativement substitué, hétéroaryle à 5 chaînons facultativement substitué, hétéroaryle à 6 chaînons facultativement substitué, hétérocycloalkyle non aromatique à 4 chaînons facultativement substitué contenant 1 hétéroatome choisi parmi O, N et S, hétérocycloalkyle non aromatique à 5 chaînons facultativement substitué contenant 1 ou 2 hétéroatomes choisis parmi O, N et S, hétérocycloalkyle non aromatique à 6 chaînons facultativement substitué contenant 1, 2 ou 3 hétéroatomes choisis parmi O, N et S, -C(=NH)NH₂, -C(=NR⁷)NH₂, -C(=NH)NHR⁸, -(=NR⁷)NHR⁸, -C(=NR⁷)NR⁸R⁹ ; et -X-Z, où
X est choisi dans le groupe constitué par les groupes méthylène, éthylène, alkylène en C₃₋₈ à chaîne linéaire ou ramifié ; chacun d'entre eux peut être, en plus d'être lié à Z, facultativement substitué par un ou plusieurs substituants indépendamment choisis dans le groupe constitué par les substituants méthyle, -CH₂F, -CF₃, éthyle, alkyle en C₃₋₆ à chaîne linéaire ou ramifié, halogène, -OH, -OCH₃, -OCH₂F, -OCF₃, -Oalkyle en C₂₋₆, - NH₂, -NHQ, -NHR¹⁰, -NR¹⁰R¹¹, -CO₂H, -CO₂CH₃, -CO₂alkyle en C₂₋₆, - OCOCH₃, -OCOalkyle en C₂₋₆, -CN, -CONHR¹², -CONR¹²R¹³, -NHCOCH₃, -NHCOalkyle en C₂₋₆, -NR¹⁴COCH₃, et -NR¹⁵COalkyle en C₂₋₆, sous réserve que l'atome dudit groupe X qui le lie à l'atome de N auquel R⁵ est lié dans la structure de formule (I) ne puisse pas être directement lié à un atome de O ou N supplémentaire ;
et
Z est choisi dans le groupe constitué par un groupe phényle facultativement substitué, hétéroaryle à 5 chaînons facultativement substitué, hétéroaryle à 6 chaînons facultativement substitué, hétérocycloalkyle non aromatique à 4 chaînons facultativement substitué contenant 1 hétéroatome choisi parmi O, N et S, hétérocycloalkyle non aromatique à 5 chaînons facultativement substitué contenant 1 ou 2 hétéroatomes choisis parmi O, N et S, hétérocycloalkyle non aromatique à 6 chaînons facultativement substitué contenant 1, 2 ou 3 hétéroatomes choisis parmi O, N et S, cycloalkyle en C₃₋₆ facultativement substitué ; où chacun des R⁷ à R¹⁵ est indépendamment choisi dans le groupe constitué par H, les groupes méthyle, éthyle, alkyle en C₃₋₆ et cycloalkyle en C₃₋₆ ;
(vi) R⁶ représente OH ou NH₂ ;
(vii) sous réserve que pour tous les composés de formule (I) ou leurs sels pharmaceutiquement acceptables qui, lorsque l'atome de R² qui le lie au cycle tétrahydropyrane auquel à la fois celui-ci et R³ sont liés dans la structure de formule (I) est un atome de O ou N, l'atome de R³ qui le lie audit cycle tétrahydropyrane ne puisse pas être un atome de O ou N ; et sous réserve que lorsque l'atome de R³ qui le lie au cycle tétrahydropyrane auquel à la fois celui-ci et R² sont liés dans la structure de formule (I) est un atome de O ou N, l'atome de R² qui le lie audit cycle tétrahydropyrane ne puisse pas être un atome de O ou N ; et
(viii) où pour chacun des groupes décrits
m = 0, 1, 2, 3, 4 ou 5 ;
n = 1 ou 2 ;
p = 0, 1 ou 2 ;
q = 1, 2, 3, 4 ou 5 ;
r = 1, 2, 3 ou 4 ; et
(ix) où en ce qui concerne tous les substituants ci-dessus et les groupes contenant le groupement Q,
Q représente 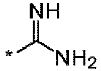
où * représente le point de liaison de l'atome de N auquel Q est lié.

2. Composé ou sel pharmaceutiquement acceptable de celui-ci selon la revendication 1, dans lequel R² est choisi dans le groupe constitué par H, les groupes méthyle, -CH₂F, -CF₃, éthyle, n-propyle, isopropyle, cyclopropyle, halogène, hydroxyle, -OCH₃, -OEt, -OCH₂F, -OCF₃, -NH₂, -NHCH₃, -NHEt, -N(CH₃)₂, -N(Et)₂, -NHCH₂F, -NHCF₃ et -NHQ.

3. Composé ou sel pharmaceutiquement acceptable de celui-ci selon l'une quelconque des revendications précédentes, dans lequel R² est choisi dans le groupe constitué par H, les groupes méthyle, éthyle, n-propyle, isopropyle, cyclopropyle, -F, hydroxyle, -OCH₃, -OEt, -OCH₂F et -OCF₃.

4. Composé ou sel pharmaceutiquement acceptable de celui-ci selon l'une quelconque des revendications précédentes, dans lequel R³ est choisi dans le groupe constitué par H, les atomes d'halogène, les groupes hydroxyle, -OCH₃, -OCH₂F, -OCF₃, -OEt, -Oalkyle en C₃₋₈, -Ocycloalkyle en C₃₋₆ et -OCH₂(cycloalkyle en C₃₋₆),
dans lequel lorsque R³ est un groupe -OEt, -Oalkyle en C₃₋₈, -Ocycloalkyle en C₃₋₆ ou -OCH₂(cycloalkyle en C₃₋₆), les groupements alkyle et cycloalkyle dans lesdits groupes R³ peuvent être éventuellement substitués par un ou plusieurs substituants indépendamment choisis dans le groupe constitué par les substituants halogène, hydroxyle, -OCH₃, -OCH₂F, -OCF₃, -Oalkyle en C₂₋₄, -NH₂, -NHCH₃, -NHalkyle en C₂₋₄, -N(CH₃)₂, -N(alkyle en C₂₋₄)₂ et -NHQ ; sous réserve que l'atome de carbone dans chacun desdits groupes R³, qui est directement lié à l'atome de O dans chacun desdits groupes R³, ne puisse être substitué que par un ou plusieurs substituants indépendamment choisis parmi les substituants halogène ; et
dans lequel lorsque R³ est un groupe -OCH₃, il peut être éventuellement substitué, sur le groupement CH₃ dudit groupe -OCH₃, par un groupe phényle facultativement substitué, hétéroaryle à 5 chaînons facultativement substitué, hétéroaryle à 6 chaînons facultativement substitué, hétérocycloalkyle non aromatique à 4 chaînons facultativement substitué contenant 1 hétéroatome choisi parmi O, N et S, hétérocycloalkyle non aromatique à 5 chaînons facultativement substitué contenant 1 ou 2 hétéroatomes choisis parmi O, N et S, ou hétérocycloalkyle non aromatique à 6 chaînons facultativement substitué contenant 1, 2 ou 3 hétéroatomes choisis parmi O, N et S.

5. Composé ou sel pharmaceutiquement acceptable de celui-ci selon l'une quelconque des revendications précédentes, dans lequel R³ est choisi dans le groupe constitué par H, les atomes d'halogène, les groupes hydroxyle, -OCH₃, -OCH₂F, -OCF₃, -OEt, -Oalkyle en C₃₋₈, -Ocycloalkyle en C₃₋₆, -OCH₂(cycloalkyle en C₃₋₆), -OCH₂(phényle facultativement substitué), -OCH₂(hétéroaryle à 5 chaînons facultativement substitué), -OCH₂(hétéroaryle à 6 chaînons facultativement substitué), -OCH₂(hétérocycloalkyle non aromatique à 4 chaînons facultativement substitué contenant 1 hétéroatome choisi parmi O, N et S) ; -OCH₂(hétérocycloalkyle non aromatique à 5 chaînons facultativement substitué contenant 1 ou 2 hétéroatomes choisis parmi O, N et S) ; et -OCH₂(hétérocycloalkyle non aromatique à 6 chaînons facultativement substitué contenant 1, 2 ou 3 hétéroatomes choisis parmi O, N et S).

6. Composé ou sel pharmaceutiquement acceptable de celui-ci selon l'une quelconque des revendications précédentes, où le composé de formule (I) est choisi parmi les composés de formules (Ia) à (If) 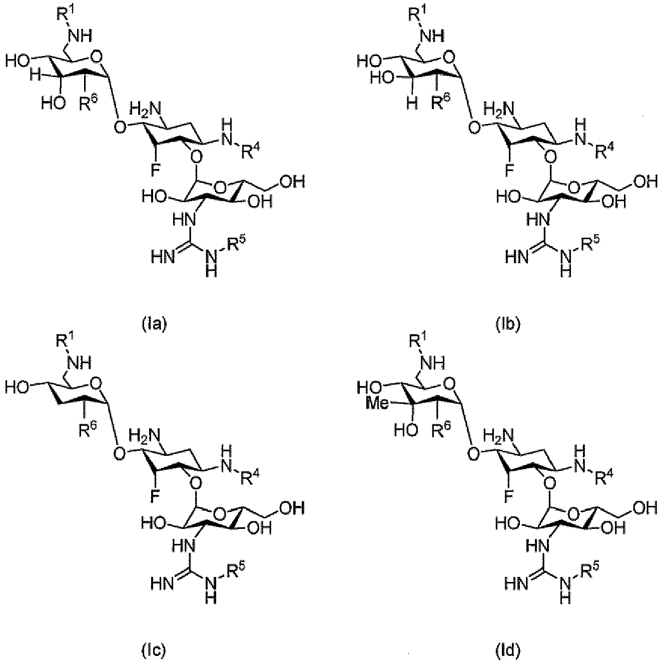 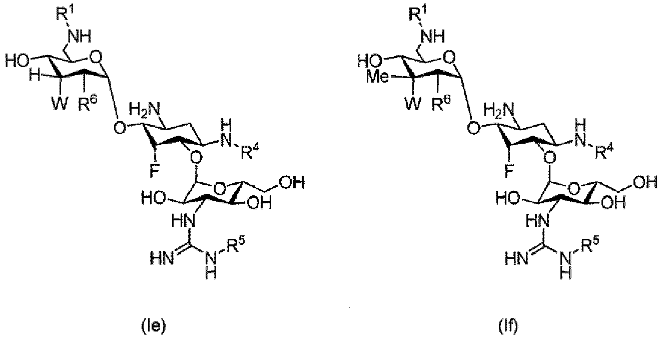 dans lesquels, indépendamment pour chacun des composés de formules (Ia) à (If), R¹, R⁴, R⁵ et R⁶ sont tels que définis dans l'une quelconque des revendications précédentes, et dans lesquels W est indépendamment choisi pour chacun des composés de formules (Ia) à (If) dans le groupe constitué par -OCH₃, -OCH₂F, -OCF₃, -OEt, -Oalkyle en C₃₋₈, -Ocycloalkyle en C₃₋₆, -OCH₂(cycloalkyle en C₃₋₆), -OCH₂(phényle facultativement substitué), -OCH₂(hétéroaryle à 5 chaînons facultativement substitué), -OCH₂(hétéroaryle à 6 chaînons facultativement substitué), -OCH₂(hétérocycloalkyle non aromatique à 4 chaînons facultativement substitué contenant 1 hétéroatome choisi parmi O, N et S), -OCH₂(hétérocycloalkyle non aromatique à 5 chaînons facultativement substitué contenant 1 ou 2 hétéroatomes choisis parmi O, N et S), et -OCH₂(hétérocycloalkyle non aromatique à 6 chaînons facultativement substitué contenant 1, 2 ou 3 hétéroatomes choisis parmi O, N et S).

7. Composé ou sel pharmaceutiquement acceptable de celui-ci selon l'une quelconque des revendications précédentes, où le composé de formule (I) est choisi parmi les composés de formules (Ig) à (Ip) 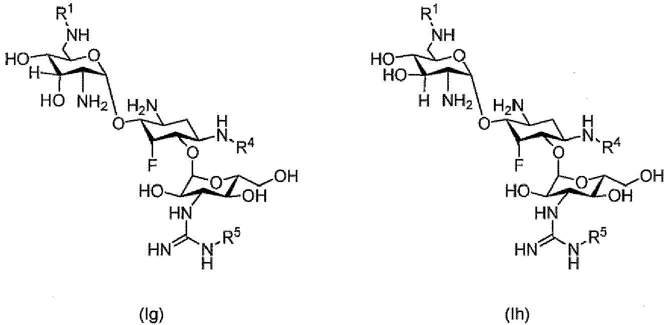 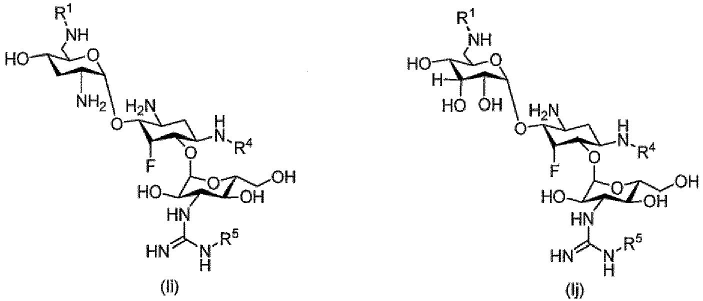 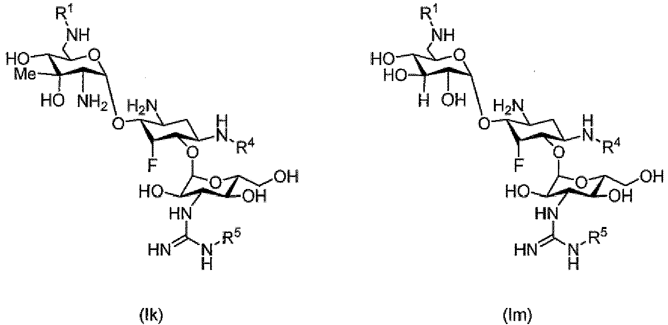 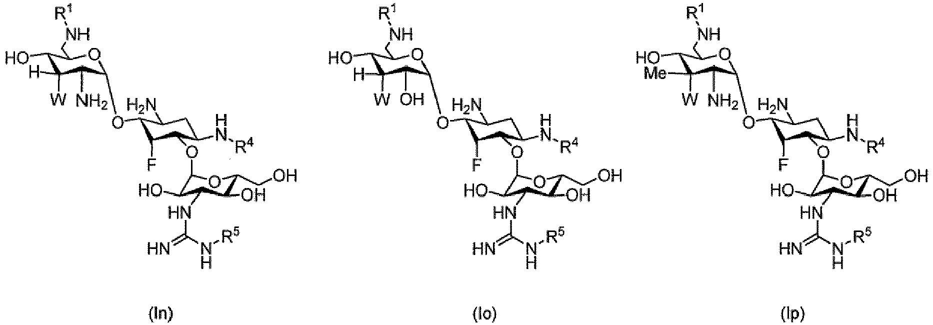 dans lesquels, indépendamment pour chacun des composés de formules (Ig) à (Ip), R¹, R⁴ et R⁵ sont tels que définis dans l'une quelconque des revendications précédentes, et dans lesquels W est indépendamment choisi pour chacun des composés de formules (In) à (Ip) dans le groupe constitué par -OCH₃, -OCH₂F, -OCF₃, -OEt, -Oalkyle en C₃₋₈, -Ocycloalkyle en C₃₋₆, -OCH₂(cycloalkyle en C₃₋₆), -OCH₂(phényle facultativement substitué), -OCH₂(hétéroaryle à 5 chaînons facultativement substitué), -OCH₂(hétéroaryle à 6 chaînons facultativement substitué), -OCH₂(hétérocycloalkyle non aromatique à 4 chaînons facultativement substitué contenant 1 hétéroatome choisi parmi O, N et S) ; -OCH₂(hétérocycloalkyle non aromatique à 5 chaînons facultativement substitué contenant 1 ou 2 hétéroatomes choisis parmi O, N et S) ; et -OCH₂(hétérocycloalkyle non aromatique à 6 chaînons facultativement substitué contenant 1, 2 ou 3 hétéroatomes choisis parmi O, N et S).

8. Composé ou sel pharmaceutiquement acceptable de celui-ci selon l'une quelconque des revendications précédentes, dans lequel R¹ est choisi dans le groupe constitué par H, les groupes méthyle, éthyle, alkyle en C₃₋₆ à chaîne linéaire ou ramifié, cycloalkyle en C₃₋₆, 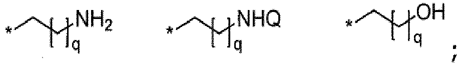 de préférence, dans le groupe constitué par H, 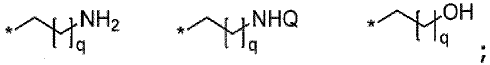 où, dans lesdits groupements R¹, q et Q sont tels que définis dans la revendication 1.

9. Composé ou sel pharmaceutiquement acceptable de celui-ci selon l'une quelconque des revendications précédentes, dans lequel R⁴ est choisi dans le groupe constitué par H, 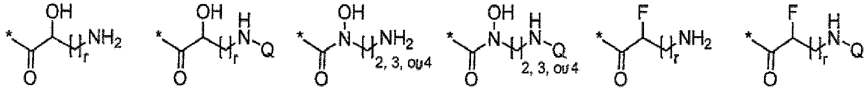 et dans lequel, dans lesdits groupements R⁴, r et Q sont tels que définis dans la revendication 1.

10. Composé ou sel pharmaceutiquement acceptable de celui-ci selon l'une quelconque des revendications précédentes, dans lequel R⁴ est choisi dans le groupe constitué par H,

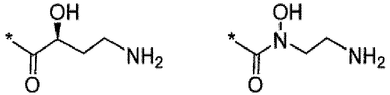

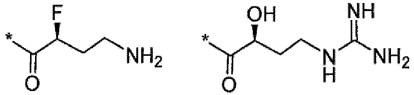

11. Composé ou sel pharmaceutiquement acceptable de celui-ci selon l'une quelconque des revendications précédentes, dans lequel R⁵ est choisi dans le groupe constitué par H, les groupes méthyle, éthyle, alkyle en C₃₋₈ à chaîne linéaire ou ramifié, alkyle en C₂₋₈ à chaîne linéaire substitué, alkyle en C₃₋₈ à chaîne ramifiée substitué, cycloalkyle en C₃₋₆ facultativement substitué, 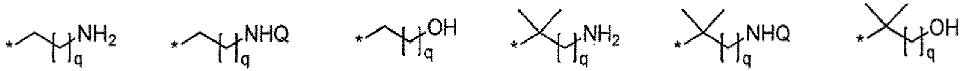 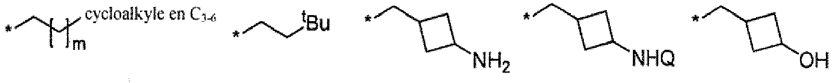 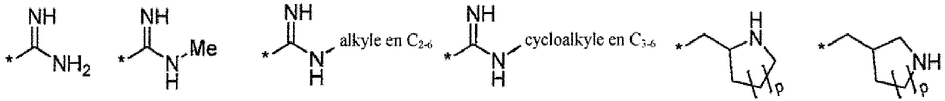 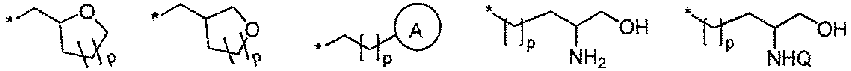 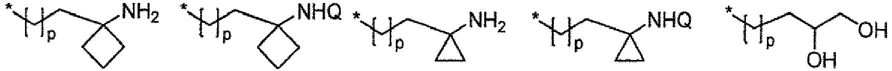 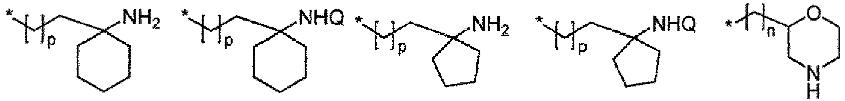 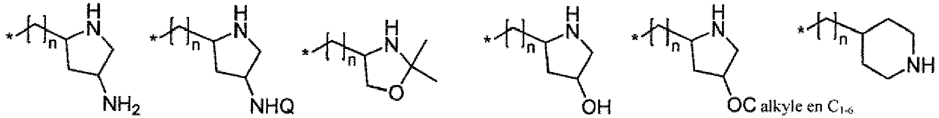 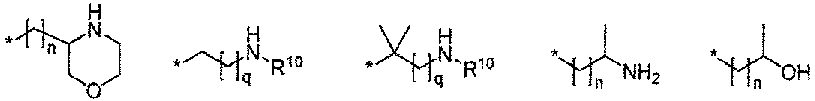 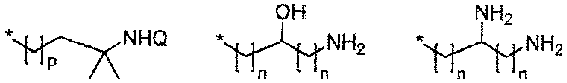
où * représente le point de liaison avec l'atome de N auquel R⁵ est lié dans la formule (I), et
où A est choisi dans le groupe constitué par un groupe phényle facultativement substitué, hétéroaryle à 5 chaînons facultativement substitué, hétéroaryle à 6 chaînons facultativement substitué, hétérocycloalkyle non aromatique à 4 chaînons facultativement substitué contenant 1 hétéroatome choisi parmi O, N et S, hétérocycloalkyle non aromatique à 5 chaînons facultativement substitué contenant 1 ou 2 hétéroatomes choisis parmi O, N et S, ou hétérocycloalkyle non aromatique à 6 chaînons facultativement substitué contenant 1, 2 ou 3 hétéroatomes choisis parmi O, N et S ; et
où m, n, p, q et R¹⁰ dans lesdits groupements R⁵ sont tels que définis dans l'une quelconque des revendications précédentes.

12. Composé ou sel pharmaceutiquement acceptable de celui-ci selon l'une quelconque des revendications précédentes, dans lequel R⁵ est choisi dans le groupe constitué par H, les groupes méthyle, éthyle, alkyle en C₃₋₆ à chaîne linéaire ou ramifié, 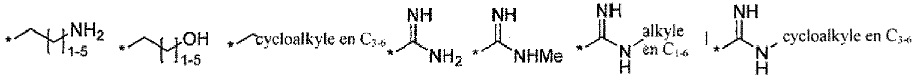 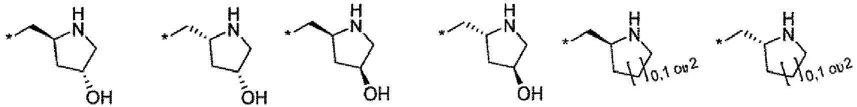 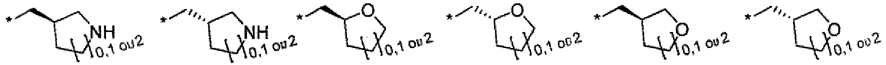 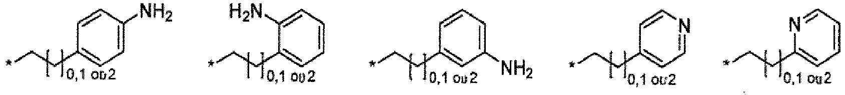 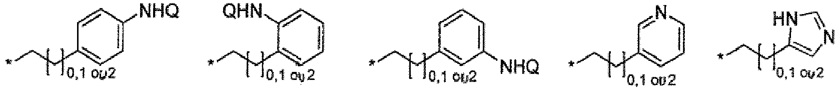 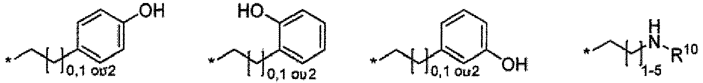 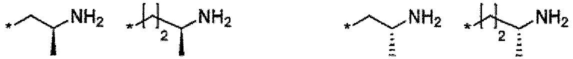 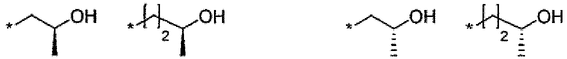 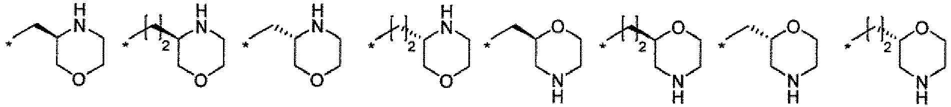 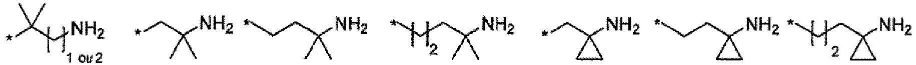 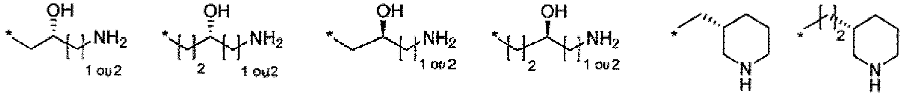 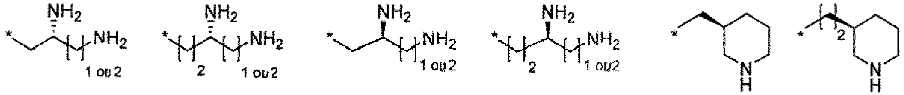 où Q et R¹⁰ dans lesdits groupements R⁵ sont tels que définis dans l'une quelconque des revendications précédentes.

13. Composition pharmaceutique comprenant le composé ou un sel pharmaceutiquement acceptable de celui-ci selon l'une quelconque des revendications précédentes et un véhicule pharmaceutiquement acceptable.

14. Composition comprenant au moins un composé ou un sel pharmaceutiquement acceptable de celui-ci selon l'une quelconque des revendications 1 à 12 et au moins un agent antibactérien supplémentaire, dans laquelle ledit au moins un agent antibactérien supplémentaire est différent dudit au moins un composé ou un sel pharmaceutiquement acceptable de celui-ci selon l'une quelconque des revendications 1 à 12.

15. Composé ou sel pharmaceutiquement acceptable de celui-ci selon l'une quelconque des revendications 1 à 12, ou composition selon l'une quelconque des revendications 13 et 14, à utiliser comme médicament.
